# EUROPEAN PATENT APPLICATION

(11) **EP 1 213 349 A1**
(43) Date of publication of application: **12.06.2002**
(21) Application number: 00956853.6
(22) Date of filing: 31.08.2000
(51) Int. Cl.: C12N 15/09

(54) **HUMAN 1p36 HOMOZYGOSITY DELETION DOMAIN**

(30) Priority: 31.08.1999 JP 24596299; 09.05.2000 JP 2000136266
(71) Applicant: HISAMITSU PHARMACEUTICAL CO. INC., Tosu-shi, Saga-ken 841-0017 (JP); CHIBA PREFECTURE, Chiba-shi Chiba 260-8667 (JP)
(72) Inventor: NAKAGAWARA, Akira, c/o Chiba Cancer Ctr.Resrh.Inst, Chuo-ku, Chiba-shi, Chiba 260-0801 (JP)
(74) Representative: Grünecker, Kinkeldey, Stockmair & Schwanhäusser Anwaltssozietät
(86) International application number: JP0005930
(87) International publication number: WO0116311

(57) **Abstract**

There are disclosed base sequence data for a deletion region at position 36 of the short arm of chromosome 1 of the human neuroblastoma cell lines NB-1 and MASS-NB-SCH-1, as a tumor suppressor gene for human neuroblastoma.

## Description

### Technical Field

This invention relates to a homozygous deletion region (hereunder also referred to simply as "deletion region") present in common at position 36 on the short arm of chromosome 1 (hereunder also referred to as "1p36") of human neuroblastomas.

### Background Art

Cancer-associated genes linked to oncogenesis are known to include tumor suppressor genes which promote oncogenesis when inactivated, and for example, the tumor suppressor genes RB and WT-1 have already been discovered and identified.

The RB gene was so named because it was discovered as the gene responsible for retinoblastoma, an ocular cancer of childhood. In most cases of retinoblastoma, region 14 of the long arm of human chromosome 13 (13q14) is shortened (Yunis, JJ. et al., Am. J. Dis. Child. 132, 161-163(1978)), and it has long been conjectured that inactivation of a gene on this portion results in predisposition to cancer. However, cloning of the gene was difficult, and RB was first identified as a human tumor suppressor gene in 1986 (Friend, SH. et al., Nature, 323, 643-646(1986)).

Wilms' tumor, which is also called nephroblastoma, is a fetal malignant tumor originating in the nephric blastema tissue in infants. Patients often also exhibit abnormalities of the genitourinary organs, but epidemiological data tend to suggest the "two-hit theory" as with retinoblastoma, leading to conjecture on the role of a recessive oncogene. In the case of patients with WAGR syndrome which also exhibit aniridia (A) and mental retardation (R) in addition to the tumor (W) and genitourinary (G) abnormalities, a microdeletion is often found in region 13 on the short arm of chromosome 11 (11p13). WT1 has been isolated as the gene located in this common deletion region (Call, KM. et al., Cell 60, 509-520(1990)).

Numerous tumor suppressor genes have since been discovered based on the data for chromosome deletion regions, but as of the current time no tumor suppressor gene for neuroblastoma has been identified from the 1p36 deletion region.

### Disclosure of the Invention

It is an object of this invention to provide data relating to a novel homozygous deletion region at position 36 on the short arm of chromosome 1 which is common to human neuroblastomas.

It is another object to provide a method for diagnosis and prognosis of tumors based on the base sequence data for this region, as well as a tumor marker which can be used therefor.

It is yet another object to develop a method of gene diagnosis and an anticancer agent, and to develop a method of analyzing the tumor pathology mechanism and a reagent to be used for analysis of the tumor pathology mechanism, based on this base sequence data.

As a result of much diligent research toward finding a tumor suppressor gene for neuroblastoma, the present inventors have discovered a novel homozygous deletion region at position 36 on the short arm of chromosome 1 of NB-1, a human neuroblastoma cell line.

This finding made it possible to design a method for tumor diagnosis and prognosis and a tumor marker which can be used therefor, and this invention was thus completed. The base sequence data for this region also allowed development of a tumor gene diagnosis method and an anticancer agent, as well as development of a method of analyzing the tumor pathology mechanism and a reagent to be used for analysis of the tumor pathology mechanism, and this invention was thereupon further completed.

More specifically, the invention relates to a homozygous deletion region present in common at position 36 on the short arm of chromosome 1 of human neuroblastomas.

Even more specifically, this region is a region characterized by being defined by at least from between a primer set at the SP6 end of the PAC clone dJ1028013 and a primer set at the SP6 end of the PAC clone dJ371E1 to between a primer set D1S2736 and a primer set at the T7 end of the PAC clone dJ142A6.

The region is also a region characterized by being further defined by at least the primer set SGC30343 and D1S2736.

The region is yet also a region characterized by being deleted in the human neuroblastoma cell lines NB-1 and MASS-NB-SCH-1.

The invention further includes a tumor marker, a tumor diagnostic reagent, a tumor prognostic reagent and an anticancer agent which are based on the base sequence data obtained from this region.

The invention still further includes a tumor pathology mechanism analysis method and a tumor pathology mechanism analysis reagent which are based on the base sequence data obtained from this region.

### Brief Description of the Drawings

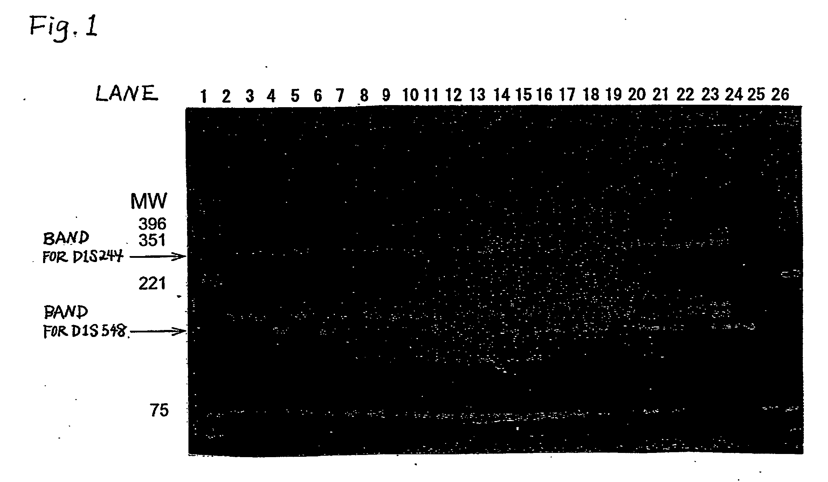

Fig. 1 is a photograph of 4% agarose gel electrophoresis of a PCR product for human neuroblastoma genomic DNA using the D1S548 and D1S244 primer sets. (Lane 1: molecular weight marker (1330, 828, 750, 696, 536, 517, 501, 396, 351, 221, 75, 65, 35, 22 bp from top), Lane 2: SK-N-BE, Lane 3: SK-N-DZ, Lane 4: RT-BM1, Lane 5: NLF, Lane 6: LA-N-5, Lane 7: IMR32, Lane 8: NMB, Lane 9: OAN, Lane 10: NGP, Lane 11: NB1, Lane 12: CHP134, Lane 13: KCN, Lane 14: NBL-S, Lane 15: CHP901, Lane 16: SY5Y, Lane 17: GANB, Lane 18: SK-N-AS, Lane 19: KAN, Lane 20: LHN, Lane 21: SAN, Lane 22: TGW, Lane 23: positive control (human lymphocytes), Lane 24: MASS-NB-SCH-1, Lane 25: negative control (sterile distilled water), Lane 26: molecular weight marker (same as Lane 1)).

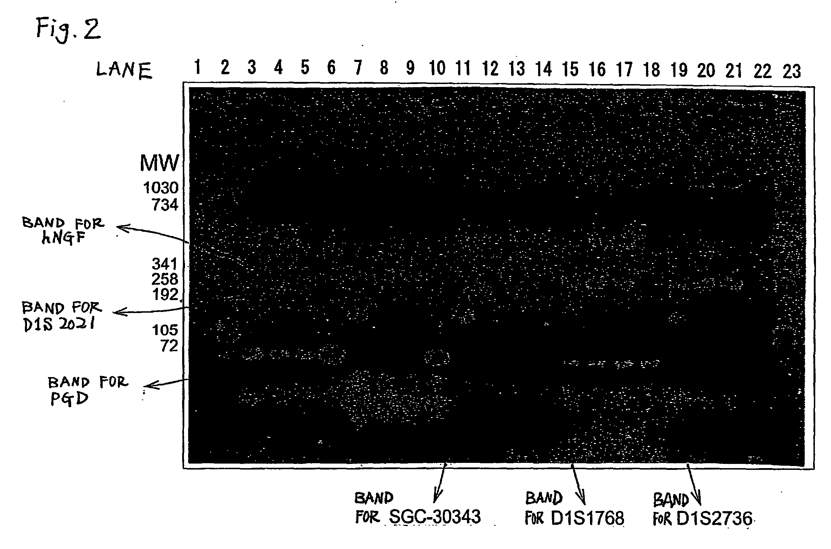

Fig. 2 is a photograph of 2% agarose gel electrophoresis of PCR products for NB-1 and MASS-NB-SCH-1 genomic DNA using the D1S2021, PGD, SGC-30343, D1S1768 and D1S2736 primer sets. All of the PCR products include primers that specifically amplify the region of human Nerve Growth Factor β (hNGFβ) as positive controls. (Lane 1: empty lane, Lane 2: molecular weight marker (1030, 734, 341, 258, 192, 105, 78, 75, 46, 36, 18, 17, 12, 11, 8 bp from top), Lane 3: positive control (SYSY), Lane 4: NB-1, Lane 5: MASS-NB-SCH-1, Lane 6: negative control (sterile distilled water), Lane 7: positive control (SY5Y), Lane 8: NB-1, Lane 9: MASS-NB-SCH-1, Lane 10: negative control (sterile distilled water), Lane 11: positive control (SYSY), Lane 12: NB-1, Lane 13: MASS-NB-SCH-1, Lane 14: negative control (sterile distilled water), Lane 15: positive control (SYSY), Lane 16: NB-1, Lane 17: MASS-NB-SCH-1, Lane 18: negative control (sterile distilled water), Lane 19: positive control (SY5Y), Lane 20: NB-1, Lane 21: MASS-NB-SCH-1, Lane 22: negative control (sterile distilled water), Lane 23: molecular weight marker (same as Lane 2)).
Fig. 3 is a photograph of 5% acrylamide electrophoresis of a PCR product for a PAC clone DNA using the D1S244 primer set. (Lane 1: molecular weight marker (1202 bp, 517 bp, 396 bp, 201 bp), Lanes 2-4: dJ142A6, Lane 5-7: dJ332G17, Lane 8-10: dJ369D13, Lane 11: positive control (human placenta DNA), Lane 12: negative control (sterile distilled water)).
Fig. 4 is a contig map for the PAC18 clone included in 1p36 found in strains NB-1 and MASS-NB-SCH-1 by the 1st screening.
Fig. 5 is a contig map for the PAC34 clone included in 1p36 found in strains NB-1 and MASS-NB-SCH-1 in the final screening.

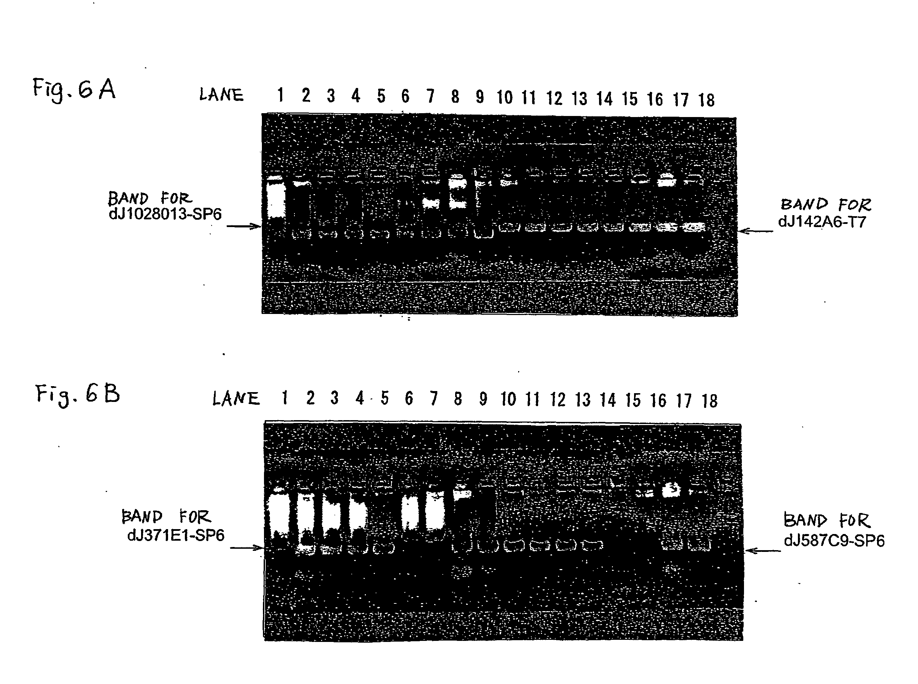

Fig. 6 is a pair of photographs of electrophoresis of PCR products for NB-1 and MASS-NB-SCH-1 genomic DNA using primers designed based on PAC clone data. The gel used was 2.5 agarose. (Lane 1: marker (1202 bp, 517 bp, 396 bp, 201 bp), Lane 2: positive control (human placenta DNA), Lane 3: normal cells derived from neuroblastoma patient without 1p36 deletion, Lane 4: tumor cells derived from neuroblastoma patient without 1p36 deletion, Lane 5: normal cells derived from MASS-NB-SCH-1 patient, Lane 6: MASS-NB-SCH-1, Lane 7: NB1, Lane 8: positive control (mouse hybridoma with human chromosome), Lane 9: positive control (human lymphocytes), Lane 10: positive control (human placenta DNA), Lane 11: normal cells derived from neuroblastoma patient without 1p36 deletion, Lane 12: tumor cells. derived from neuroblastoma patient without 1p36 deletion, Lane 13: normal cells derived from MASS-NB-SCH-1 patient, Lane 14: MASS-NB-SCH-1, Lane 15: NB1, Lane 16: positive control (mouse hybridoma with human chromosome), Lane 17: positive control (human lymphocytes), Lane 18: empty lane). In A, dJ1028013 was used as primer for Lanes 1-8; dJ142A6-T7 was used as primer for Lanes 9-17. In B, dJ371E1-SP6 was used as primer for Lanes 1-8; dJ587c9-SP6 was used as primer for Lanes 9-17.
Fig. 7 is a diagram showing the genome deletion region area for 1p36 elucidated by this invention.
Fig. 8 is a diagram showing the positions for a minimal PAC clone group included in the genome deletion region area for 1p36 elucidated by this invention.
Fig. 9 is a diagram showing the positions for a minimal PAC clone group included in the genome deletion region area for 1p36 elucidated by this invention, which differs somewhat from the combination shown in Fig. 8.

### Best Mode for Carrying Out the Invention

### (Novel homozygous deletion region for 1p36)

The present inventors focused on the fact that the human 1p36 deletion has been found in at least 14 different human tumors (colon cancer, breast cancer, brain tumor, etc.), that several tumor suppressor genes are predicted to be present in this region and that the deletion has a high correlation with poor prognosis of neuroblastoma (Schwab M. et al., Genes Chromosomes Cancer 16, 211-229(1996), Fong C.T. et al., Cancer Res. 52, 1780-1785(1992)), and also focused on the fact that normalization of cancer cells has been achieved by introduction of normal 1p36 in colon cancer (Tanaka K. et al., Oncogene, 8, 2253-2258(1993)) and that the 1p36 deletion is found from the initial stages of liver cancer and colon cancer (Kuroki T. et al., Genes Chromosomes Cancer 13, 163-167(1995)), which suggested a high possibility that the 1p36 deletion plays a role in the early stages of canceration of normal cells.

Identification of the tumor suppressor gene is possible by analysis of Loss of Heterozygosity (hereunder also abbreviated as LOH). The chromosomes of human cells, except for the sex chromosomes, exist as pairs, and two copies of each gene are present, one from the mother and one from the father. According to the "two-hit theory" advocated by Kundson A.G. et al. (Kundson A.G., Pediatr. Res. 513- (1976)), inactivation of a gene is only found to occur when both of each copy from the mother and father are inactivated, and LOH is observed most often by this mechanism. LOH refers to a pattern whereby a small genetic abnormality (deletion or substitution of a base or part of the gene) exists in one copy, while the chromosome on which the other copy resides undergoes the entire deletion of a large region including the gene. Consequently, in patients experiencing onset of cancer due to inactivation of a tumor suppressor gene, it is thought that there are two copies of the chromosome on which the tumor suppressor gene resides in normal cells, and one copy in tumor cells.

However, the commonly observed range for LOH is very large in terms of the molecular genetic distance, and the numbers of genes and numbers of bases contained therein are exceedingly huge. The current actual deletion region of 1p36 is limited to the approximately 8-10 Mb region of 1p36.2-1p36.3 according to LOH analysis, but this region must be further narrowed down.

In order to identify the tumor suppressor gene, the present inventors analyzed neuroblastoma cell lines and discovered an approximately 700 kb homozygous deletion region on the neuroblastoma chromosome 1p36. These 700 kb constituted part of the deletion region (approximately 8-10 Mb) elucidated by analysis of loss of heterozygosity.

### (Application example for human neuroblastoma 1p36 homozygous deletion region)

### (1) Tumor gene diagnosis, method of prognosis, and tumor marker to be used therefor

The base sequence disclosed by this invention may be used to investigate the presence or absence of the 1p36 homozygous deletion region in cell specimens to allow diagnosis and prognosis of tumors. For investigation of the presence or absence of the deletion region there may be applied all of the methods using any desired sequence of the 1p36 homozygous deletion region and base sequences (probes) that can hybridize thereto. When necessary, these probes may be labeled with radioisotopes, enzymes (for example, alkali phosphatase or peroxidase), fluorescent compounds (for example, fluorescein) or chemiluminescent compounds (for example, acridinium esters or isoluminol). That is, when nucleic acids that hybridize to the probe sequences are not present in the cell specimen, a high possibility exists for tumor onset, while in specimens from known cancer onset cases the prognosis can be predicted to be poor. As hybridization methods there may be used, for example, Southern blotting, Northern blotting, dot blotting, Fluorescence *in situ* Hybridization (FISH) or the like.

For investigation of the presence or absence of the deletion region there may also be used a Polymerase Chain Reaction (PCR) with any desired sequences from the 1p36 homozygous deletion region as the primers.

Identification of loss of heterozygosity may be accomplished using, for example, Restriction Fragment Length Polymorphism (RFLP).

These methods are carried out according to protocols known by skilled professionals. Reference may be made to, for example, Molecular Cloning, A Laboratory Manual (T. Maniatis: Cold Spring Harbor Laboratory Press) and Idenshibyo Nyumon [Introduction to Genetic Diseases] (F. Takahisa: Nankodo Publishing).

### (2) Development of anticancer agent

Cells having the 1p36 homozygous deletion or loss of heterozygosity can be found using base sequences disclosed by the invention, thus allowing screening of anticancer agents. Because the cells are cancerous, their cell division occurs faster than that of normal cells. By adding candidate anticancer agents to the cell culture solution and observing the rate of cell division, it is possible to screen for anticancer agents against tumors having the 1p36 homozygous deletion or loss of heterozygosity. The cell division rate can be measured based on, for example, the amount of uptake of radioisotope (for example, tritium)-labeled thymidine.

### (3) Method of analyzing tumor pathology mechanism

Cells having the 1p36 homozygous deletion or loss of heterozygosity can be found using base sequences disclosed by the invention, thus allowing analysis of the tumor pathology mechanism. The proteins may be extracted from these and normal cells, and the protein expressed only by the normal cells identified by, for example, Western blotting. This protein may then be used for identification of other proteins that specifically interact therewith by, for example, affinity chromatography. By repeating such methods it is possible to identify proteins connected with oncogenesis and thus analyze the tumor pathology mechanism through analysis of their intracellular function.

The 1p36 homozygous deletion region common to neuroblastoma which was discovered by this invention will now be described in detail by way of examples.

### Examples

### (Example 1) Extraction of genomic DNA from human neuroblastomas

Each of the following human neuroblastoma-derived cell lines was cultured on a 10 cm culturing plate to general confluency. The names and sources of the cell lines used are summarized below.
SK-N-BE (ATCC Number: CRL-2271)
SK-N-DZ (ATCC Number: CRL-2149)
RT-BM1
NLF: donated by Garrett M. Brodeur
LA-N-5: (Riken Cell Bank (The Institute of Physical and Chemical Research): RC0485)
IMR32: donated by Garrett M. Brodeur
NMB: donated by Garrett M. Brodeur
OAN: donated by Garrett M. Brodeur
NGP: donated by Garrett M. Brodeur
NB1: donated by Dr. Takafumi Matsumura of Kyoto Prefectural University, Dept. of Pediatrics (Considered one of the oldest neuroblastoma cell lines in Japan, established at the Niigata University Brain Research Laboratory)
CHP134: donated by Garrett M. Brodeur
KCN: donated by Garrett M. Brodeur
NBL-S: donated by Garrett M. Brodeur
CHP901: cell line established from patient surgically extracted specimen
SYSY: donated by Garrett M. Brodeur
GANB: cell line established from patient surgically extracted specimen
SK-N-AS (ATCC Number: CRL-2137)
KAN: donated by Garrett M. Brodeur
LHN: donated by Garrett M. Brodeur
SAN: donated by Garrett M. Brodeur
TGW: donated by Garrett M. Brodeur
MASS-NB-SCH-1: donated by Dr. Hisayuki Hiraiwa of Shizuoka Children's Hospital, Clinical Pathology Section. Cancer 79, 2036-2044 (1997)
KP-N-NB: donated by Dr. Takafumi Matsumura of Kyoto Prefectural University, Dept. of Pediatrics
NB69: (Riken Cell Bank: RC0480)
NBTu-1: donated by Dr. Kinji Yokomori of Japanese Red Cross Medical Center, Dept. of Pediatrics
SK-N-MC (ATCC Number: HTB-10)
SK-N-SH (ATCC Number: HTB-11).

Two of the obtained confluent culturing plates were used to obtain cells by trypsin treatment. The obtained cells were suspended in 5 ml of TEN buffer (TEN: 50 mM Tris-HCl (pH 8.0), 1 mM EDTA, 100 mM NaCl) and homogenized. To the homogenized suspension there were added 750 µl of SDS (10%) and 125 µl of Proteinase K (MERCK Co., 20 mg/ml), and the mixture was gently mixed by inversion and incubated overnight at 50°C for lysis of the cells.

The next day, 5.1 ml of saturated phenol was added, and then the mixture was mixed for one hour and centrifuged at 10,000 rpm for 10 minutes, after which the supernatant was collected. This was further centrifuged at 3,000 rpm for 10 minutes and the supernatant was collected.

After adding 5.1 ml of chloroform:isoamyl alcohol = 24:1 to the collected supernatant and mixing for one hour, the mixture was centrifuged at 3000 rpm for 10 minutes and the supernatant was collected.

Next, 10.2 ml of ethanol which had been precooled at -20°C was added to and mixed with the collected supernatant, the resulting filamentous DNA was collected with a Pasteur pipette, the excess ethanol was removed and the DNA was dried. An appropriate amount of TE buffer (Tris-HCl (pH 8.0), 1 mM EDTA) was added to the dried DNA, and mixing was effected at room temperature for 1-2 days to dissolve the DNA.

### (Example 2) Purification of genomic DNA extracted from human neuroblastoma cell lines

The genomic DNA extracted from human neuroblastoma cell lines was purified under the following conditions using a QIAamp Blood Kit (QIAGEN).

The extracted genomic DNA was quantified by ultraviolet absorption at 260 nm, and to 5-10 µg of the DNA there was added an appropriate amount of TE buffer to 225 µl. After further adding 210 µl of ethanol, the mixture was stirred.

The stirred solution was transferred to a QIAamp column (kit accessory, QIAGEN), and centrifugation was performed at 8,000 rpm for one minute to adsorb the genomic DNA onto the column. A 500 µl portion of Buffer AW (kit accessory, QIAGEN) was added to the column, and centrifugation was performed at 8,000 rpm for one minute. After then further adding Buffer AW and centrifuging at 15,000 rpm for 3 minutes, the column was transferred to a 1.5 ml fresh tube, 200 µl of Buffer AE prewarmed to 70°C was added, and the column was allowed to stand at room temperature for one minute and then centrifuged at 8,000 rpm for one minute to purify the DNA.

The purified genomic DNA was quantified based on the ultraviolet absorption at 260 nm.

### (Example 3) Identification of deletion lines by 1p36 site-specific PCR using human neuroblastoma genomic DNA

The genomic DNA of the 27 human neuroblastoma cell lines extracted and purified in Examples 1 and 2 was used for identification of the 1p36 deletion by the following site-specific PCR. The PCR primers used were products of Sawady Technology Co., Ltd., unless otherwise specified.

A 10 µl mixture was prepared by combining 1 µl of genomic DNA solution (2 ng/µl), 1 µl of 10xPfu buffer, 1 µl of dNTPs (2 mM), 0.2 µl of D1S548F (20 pmole/µl), 0.2 µl of D1S548R (20 pmole/µl), 0.2 µl of D1S244F (20 pmole/µl), 0.2 µl of D1S244R (20 pmole/µl), 6.12 µl of sterile distilled water and 0.08 µl of Pfu.

The D1S548 and D1S244 are both known primer sets capable of specifically amplifying the 1p36.23-31 region, and their sequences are shown in Table 1.

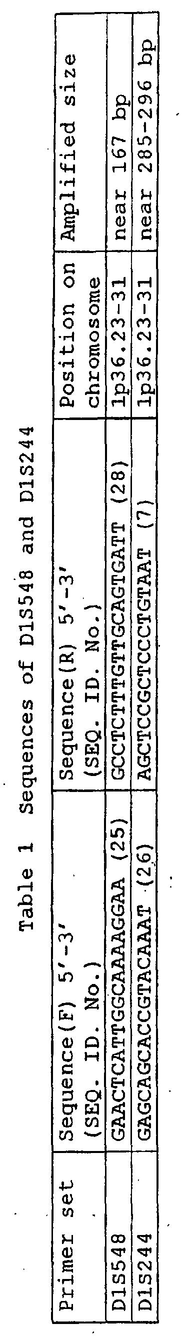

The 1.3 Mb chromosome telomere end was amplified by D1S548 and D1S244. F and R indicate the directions of the primers.

When absolutely no deletion is present in the 1p36.23-31 region, an amplified band is observed near 167 bases for D1S548 and near 285-296 bases for D1S244. When a deletion is present in the 1p36.23-31 region specified by this invention, a band is observed near 167 bases for D1S548, but no amplified band is observed near 285-296 bases for D1S244.

After denaturing the mixture at 96°C for 3 minutes, it was provided for PCR with 30 repeated cycles of 94°C for 30 seconds, 56°C for 45 seconds and 72°C for one minute, followed by a final incubation at 72°C for 10 minutes. The obtained PCR product was subjected to electrophoresis using 4% agarose gel. The results are shown in Fig. 1.

A band near 169 bases amplified by D1S548 as the positive control primer set was observed in all of the cell lines. In contrast, a band near 285-296 bases amplified by D1S244 was observed only in lines NB-1 and MASS-NB-SCH-1.

### (Example 4)

A PCR was conducted with each of 5 other primer sets, SGC-30343, D1S2021, D1S1768, PGD and D1S2736, which are known to specifically amplify the 1p36.23-31 region of the two cell lines NB-1 and ESS-NB-SCH-1. As a positive control, there were used two different types of human Nerve Growth Factor β (hNGF β) located at position 13 of the short arm of human chromosome 1 (1p13). hNGFβ 60s is a primer with an annealing temperature near 60°C while hMGF β 57s is a primer with an annealing temperature near 57°C, and the bands are amplified near 253 bp and 229 bp, respectively. The sequences for these primers are shown in Table 2.

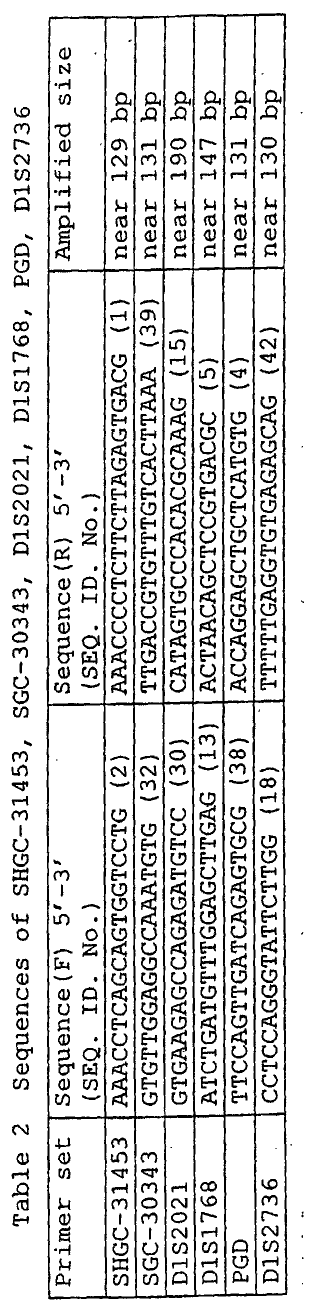

As seen by the results (Fig. 2), there was detected a band for amplification with the hNGFβ positive controls, but no band was observed for amplification in the 1p36.23-31 region.

Based on these results it was determined that the human neuroblastoma lines MB-1 and MASS-NB-SCH-1 have deletions at least in the region from SGC-30343 to D1S2736 of 1p36, on both chromosomes.

### (Example 5) Selection of PAC clones containing sequence of deletion region

The primer set D1S244 which was used to confirm the 1p36.23-31 region deletion in Example 3 was used for PCR with PAC clones (P1-derived artificial chromosomes, product of Oligo Service, Tsukuba Laboratory) having the human genome at random, using an LA PCR kit (Takara Shuzo), and PAC clones containing the deletion region sequence were selected. A 20 µl mixture was prepared by combining 5 µl of PAC DNA pool solution (Z1-Z4: Oligo Service, Tsukuba Laboratory), 2 µl of 10xLA PCR buffer, 2.5 µl of dNTPs (2 mM), 0.4 µl of D1S244F (100 pmole/µl), 0.4 µl of D1S244R (100 pmole/µl), 9.6 µl of sterile distilled water and 0.1 µl of LA Taq (5 u/µl).

After denaturing the mixture at 94°C for 3 minutes, it was provided for PCR reaction with 35 repeated cycles of 94°C for 30 seconds, 56°C for 30 seconds and 72°C for one minute, followed by a final static incubation at 72°C for 5 minutes.

The PCR product was subjected to electrophoresis, and then only the PAC DNA pools for which an amplification band near 285-296 bases was observed were subjected to a second PCR by the same procedure to narrow down the clones.

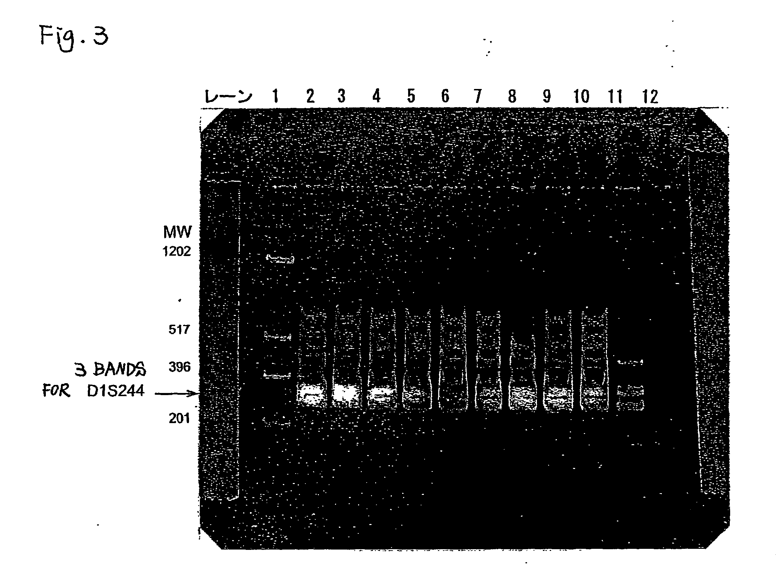

The results are shown in Fig. 3. An amplified band near 285-296 bases was observed for the 4 PAC clones dJ142A6, dJ332G17, dJ345P21 and dJ369D13. Fig. 3 shows the results for only dJ142A6, dJ332G17 and dJ369D13.

As a result of screening in the same manner with the other primer sets used to confirm the 1p36.23-31 region deletion, SHGC-31453, SGC-30343, D1S2021, D1S1768, PGD and D1S2736, there were isolated 14 clones for which amplification was observed with the five primer sets excluding SHGC-31453.

The clones obtained in this manner were taken as the 1st screening. The 18 clones obtained by the 1st screening are shown in Table 3.

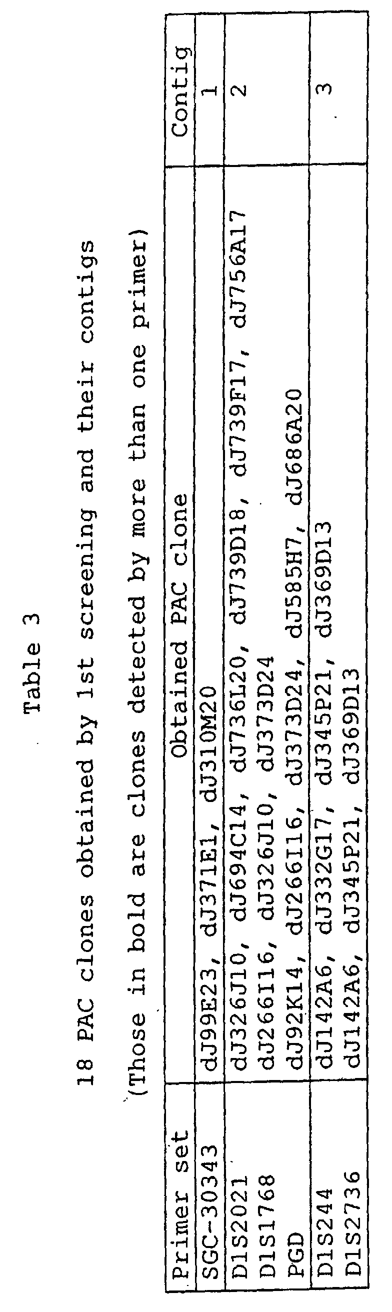

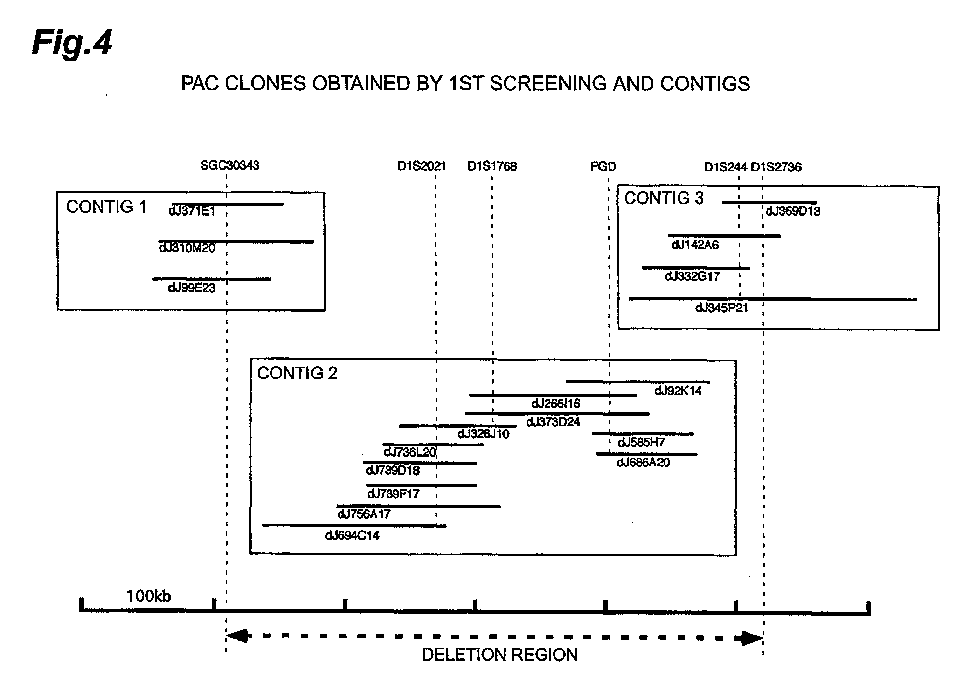

The total of 18 clones obtained by the 1st screening are believed to retain the genome sequence of the deleted region of the two strains NB-1 and MASS-NB-SCH-1, and since some sequences were detected by multiple primer sets, 3 general contigs were formed (respectively designated as Contig 1, Contig 2 and Contig 3). The contigs for the 18 PAC clones obtained by the 1st screening are shown in Fig. 4.

RPN2434 (Kit: Amersham Co.) was used according to the procedure described below to determine the base sequences at both ends of a total of 9 PAC clones, namely the 2 clones dJ99E23 and dJ371E1 from Contig 1, the 4 clones dJ326J10, dJ739D18, dJ756A17 and dJ373D24 from Contig 2 and the 3 clones dJ369D13, dJ142A6 and dJ345P21 from Contig 3. Sterile distilled water was added to a mixture of 2 µg of PAC DNA, 6 pmol of SP6 or T7 primer and 2 µl of each reagent for A, C, G and T (kit accessories), to prepare a total of 6 µl of the mixture. After denaturing the mixture at 95°C for 2 minutes, it was provided for PCR reaction with 30 repeated cycles of 95°C for 30 seconds, 50°C for 30 seconds and 72°C for one minute. After adding 5 µl of reaction suspending solution (kit accessory), the reaction solution was denatured at 95°C for 2 minutes, cooled on ice, subjected to 6% acrylamide gel electrophoresis and sequenced. Ten new walking primer sets were designed from the obtained base sequence data.

Table 4 shows the selected clones and the sequences of their respective primer sets.

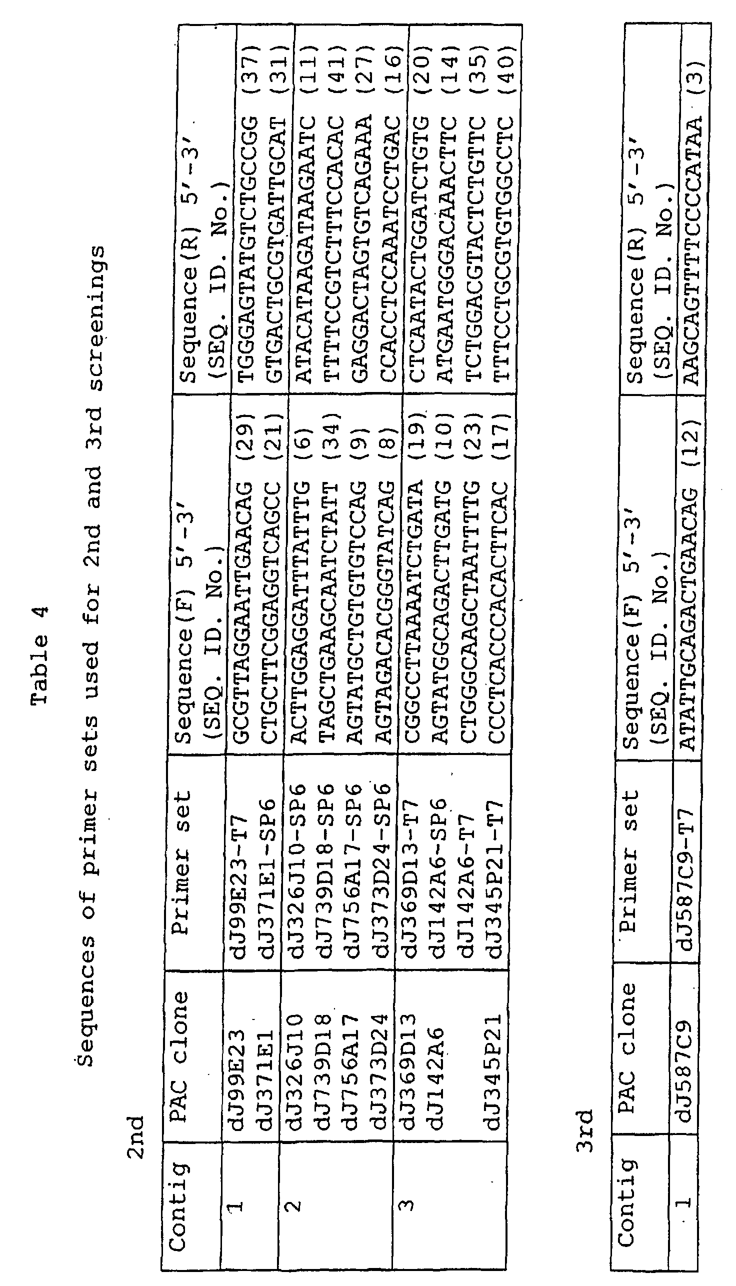

The newly designed primer sets were used for reselection of PAC clones from PAC DNA pools by PCR, and the process of designing of walking primers based on both end sequences of the selected clones and determination of contigs based on walking was repeated. These were taken as the 2nd and 3rd screenings. Table 5 shows the selected clones and the sequences of their respective primers sets.

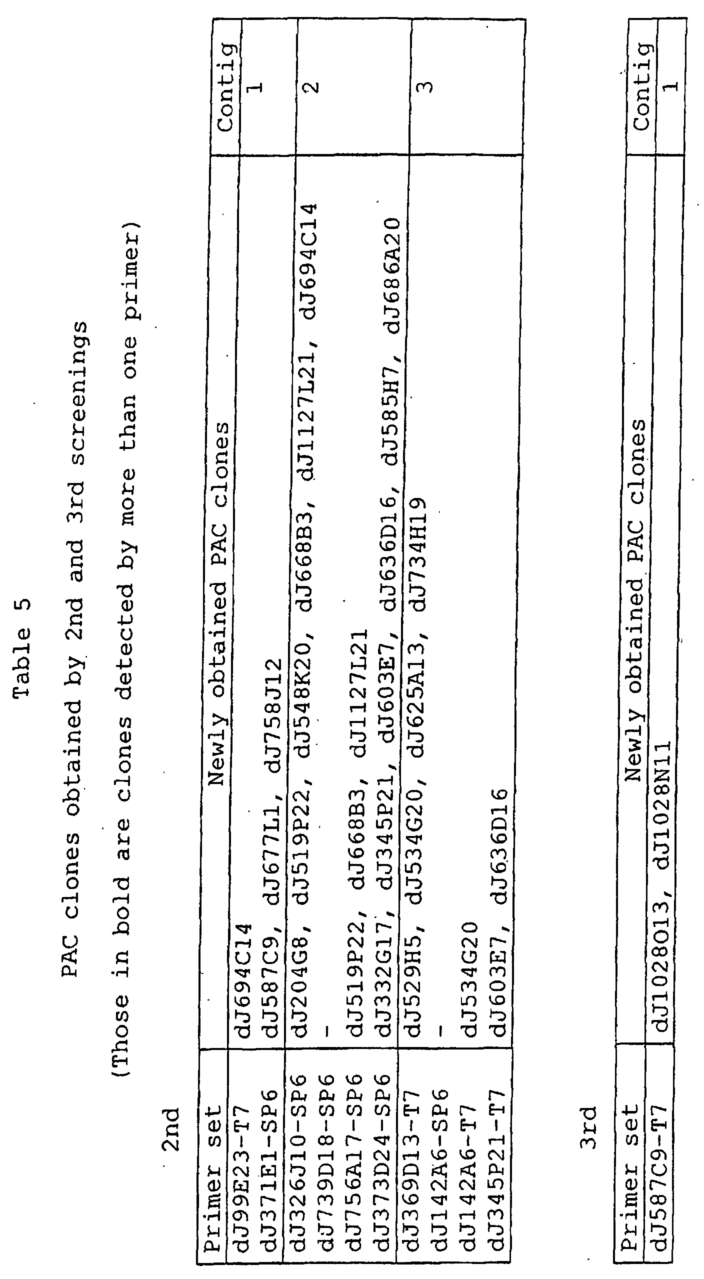

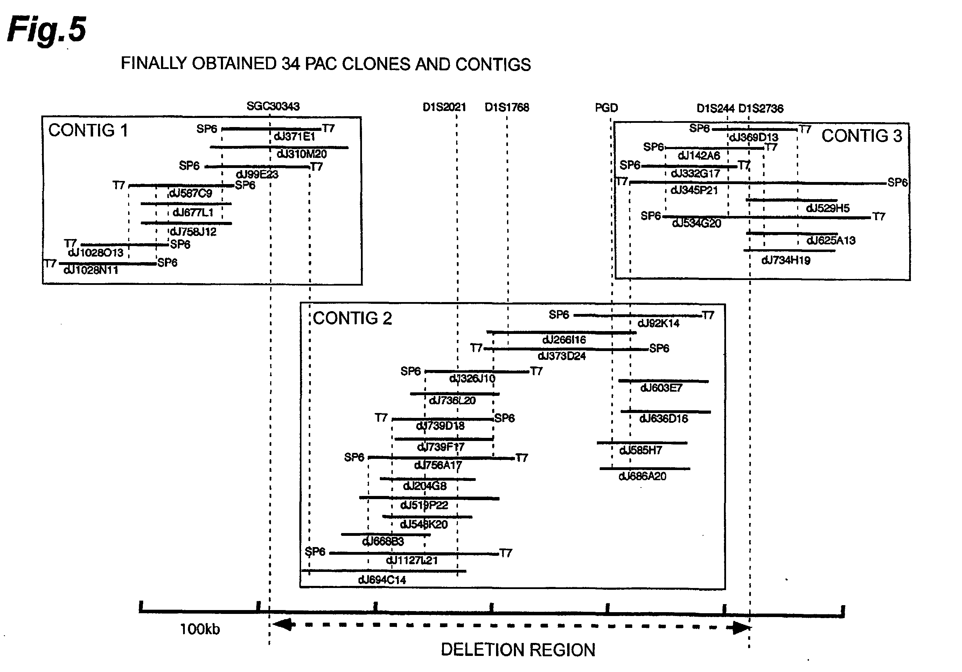

The sequences of the walking primers used for the 2nd and 3rd screenings are shown in Table 4. Finally, one contig of 34 PAC clones was obtained. The results are shown in Fig. 5.

### (Example 6) Identification of genome deletion sites on 1p36 of NB-1 and ESS-NB-SCH-1

Based on Example 4 it was judged that NB-1 and MASS-NB-SCH-1 have deletions on both chromosomes in at least the regions defined from SGC-30343 to D1S2736 of 1p36, but the following procedure was carried out next to identify both ends of the deletion regions.

New primers were synthesized near the deletion region based on the contig data for the PAC clones obtained in Example 5 and the NB-1 and MASS-NB-SCH-1 strains and positive controls were subjected to PCR under the conditions described below, after which the presence or absence of specific band amplification was investigated. An LA PCR kit (Takara Shuzo) was used for PCR in the following manner.

A 10 µl mixture was prepared by combining 1 µl of genomic DNA solution (100 ng/µl), 1 µl of 10xLA PCR buffer, 0.6 µl of dNTPs (2.5 mM, kit accessories), 0.1 µl of Primer F (100 mM), 0.1 µl of Primer R(100 mM), 7.15 µl of sterile distilled water and 0.05 µl of LA Taq (5 u/µl, kit accessory). After denaturing the mixture at 95°C for 2 minutes, it was provided for PCR with 30 repeated cycles of 95°C for 30 seconds and 58°C for one minute.

The PCR reaction product was subjected to electrophoresis using 2.5% agarose gel. The results are shown in Fig. 6.

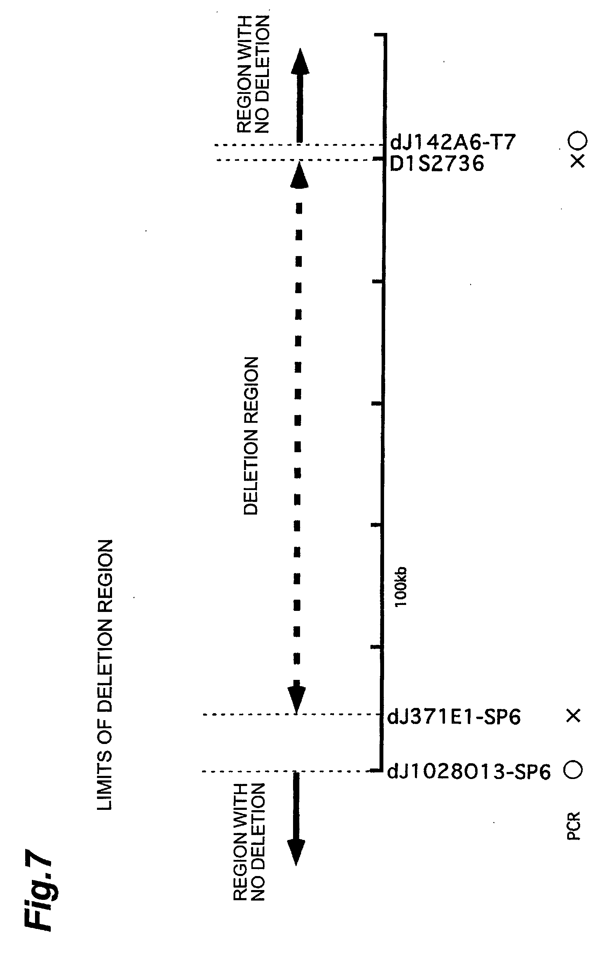

The results gave a range for the genome deletion region of 1p36 as defined by from between the primer set at the SP6 end of the PAC clone dJ1028O13 and the primer set at the SP6 end of the PAC clone dJ371E1 to between the primer set D1S2736 and the primer set at the T7 end of the PAC clone dJ142A6, as shown in Fig. 7.

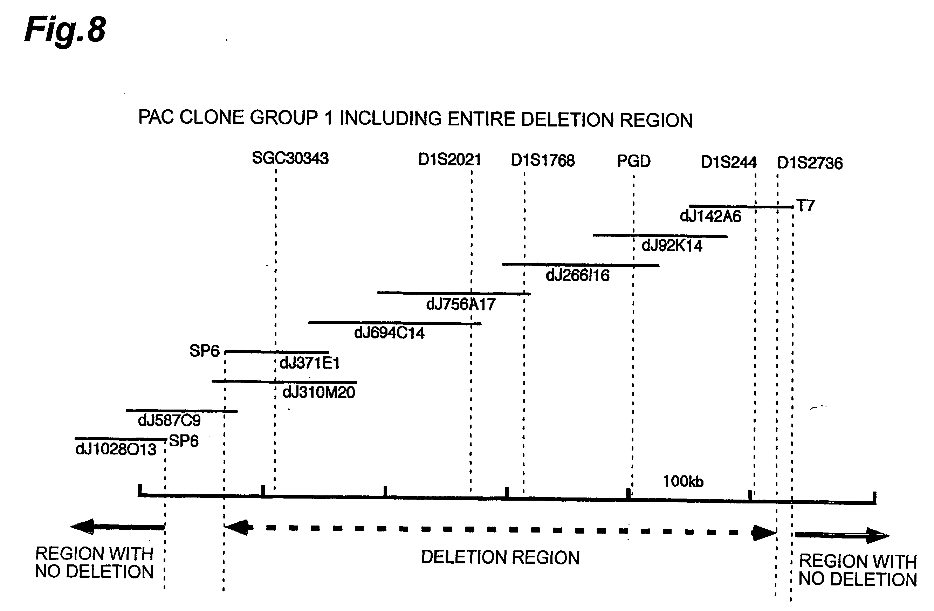

The minimal PAC. clone group including the entire deletion region is shown in Fig. 8. Sequencing of these PAC clones yields the base sequence contained in the deletion region of NB-1 and MASS-NB-SCH-1.

Table 6 shows the sequences of the primer sets used to determine the 1p36 genome deletion region range as elucidated by this invention.

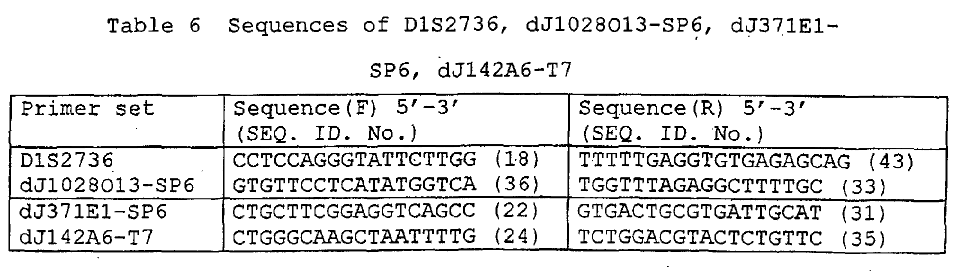

### (Example 7) Base sequence data for 1p36 deletion region

In order to obtain the base sequence data for the deletion region, a search was made of an internet gene map database (a database containing recorded human genome data for over 30,000 mapped genes, ESTs etc. according to chromosome: http://www.ncbi.nlm.nim.nih.gov/genemap99/) with the keywords "primer D1S244", etc. which were used in the aforementioned examples, resulting in numerous hits for genes and ESTs. Upon conducting a DNA sequence homology search through the internet [NCBI BLAST (National Center of Biotechnology Information USA, http://www.ncbi.nlm.nih.gov/BLAST)] for portions of the genome sequence, some ESTs were found. The data for these sequences are shown in Table 7, and those with recorded base sequences are listed in the Sequence Listing.

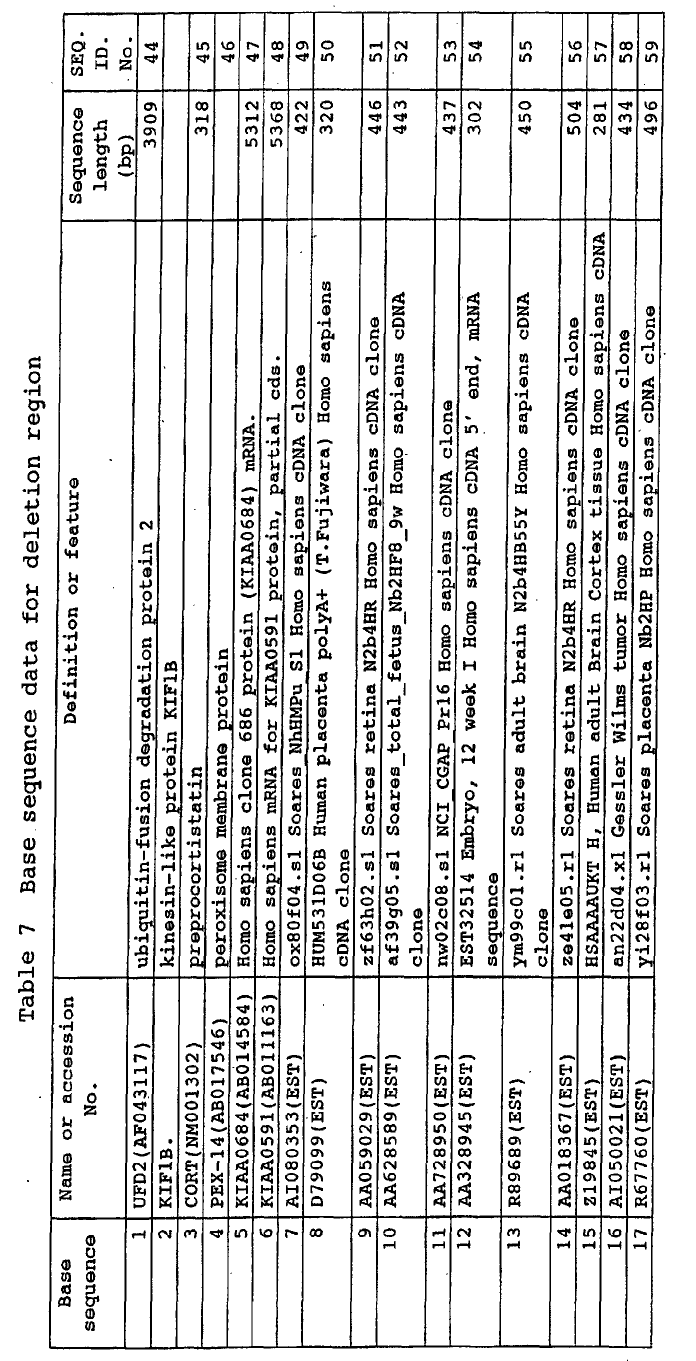

### (Example 8) Sequencing of 1p36 deletion region

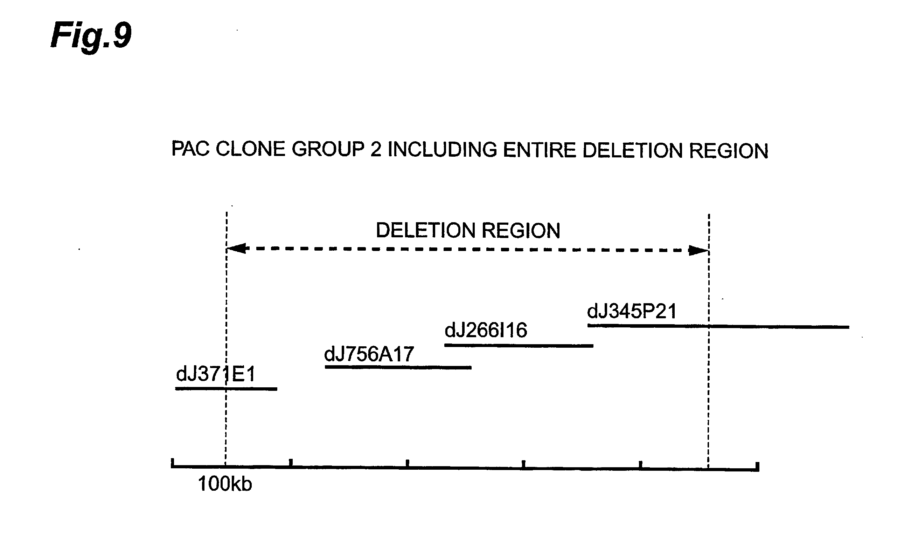

A PAC clone group was selected which included essentially all of the deletion region, in the same manner as Example 6. These PAC clones were dJ371E1, dJ756A17, dJ266I16 and dJ345P21, the overall structural map of which is shown in Fig. 9. Thus, by sequencing each of these PAC clones and aligning their sequences, it is possible to determine approximately the entire length base sequence of the deletion region.

The sequences of each of the clones were determined by the nested deletion (ND) method. See Hattori, M., Tanpakushitsu, Kakusan, Kouso [Proteins, Nucleic acids, Enzymes] 42, 17, 296-303(1997) or Hattori, M. et al., Nucl. Acids Res. 25, 1802-1808(1997).

In this manner, the base sequences of each of the clones, including the deletion region, were determined. Specifically, the base sequences of 5 contiguous partial sequences including the deletion region were found for PAC clone dJ371E1. Their partial sequences were designated as dJ371E1-1 (SEQ. ID. No.60), dJ371E1-2 (SEQ. ID. No.61), dJ371E1-3 (SEQ. ID. No.62), dJ371E1-4 (SEQ. ID. No.63) and dJ371E1-5 (SEQ. ID. No.64). The respective base sequences are listed in the Sequence Listing. The base sequences of 7 contiguous partial sequences including the deletion region were found for PAC clone dJ756A17. Their partial sequences were designated as dJ756A17-1 (SEQ. ID. No.65), dJ756A17-2 (SEQ. ID. No.66), dJ756A17-3 (SEQ. ID. No.67), dJ756A17-4 (SEQ. ID, No.68), dJ756A17-5 (SEQ. ID. No.69), dJ756A17-6 (SEQ. ID. No.70) and dJ756A17-7 (SEQ. ID. No.71). The respective base sequences are listed in the Sequence Listing. The base sequences of 9 contiguous partial sequences including the deletion region were found for PAC clone dJ266I16. Their partial sequences were designated as dJ266I16-1 (SEQ. ID. No.72), dJ266I16-2 (SEQ. ID. No.73), dJ266I16-3 (SEQ. ID. No.74), dJ266I16-4 (SEQ. ID. No.75), dJ266I16-5 (SEQ. ID. No.76), dJ266I16-6 (SEQ. ID. No.77), dJ266I16-7 (SEQ. ID. No.78), dJ266I16-8 (SEQ. ID. No.79) and dJ266I16-9 (SEQ. ID. No.80). The respective base sequences are listed in the Sequence Listing. The base sequences of 8 contiguous partial sequences including the deletion region were found for PAC clone dJ345p-21. Their partial sequences were designated as dJ345p-21-1 (SEQ. ID. No.81), dJ345p-21-2 (SEQ. ID. No.82), dJ345p-21-3 (SEQ. ID. No.83), dJ345p-21-4 (SEQ. ID. No.84), dJ345p-21-5 (SEQ. ID. No.85), dJ345p-21-6 (SEQ. ID. No.86), dJ345p-21-7 (SEQ. ID. No.87) and dJ345p-21-8 (SEQ. ID. No.88). The respective base sequences are listed in the Sequence Listing.

### (Example 9) Diagnosis for prognosis of human neuroblastoma based on base sequences included in deletion region

Cells of patients with pediatric neuroblastoma were used to examine the correlation between the presence or absence of LOH for 1p36 demonstrated here and various factors which are associated with tumor prognosis. The results are shown in Table 8.

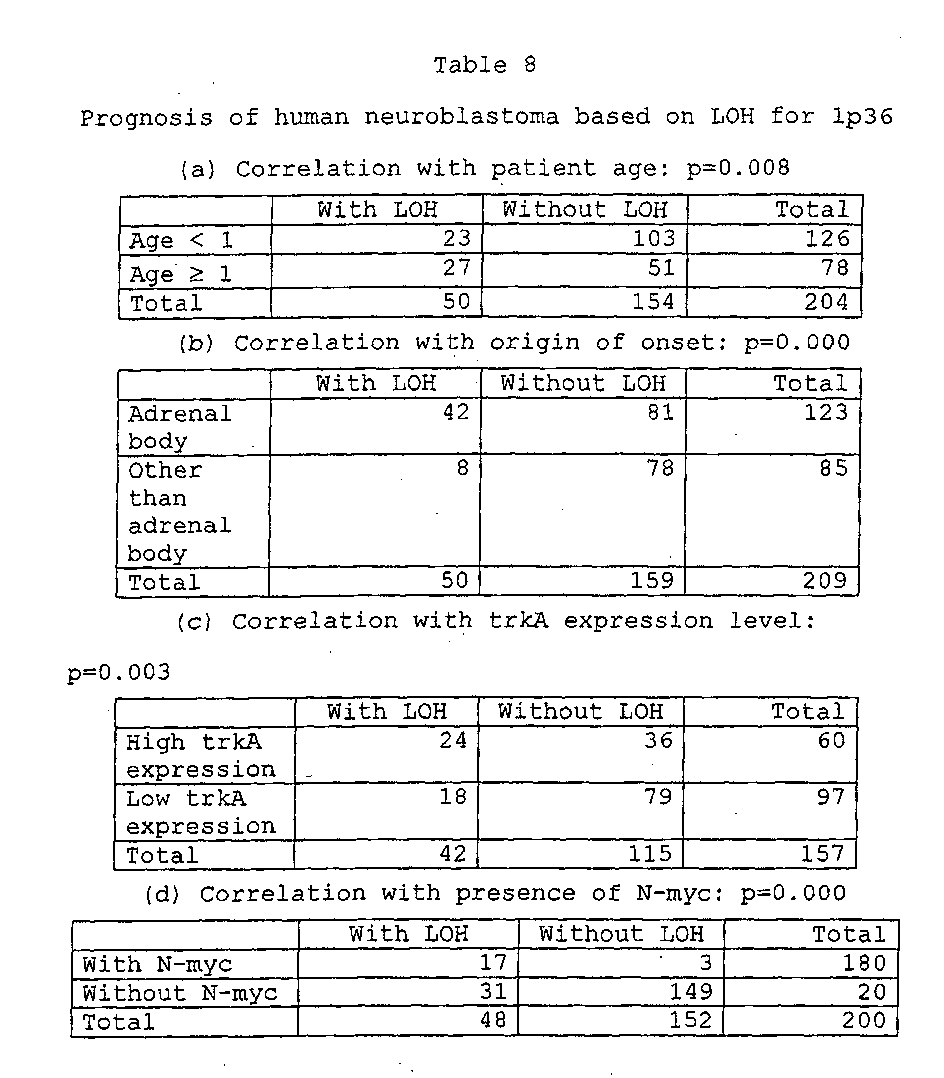

Generally speaking, patients with high expression of the oncogene trkA are considered to have poor prognosis. See Nakagawara, A. et al., Cancer Res. 52, 1364-1368(1992), Nakagawara, A. et al., N. Engl. J. Med. 328, 847-854(1993).

In fact, upon examining LOH for 1p36 across 157 pediatric neuroblastoma patients based on comparison of the degree of trk gene expression, among 42 patients who had LOH on 1p36, 24 patients had high trk expression while 18 had low trk expression. Conversely, among the 115 patients with no LOH on 1p36, 36 patients had high trk expression while 79 had low trk expression. A good correlation was therefore obtained between LOH on 1p36 and trk expression.

Prognosis is generally also poor for patients with high age of onset (Nakagawara A. et al., Med. Pediatr. Oncol. 31, 113-115(1998)), patients with primary lesion in the adrenal body (Nakagawara A. et al., Surgery 104, 34-40(1988)), and patients with high expression of the oncogene N-myc (Brodeur GM. et al., Science 224, 1121-1124(1984)), and a very stable correlation was found with the presence or absence of LOH for 1p36.

This confirms that the presence or absence of LOH for 1p36 can serve as an excellent marker for prognosis and identification.

### Industrial Applicability

According to this invention, there has been identified a homozygous deletion region present in common at position 36 on the short arm of chromosome 1 of human neuroblastoma. Based on the base sequence data of the deletion region it is possible to design a method for diagnosis, prognosis of tumors, as well as a tumor marker which can be used therefor, while the base sequence data of the deletion region can also be used to develop a method of tumor gene diagnosis and an anticancer agent, and to develop a method of analyzing the tumor pathology mechanism and a reagent to be used for analysis of the tumor pathology mechanism.

## Claims

1. A homozygous deletion region present in common at position 36 on the short arm of chromosome 1 of human neuroblastoma (1p36).

2. The region according to claim 1, **characterized by** being defined by at least from between a primer set at the SP6 end of the PAC clone dJ1028013 and a primer set at the SP6 end of the PAC clone dJ371E1 to between the primer set D1S2736 and a primer set at the T7 end of the PAC clone dJ142A6.

3. The region according to claim 1 or 2, **characterized by** being further defined by at least the primer set SGC30343 and D1S2736.

4. The region according to claim 1 or 2, **characterized by** being deleted in the human neuroblastoma cell lines NB-1 and MASS-NB-SCH-1.

5. The region according to claim 3, **characterized by** being deleted in the human neuroblastoma cell lines NB-1 and MASS-NB-SCH-1.


SEQUENCE LISTING
<110> Hisamitsu Pharmaceutical Co., Inc.

<120> Human 1p36 Homozygous Deletion Region

<130> FP00-0197-00

<140> PCT/JP00/05930
<141> 2000-08-31

<150> JP 11/245962
<151> 1999-08-31

<150> JP 12/136266
<151> 2000-05-9

<160> 88

<170> PatentIn Ver. 2.1

<210> 1
<211> 23
<212> DNA
<213> Artificial Sequence

<220>
<223> PCR primer

<400> 1
aaacccctct tcttagagtg acg 23

<210> 2
<211> 20
<212> DNA
<213> Artificial Sequence

<220>
<223> PCR primer

<400> 2
aaacctcagc agtggtcctg 20

<210> 3
<211> 18
<212> DNA
<213> Artificial Sequence

<220>
<223> PCR primer

<400> 3
aagcagtttt ccccataa 18

<210> 4
<211> 19
<212> DNA
<213> Artificial Sequence

<220>
<223> PCR primer

<400> 4
accaggagct gctcatgtg 19

<210> 5
<211> 19
<212> DNA
<213> Artificial Sequence

<220>
<223> PCR primer

<400> 5
actaacagct ccgtgacgc 19

<210> 6
<211> 18
<212> DNA
<213> Artificial Sequence

<220>
<223> PCR primer

<400> 6

acttggagga tttatttg                                                    18

<210> 7
<211> 18
<212> DNA
<213> Artificial Sequence

<220>
<223> PCR primer

<400> 7
agctccgctc cctgtaat                                                    18

<210> 8
<211> 18
<212> DNA
<213> Artificial Sequence

<220>
<223> PCR primer

<400> 8
agtagacacg ggtatcag                                                    18

<210> 9
<211> 18
<212> DNA
<213> Artificial Sequence

<220>
<223> PCR primer

<400> 9
agtatgctgt gtgtccag                                                    18

<210> 10
<211> 18
<212> DNA
<213> Artificial Sequence

<220>
<223> PCR primer

<400> 10
agtatggcag acttgatg 18

<210> 11
<211> 18
<212> DNA
<213> Artificial Sequence

<220>
<223> PCR primer

<400> 11
atacataaga taagaatc 18

<210> 12
<211> 18
<212> DNA
<213> Artificial Sequence

<220>
<223> PCR primer

<400> 12
atattgcaga ctgaacag 18

<210> 13
<211> 21
<212> DNA
<213> Artificial Sequence

<220>
<223> PCR primer

<400> 13
atctgatgtt tggagcttga g 21

<210> 14
<211> 18
<212> DNA
<213> Artificial Sequence

<220>
<223> PCR primer

<400> 14
atgaatggga caaacttc 18

<210> 15
<211> 20
<212> DNA
<213> Artificial Sequence

<220>
<223> PCR primer

<400> 15
catagtgccc acacgcaaag 20

<210> 16
<211> 18
<212> DNA
<213> Artificial Sequence

<220>
<223> PCR primer

<400> 16
ccacctccaa atcctgac 18

<210> 17
<211> 18
<212> DNA
<213> Artificial Sequence

<220>
<223> PCR primer

<400> 17
ccctcaccca cacttcac 18

<210> 18
<211> 18
<212> DNA

<213> Artificial Sequence

<220>
<223> PCR primer

<400> 18
cctccagggt attcttgg 18

<210> 19
<211> 18
<212> DNA
<213> Artificial Sequence

<220>
<223> PCR primer

<400> 19
cggccttaaa atctgata 18

<210> 20
<211> 18
<212> DNA
<213> Artificial Sequence

<220>
<223> PCR primer

<400> 20
ctcaatactg gatctgtg 18

<210> 21
<211> 18
<212> DNA
<213> Artificial Sequence

<220>
<223> PCR primer

<400> 21
ctgcttcgga ggtcagcc 18

<210> 22

<211> 18
<212> DNA
<213> Artificial Sequence

<220>
<223> PCR primer

<400> 22
ctgcttcgga ggtcagcc                                                    18

<210> 23
<211> 18
<212> DNA
<213> Artificial Sequence

<220>
<223> PCR primer

<400> 23
ctgggcaagc taattttg                                                    18

<210> 24
<211> 18
<212> DNA
<213> Artificial Sequence

<220>
<223> PCR primer

<400> 24
ctgggcaagc taattttg                                                    18

<210> 25
<211> 20
<212> DNA
<213> Artificial Sequence

<220>
<223> PCR primer

<400> 25
gaactcattg gcaaaaggaa                                                  20

<210> 26
<211> 18
<212> DNA
<213> Artificial Sequence

<220>
<223> PCR primer

<400> 26
gagcagcacc gtacaaat 18

<210> 27
<211> 18
<212> DNA
<213> Artificial Sequence

<220>
<223> PCR primer

<400> 27
gaggactagt gtcagaaa 18

<210> 28
<211> 20
<212> DNA
<213> Artificial Sequence

<220>
<223> PCR primer

<400> 28
gcctctttgt tgcagtgatt 20

<210> 29
<211> 18
<212> DNA
<213> Artificial Sequence

<220>
<223> PCR primer

<400> 29
gcgttaggaa ttgaacag                                                    18

<210> 30
<211> 20
<212> DNA
<213> Artificial Sequence

<220>
<223> PCR primer

<400> 30
gtgaagagcc agagatgtcc                                                  20

<210> 31
<211> 18
<212> DNA
<213> Artificial Sequence

<220>
<223> PCR primer

<400> 31
gtgactgcgt gattgcat                                                    18

<210> 32
<211> 19
<212> DNA
<213> Artificial Sequence

<220>
<223> PCR primer

<400> 32
gtgttggagg ccaaatgtg                                                   19

<210> 33
<211> 18
<212> DNA
<213> Artificial Sequence

<220>

<223> PCR primer

<400> 33
tggtttagag gcttttgc 18

<210> 34
<211> 18
<212> DNA
<213> Artificial Sequence

<220>
<223> PCR primer

<400> 34
tagctgaagc aatctatt 18

<210> 35
<211> 18
<212> DNA
<213> Artificial Sequence

<220>
<223> PCR primer

<400> 35
tctggacgta ctctgttc 18

<210> 36
<211> 18
<212> DNA
<213> Artificial Sequence

<220>
<223> PCR primer

<400> 36
gtgttcctca tatggtca 18

<210> 37
<211> 18
<212> DNA
<213> Artificial Sequence

<220>
<223> PCR primer

<400> 37
tgggagtatg tctgccgg                                                    18

<210> 38
<211> 20
<212> DNA
<213> Artificial Sequence

<220>
<223> PCR primer

<400> 38
ttccagttga tcagagtgcg                                                  20

<210> 39
<211> 22
<212> DNA
<213> Artificial Sequence

<220>
<223> PCR primer

<400> 39
ttgaccgtgt ttgtcactta aa                                               22

<210> 40
<211> 18
<212> DNA
<213> Artificial Sequence

<220>
<223> PCR primer

<400> 40
tttcctgcgt gtggcctc                                                    18

<210> 41
<211> 18

<212> DNA
<213> Artificial Sequence

<220>
<223> PCR primer

<400> 41
ttttccgtct ttccacac                                                    18

<210> 42
<211> 20
<212> DNA
<213> Artificial Sequence

<220>
<223> PCR primer

<400> 42
tttttgaggt gtgagagcag                                                  20

<210> 43
<211> 20
<212> DNA
<213> Artificial Sequence

<220>
<223> PCR primer

<400> 43
tttttgaggt gtgagagcag                                                  20

<210> 44
<211> 5314
<212> DNA
<213> Homo sapience

<400> 44
   gaattcccct gctctccttc ctaccctttg gtctcgagaa cagaaggatc tctccttaac 60
   gcctttcacc attaagagga aagcgatgga ggagctgagc gctgatgaga ttcgacggag 120
   gcgccttgca cgacttgctg gtggacagac ctctcagcca accaccccac tcacctctcc 180
   ccagagggag aaccctccgg ggcctcccat agcggcatca gccccaggac cctctcagag 240
   tcttggtctc aatgtccaca acatgacccc agctacctcc ccaataggtg catcaggagt 300
   agcccatcga agccagagca gtgaaggagt cagttctctc agcagctcgc cctctaatag 360
   ccttgaaacg caatctcagt ctctctcacg ttcccagagc atggatatcg atggtgtctc 420
   atgtgagaaa agcatgtccc aggtggatgt ggattcagga attgaaaaca tggaggttga 480
   tgaaaatgat cgaagagaaa agcggagcct cagtgataag gagccttcct cgggccctga 540
   agtgtctgaa gagcaggcct tacagctggt ctgtaagatc ttccgtgtct cttggaagga 600
   ccgggacaga gatgtcatct ttctttcttc tctttctgca cagtttaagc agaacccaaa 660
   agaagtattc tccgatttta aggacttgat tggccagatt ttaatggaag tgctaatgat 720
   gtccactcag accagagatg aaaacccatt tgccagtctg acagccacat cacagccaat 780
   tgctgcagca gcacggtcac cagacagaaa tctcttgcta aacactggct ccaatccagg 840
   aacaagcccc atgttctgca gcgtggcttc ctttggtgcc agctctttgt ctagcctcta 900
   tgaaagtagt ccggctccca ctcccagttt ctggagctct gttcccgtga tgggcccgtc 960
   tcttgcctca ccttcccgtg cagccagcca gttggctgtg ccttccactc ccctcagtcc 1020
   tcacagtgca gcctctggaa ctgctgcggg aagccagcct tcatccccgc ggtatcgccc 1080
   ctacactgtc actcacccat gggcgtcctc aggcgtctcc attctgtcga gctccccaag 1140
   tccccctgcc ctcgccagta gcccccaagc agtgcccgcc agcagttcca gacagaggcc 1200
   cagcagcacg ggtccacccc taccacccgc ctcacccagt gccacgagca gacgcccctc 1260
   ctccctgagg atctctccta gtttgggagc ctctggtgga gcaagtaatt gggattccta 1320
   cagtgaccat ttcaccattg aaacctgcaa agagacagat atgctgaact acctcatcga 1380
   gtgtttcgac cgagttggaa tagaggaaaa aaaagcacca aagatgtgca gccagccagc 1440
   agtcagccag cttctgagca acatccgctc acagtgcata tcccatactg ctttagtact 1500
   acaaggctcc ctaacacagc ccaggtcctt gcagcagccg tccttcctag tgccgtatat 1560
   gctgtgtagg aatctcccat atggcttcat tcaggaactg gtgagaacca ctcaccagga 1620
   tgaagaagtg ttcaagcaga tatttatccc cattttacaa ggcctggctc ttgctgccaa 1680
   agagtgctcc ctcgacagtg actactttaa ataccccctc atggcactag gtgagctctg 1740
   tgaaaccaag tttgggaaga cacaccctgt gtgcaatttg gttgcttctt tgcggttgtg 1800
   gttgccgaaa tccttaagtc ctggctgtgg gcgggagctg cagagactct cttacttagg 1860
   ggctttcttt agcttctcag tctttgcaga agatgatgtt aaagtggttg aaaaatactt 1920
   ctcagggcct gccattaccc tggaaaacac tcgtgtggtt agccaatcat tgcagcatta 1980
   cttagagctc ggaaggcaag agctttttaa gattctgcat agtattttgt taaatggcga 2040
   aacccgtgag gctgctctca gttacatggc ggctgtcgtc aatgccaata tgaagaaagc 2100
   acagatgcag acagatgata gattggtgtc tacagatgga tttatgctga atttcctttg 2160
   ggtactgcag cagctaagta caaaaatcaa gttagaaaca gttgatccca cgtatatttt 2220
   tcacccaaga tgtcggatta ctcttcccaa tgatgagacg cgtgtgaatg caacgatgga 2280
   agatgtgaat gactggctga ctgaactcta tggcgatcag cctccatttt ctgagccgaa 2340
   attccctacg gagtgcttct ttctcaccct gcatgctcac cacctctcta ttctgcctag 2400
   ttgccgtcgc tatatccgca gactccgggc tatccgggag ctcaatagaa ctgtagaaga 2460
   tttgaaaaat aatgaagccc aatggaaaga ttccccactg gcaactagac accgcgaaat 2520
   gctgaagcgc tgtaaaactc agcttaagaa actggtacgg tgcaaggcct gtgctgatgc 2580
   tggcctactt gacgagagct tcctgagaag atgtctgaat ttttatggcc ttctcattca 2640
   gctgctgctc cgcatcctgg accccgcata tcccgatata acactgcctt taaattcaga 2700
   tgtccccaag gtatttgcag cgttgcctga gttttatgta gaagatgttg cagaattttt 2760
   attttttatt gtacaatact ctccccaggc gctttatgag ccctgtactc aggatattgt 2820
   gatgttcctt gttgtgatgt tgtgcaacca gaactacatc cgaaacccat atttggtggc 2880
   caaactggta gaagtcatgt ttatgaccaa ccctgctgtt cagccacgaa cccagaagtt 2940
   ttttgaaatg attgagaacc atcctctctc caccaagttg ttggtacctt ccctgatgaa 3000
   gttttataca gatgttgagc ataccggagc caccagcgag ttttatgaca agttcacaat 3060
   tcgctatcat attagcacca tttttaaaag cctttggcaa aacatagctc accatggcac 3120
   ctttatggag gagttcaact ccgggaagca gtttgttcgc tatataaaca tgttgataaa 3180
   cgacacgacg tttttgctcg atgaaagtct ggagtctctg aagcgaatcc atgaagtgca 3240
   ggaagagatg aagaacaaag aacagtggga ccagttgccc cgggatcagc agcaggctcg 3300
   tcagtctcag cttgctcagg atgagcgtgt gtcccgctct tacctcgccc tggccaccga 3360
   aaccgtggac atgttccaca tcctcacgaa gcaggtccag aagcccttcc tcagaccgga 3420
   gcttggaccc cgattggctg caatgctgaa ctttaatctt cagcaacttt gtggccccaa 3480
   gtgccgtgac ctgaaagttg aaaaccctga gaaatacggc tttgaaccaa agaagctgtt 3540
   ggaccaactg acggatattt acttacagct ggactgtgct cggttcgcga aagccattgc 3600
   tgacgaccag agatcctaca gtaaggaatt gtttgaagaa gttatttcaa agatgcggaa 3660
   ggcagggatc aaatccacaa tagcaataga aaaatttaag ctgctcgccg agaaagtgga 3720
   ggagatagtg gccaagaacg cacgcgcaga aatcgactac agcgacgctc ctgatgagtt 3780
   cagagacccc ctgatggaca ccctcatgac agaccccgtg cggctgccct ctggcaccat 3840
   catggaccgc tccatcatcc tgcggcacct gctcaactcc cccacggacc ccttcaaccg 3900
   gcagacgctg acagagagca tgctggaacc agtgccagaa ctgaaagagc agattcaggc 3960
   gtggatgaga gagaaacaga acagcgatca ctaaaccgtt ccgccgccca ccctctgcta 4020
   gacacagcca aggccaacga ggcaagcaga agcagcggcc gcagcgaagc tgctgttcat 4080
   gtgttggagg ccaaatgtgg caaaccaacc ccaggcccac ccagagcgag caaacgctga 4140
   gacctgaaag gacatggatg agaagaggag cccgcttcct gtacatatat ttaagtgaca 4200
   aacacggtca aaagcttaag ggacaggttt tatggttgct tgtgtaataa agcatgtcct 4260
   tcgtatgtca cagtttgggg caacggaagt cttttagtga tggctaatgg gtctgggcag 4320
   catcccttca tgaatttttt tttaatccaa tatccgttga tttgattgtg attagagaac 4380
   cttggacatt ttgctgctaa agaatctttt ttcccctctc ccccttcctg accctacttg 4440
   cactgctgtg gattttttta agagaagcaa aaacaaaagt aaactccttt ccctggccct 4500
   ccaaacatat attctgtgag ataactgtgc ctgctaccaa gtgttaatcc tgggatcgta 4560
   tttttatatc atattcacat atttgttttt ttaattggtg ttagatgaca tgattaataa 4620
   aaaaggcaag atattttcag aatttgaatt tcagtttttt ttttcttttg aaatgtccct 4680
   ttaagatttt tttattctaa acaaaataaa gaaaatcctg ctgctctggt ccttgtgata 4740
   agcctctctt cggcatctga ggagcagctg cagcaaaatc taggggtgta agtgtatcag 4800
   gacttatgtg acttatattt tggggagatg agggttgggt ttttttttaa tgctacgtga 4860
   cagtttgaaa ccttcacagt tatcctctgg gggtaaatca attcttcaac ccttgggtgt 4920
   gtgagtttga ggcagggtca tttgtgtgat gtgtttggcc ttaccaaagc aaaagagggt 4980
   gcaagaatgt gggagtatgt ctgccggttt caacacacac agacaaacac agccacacgc 5040
   gcacacaagt ataagacttt ttgtattact gctccctact taacatactt gaattctcaa 5100
   atttcctttg ggtaaaaaaa aaaaaaagga tttgaaacca taaagtgttc tgaagaaatt 5160
   aagtctataa aaagcatact ttcttttttc ttttcctttt ttccctccac agacaatgtc 5220
   ctctgttcaa ttcctaacgc aaactacaat aaatggtgac acacgttcag aagaaaaaaa 5280
   aaaaaaaaaa aaaaaaaaaa aaaaaaaaaa aaaa 5314

<210> 45
<211> 709
<212> DNA
<213> Homo sapience

<400> 45
ggcacgaggc caaacattga tttcagggct gccaggaagg aagagcagca gcagggtggg 60
agagaagctc cagtcagccc acaagatgcc attgtccccc ggcctcctgc tgctgctgct 120
ctccggggcc acggccaccg ctgccctgcc cctggagggt ggccccaccg gccgagacag 180
cgagcatatg caggaagcgg caggaataag gaaaagcagc ctcctgactt tcctcgcttg 240
gtggtttgag tggacctccc aggccagtgc cgggcccctc ataggagagg aagcccggga 300
ggtggccagg cggcaggaag gcgcaccccc ccagcaatcc gcgcgccggg acagaatgcc 360
ctgcaggeac ttcttctgga agaccttctc ctcctgcaaa taaaacctca cccatgaatg 420
ctcacgcaag tgtaatgaca gacctgaata aaatgtatta agcagcagtg atctttcctc 480
tcctccttcc caagtcattg aaaagtgttt gttatttaaa ttccaataat gcccaatact 540
gacgtgtctt gagtaatttg gaacccaaaa gtgaagatct ttgataaaga ttttttttgt 600
ggttcgactg gactgtgctg agtgcgggca ctgggctttt cttctgatgt tcattatggt 660
gctgggaagc tctgtctttg atttaaaata aaatagctaa aggctacac 709

<210> 46
<211> 1908
<212> DNA
<213> Homo sapience

<400> 46
gaaagatggc gtcctcggag caggcagagc agccgagcca gccaagctct actccaggaa 60
gtgaaaatgt gctgcctcga gagccgctga ttgccacggc agtgaagttt ctacagaatt 120
cccgggtccg ccagagccca cttgcaacca ggagagcatt tctaaagaag aaagggctga 180
cagatgaaga gattgatatg gccttccagc agtcgggcac tgctgccgat gagccttcgt 240
ccttgggccc agccacacag gtggttcctg tccagccccc tcacctcata tctcagccat 300
acagtcccgc aggctcccga tggcgagatt acggcgccct ggccatcatc atggcaggca 360
ttgcatttgg ctttcaccag ctctacaaga aatacctgct ccccctcatc ctgggcggcc 420
gagaggacag aaagcagctg gagaggatgg aggccggtct ctctgagctg agtggcagcg 480
tggcccagac agtgactcag ttacagacga ccctcgcctc cgtccaggag ctgctgattc 540
agcagcagca gaagatccag gagcttgccc acgagctggc cgctgccaag gccaccacat 600
ccaccaactg gatcctggag tcccagaata tcaacgaact caagtccgaa attaactcct 660
tgaaagggct tcttttaaat cggaggcagt tccctccatc cccatcagcc ccgaagatcc 720
cctcctggca gatcccagtc aagtcaccgt caccctccag ccctgcggcc gtgaaccacc 780
acagcagcag cgacatctca cctgtcagca acgagtccac gtcgtcctcg cctgggaagg 840
agggccacag ccccgagggc tccacggtca cctaccactt gctgggcccc caggaggaag 900
gcgagggggt ggtggacgtc aagggccagg tgcggatgga ggtgcaaggc gaggaggaga 960

agagggagga caaggaggac gaggaggatg aggaggatga tgatgtgagc catgtggacg 1020
aggaggactg cctgggggtg cagagggagg accgccgggg cggggatggg cagatcaacg 1080
agcaggtgga gaagctgcgg cggcccgagg gcgccagcaa cgagagtgag cgggactagg 1140
gctgcgcctg ctgcctccag ccctgaggat ggcatctagt gtgcccgtgc gtggccatac 1200
cctgcctccc tctctggccc tgggagggca gcttggagcc caggtagggg gcagagctgt 1260
cctcagctgc actgcggcct ggtggcagtg tggggagtca cacttctgtc cacctggcct 1320
cctctcgcct ggccgccagc cccagcccca gccccagccc caggcccagc tgcctttggc 1380
tttgatctca agtcaggctg aaggcagcga agcctcgggg cccaagcccc tccccagccc 1440
cctctcccgg acagacgcct tgcccagggt gtgtttgctg agtgtcttga ctaccgtgac 1500
accacgcatg gccagagcta gcgtccctac tgcctcccga ctcctcagtg gaggaggagc 1560
tgcggtccct ctggtgtctg ccatccccct ccctccctgg gcccggccct ggacccgtca 1620
ggtgcctgtc cccagcccca accccactca tgccccgtcg tcctcccaga caaatgaaac 1680
cacgctgcgc ttccgatgcc cccgcttgcc gtgtaatggt tcagctaatc ccatggcgag 1740
atgggggctc attccggagg aggagccagg cagcagggcc ttcctgacca acagccagtt 1800
ttgtccttcc ccccaggaaa aaaatgttca tttgtgtgat catgtataga cctcagaacg 1860
gaagatagga ctgtatataa ttgtaataaa taccagttgc cactattt 1908

<210> 47
<211> 3281
<212> DNA
<213> Homo sapience

<400> 47
cgttcccaga gcatggatat cgatggtgtc tcatgtgaga aaagcatgtc ccaggtggat 60
gtggattcag gaattgaaaa catggaggtt gatgaaaatg atcgaagaga aaagcggagc 120
ctcagtgata aggagccttc ctcgggccct gaagtgtctg aagagcaggc cttacagctg 180
gtctgtaaga tcttccgtgt ctcttggaag gaccgggaca gagatgtcat ctttctttct 240
tctctttctg cacagtttaa gcagaaccca aaagaagtat tctccgattt taaggacttg 300
attggccaga ttttaatgga agtgctaatg atgtccactc agaccagaga tgaaaaccca 360
tttgccagtc tgacagccac atcacagcca attgctgcag cagcacggtc accagacaga 420
aatctcttgc taaacactgg ctccaatcca ggaacaagcc ccatgttctg cagcgtggct 480
tcctttggtg ccagctcttt gtctagtttg ggagcctctg gtggagcaag taattgggat 540
tcctacagtg accatttcac cattgaaacc tgcaaagaga cagatatgct gaactacctc 600
atcgagtgtt tcgaccgagt tggaatagag gaaaaaaaag caccaaagat gtgcagccag 660
ccagcagtca gccagcttct gagcaacatc cgctcacagt gcatatccca tactgcttta 720
gtactacaag gctccctaac acagcccagg tccttgcagc agccgtcctt cctagtgccg 780
tatatgctgt gtaggaatct cccatatggc ttcattcagg aactggtgag aaccactcac 840
caggatgaag aagtgttcaa gcagatattt atccccattt tacaaggcct ggctcttgct 900
gccaaagagt gctccctcga cagtgactac tttaaatacc ccctcatggc actaggtgag 960
ctctgtgaaa ccaagtttgg gaagacacac cctgtgtgca atttggttgc ttctttgcgg 1020
ttgtggttgc cgaaatcctt aagtcctggc tgtgggcggg agctgcagag actctcttac 1080
ttaggggctt tctttagctt ctcagtcttt gcagaagatg atgttaaagt ggttgaaaaa 1140

tacttctcag ggcctgccat taccctggaa aacactcgtg tggttagcca atcattgcag 1200
cattacttag agctcggaag gcaagagctt tttaagattc tgcatagtat tttgttaaat 1260
ggcgaaaccc gtgaggctgc tctcagttac atggcggctg tcgtcaatgc caatatgaag 1320
aaagcacaga tgcagacaga tgatagattg gtgtctacag atggatttat gctgaatttc 1380
ctttgggtac tgcagcagct aagtacaaaa atcaagttag aaacagttga tcccacgtat 1440
atttttcacc caagatgtcg gattactctt cccaatgatg agacgcgtgt gaatgcaacg 1500
atggaagatg tgaatgactg gctgactgaa ctctatggcg atcagcctcc attttctgag 1560
ccgaaattcc ctacggagtg cttctttctc accctgcatg ctcaccacct ctctattctg 1620
cctagttgcc gtcgctatat ccgcagactc cgggctatcc gggagctcaa tagaactgta 1680
gaagatttga aaaataatga aagccaatgg aaagattccc cactggcaac tagacaccgc 1740
gaaatgctga agcgctgtaa aactcagctt aagaaactgg tacggtgcaa ggcctgtgct 1800
gatgctggcc tacttgacga gagcttcctg agaagatgtc tgaattttta tggccttctc 1860
attcagctgc tgctccgcat cctggacccc gcatatcccg atataacact gcctttaaat 1920
tcagatgtcc ccaaggtatt tgcagcgttg cctgagtttt atgtagaaga tgttgcagaa 1980
tttttatttt ttattgtaca atactctccc caggcgcttt atgagccctg tactcaggat 2040
attgtgatgt tccttgttgt gatgttgtgc aaccagaact acatccgaaa cccatatttg 2100
gtggccaaac tggtagaagt catgtttatg accaaccctg ctgttcagcc acgaacccag 2160
aagttttttg aaatgattga gaaccatcct ctctccacca agttgttggt accttccctg 2220
atgaagtttt atacagatgt tgagcatacc ggagccacca gtgagtttta tgacaagttc 2280
acaattcgct atcatattag caccattttt aaaagccttt ggcaaaacat agctcaccat 2340
ggcaccttta tggaggagtt caactccggg aagcagtttg ttcgctatat aaacatgttg 2400
ataaacgaca cgacgttttt gctcgatgaa agtctggagt ctctgaagcg aatccatgaa 2460
gtgcaggaag agatgaagaa caaagaacag tgggaccagt tgccccggga tcagcagcag 2520
gctcgtcagt ctcagcttgc tcaggatgag cgtgtgtccc gctcttacct cgccctggcc 2580
accgaaaccg tggacatgtt ccacatcctc acgaagcagg tccagaagcc cttcctcaga 2640
ccggagcttg gaccccgatt ggctgcaatg ctgaacttta atcttcagca actttgtggc 2700
cccaagtgcc gtgacctgaa agttgaaaac cctgagaaat acggctttga accaaagaag 2760
ctgttggacc aactgacgga tatttactta cagctggact gtgctcggtt cgcgaaagcc 2820
attgctgacg accagagatc ctacagtaag gaattgtttg aagaagttat ttcaaagatg 2880
cggaaggcag ggatcaaatc cacaatagca atagaaaaat ttaagctgct cgccgagaaa 2940
gtggaggaga tagtggccaa gaacgcacgc gcagaaatcg actacagcga cgctcctgat 3000
gagttcagag accctctgat ggacaccctc atgacagacc ccgtgcggct gccctctggc 3060
accatcatgg accgctccat catcctgcgg cacctgctca actcccccac ggaccccttc 3120
aaccggcaga cgctgacaga gagcatgctg gaaccagtgc cagaactgaa agagcagatt 3180
caggcgtgga tgagagagaa acagaacagc gatcactaaa ccgttccgcc gcccaccctc 3240
tgctagacac agccaaggcc aacgaggcaa gcagaagcag c 3281

<210> 48
<211> 5368
<212> DNA
<213> Homo sapience

<400> 48
ggaatcagag aagatcattg ctgagttgaa tgaaacttgg gaagagaagc ttcgtaaaac 60
agaggccatc agaatggaga gagaggcttt gttggctgag atgggagttg ccattcggga 120
agatggagga accctagggg ttttctcacc taaaaagacc ccacatcttg ttaacctcaa 180
tgaagaccca ctaatgtctg agtgcctact ttattacatc aaagatggaa ttacaagggt 240
tggccaagca gatgctgagc ggcgccagga catagtgctg agcggggctc acattaaaga 300
agagcattgt atcttccgga gtgagagaag caacagcggg gaagttatcg tgaccttaga 360
gccctgtgag cgctcagaaa cctacgtaaa tggcaagagg gtgtcccagc ctgttcagct 420
gcgctcagga aaccgtatca tcatgggtaa aaaccatgtt ttccgcttta accacccgga 480
acaagcacga gctgagcgag agaagactcc ttctgctgag accccctctg agcctgtgga 540
ctggacattt gcccagaggg agcttctgga aaaacaagga attgatatga aacaagagat 600
ggagaaaagg ctacaggaaa tggagatctt atacaaaaag gagaaggaag aagcagatct 660
tcttttggag cagcagagac tggactatga gagtaaattg caggccttgc agaagcaggt 720
tgaaacccga tctctggctg cagaaacaac tgaagaggag gaagaagagg aagaagttcc 780
ttggacacag catgaatttg agttggccca atgggccttc cggaaatgga agtctcatca 840
gtttacttca ttacggaact tactctgggg caatgccgtg tacctaaagg aggccaatgc 900
catcagtgtg gaactgaaaa agaaggtgca gtttcagttt gttctgctga ctgacacact 960
gtactcccct ttgcctcctg aattacttcc cactgagatg gaaaaaactc atgaggacag 1020
gcctttccct cgcacagtgg tagcagtaga agtccaggat ttgaagaatg gagcaacaca 1080
ctattggtct ttggagaaac tcaagcagag gctggatttg atgcgagaga tgtatgatag 1140
ggcaggggag atggcctcca gtgcccaaga cgaaagcgaa accactgtga ctggcagcga 1200
tcccttctat gatcggttcc actggttcaa acttgtgggg agctccccca ttttccacgg 1260
ctgtgtgaac gagcgccttg ccgaccgcac accctccccc actttttcca cggccgattc 1320
cgacatcact gagctggctg acgagcagca agatgagatg gaggattttg atgatgaggc 1380
attcgtggat gacgccggct ctgacgcagg gacggaggag ggatcagatc tcttcagtga 1440
cgggcatgac ccgttttacg accgatcccc ttggttcatt ttagtgggaa gggcatttgt 1500
ttacctgagc aatctgctgt atcccgtgcc cctgatccac agggtggcca tcgtcagtga 1560
gaaaggtgaa gtgcggggat ttctgcgtgt ggctgtacag gccatcgcag cggatgaaga 1620
agctcctgat tatggctctg gaattcgaca gtcaggaaca gctaaaatat cttttgataa 1680
tgaatacttt aatcagagtg acttttcgtc tgttgcaatg actcgttctg gtctgtcctt 1740
ggaggagttg aggattgtgg aaggacaggg tcagagttct gaggtcatca ctcctccaga 1800
agaaatcagt cgaattaatg acttggattt gaagtcaagc actttgctgg atggtaagat 1860
ggtaatggaa gggttttctg aagagattgg caaccacctg aaactgggca gtgccttcac 1920
tttccgagta acagtgttgc aggccagtgg aatcctccca gagtatgcag atatcttctg 1980
tcagttcaac tttttgcatc gccatgatga agcattctcc acggagcccc tcaaaaacaa 2040
tggcagagga agtcccctgg ccttttatca tgtgcagaat attgcagtgg agatcactga 2100
atcatttgtg gattacatca aaaccaagcc tattgtattt gaagtctttg ggcattatca 2160
gcagcaccca cttcatctgc aaggacagga gcttaacagt ccgcctcagc cgtgccgccg 2220
attcttccct ccacccatgc cactgtccaa gccagttcca gccaccaagt taaacacgat 2280
gagcaaaacc agccttggcc agagcatgag caagtatgac ctcctggttt ggtttgagat 2340
cagtgaactg gagcctacag gagagtatat cccagctgtg gttgaccaca cagcaggctt 2400
gccttgccag ggacattttt tgcttcatca gggcatccag cgaaggatca cagtgaccat 2460

tatccatgag aaggggagcg agctccattg gaaagatgtt cgtgaactgg tggtaggtcg 2520
tattcggaat aagcctgagg tggatgaagc tgcagttgat gccatcctct ccctaaatat 2580
tatttctgcc aagtacctga agtcttccca caactctagc aggaccttct accgctttga 2640
ggctgtgtgg gatagctctc tgcataactc ccttcttctg aaccgagtga caccctatgg 2700
agaaaagatc tacatgacct tgtcggccta cctagagctg gatcattgca tccagccggc 2760
tgtcatcacc aaggatgtgt gcatggtctt ctactcccga gatgccaaga tctcaccacc 2820
acgctctctg cgtagcctct ttggcagcgg ctactcaaag tcaccagatt cgaatcgagt 2880
cactggcatt tacgaactca gcttatgcaa aatgtcagac acaggtagtc caggtatgca 2940
gagaaggaga agaaaaatct tagatacgtc agtggcatat gtgcggggag aagagaactt 3000
agcaggctgg cggccccgtg gagacagcct catccttgag caccagtggg agctggagaa 3060
gctggagctc ctacatgagg tggaaaaaac ccgccacttt ttgctgctgc gtgagagact 3120
tggtgacagc atccccaaat ccctgagcga ctcgttatcc cccagcctca gcagtgggac 3180
cctcagcacc tccaccagta tctcctctca gatctcaacc actacctttg aaagcgccat 3240
cacacctagc gagagcagtg gctatgattc aggagacatc gaaagcctgg tggaccgaga 3300
gaaagagctg gctaccaagt gcctgcaact tctcacccac actttcaaca gagaattcag 3360
ccaggtgcac ggcagcgtca gtgactgtaa gttgtctgat atctctccaa ttggacggga 3420
tccctctgag tccagtttca gcagtgccac cctcactccc tcctccacct gtccctctct 3480
ggtagactct aggagcaact ctctggatca gaagacccca gaagccaatt cccgggcctc 3540
tagtccctgc ccagaatttg aacagtttca gattgtccca gctgtggaaa caccatattt 3600
ggcccgagca ggaaaaaacg aatttctcaa tcttgttcca gatattgaag aaattagacc 3660
aagctcagtg gtctctaaga aaggatacct tcatttcaag gagcctcttt acagtaactg 3720
ggctaaacat tttgttgtcg tccgtcggcc ttatgtcttc atctataaca gtgacaaaga 3780
ccctgtggag cgtggaatca ttaacctgtc cacagcacag gtggagtaca gtgaggacca 3840
gcaggccatg gtgaagacac caaacacctt tgctgtctgc acaaagcacc gtggggtcct 3900
tttgcaggcc ctcaatgaca aagacatgaa cgactggttg tatgccttca acccacttct 3960
agctggcaca atacggtcaa agctttcccg cagatgcccg agccagtcga aatactaagt 4020
gactctgccg agtgccctca ctcgccttcg agagataaag aaagcgttac ctctcatttc 4080
tctttgtgat tcttgacggt gactcttgta tgtaatcctg tggcttaact acttctccct 4140
ccttgtccag cacttttcta gctctcccgt tccccatctc cattgctctg tactcttttc 4200
ttttttcttg tgctgagaat ctcgttagta gcatgtggcc taacaaaagg aaaaaatgtt 4260
tttaaacaca cacacacaca cacacacaca cacacacata cacagacaaa aacacaaaaa 4320
ctctgagggg atctggtgaa tctccaaatt attgtgggtg tactttggct tccttttgta 4380
tgataggtcc ccatcatgac cacctctgat gtctgtgctg ctgtcaccag gcacctttgt 4440
ttttcaagac aacatacttt ttttttcttt tctctgtttg tgatatcact ttaatttttc 4500
ttgggtggct tagagactaa gggaggagac atctggcctt tttagaacct gagaggaaaa 4560
aaagagtctt tttttcccct ctgtctcttt ttgccatggc taatccctgc atttccattc 4620
agggaaaagg tggtagtgag catagaactg caacagttat attctgagtc aaagttgggg 4680
ctttttacgg cataattatg gaatttttat ttactggtag agaggagacg agaggctttt 4740
tcagtgggcc tgggacagtg gctgctcttg actttgtgtg aagggaaatg ccaaggatgc 4800
ttctggtgga cttcagggga ccccagggtt tggccgtggg ccgtgatggc agcaggcggt 4860
gggatgcttg tagctcctca cagcaggatt cctgcccact gttttttctc tgttgggagg 4920
gaagctcttt tctaggagtg tctcagttct gcttttggca ttagtgatgg tggtggtaca 4980

gttggaatta gtgccatgtc atacacaaat gttccacaag gcgggagtgt ttcactttct 5040
ggtgataaac ttgatggtca ttgttatgat taagataatg ccgggcaggc cgggcacagt 5100
ggctcacgcc tgtaatccaa gcacttgggg aggccgaggc gggcagatca cgagatcagg 5160
agttcaagac cagcctggcc aatgtgatga aaccccgtct ctactaaaaa tacaaaatta 5220
gtcgggtatg gtggcacatg cctgtaattc cagctgcttg ggagcctgag gcaggagaac 5280
tgcttgaacc caggaggcag aggttgcagt gagccaagat cgcgctattg cactccagcc 5340
tgggtgacag agcaagactc tgcctcag 5368

<210> 49
<211> 422
<212> DNA
<213> Homo sapience

<220>
<223> EST

<400> 49
aaacagctaa ataatgtggt tatttattaa agatacatta ataattcttg taattgatgc 60
aggttttctt ttttctacag actactatta ttttattctt gtctctctgg aaaatgaaaa 120
atccattagt ttttctcttt tgcttttacc cagaaattaa aggttggtaa gtggtaatta 180
gcgtagtcct gtcaaagcca gttgcctcat gaggaccgac atttgactta agctgaaata 240
atggaatatg gctaatgtaa agttcattag tcccagctgc tttctttgac attcttttct 300
tctaccacaa gaacgggcat gccctgccct ctctcactcc tatttttaga cgtattgctg 360
cgctcctatg tgtctattct acaggctgga ggcacagtgg ttatagggaa ggtaatggta 420
at 422

<210> 50
<211> 320
<212> DNA
<213> Homo sapience

<220>
<223> EST

<400> 50
gtaagtgggc ccacgtcaca cactggcccc agtgatcgta gcgttgtagg aagcgctgcc 60
atatttcatt gaatttagcc attctggaat gacgtgttca tgaagtcggc tcttgcatta 120
ccgtggcctt tattattgct aatgcattct gacattacag gtgtagcctt ggctctccca 180
gaaagggcaa ggctgctctt accaaacgga agagcagaat tcatgatact cttacacagt 240
gaggccagtg ttcaccttta gttactaagc agagtaaggg aaataaatgc ttttatagaa 300
aagatagcca gtgacagcag 320

<210> 51
<211> 446
<212> DNA
<213> Homo sapience

<220>
<223> EST

<400> 51
gttttaactg ttcaatttta ttattagtta acactgttaa tctcttagtg tacctaactt 60
ataaattaaa ctttaccaaa ggtatgcaag cataagaaaa acatagtaca tacagagttc 120
ggtactagcc gaggtttcag gcatccactg gaggtcttgg aacgtctcct ccgtggttaa 180
ggcggggact aacgcaccca taatttgcgc catattgcac aaaatgcttc cctccatggc 240
agctagagtt ctggatggga attaggtacc accaattagg tgcactcatg gacaatttgg 300
aaagtggaaa tgaggcagag gactgctgtt cttgctgctt ttgattgctg caaatatcag 360
cagatgggca ggcaggatat tggaaacact gaggtttcct attgtaatgt ccagtctcca 420
gcttcatggg gggtcaagag ccaatt 446

<210> 52
<211> 443
<212> DNA
<213> Homo sapience

<220>
<223> EST

<400> 52
ttgaagattt tacattgata agtcacattt acggactcac ctctcaaaaa aatgttaggt 60
ttttatattt tacggttttc agtatagccg taatcattca agtttaacca ctttacttca 120
tttgaattaa aaagtcaatg tagtatttta atattaagac atttagtatt ttcagaaagt 180
attccttata aaagacttgc tcaactgaca acttatagtt agaataaatg actctttaac 240
cactttttcc cccatagaca aaatgaagta aaatctcagg gaactaacag aggactttat 300
ttctgatgaa agaggagcag ctatggcttg agactgctaa ccctactcca aacacacaaa 360
agcagctacc tctctcctgg cccaaatgca cacacaggat gacttagcat ttctagaagg 420
aagctttgta agatgactgg tgc 443

<210> 53
<211> 437
<212> DNA
<213> Homo sapience

<220>

<223> EST

<400> 53
gctcgaccag acacaagcat tttgagtaag agagtcagga atatgtctaa catgcaaatg 60
acaagaaacc ccatggccag agtggagaaa gtaaattaag gagtattggc tagatagtgt 120
aaactttaga atgacagtcc gaggcaagag tttggaactt ttgttgccat ggacctcttt 180
ggcagtaacg aagccttaga attatgtttt caaatgtgag aaataaagta tttaggatta 240
caaaggaaac cgataatatt gaaatacagt tgttaaaaca ttaagctctt ctttactgat 300
gcatgaaata acaagattta gcagttattc taattaccat aatttccaag tagtgatcaa 360
tgaaaaagat atcttgcgtc caggcacggt ggctcacgcc tgtaatccca gcactttagg 420
aggctgagat gggcgga 437

<210> 54
<211> 302
<212> DNA
<213> Homo sapience

<220>
<223> EST

<400> 54
cgtccctcaa gttccctaag cttgccaggg gctgaaaccc agatcaaagc tttttggagg 60
aatcagcctt ctgaggactt tcctctgcat ctgctcagtg ggtggcatct ggaaatnaca 120
ggcacttctc gcctctgtcc actgctctct gtgggcatcc cagtctacct ggacagtcct 180
cctgcccaag cagggcaagc ctctnacgga ttcccctaat gggcagcatg gggctnggca 240
gggcaggcca tccagagtga gaccaagcct ncagcaggag agaaggccgg tcgctggttt 300
gt 302

<210> 55
<211> 450
<212> DNA
<213> Homo sapience

<220>
<223> EST

<400> 55
nattcggaac agggccagtc ggtgcagcaa acttcaggga agccatgcgc attggagcag 60
aggtttacca caacctgaag aatgtcatca aggagaaata tgggaaagat gccaccaatg 120
tnggggatga aggcgggttt gctcccaaca tcctggagaa taaagaaggc ctggagctgc 180
tgaagactgc tattgggaaa gctggctaca ctgataaggt ggtcatcggc atggacgtag 240
cggcctccga gttcttcagg tctgggaagt atgacctgga cttcaagtct cccgatgacc 300

ccagcaggta catctcgcct gaccagctgg ctgacctgta caagtccttc atcaaggact 360
tacccagtgg tgtctattcg aagatccctt tgaccagggt gactggggag cttggcagaa 420
tttnacagcc atgcaggaat tccaggtagt 450

<210> 56
<211> 504
<212> DNA
<213> Homo sapience

<220>
<223> EST

<400> 56
aattcggaac gaggctgaag tggggacaaa taagccccgc cccttctagg tactttgaca 60
aaaacaggaa cacaataaaa gagcagacct ctggaggctg aggcaggaga atcacttgaa 120
cccggcatga ggaggttgca gtgagccaag atcatgccac cctactccag cctgggtgac 180
aaaagcaaga ctctgtctca aaaaaaaaaa aaaaaaaaac agatctcaca atgatacagt 240
cagttctcaa gtggcatctg ttgaagcagt ggatgtcgtc acccaatatt agaatatgtc 300
ttaaaccaaa ttaaaacttt acttttacac aggagntttt atgggncaca ttgaaagggn 360
ttcctncctg gtgcccaggg gagcccaaga acttattaga aaggccttca ttggtccatc 420
catttctgtt cctgtttggg gtcacttggt ggctttgtct ttgacttgtt agttgtttct 480
ctatcttctg tgtctttggt agaa 504

<210> 57
<211> 281
<212> DNA
<213> Homo sapience

<220>
<223> EST

<400> 57
aaaaacaatg tcattttgta cagacaaaat ttagcagact caagcttcca cagttagtgc 60
gagagcctgt ggatggcata gttacataag agtctgttgt ggattcaaat gtcacctctt 120
ccaggcagtc tttcctgact taccctggca gctttgtgtt cccacattac tagtaatgtt 180
tttttcctta atagcaatga tttcactgta ataatgtatt tatatgcctc tctcccccag 240
acagcaagcg ttttcccaca ggtctcgaca cacagaagat a 281

<210> 58
<211> 434
<212> DNA
<213> Homo sapience

<220>
<223> EST

<400> 58
aagaaagctt atattaagca cacattacat taaagtactg tttgcacaca cagtaaagat 60
gagattgcct ttctgcctat ggcaatggct agtccaagtg tgggagcatt gaaggccatt 120
attatagatt tcccttttta gataattctg taattctatg gcagggacat cagctccatg 180
actacacaat cacacattca ctagatagca ccttgttatg ttaccagaat atagccctta 240
gctccaaatc aatgctatat aaacattcta ttctgaagct ttttaaacag aattttcata 300
attactctgc atttttgaga atccttatac tttgacctag cagactgaag gagaacaggg 360
atgagagagg ggaatatgga aaggatacgt tagcagaaag ttcaatgaag ttacttcaaa 420
aagtggaaca aact 434

<210> 59
<211> 496
<212> DNA
<213> Homo sapience

<220>
<223> EST

<400> 59
ggaagatcca agagcaagct tccaagagga caagacagaa gctgtgtatt gcccttttac 60
agcctaggct tggaagtcac atagtgtcac ttcagcagca gtcacaagcc tgcctggatt 120
gaaggggaag aaatgcagaa cccgcctcat gctgggaggg aggtcaacgt cactctgtga 180
gaagatcttc tgggatggga gacgtcgttc tgaccatctt gcaaataaca tttgccacgt 240
tcctcctctg gcaacaattt acattcttca tatctgcaaa tgatgcctta tttccttccc 300
gtgagattcc caaaccgcat cccatcaggg catccgcttt caagtccagc atctcattca 360
ttgaaaattc aagtccagct ggcagatgga ggcttcttca ggatggggat tctttggaac 420
gtagttcctt tggagtgtta atncttaacg ggatttttaa agatctgtta attgaaaggt 480
accaagttta cctacg 496

<210> 60
<211> 51474
<212> DNA
<213> Homo sapiens

<400> 60
   aagtgaattc tggtgattca tagcaaaaaa tataatactg ttgttctcaa taattctact 60
   tttggggtta tattcccaaa gttggaggaa aaaggaataa ttttgtttac ataaatcctc 120
   ccagtagtaa cattatttat aatagaaaaa aattagaaaa aagaaatatg cagggaaacg 180
   gctctggaat cagtagttta ttaacataat ttaataccat ttcgagattt aaatatttct 240
   gtattgtgtt gaaaaatgga gattttatac aatacatcag taaggaacat caccaaagat 300
   tgttgatggg ctgggaatgg tggctcacat ccataatccc agcacttttg gaagctgagg 360
   tgggaggaga gcttgagccc aggagttcaa gaccagcctg ggcaaaataa ggaaacccca 420
   tgtctacaaa gaaaaaaatt agcccagcat ggtggcacat gcctgtgttc ccagccaccc 480
   aggagactga ggcaggagga ttgcctgagc ccaggaaatt gaggctgcag tgatctgtga 540
   tcttaccact gcactgcact gcagcctagg tgacactagg tgacagagta agaccctctt 600
   aaaaaaaaaa aaaaaaaaag attgtgatgg acatctgtat gcacatttaa cgaggtagga 660
   cagaatacgg tggtccttga atggagctaa caggaattct cttcttcttg tagtattgtt 720
   taagtttata attgcaaaag tatccttaaa cttctggcgg ggcatggtgg ctcacgcctg 780
   taatcccagc actttgggag gccaaggagg gtagattgcc tgaggtcagg agttcgagac 840
   cagtctgagc aacatggtga aacctcgtct ctcctaaaaa tgcaaaagta gccaggtgtg 900
   gtagcacacg cctgtaatcc cagcttacta gggaggctga ggcaggagaa tcacttgaac 960
   ctgggaggcg gaatttgcag taagccaaga tcgtgcaact gcacttctgc ctgggtgacg 1020
   agagcaaaag tccatctcaa ataaataaat aaataaataa acaaataaaa agtatcttta 1080
   aacttcataa aaattcaaat gtaattttct cttctgaatt cagctttttc tctgattgag 1140
   acatgtaata gattttattt agggaatgat gttatttttc ctagatggcg atcagcctcc 1200
   attttctgag ccgaaattcc ctacggagtg cttctttctc accctgcatg ctcaccacct 1260
   ctctattctg cctagttgcc gtcgctatat ccgcagactc cgggctatcc gggagctcaa 1320
   taggtatgtg ccatgatacc gtgtcctggg attgcctgag ttaccacttt ttctcagggg 1380
   caggcaattc cattgtgaac agtagttttg aatgttgggg gaggtatgta cgttatatag 1440
   tgttctgtct gcctccaccg ccacatccat gttgctcagt tgtccagagt tctttacctg 1500
   gtgaagtgat ccaaaccttg atttctgaga gttctgaacc ctggatattt cactctgttt 1560
   atggttacta cagacttcca ttaacttttc ccactgggca tacattacca ggaggcactt 1620
   caaactttaa aagtttaaag gatcccctgg gtttcagaca taccccttct tccctggact 1680
   gtgaaaaagt aaccatggtt tcccttgata accaggatca tttactcctg cctacatgta 1740
   attctctttt tggcttgttc tgtggcatga ggggtccaaa atggccagat agcagcctca 1800
   aatttccaat tcattagcac catggttgtg tttcttactc taagcatttt ttcctgtgta 1860
   ctaacaccaa caaacagaag tcacaggaac acagaagcaa atatttttga gcaggttatc 1920
   tggaataata atgatgccac ttctgctttt tggtctagaa cctgcgtagt ctgtctatga 1980
   gagaaactga atcatattta tatctctgta tctaccttgc ttaaatcact tactaattct 2040
   tttttttttt tttttttttt tttttttttt gagacggagt ctcgctctgt tgcccaggct 2100
   ggagtgcagt ggtacgatct cagctcggtg caacctctgc ctcccaggtt caagcgattc 2160
   tcctgcttca gcctcccgag tagctgggat tacagatgtg caccaccacg cctggccaat 2220
   tttttttttt tttttgatgg agtctcactc tgtagtccag gctggagtcc aatggcatga 2280
   tctcggctca ctgtaatctc tgccgcctgg gttcaagcaa ttctcctgtc tcagtctcct 2340
   gagtagctgg gactacaggc gcccaccacc acacccagct aattttttgt atttttagta 2400
   gagacggggt ttcaccgtgt tagccaggat agtcttgatc tcctgacctc gtgatccgcc 2460
   cgcctcagcc tcccaaagtg ctggaattac aggcgtgagc ccatgcacct ggcccaattt 2520
   tttgtatttt ttgtagagac ggggtttctc catgttggtc aggctggtct caaattcctg 2580
   acctcaggcg atccgactgc ctccgcctcc caaagtgctg agattacagg tgtgagccac 2640
   cgtgcctggc ctaaaatcac ttactaattc taatagttaa ctctgcaaat tctttatgat 2700
   ttactttcta cacataatca tgtctataaa tgatgacaat tttatttctt ttactttttt 2760
   tttcatgcct tagtgcccag gctgtaattt ttagtacaat gttgaataga aataaccttg 2820
   aaatttccat cttgtttcag ttggtggagg aaagctttct atatatcctc agttaccaga 2880
   ttaagaaaat ttccttttat ttcaatagtt agttccctta gcactggttg aattttatta 2940
   aatacttttg ccatatctgt tgaggagact agactttttt cttctctatt ctgttaatgt 3000
   ggttgaatta tatcagttgg ttttctgtta ttaaaccagt ctttctttcc tggagtaaac 3060
   ttaactttta tattaatgtt tttatatcta attggatata caatttgcta atattctgtt 3120
   taggattttt gcatctgttc aaaagaaagc ctgacctgta attttcattt cttgttaagt 3180
   gcatctcaaa ttttggtatc aaggttatgc tgccttatag aagaaactgg gggctgggca 3240
   cagtggctta cgcctgtaat cccaacactt tgggaggccg aggcagacgg atcacctgag 3300
   gtcaggagtt ggatgctagc ctggccaaca tagtgaaaac ccgtctctac tacaaaaatt 3360
   agctgggcat ggttggcgca tgcctgtaat cccagctact tgggaggcta tagcaggaga 3420
   gtcgcttgaa cccgggaggc ggaggttgta gtgagccaag accgagccac tgtactccag 3480
   cctgagcaag agtgagactc tgtctcaaca aaagaaaaaa aaaaagaaag aactggagaa 3540
   tattctcctg ttttttatgt tccctggaag agtttattgt ataagaatta tgttgtttct 3600
   tccttaaatg tcagaaaaaa ttcagtgatg aagttgtcag ggcttaagag ttttctgtgt 3660
   ggggatgttt ttaattatgg atttaagaca gttaatatat ataagactat ttagattttc 3720
   attacatctt gtgtcagttt ttctaaagtt tgaaaatgtt ttggcatgaa gttgttcata 3780
   atatcctttt atcattttaa tgtctgtagg atccttatta tttctgcttt tatttattta 3840
   ttttagtttt tgagaggagg ttcactcttg ttgcccaggc ttgttgccca ggcaggagtg 3900
   caatggtgca atcttggctc acctcaacct ctgcctccca ggttcaagtg attctcctgc 3960
   ctcagcctcc tgagtagctg ggattacagg catgtgccac cacgcccagc taattttgta 4020
   tttttagtag agacagggtt tctccatgtt ggtcaggctg gtctcgaact cctgacctca 4080
   ggtgatttgc ctgcctaggc cttgctggga ttacaggtgt gagccactgc acccggccca 4140
   ttttaattgg ctttctactg cctcttgaga tggatattta gatcatcctt tccaaccctt 4200
   tcgcttatgt gtatatttaa gactattttg tttccctctt agtgtgacct tagctgcatc 4260
   cctcattttt gttatatcat atcttattac tcagttcaaa atgttttcta atttttgttt 4320
   gttcttatgt aacctgtggg ttatttcaaa atatattatt taaattctag gttcttaggg 4380
   tatttgtcta tgtatctttt gttaattgat ttctagataa attccatcat ggtaagaaaa 4440
   tatattttat gtgatttgtg tcttttgaag tttattgaaa tttactttat agccttttac 4500
   attagtagac ctgaaaagac tgctatctgt gttgttttta cagtgttcca catatattat 4560
   ttaggtcaag tttgttaatt ttctgttcaa gttttcctta tatctaatga ttttatcagc 4620
   ttataatatc aatcactgag agaagcttat aaaaaattca aactatgatt gtggatttat 4680
   ctgtttcttt atcctccgat ggattacctc ctgggataca gtcactcccg ctggctttag 4740
   agggtccatt tcaatggaac catctagaac ctttgctcct ggcttgcagc agatagcctt 4800
   tattgtgctt tccagagaca tttatgttca cctcatctgt gtatttttca atgctgtgat 4860
   ttaagaagta tcttctagga catagttggg gtgtgtatat gcgtattgga aagaggatta 4920
   tctgagcaaa tcacataaag taaatccacc ttaattttcc aagctcttta agttttgaat 4980
   catttcctct gaattagatc aaagaatatt tggcaactca gcttcattca gtataggtca 5040
   ctgtcaattc aaagtttggg tcaactagtt ggttgcataa tatgaacctc tcccaactca 5100
   gaataatact gaattcaggg tctctggtag gtataatcat gtcagtgatt ttagccctac 5160
   atagaagtat atcagcattt tcttctcttg ctgatcaagc atcctcagaa tccattccca 5220
   tacataatcc tacataaccg tcatgtttct gcagtacata tgtggttggt agagtaatgg 5280
   catctcaaag gtgcccacat cctggtctcg gaacctgtga atatgttatc taacttgatt 5340
   aaagggactt tgcagctgtg atatcaggtt catgatcttg aggtggggag attatcctgg 5400
   attatgtggg taggtccagt gtcattgcaa aggtcttcat aagagggaat caggagggtc 5460
   acagggagag acggaatgtg atggggaagc agaggttgga gtggtttggg gtcatgagcc 5520
   aatccggtaa cctctggaag ctggaaaagg cgaggaaact tgttctcccc tagagcctcc 5580
   agaaggaacc atccctgctg acccctttta gacttccaga actctaagac tataattgtg 5640
   tggtttggag ccactgaggt tgtggtaact tgttacagca gcaatagaaa actagcataa 5700
   cacattagca aatcttgcca tttttttctt tcaaatatgt gcttctgttg catccaattt 5760
   tgtaataatg ccataaggac agtccttgat ctgactctag ctgtggttct ggaagcgaag 5820
   agggatgatg ggttagaacc taggggaaaa tagcattacc ctgcaaggtg actatctctg 5880
   atgaggtcat tatagaaact gcttctgcta gcaagcaagg ctcaggaaat ttagagattt 5940
   aggtacttgg agtcagctag ttctcccaaa ctcctccatt tgaactcttt gggtcccagt 6000
   ctttgcctta actttcagat aagaggtcca gcaaagttat taatttcttc tgtactgtaa 6060
   tttgtagaac tggagctgct cctttccttt aacaacagtc ttagaaactc ctgattaatt 6120
   cacatttcct ggagataaga atttaaaatc tgaagcctgt catttttttt tttctataag 6180
   ctccaccaca taattagagg tagccactcc acatcctcat tccagatcac cagggatcat 6240
   gtatatagct gtttttctac tcagagcctt gggctaccag tgttccatcc tcaaatacta 6300
   attgtatctt caccatgcag cccaatcaaa gaactgatcc cagagcccca tttttttcat 6360
   gaaggcctgt tacctataaa ctcttgtgat acccgtagcc atataggtca ttatcttagt 6420
   ttcctatgac tgcaacaaca aattaccaca actttagtca cttaaaacaa cacatattac 6480
   agttctgaag gtcaggagtc ctaaaatcgg cccagcgcag tggctcacgc ctgtaatccc 6540
   aacactggga ggccgaggtg ggcagatcac atgaggtcag gagttcgaga ccagcctggc 6600
   caacatggtg aaacctcgtc actactaaaa atacaaaaat tagccggtca tggtagtgca 6660
   tgcatgtagt cccagctact caagaggttg aggcaggaga atcgcttgaa cacgggagac 6720
   ggaggttgca gtgagctgag attgcgccac tgcactccag cctgggagac agagcgagac 6780
   tgtctcaaaa aaagaagatg aaggttgcat tccctgcaga ggctctaggg gagaatctcc 6840
   tttgcctttt ccatcttctg aaagctgcct acgttcctct gctccttatc accccccacc 6900
   ccaccccacc ttactgtatt ttcaaagcca gcaacatagt atctttagat ctccttcgct 6960
   tgcctggtgt atcgcagcac tttgggaggc tgaggtgggt ggatcacttg aagtcaggag 7020
   tttgagaccc gcctggccaa catggtgaaa cccttttcta ccaaaaaata caaaaattag 7080
   ccaggcatgg taacgcaccc ctgtagtcgc cactactcag gaggctgagg tacgagaatt 7140
   atttgaaccc aggaggtaga ggttgcaatg agctgagatc gtgccactgc actccagggc 7200
   aacagagtga gagaccctgt ctcaaaaaag caaaaaacaa ggctgggcac gtgtctgaca 7260
   cctgtaatcc catcactttg ggaggccaag gcaggtggat catttgaggt cagaagttcg 7320
   agatcagcct ggccaacatg ttgaaacccc atctctacta aaaatataag ttagccaggc 7380
   ctggtggcag gtgtctataa ttccagctac ttgggaggct gtggcaggag aatcgcttca 7440
   acccgggagt cagaggttgc agtgagccag atgatgccag tttactccag cctgggcaat 7500
   agagtgaggc tcagtctaaa aaaaaaagac aacaacaaga tctccttctc tctctgacct 7560
   ttgcttccat tgtcccattg tcacatctcc ttctctgtct ctgagtcctt ttttctctta 7620
   gaaggacctt tgtgattata ttgggccctc caaaatcatc caagatactc ttcccatctc 7680
   aaatccatac ccttaatcac atctacaacg tccctttcgc catgggagat cacatgttca 7740
   caggttccct cgaaggattc ggatgcgaca agtttggagg agccattctt ttgtctacca 7800
   tactcatgtt tcagttgtag acgaaagaat ccaattcttc tgggataagc agaaagggga 7860
   actagttggt ttactgaatc attggaatgg ctataataga agattcccag agctatactt 7920
   tgaacaagga agctgcttcg gaggtcagcc tgacagcaca gcctctgcca cagcttcagc 7980
   caaaacccct ctaccactgt acttccagaa ggatcatgca atcacgcagt cacttcacag 8040
   tactcactct gccttcagtg agggttcatc ttggtgaaaa ctaggtctta cagaaatgct 8100
   ggcaggaggg gagtctagga aatgtagctt ttggagattt agcagcctct agtattacac 8160
   acaacatcat gatagaaagg ggttgaaata gtgttaagag agtcggtctt caaaatatgc 8220
   agcctattag gtgatgggga agcctgttac tgcagagaca gatggtgttt gagcagacac 8280
   ctgaggggag tgaaggagca aagccagaga taacctcaga gaagagcatt gcgagcagag 8340
   ggaacagttg taaaggacga tgccgttagt aaaatagcct tttgttttgg gtaattgaat 8400
   aggcaaagta acctcgaatt ttcatgagag acttcttttt ttccctccca gaaaaataag 8460
   ggaacaatgt actttcttgc aacaaaaaag atgattatat tcaccaaatc tgtagtgcca 8520
   tccattcaaa gacacatcat tgttttagga accaataaaa aagaaaaata tggcccgacg 8580
   cagtggctca tgcctgtaat cccagcactt tgggaggctg gggcaggcag accacaaggt 8640
   caggagtttg agaccagcct gcccaacata gtgaaacctc atctctacta aaaatagaaa 8700
   aaattagctg ggcatggtgg tgagcacctg taatcccagc tacttgggag tttgaggcag 8760
   gagaatcact tgaacccagg aggcggaggt tgcagtgagc tgagattgca gcactgcact 8820
   ccagcctggt gacagagcga gactctgtct taaaaaaaaa aaaaaaagaa agaaagaaag 8880
   aaaaatgctg ccaattaaac tatgatttgc cgctggttgt aagatatact ctgattttaa 8940
   agatgttaca atgcgaaaac aaacaactgc gtgtgagagt cagtgaaatg agcaagatga 9000
   atgggtaaga tggatccggc tgttttcttt catttgactg ttagtctttg atttcagaac 9060
   tgtagaagat ttgaaaaata atgaaagcca atggaaagat tccccactgg caactagaca 9120
   ccgcgaaatg ctgaagcgct gtaaaactca gcttaaggtt tgtatagcta agtggaataa 9180
   ttatagacca gcctcatagt gataattttc ccaacagaat atatgccttg agtgagcagt 9240
   ttagtgtcct cactttttct ttgttattct tttcccttcc aataaaatag tatattttaa 9300
   aacttgttga taccttactt atatgtgtat ataatgattt cagtctcagg acagaatatg 9360
   gatagatatg cttctgctgt gattatgaat tcttaatggg ttttatgatt ctcagtttaa 9420
   catcacctcc cttggccggg cgcagtggct cacacctgta atcccagcac tttgggaggc 9480
   ctaggcgggc ggatcacgag gtcaggagat ggagaccatc ctggctaaca cggtgaaacc 9540
   ccgtctctac taaaaataca aaaaaaaatt agccgggcgt gatggcgggc gcctgtagtc 9600
   ccagctactc aggaggctaa ggcaggagaa tggcatgaac ccaggaggca gagcttgcag 9660
   tgagccaaga tggcgcccct gcactccagc ctgggcgaca gagcgagact ccgtctcaaa 9720
   aaaaaaaaga caacaacaac aaaaatcacc tctccttccc tgccttatgc agggccctcc 9780
   tcacctgctg tctcccaccc ctcgccccaa cactcatcca gtatatctag ccatgcattt 9840
   ccatgatatt tgacaaagag tatctgtggt agtggaaaga tctctttcct ttgcccctca 9900
   ttctttcttt ttaattgaaa ttgattcatc acacaaaacc taactataga tgggaatatt 9960
   cttgctggtg agacatagct aaaaacatat gccagctgac attgtacaaa taaagcatag 10020
   atagcaaatt tatgcatttt tcttctattc atgttccagt tataaacctg agtgttagct 10080
   ggtcatgcat tctgttgaac agtaggataa tatgtggaaa gtttacctga catagctttc 10140
   tagtaccagt taagatttca gttttattta ctaatagact agacttcttc aagatgcaca 10200
   attaacaagg ggcccttatc tggggtctgt gcacccccct aagcagtcca cagatgtgtt 10260
   tcaaggggat ctataaactg cttaaattgc caggcatggt ggctcacacc agtaatccca 10320
   gcactttgag aggccgaggc aggcagatca cctgaggtca ggagttcgag accagcctgg 10380
   ccaacatggt gaaaccccgt ctctactaaa aatacaaaaa ttagctgggt gtggtggcac 10440
   acgactgaaa tctctgccat tcgggagact gaggcaggag aattgcttga acctggtagg 10500
   cagaggttgc agtgagccaa gatcgcgcca ctgcacttca gcctgggcaa cagagcaaga 10560
   ctctgtctca aaataaataa ataagaaaca taaaaactgc ttaaatgtat acatcagtgt 10620
   gtgcatgcat gtgtgtgtaa ttctgtcaac aggcctctag gtaggaattc catccttagt 10680
   ggccagcatt ttgcccgagg gcagctcgta agaggcgaca ctggaattca gacactgatt 10740
   gtttggcttt ttcccactaa ccaccctgac acccaaaggg cctccctgtc gccaccctct 10800
   cctcatcagg ttatttactt gtgactgttg gaactcaaca ttttgagctt taactgtgtg 10860
   ctagacctgg tggtactcgg tggtttgctt gccacgcctt tgtttttctc aggaaaaagg 10920
   tcttgagaca caaccagcat cttatagcta gttaggagat gaagctctag aagctctgga 10980
   acccaaattt cccatctcct ggccccagtc cctgcccagt ccccctgtag ggacttagct 11040
   actgactgat ctttgggtgg ggacatccct gcttccacag aaactggtac ggtgcaaggc 11100
   ctgtgctgat gctggcctac ttgacgagag cttcctgaga agatgtctga atttttatgg 11160
   ccttctcatt cagctgctgc tccgcatcct ggaccccgca tatcccgagt gagtgtgctt 11220
   cttcctgttt cccttgtccc tccttggctg ggctctgttg tctttattgt ccttcaaagt 11280
   tgataactaa aaccaaaacc ctttctttat caagaattca cttctctgca aggcgcagtg 11340
   gttcacgcct gtaatcccag cactttgggg gaggctgagg cagatggata acttgaggtc 11400
   aggagttcaa gaccagcctg gccaacatgg cgaaaccgtg tctctactaa aaatacaaaa 11460
   attagccggg cttggtggca gatgcctgca gtcatagcta ctcaggaggc tgaggcatga 11520
   gaatcgtttg aacccgggag gcggaggttg cattgagccg agatcatgct actgcactcc 11580
   agcctgggcg acagagcgag actctgtccc cacccaaaaa aaaagataat tcacttctca 11640
   gtttccagat tgcatttata tagattatgt agttatattg ataggtgttt tccctgtaat 11700
   tagttacaat aataggcaaa tgtcaacacc tgttatttta attttgacaa gttttcttta 11760
   aaatttgcta taaagtcttt gattgataga aagagaaatc tataaattgg aagccaaaag 11820
   ctggagataa tattaggccc tcctaagcac tgggtccagg aatacccacc taattcagag 11880
   agtgagctaa gaacattgtt gatcactttg tcatcaactg gtcatctcct ccaggcaaac 11940
   catttttgtt ttatttatta ttattattat tttactgtta tgaagtctct ggttcactga 12000
   tgttggtgag attggcccaa gacccgtgaa ggcgtggaaa atattattta aaatatgtgt 12060
   gtaggccggg tgcggtggct cacgcctata atcccagcac tttgggaggc cagggcgggt 12120
   ggatcacatg gtcaggagat cgagaccatc tttgctacac ggtgaaaccc cgtctctact 12180
   aaaaatacaa aaaatagctg gcgctgtggc gggtgcctgt agtcccagct actcgggagg 12240
   ctgaggcagg agaatggcgt gaacctggga ggcggagctt ccggtgagcg gagatcacgt 12300
   ctctgcactc cagcctgggt gacgagcaag actccatctc aaaaaaaaaa aaaaatgtgt 12360
   gtagctctgt tgtttccgtt tgagttgcac aaaatgacca aataggacct ttttatttcc 12420
   ttcccatttt acttattgca ttaaccttag caagaagtca agaaggctgg gcgtggtggc 12480
   tcacgtctgt aatcccagta ctttggaagg ctgaggcagg cggatcactt gaggtcagga 12540
   gttcgagacc atcctggcca acgtggtgaa acaccgtctc tactaaaaat acaaaaatta 12600
   gccaggtgtg gtggtgggcg cctgtaatcc cagctactcg ggaggctgag gcacaagaat 12660
   tgcttgaacc caggaggcag aggttgcagt gagccgagat cacgccactg tactccagcc 12720
   tgggtgacag ggtgagactc tgtctcaaaa aaaaaaaaaa aatcaagaaa agggccaggt 12780
   gtggtggctc acacctgtaa tcccagcact ttgggaggcc aagatgggtg gatcacttga 12840
   ggtcaggagt tcgagaccag cctggccaac atggtgaaac cctgtctcta ttgaaaatac 12900
   aaaaattagc caggtatggt ggcacgtgcc tgtaatctca gctattcggg aggctgagac 12960
   atgataatcg attgaactgg ggaggtggag gttgcagtga gccaagattg caccactgca 13020
   ctccagcctg ggtgacagag tgagactcca tctcagggga gaaaaaaaag aaatcaagaa 13080
   aaggaaaaca tttcacacat acccttgcat atgcactgtc ttcattattc cctgttcatt 13140
   tctggttgta gtttctttgc ctacatattt attacatagt attacagaat gattttcttc 13200
   cccaaatgaa atacttatcc gatggtaatg taatactcct tgtagtttga tgtaccataa 13260
   tttcatttaa taaattgtcc tttttttctc tttgacagta taacactgcc tttaaattca 13320
   gatgtcccca aggtatttgc agcgttgcct gagttttatg tagaagatgt tgcagaattt 13380
   ttatttttta ttgtacagta agtgccttta atattttaca tagttctaat atgtttttat 13440
   gcataatagt ataatattct gagctatgtt gcaaacctcc atttcatgcc tgaaatttga 13500
   tgtaactgtt ttaactgaat gctgcctaga gactggaaaa aataaatcca actgtggcat 13560
   cagtccaatt ccaataatat ctcaaagaca gaggcaggat taacagtgca gataatgtat 13620
   tccttgaaaa ttgagttgaa tttctaatag ttaaatatgt gtttctgagc aacagaagga 13680
   ggaaacagtt tataaatgtc tgaatgaaca tcccaaaacc agaaatatcc acatctatat 13740
   tttgtactaa aatttgtctc aattgtaaaa ttggagaatg cttaccaaga aatggtttga 13800
   atttctaact ggtattgtag ggttttaatt tttttttcaa atccttattt ggtttatcaa 13860
   attaacattt tatttctaaa acattacagg ttagaagaat tacaatttca atcccttggc 13920
   accatgatag cacctatgac tagctgaggg agtggtatgt gaagtctatt gtgaaaagta 13980
   aaagtcatgc tgggcactgt ggctcatgct tgtagtccca gacatgcagg aggattgctt 14040
   gagcctagtt caagaccagt ctgggcaatg cagcaagtct gcatctcttt aaaaaataaa 14100
   taaataaata gtataggtcc ccagtcacag aatacaaatt gtgaaagttt tgtaacaaac 14160
   aacaggaaaa aaacaaaatt agtataaatg attttaaaat aagggccaaa agtaaatgga 14220
   gcaagttaat attgctgtca taaaagctgc ctttaaaaaa aagcccaaat caaggcagag 14280
   gttgcaatga gcagagatca tgccactaca ctccaacctg ggcaacatac acaaaaaaac 14340
   ccaaatcatt ggtgacacgt gattttgatt ggtatgaaat tttaaaagtg actcaaaaac 14400
   gtggtttatt tgttgaaaaa ccaaccttgg aatttaagca actcaagtca catttaaaaa 14460
   tttgttctat gcacagtgat acaaaagaca cctatttgaa aaatttcatt tattctcatt 14520
   ttcagcagag attaaaaaaa aactatcaaa atttttatgt tttggtagtg atgatctttt 14580
   gtgaagttta tctgatttga tcataggttt tgtttatagt taatataaca tgctacagcc 14640
   agtaaccatg tttggtttta aatctgtctt gttacagtgt gaatagtcac tgtaggctgg 14700
   gtgcaatggc tcatgcctgt aatcccagca ctttgggagg ccaaggtggg caaatcacga 14760
   ggtcaaaaga tcaagaccat cctggccaac atggtgaaac cccatctcta ctaaaaatac 14820
   aaaagttagc tgggcatggt ggcaggcacc tgtagtccca gctactcagg aggctgaggc 14880
   aggagaatcg cttgaacttt ggaggcggag gtttcagtga gccgagattg tgccactgca 14940
   ctccagcctg ggcaacacag cgaggctcca tctcaagaaa aaaaaaaaaa aataggctgg 15000
   gcgcagtggc tcatgcctgt aatcccacca ttttgggagg tcgaggcggg cggatcacga 15060
   ggtcaggaga tcgagaccat cctggctaac atggtgaaac cccatctcta ctaaaaatac 15120
   aaaaaaaatt agccgggcat ggtggcaggt gcctgtagtc ccagctactc gggaggctga 15180
   ggcaggagaa tggcgtgaac ctgggaggcg gagcttgcag tgagccgaga tcgcgccact 15240
   gcactccagc ctgggcgaca gagcgagact ctgtctcaaa aaaaaaaaaa agtcactgta 15300
   aactgaatga gttcaagggc actcccaaac ctacttatcc tgccttcccc tcctcactta 15360
   ctgacaccag ccttcaccca agatgagttt ctcagcagtt tctcagcggt ctctttcttg 15420
   ctcttcttgc ctctgttcag atactctccc caggcgcttt atgagccctg tactcaggat 15480
   attgtgatgt tccttgttgt gatgttgtgc aaccagaact acatccgaaa cccatatttg 15540
   gtggccaaac tggtagaagt catgtttatg accaaccctg ctgttcagcc acgaacccag 15600
   aagttttttg aaatgattga gaaccatcct ctctccacca agttgttggt accttccctg 15660
   atgaagtttt atacaggtag gttgctggaa cacagtgtag cacatggcag gccaacttag 15720
   gtaaggtcat ctaaagctag tggacgacgt tgctctaagc ctgttaccta atatcctgct 15780
   acctgtgtgt atagcctact aacctgtggc ctgcagagga tgtacttttg gttcttcttg 15840
   ccctgagtag ttaggaatag gacaaccatt ccacctactg gtctctgatg attcagcagg 15900
   aactactgaa agtcagatga ttggcaccga gacccagggt atcaagagca caagttggta 15960
   gtgttatctg aatggaatta tctggttcat tttacctttg aacttacagc atttgttttt 16020
   taacactatc ctataaccca ctttccaaag tgtgggtgaa acagggagct tgttaaaaat 16080
   gcatattctt ggtccgggtg cggtggctca tgcctgtaat cccagcactt tgggaggccg 16140
   aggcgggcgg atcacaaggt caggagttca agaccagcct ggccagtatg gtgaaacccc 16200
   atctctacta aaaatagaaa aaaaaattaa attagccggg catggttgca ggtgcctgta 16260
   gtcccagggc ggttgaggga ggagaattgc ttgaacccgg gaggcagaag ttgcagtgag 16320
   ccgagatccc gccactgtat tccagcctgg gcaagagaac aagactccgt ctcaaataat 16380
   aataataata ataataataa taataataat aataccactt ttcacctgtc aaatttattt 16440
   cttaagttga aaatacctgg tacaaacatg gttgtggaga agtaagctaa cctgcaaaaa 16500
   aacctgctta atctacccta tagccagtgt gcagatttag tgctatgaaa ctctcatgag 16560
   aatgcaaatt taaagccttt ctgaaaggca gtttagcagc ccacaccaag agccttaaaa 16620
   atgcttttat cggggccagg tgcggtggct catacctgta atcccagcac tttgggaggc 16680
   cgaggcgggt ggatcacctg agatcaggaa tttgtgacca gcctggccaa catggagaaa 16740
   ccccgtctct actaaaaata caaaaattag ctgggtgtgg cgggcggcgc ctgtcatccc 16800
   agctactcag gaggccgagg cacgagaatc gagtgaacct aggaggcaga ggttgcattg 16860
   aactgagatc acaccagtgt gctccagcct aggagaaagg gcaagactcc gtctcaaaaa 16920
   agaaaaaaaa aaagcactgg gataacaaat agtaggtgca ggaagtagcc atcttctatc 16980
   cctcagtctg ggacccaagg ggaagagcct ggggatctca gaacccagct gccctgatga 17040
   gggcttggac ggagctagga cccagtcctc tgtggaaggc ccttggccag taggtgctgg 17100
   tgtctctctc gggggttgtg cacggttgct agctctgggg gtacgagaca aagctgaaaa 17160
   ttggaaccaa ctgctactgc tgacagtaag tgcaaggtcc taagtccctt gtttctcctt 17220
   cagttttcca gttctcctgc agcaccccct attggcaacc ctagagaggg cgggctgcca 17280
   aaggaaagat tctgcagtgc cagccccaga atcacaaagc caactacaaa gagtgcatgt 17340
   gggctgggca cggtgactcc agctaataat cctagcactt taggaggcca aggtgggagg 17400
   attgtttgag cctaagagtt ggagaccagc ctgggcaaca tggcgagacc ccatctctac 17460
   gaaaaattta aaaattagat gcctgttgtc ccagctacta aggaggcaga ggtgggagga 17520
   aggcttgagc ccaggagttc aaggctgcag tgagctatga tcttgctgct gcgttccagc 17580
   ctgtgaaaca aagcaagacc ctgttctaaa aaaaaaaaaa aaaaaaaaaa aaaaagggta 17640
   aaaagactgg gtttcggcca ggcacagtag cttacacttg taatcccagc actttgggag 17700
   gccaaggcag tggatcacct gaggtcagga gttcgagact agcctgacca aaatggtgaa 17760
   tcctagtctc tactaaaagt aaaaggccgg gcgtggtggc tcacgcctgt aatcccagca 17820
   ttttggaaga ctgaggtggg tggatcacaa ggtcaagaga tcgagatgag cctcgccaac 17880
   atggtgaaac cccgtctcta ctaaaaatac aaaaattagg cctggcacag tggctcacgc 17940
   tgtaatccca gcactttggg aggctgaggt gggcgaatca cctgaggtcg ggagttcaaa 18000
   accagcctga ccaacatggt gaaaccctgt ctctactaaa aatacaaaat tagcctggca 18060
   tggtggcaca tgcctgtaat cccagctact tgggaggctg aggcaggaga atcacttgaa 18120
   cccgcgaggc agaggttacg gtgagccaag atcgcgccat tgtaatccag cctgggcaac 18180
   aagagcaaaa ctccgtctca aaaacaaaaa ttagctggac atggtggcat gtgcctgtag 18240
   tcccagctac tcgggaggct gaggcaggag aatctcttga acccgggagg cagaggttgc 18300
   aatgagccaa gatcgcgcta ctgcacttca gcctggtgac agagcaagac ttcatctcaa 18360
   aaacaaaaca aaaaacaaaa caaaacaaaa ttagccgggt gacgcatgcg tgtaatccca 18420
   gcaacttgag aagctgaggc aggagaatag cttgaacccg ggaggtggag gttgtagtta 18480
   gccgagatct aaaaaaattt taaaaaaaaa gagtggattt ggagctacga gacaacagct 18540
   gaatagctat actctgacat aagaatttaa tttcttgaaa tttattcaat ggaaataatt 18600
   acgaatgttt gtaagtgatg ttctccacag cattatttat aaagcagtgc agtgaaaata 18660
   ataaaaacaa ctggccgggc tcagtgactc acgcctgtaa tcccagcact ttgggaggct 18720
   gaggcaggag gatcacctga ggtcaggagt tggaggctta gtctggccaa aatggtgaaa 18780
   ccccgtctct actaaaaata caaaaaaatt tagctgggcg tagtggtgca cacctatagt 18840
   cccagcttct ctggagtcta aggcagatga atcacttgaa tcagggaggc agaggttgca 18900
   gtgagccaag atcgtgccat tgcactccag cctgggctac aagagcaaat ctccatctca 18960
   aaaaaaaaaa aaaggaatgt agggaaggac atataccaaa atagtaacag cgcattggtt 19020
   atttttgaat ggtagcatta taaatagttt ttatttttat tttattatct gaacttctta 19080
   atttttctac agtaaacatg tattgttagt agtaaagaca aaacaatttt agttaaattt 19140
   attttgtagt cagtgttctg ggcctgtcac tgtggcttca ggagagagag agagagagag 19200
   agtgtgtgtg tgtgtgtgtg tgtgtgtttg tgtatttgtg tttacacaac agaactgggc 19260
   ctgttgccta taagacgagt gctgggccta gtggagtcct gctgggaatg ttccagaagg 19320
   aattgggtgg gggttgttct ctctttcctc tttgccatgt gtgccctctg gcctggacca 19380
   cctcttcagt catagatgga tgacatctgt actaataata gccaccactt attgaatgct 19440
   tactaatcct ttcacttaat ccccacggca gtcctaaaga tagtactata aatacatcca 19500
   ctttacaaat gaggaaactg aagcttaaaa gggtcaagaa gcttgctgaa ggtcagtggc 19560
   tagcaagtgg cagcaacctc gcagatgccc tttagcacag gtccccctgg ctactgtgct 19620
   ggtcttccta cagctactaa gggaccggtg tgagggtacc cctgagggag gcatcacaga 19680
   catagtcccc agtgctcatc acctccatgt gaagacctgg ctgattaggt cctggtccct 19740
   gagcagatgg gaccctcaga tataaattaa aagttgccac acacaagatc agtatgtgga 19800
   acaagctcct cttaacacca cagagatagt attctagctt agaaaatgct caagttaggc 19860
   tgggcgtggt ggctcacgcc tgtaatccta gcactttggg agtccgagac aggtgaatca 19920
   cttgaggcca ggagtttgag accagcctgg ccaacatggc gaaaccctgt ctctactaaa 19980
   aatacaaaag attatccggg tgtggtggtg ggggcctgta atgccagcta cttgggaggc 20040
   tgaggctggg gaattgcttg aaccgggacc tgggaggtgg aggtcgcagt gagccaagat 20100
   cttgccactg tactccagcc tgggctacag agtgagactc catctcaaaa aaaaaaaaaa 20160
   agaaagaagg cagcaagaat acctgggggg aggagggaga ggttcaacac aagtctagtc 20220
   cacctacggc cttctagaac aaggacctgg gaattagaac ttgggctggt atgactttga 20280
   gggaacagta gaagtgaata agaagaaaaa aaaactgttg ggagaaatgt atattgtttt 20340
   gtttcatttt cttttctttt tttttttttt ttttgagcta gggtcttgct ctgtcaccca 20400
   cgctagagtg tagtggtaca attacagcac catgcactct tgacctcccg ggctcaagtg 20460
   atcctcctac ctcagcctcc cctgtagctg ggactgcagg catgtgccac cacacccagc 20520
   taatttttgt ggtttttgta gagacggggt ttcacgatgt tcaccaggct ggtctcaaac 20580
   tcctgagctc aagtgatctg cccacccggg cctcctgaag tgctggaaat acagatgcgg 20640
   ggccctgctg agatacatat tgctacataa taaattaaca tttacttagt ctctactata 20700
   ttcagaattg ggtgcagata ttggggcatt ttagatgata tgaggcgttg ggttggtcct 20760
   cctgtatctt cctcttgtag gctatcctta tgtcaacact ccacacttta gctcatgctt 20820
   ttatacaatt aataaatgac atttagttta ttttaaggaa aggttactta atgaatatac 20880
   ataagtactt tcggatctta gtatgatagg tgttaattat cttattttat ttttaaatag 20940
   ctgtttcctt ttttggtcac acacaaaaaa atgtgttctt tggaaaatac agaaaatatc 21000
   aaagacaagg tatagtggct cacacctgta atctcagtac tttggaagga cgatgtagga 21060
   ggatcgcttg aggcaggaat ttgaaaccag cctaggcaac atagtgagac cccatctgta 21120
   aaaaaattaa aagtgaaaaa aaaaattttt gagacagagt cttgctttgt cgcccaggct 21180
   ggagtgcagt ggcatgatct tggctcactg caacctctgt atctggattc aagcaattct 21240
   cctgcttcag cctcctgagt agctggaatt atgggcacac accaccatgc cctgctagtt 21300
   tttgtatttt tattaatagt agaggcgggg ttttgctatg ttggccaggc tggtctcgaa 21360
   ctcctggcct caagtgatct gcctgcctcg gcctcccaaa gtgctgggat tataggcgag 21420
   agccaccacg ctcagccata aaaaataagc cacgcgtggt ggatacacct ccagtcccag 21480
   ctacttggga tgctgaggtg ggaggatcac ttgagcccga gaggttgagg ctgcagtgag 21540
   ccattattca caccactgca ctttaccctg ggtgacaggg caagaccctg tctccaaaaa 21600
   aataataata aaatagttgg gcacggtggc tcacacctgt aatcccagca ctttgggagg 21660
   ccaaggtggg tggatcatga ggccaagaga tcgagacaat cctggccaat atggtgaaac 21720
   cccatcttta ctaaaaatac aaaaattagc agggcgtggt ggcgcatgtc tatagtccca 21780
   gctactaggg aggctgaggc aggagaattg cttgaacccc gaggcagagg ttgcagtgag 21840
   ctgagatcgt gctactgcac tccagcctgg cgacagagcg ggactctgtc tcaaaaataa 21900
   taataataat aataaaattt gtcatgaatt ctgaaatttc tcatgcctaa gcttttgcca 21960
   tttggacaaa caagtttgat ttttatccct ctttaataga tttagggatg atagacacat 22020
   tggtgttttt tcaacaccaa aaatggtcaa tagatagaca catttagtaa atacatggcc 22080
   ctcaataaaa ttaaacctaa ctttttatcc gctgaagtat gtccatttga ggtgccatag 22140
   aatttctgta aattaataac ttaaaattag gtggggggag tatgataatt cagcttttgg 22200
   tcattggtaa gttgatgttg tcctaatgat gggagttttc ctagccccgt tcctttaggt 22260
   gttgtgtcat tgtcaaatct tcattgtgaa acttttgtct ttaaaagcag cttataattt 22320
   ctgtcgtgcc ctacatgcaa aagaagtgca aaatttcacc agctctctga ttttgcaggt 22380
   ttacattcac tcagtatcat tgttggggca ataactggat ttggaaagca agaaaataca 22440
   tatccctcag tacctttgtc tttcttcttt agatgttgag cataccggag ccaccagtga 22500
   gttttatgac aagttcacaa ttcgctatca tattagcacc atttttaaaa gcctttggca 22560
   aaacatagct caccatggca cctttatgga ggagttcaag tgagtatggg gcccctcgtg 22620
   tcacaacttg ctttcttgca aatcgcaggt aggattgcag cttaaaagca tgcacagctg 22680
   ggttcttgtt gactgtttgc ttggtctcag actcctttga gtttaccaca agatgccact 22740
   aaatgtgggc aggatttttt aaatggcttt acttgcaaga tattgctgaa ttttagattt 22800
   gtcaagatac tttgggggtg gtacacaagc gaagattgta tagggcaggg cgtggtggct 22860
   cacgcctgta atcccagcac tttgggaggc agaggcgggc ggatcacttg aggtcaggag 22920
   ttcaaaacca gcctggccaa catggtgaaa ccccatctct attaaaaata caaaacatta 22980
   actgggtgtg gtggcgcacc gtctgttatc ctagctactt gggaagctga ggcaggagaa 23040
   tcacttgaac ccgggaggca gaatttgcag tgagccgaga ttgtgccagt gctctccagc 23100
   ctgggtgaca gagcgagact ccgtctcaaa aaaaaaaaaa aaaaggaaaa gaaaagatcc 23160
   tataaatatg ctgagatgat gggttaagtg tttttttggt gttttctacg atgacagtgt 23220
   taaatgaagt tgtcagaatg agtgaaatga aatcttagtt cctgaatgtt ggaaggactt 23280
   tagattttgc aacaattagg tcagagttgt catttaaagt atcattcttt gtggtaaggc 23340
   cctaaacctt aattaaagac ttttcatata cctacctgaa atctgagtga ctgcttaaac 23400
   ctggacatta gtaattccat ctaaattatc tgtcagtgcc taaaaacaaa agggagatga 23460
   agatgctgaa ctcttgaatt attcttcaaa aattttatcc catctaaggg ccgggcgtgg 23520
   tggctcatgc ctgtaatccc agcactttgg gaggccgagc agggcggatc aggaggtcag 23580
   gagatcgaga ccatcctggc taacatggtg aaaccccgtc tctactaaaa ataccaaaaa 23640
   attagccagg catggtggtg ggcgctgtgg cgggcgcctg tagtcccagc tactcaggag 23700
   gctgaggcag gagaatggcg tgaacctggg aggcggagct tgcagtgagc cgagatggtg 23760
   ccactgcact ccagcctggg cgacagagtg agactccgtc tcaaataaat aaataaataa 23820
   ataaaaattt tatcccacct acttggatct tttgcaaccc atgttagatc tttgatcaaa 23880
   tctagctata cttgttaacc gaattactag tcaataatat acaaattacc agtggaataa 23940
   ctatttgcca gatagatttc tgtgaagcaa actttttaac tttttgtttc cataaactgc 24000
   tcttatggtc agaattgtgt tccagctaaa gcctggcacg aggcaggccc atggcttggt 24060
   ctgaatggtg ctgattggag cgcttgtcct gaacttgcca tgttcctgag atcgaggcct 24120
   cctgcacttg ttcacaggaa cttctcttct ccagcattgg cttaccaccc cgtagttgca 24180
   cagttgtaga ccctgaagtg tagcttcttt ttttctctac gaatctggca tctccagagt 24240
   ctaacagagg taggccaagc tatttcagtt caccagcaag ttctcattat aacacgatct 24300
   gcagaacagt tccttagatt ggaggcttcc agttttaaag taagttaata tcccacttta 24360
   tcaatattac aggttccaat ttttcttcca tgcgtcagtc tttgtgataa ggaataacta 24420
   tatcagttgt tttcttatct gttgtatgtg gttaattatt tgcattttct tggtatattg 24480
   gtgagtatat gcgtttttcc tgttctagtc tggaagaagt gttttctctc cttaaaaaca 24540
   ttgagggagt gagctagggg tgtgctggta agcctgctct gggaagcagg gtgggggagt 24600
   cagcatttgc tggttttcca tggtgtgtct gtttcctctg ttgcccaatt taaggtgcca 24660
   ggggaatctg gttataaaat tctggaatat ttagcacttg actgtcatca gccgtgcaag 24720
   gtggctccaa ataccactgg aatgagctta tcataaacaa tttaaaagaa taacaaaatc 24780
   caggccaggt gtggtggctc atctctgtaa tcccaacatt ttgagggcct gaggtgggag 24840
   gaacgcttga gcccaggagt ttgagaccag ccctggcaac ataacaagtc cctgtctcta 24900
   caaaaaatac aaaagctggg cgtggtggtg tgtgcctgta gttccagcta cttaggaggc 24960
   tgaggcagga agattgcttg agccctggag gtcgaggatg cagtgagctg tgattgtgcc 25020
   actgcattcc aggctgggcg acagagtgag accctgtctc aaaaagaaac aagattaaca 25080
   taatctaaag agaagcttgg tggtccttga attccatagt gcctgacttg tgccagggta 25140
   gccaggcttt tagggtgaga tagttgcagt ctgggtggag gtgcttgttc cctgggattt 25200
   gctgtggcat tttgcttcag ggagatgtat gaccatgtac aatataattg cctttccagc 25260
   tccgggaagc agtttgttcg ctatataaac atgttgataa acgacacgac gtttttgctc 25320
   gatgaaagtc tggagtctct gaagcgaatc catgaagtgc aggaagagat gaagaacaaa 25380
   gaacagtggg accagttgcc ccgggtgagg acgtggtcca gaggcttgga cagctttgca 25440
   ggccatctgc ctccacacac gggcagagct gctttggggc tgcatttgtg ggtctgatga 25500
   tatgcgatct gacatgctgg gattttcctt ccatgaaatc atattgcagg attggaatag 25560
   gaaaattctt aaatacctta tctgaagcta agaaatgggt tgagaagtct ttgaaaatgt 25620
   acactgaaaa ctttctctgc ctttttaatt ttaaagggct tttttggaga tgagctttta 25680
   ttgtctgaat atattaatat tcatgaacat aattaggaaa gaaatctcta gaattatgtc 25740
   tgctggacaa gaatttgatt tgttcctatg ggcttctcta ggcaactgtg tgaatcactc 25800
   aggatcctgg cggggtgttt tttccctttc agttgatctt gggttttata ttcagttatt 25860
   aatagaaaag aggcctggct tgccaggttc catggaaagc acgtgccgtg ccagtaccag 25920
   tgctctttcc tgacgtgcgt agcagccact gagatacttc cttggtcgct tatgaggccc 25980
   ttgacagttg ctggtgactt cttagattgg cttaggttta ttgattctgt tcttggaatt 26040
   gtcaggctca gaatcagaac tgatttccat aaggggttct catgtcaaag tggggactgc 26100
   tttttcttca ctttttcttt tttttttttt tttgagacgg agtttcactc ttgtcgccca 26160
   cgcaggagta cagtggtgcg atctcttctc actgcaatcc ccgcttctcc ggttgaagcg 26220
   attttcgtgc ctcagccttc tgaatagctg ggattacagg cgcctgccac cacacctggc 26280
   tagtttttgt attttgtttg tttgtttttg agacggagtc ttgctctttc acccatgctg 26340
   gagtgcagtg gcacgatctc agctcactgc aatctttgcc tcctgggtac aagtgattct 26400
   cctgcctcag cctcccaagt agttgggact acaggcacct gccactacgc ctggcttatt 26460
   ttttgtattt ttagtagaga tggggtttca ccgtgttagc caggatggtc tcgatctcct 26520
   gaccttgtga tccgccttcc tcggcctccc aaagtgctgg gattacaggc gtgagccatt 26580
   gcacccggac aatttttgta tttttagtag tgacggggtt tcaccatgtt ggccaggcct 26640
   gtcttggact cctgacctca ggtgatccat ccgcctcagc ctcccaaagt gctgggacta 26700
   caggcgtgag tcaccgcgcc cagacttttt tttttttttt ttctttttta atttcaactt 26760
   ttattttaga ttcaggggat ggggtacatg tgcaggtttg ttacatgggt ataccgtgat 26820
   gctgaggctt tatgattcag cctgtcaccc agggagtgag cataatacct aataggtagt 26880
   tttcaaccct cactcctccc tcactcccct cccttgtagt ccccagtgtc tgttattccc 26940
   gtctccgtcc atgtgtacca atgtttagct cccatttata cctgcttttc ttcttatgta 27000
   ttaaagttct acccattctc tgagtaaggt cctatccaaa tactccctca ggtttgtctt 27060
   cgccaatcag catagctgaa aattctcacc tttctcctta cttctgtaac acccacttta 27120
   taccattcac agtgcttggt atgcccagct tatattttat ttacttattt gttttttatt 27180
   attattaata aataaagaca gagtgttgat ctgtcgcctt ggctggagtg cactggcagg 27240
   atcatagcac accacagcct caacctcctg gactcaagtg atcctcctgc ctcagccttt 27300
   gtagatccaa agagctggat ctacaagcat gtaccaccac gcttggctaa ttaaaaaaat 27360
   tttttttata gagacaaagt ctcacatgct tgcttcagca gcacgtatac taacattgga 27420
   acaatgcaga gaagattacc atggcccctg ctcgaggatg acatgcaaat tcataaagca 27480
   ttccatcctt taaaaataaa tattaggcca ggggtggtgg ctcacgcctg taatcccagc 27540
   actttggaag gccgaggcga gtggatcaca atgtcaagag atcaagaaca tcctggccaa 27600
   catggtgaaa ccccgtctct actaaaaata caaaaattag ctgggtgtgg tggcacgccg 27660
   ctgtagtccc agctacttga gaggctgagg caggataatt gcttgaaccc gggaggcgga 27720
   ggttgcagtg agccaagatc gcaccactgc actccagcct gggcgacaga gtgagactcc 27780
   gtctcaaata aataaataaa tattaaagag agagagtctt gctagttttc ccaagctggt 27840
   cttatatttt atttacttac tatttttatt attaaattat tttttatttt attttatttt 27900
   attttttgag acagagtctc actctgtcac ccaggttgga gtgcagtggc gcaatcttgg 27960
   ctcaatttat gcctgctggg attacaggcg tgcaccacca cgtccggcta atttttgtat 28020
   ttttagtaga gatggggttt tgccacattg gccaggctgg tcttgaactc ctggcctcaa 28080
   gcaatccacc cacctcagcc tcccaaagtg cgggggttac agacatgagc caccatgccc 28140
   agccttatta ttatttttta atagaaacag agtcttgacc gggcacagtg gctcatgcct 28200
   gtaatatcag cactttggga ggccgaggcc ggcagatcac ctgaggccag gagtttgagc 28260
   cagcctggcc aacatggtga aacccagtgt ctactaaaaa tacaaaattt agccaggcat 28320
   ggtgacaggt gcctgtaatc ccagctattt ggaaggctga ggtgggagaa tcacttgaac 28380
   ccaggaggca gaggttgcag tgagctgata ccatgccact acatttcaca tttcagcctg 28440
   ggcaacagag cgagactccg tctcaaaaaa gaaaaaaaaa aagaaagaaa gagtcttgct 28500
   ctgtcaccca ggctggagtg caatgacaga tcttagctca ctacagccta caacctcctg 28560
   gcctcaagca gtcctcccac ctcagcctcc caaaatgctg ggattatagt tgtgagccac 28620
   catggctggc cagcgtatat tttagatgtt ggaatgcagg tctatagcac attctcgtat 28680
   tcatagtcag tacttaatag catttgttta tgagccaact gaacaaataa tattgatttt 28740
   tcctgtgtta atggatgaaa aaggcccaaa taattcccac aattgctatt tcagaaataa 28800
   tttttatttg gctttgaagt cactttctac ctcccactgc cctggctgtt cctgctcatc 28860
   tccctgacgt ctgagcttgg tgagctccag cactcagccc ttaggcttca atttctttcc 28920
   cactcatgct ctctaggtta gtgtttctca acgagggcac tagttggaat tttgagccta 28980
   gtggaatttg gtataataat aactcatgat gagaaaccat cccatatatt gcaggacatt 29040
   taggatctct gtttcctgcc atttaatgtc agggatgtac cctcctaccc ccagccttca 29100
   ggcaacgaga aacactgcca cacataacct gatgtccccc ggtgaacatc actgcttggt 29160
   gagctcaggt ataccaaggc attaaatacc tgtgttctaa agactccaga atatatctcc 29220
   ctggctcaga tctgactgca gacttgcaga ccaagtattt gtctaatcag tgtctcaaac 29280
   tcactgtgtc caaaacggaa cccttgatgt tgtccacatc tcctgcctga gtctgcccaa 29340
   tctcagtaaa tggcgccttc attctaccag ctgctcaggc tcgatgcttt ggagttgctc 29400
   ttgatttctc tgcgtctctc agcaaatgcc tctaagctct gtcttaaaat atatccatta 29460
   tctgacctct tctcagcacc tccagaccgt tggcatctct tacctgggtt attgcagtag 29520
   cccactgctt ggtcttcaac ctccactctt gagcatgtcc atatcaggga tggtgttaaa 29580
   catctcagtc tgatcaacat caccactcag ctcagccatt ttgggcttcc cttatcactc 29640
   agagccaaat cctgtggtga gagcagccta cagcacgcag gaggacctgg ccccacaatg 29700
   tgtttctgct ctcagctttg gacagtctct tctccactca ctctacccca gccagactca 29760
   cctcctggcc cttccactga cacgtgaggg cctttacacc tgctcttccc cctacctgga 29820
   atgctcttcc ctcagatttt ccgactcctt cagatatacc ctccctcagg gataccttcc 29880
   ctgaccaccc tttccagaat agcattccct gactcctgac cctgcttcat ttttcctcat 29940
   cactgtcaga cttactgtgt gtctgtctgg gtatttgttt ctatgaagca gatcctccct 30000
   aacggaatct aagctgcatg agagctggga gcctttttat tctatctcca gtgcccaaag 30060
   gctgtagaag ggcctgttga gacacccagg tacacattta ggtagttcac gcctggctgt 30120
   tcttgtgtct ttcctggctc tgcctttctt tcagtaggct cattcttgac cagggaacgg 30180
   caggcccccc agtatccctg cagaatctcc atcaagcaca gactggagca ctttcccaga 30240
   gggcactcac cggccagctt cagctctgcc agctgtgttc ctttcccaac aagaaaaggc 30300
   ctctgaacag atgtaagaat tgaggctttt cctgatgggt ctgtgtttga cccatttcag 30360
   agcctgcagt gcagtacctg attgtatcca tgagtcaaac agctcttatt atctcctccc 30420
   cagcccatgg cagagttgaa ggaggaaagt gcgccagagg taagcgtgct gacagttctg 30480
   tggtgtgaca gcctcacagt ttggaaagac aaaattttaa aagcttagta gtccacaggc 30540
   acttgtagag gcagagaggc agacccgata ggcctcaata cggtggcaca ctgcatctca 30600
   tagttttaaa ataatgaaga ctggctgagc acggtggctt acacttgtaa tcccagcact 30660
   ttgggaggcc taggtgggca gatcagttga ggtcaggagt ttgagaccag cctgatcaac 30720
   atagtgaaac cccatctcta ctaaaaatac aaaaattagg ccaggcgcat tggctcacgc 30780
   ctgtaatccc agctctttgg gaggttgaag tggatggatc acctgaggtc aggagttcaa 30840
   gaccagcctg gccaacatgg cgaaacccca atctttacca aaaatacaaa aattagtcaa 30900
   gcatggtggc acgcacgtgt aatcccagct actcaggagg ctgaggcagg agaatggctc 30960
   gaacccggga ggcagagatt gcagtgagcc aagatcgcgc cactgcactc cagcctgggc 31020
   aacagagtga gaccctgtct caaaaaaaat aaataaatca cacacacaca cacacacaca 31080
   cacacacaaa aaaaaaaaat tagctgggca tggtggtcca ctcctgtaat cccagctact 31140
   tgggaggctg agacatgaga atcacttgaa cctgggaggc ggaggttaca gtgagccgag 31200
   attgtgccac tgcactctag cctgggtgac agaggaagac tccatctcaa aaaaaaataa 31260
   caataataat agagggccgg gcacggtggc ttacgcctgt aatcccaaca cttcgggagg 31320
   ccaaggcagg ctgatcacct gacgtcagga gttcgagacc agcctgacca acatggagaa 31380
   accccgtctc tactaaaaat acagaattag ccgggcatgg tgacacatgc ctgtagtccc 31440
   agctactcgg gatgctgagg cagaagaatc acttgaacct gggaggcgga ggttccggtg 31500
   agccgagatc gcgccattgc actccagcct gggcaacaag cgaaactccg tctcaaaaaa 31560
   taataataat aataataata ataataataa tgactaagta aaagttatta ttaagggaaa 31620
   taaactaccc tttcttagtg ccatgatgtg tgtcagatac tatgttccat tatcttattt 31680
   catcctgaat agaagttgat ggtgactttt tttttttttt tttttttgga gacggagtct 31740
   cactctgtca ctaggctgga gtacagtggc gcgatctcag ctcactgcag cctcagcctt 31800
   ctgggttcaa gcagttctcc tgcctcagcc tcccaagtag ctgggactac agccacgcac 31860
   caccaccccc agctaatttt tgtattttta gtagcgacgg ggtttcacca tgttggccag 31920
   ggtggtctcg atctcgacct cgtgattcgc ccaccttggc ttcgcaaagt gctgggatta 31980
   cacgcgtgag ccaccacgcc cggcagatgg tgacttcttt tatcattgag gaagcagagt 32040
   taaataaggc accaacatca cctggctgga agtctcaaag agctccgatg ctgaaaggat 32100
   gaaacttcag ttatctggga catgtggcag gcggttctgt cattccctaa gcatgttggg 32160
   tttatcacgc actttccagt tatgtgcttg gcgctttgct gagctgatga ccaggaccga 32220
   gccttactca gcgtctgttc gatgtgtcct aggatcagca gcaggctcgt cagtctcagc 32280
   ttgctcagga tgagcgtgtg tcccgctctt acctcgccct ggccaccgaa accgtggaca 32340
   tgttccacat cctcacgaag caggtccaga agcccttcct cagaccggtg agtagaaacc 32400
   cggggctctg tttggtggtt tggactccac attcagactc tctcacttat aactttagca 32460
   gttgttgaag ttctggaaat tttaggatca cagtcatcaa taagtgaaat tctgtgactg 32520
   attttgttcc cagttatgct aattgatacc agactctgtt gatggactga agagcagatg 32580
   tctgatgtca ccacatagtc tacagccact tccaaatgcc ttactgcact ttgtgcttag 32640
   acagtacatt ggattattca tgttgatgtc atgacatatt tttaccatta ttgattccta 32700
   tattttactt atagaaaaag cataaggtgc cgggtgcggt ggctcacgcc tgtaatccca 32760
   gcactttggg agcccaaggc gggcagatca cgaggtcagg agatggagac catcctggct 32820
   aacacagtga aaccccatct ctactaaaag tacaaaaaat tagctgggcg tggtggtggg 32880
   cacctgtagt cccagctact caggaggctg aggcaggaga atggcgtgaa cccgggaggc 32940
   agagctggca gtgagccgag atggtgccac tgcactccag cctgggcgac agagtgagac 33000
   tccatctcaa aaaaaaaaaa aaaagaagaa gaaaaagcat aaggtaacag ctgttgacaa 33060
   tggaaggata ttaagtagaa taattgagac cgtgtaaaat ctttggctct cttagctgta 33120
   cctttgttac actcaggaag ccaaaggtta atgggagccg gccatcacta gggtttatgg 33180
   tggattgcaa atagtcagaa tgtttgttct caactagtaa gacctggttc ctatttctaa 33240
   aattgaaact gagcagcatc actgaagatt tctgccctgc ttcagccacc cccaccacaa 33300
   agttgaaaag ttttatggtg tggccaggac aagggtagtg aggaaggcaa gacggggacc 33360
   taagattcgg tagccatttg cctatgtgga gacatgttta ctttacaatg gaatatgacg 33420
   ttactgcata tttcccctga ttttctgtat ttgctcttac cttcgttatg tgactctctg 33480
   ttatgtgact cctgtcatca gcagaggcac tattgccact ccctggcctg gggacaactc 33540
   ttttccactc ttttaacttt ccacctcatg tctcttcata aacagatttc agtgcaagag 33600
   ccatggaaca cgaaacaaaa cagctcagac actggcctcc cttaaaagtt tgcaaagtgc 33660
   aaggccatgg agcatgaaac aaaacagctc actggccaga cactggcctc ccttcaaggt 33720
   ttgcagaatt ctgtgaaggc cagatagtaa atagttgatt aattcctttg taacaaggtc 33780
   aacacccacc tattttgtac acttgttggg aagactaagc gaaaacacac atagaagtac 33840
   ctggcataag gccaagcacg gtggctcatg cctgtaatcc cagcactttg agaggccaag 33900
   aagggtggat cacctgaggt caggagttcc agaccagcct ggccaacatg gcgaaacccc 33960
   gtctctacta aaaatacaaa aattagccag gcgtggtagt gcatgcctgt aatcccagct 34020
   acttgggaag ctgtggcagg agaatcactt gaacccaaaa ggcagaggtc gcagtgagcc 34080
   gagatcacgc cactccaacc tgggtaacag agcaagactc ttctcaaaaa taaataaata 34140
   aatttatttt aaggcctgct catagacctg agccatctgt acactgtagc cctgttcttt 34200
   tggccccagc ctgtgcctgt gcctgctgtg atgttttcat ttcctttgct acactattta 34260
   ctatctggcc tttgcagaca aagtgtaaag gcatttcatt ttgcaaacct ttaaggtttg 34320
   cattttcacc ttctttactg actagctcag atctctccta cttcaaacag aatgaccaac 34380
   tctgtcctgt tatgactctt ttatttcaca gcttctcaac agtgaaatac aggagtactc 34440
   tgtgcaaaag caaattcttg aaaagctaag tatatgttga atttgtataa atccagttat 34500
   tacatttcat gaaaaccttg tgacttaaag gaatgctttc gtgactccct tttatggcca 34560
   gtgtttaatg ctctctaagc cacatcttcc tgtttcaggt ttgcttaaaa ccatgtatgc 34620
   ccaaccgtag tgggtactga gtcaccttgt gagattacag catagattat tactattttc 34680
   caggtgtttc atttaagttt actctgtctc attgactaga tttaaaacta ttcaaggcat 34740
   aacttttatc ttctttcctt gcattacttt gttatttgta tctgataagt acataagaat 34800
   gacacagact gaactgccag gggcttttaa catttatggg gaaaaaagtt tatgaaatac 34860
   tggctggaag agctgactca aaactttttt tgctcagata atggtaagat ttgtataatt 34920
   ttctctcttt tacttgtttc tcaatgaatt tggagtgata ctttttttaa ttaaaagcat 34980
   aaatgtaaag aaaagtttgg cttcttttgg tatttataaa taaatatttc atcagcctgc 35040
   aacattccat taagtattag aattatcaga tcagaaatgg ggagtaaagt tgaaatataa 35100
   tgttgtcttt cttcagctta tgatacacag cacctgtaag gattctttga agattaatcc 35160
   aaccaattcc tgttcctcca ctgaaatggg agtttttggt ccttttcact tgtctcaaca 35220
   gcatgaattg tcctattatc ctgtgatttc ctaggagctt ggaccccgat tggctgcaat 35280
   gctgaacttt aatcttcagc aactttgtgg ccccaagtgc cgtgacctga aagttgaaaa 35340
   ccctgagaaa tacggctttg aaccaaagaa gctgttggac caactgacgg atatttactt 35400
   acagctggac tgtgctcggt tcgcgaaagc cattgctgac gaccaggtca gtgagttgag 35460
   ttggtctctc tgtgagttta ctggcagatt tggagataat aaccacagtg gccagggaac 35520
   tcgtctggtg taaatgaaga tgagtgggtt gttttccttt gagtgtgaag agcagatttg 35580
   ggctgggcgc ggtggctcag gcctgtaatc ccaacattct gggaggccaa ggcaggggga 35640
   ttgcttgagg ccagactgca gtgagccgtg attgcaccac cacactccag aatgggtgag 35700
   agagtgagat cctgtctcaa aaacacggtg gctcacgcct gtaatcccaa cactttggga 35760
   ggtcgaggcg ggcagatcaa gaggtcagga gtttgagacc agcctggcta acatggtgaa 35820
   accccatctc tactaaagat acaaaaaatt acctcagtgt ggtggtgcat gcctgtaatc 35880
   acagctactc gggaggctga ggcaggagaa tcgcttgaac ccaggaggca gaggtttcag 35940
   tgagctgaga ttgtgccact gcactccagc ctgggcaaca gggtgagact ctgtctcaaa 36000
   ataaataaat aagtaagtaa gtaagtaaaa aacaaaagta aagagcaggt ttggcgctct 36060
   gagccctctc cagttgagtt cttaccccat ctttttaagg gccttcttga caaatccatt 36120
   acatttctga aaagagcccg gtttctcaag ggatcagaat cttctttcct agtctcctgc 36180
   tagagtgctg ttatttttgg ggttgtccag attaaaaatc ttgtgtggtt ttctttacat 36240
   tcttccctct cctttattcc gtgggtctcc tgtgagcaga tctgtctgtg tgtgcttcat 36300
   ttccacagcc accgatttag gctaagctgt ccctgcgtga tcttctagtc atcccataaa 36360
   gggctggccc cctccatggt ccgttctgcc tcattttcaa cgtcctcaca gctaatgttt 36420
   attaactgta cacctcattt cacccacttt tcgtatctcc acccatttag tcctcacaaa 36480
   atcctttgag gtaggttctg taaagtcttt gttttaaagt gaggaaagag gcacagagag 36540
   gttaaatagc ttgctcgagg tcacagagtt agtgtgggac agaggcatga tccatgtcca 36600
   ggcagcctgg ccttgaaccc atatcctcag tctctctgct gtccacttaa aatgcccaca 36660
   aagaaccatg cttgtctctg tgtctctgtc tttgttcaca ctacttatcc aaatactatt 36720
   catggctcaa gttaatcatc agtaggcatt ctagaaggtt ctttacccat atctcatcca 36780
   aaggagtaat attacaatag aagcattgta ggtacttttt tttaatgaca gctttattga 36840
   gctataactc acatatgtag gtgctttctg atgtagcagg ggaagtggtc ttaacaaagg 36900
   atttgcagtg aaaaaataca tagtcatgtg ctgtttctgt cgatgctgga ccacaaagac 36960
   catgtggccc catcatatta taataccgta ttttcactgt accttttctg tgtttagaat 37020
   actcaccatt gtgttacaac tgccgacagg attcagtaca gtcccacact gtacagtttt 37080
   gtagcctagg agcaataggc tataccatag agcctcgacg tggtgtaggc cacgccatct 37140
   aggtttgtgt aagtaaattc tatgatgttt acacagcaac gaaattgcct aacaagacat 37200
   ttctcagagc gacacatgac tgtgtgtcta aagcagtttt atcatacaaa gtagtggctc 37260
   gtaagagggt taggaaattt tagttacttg tagatgttaa aatatatact gctggccgag 37320
   cgcggtggct catgcctgta atcccagcac tttgggaggc cgaggcgggt ggatcacaag 37380
   gtcaggagat cacgaccatc ctggctaaca cggtgaaacc ccgtctctac taaaaaaaat 37440
   acaaaaaaat tagccgggcg tggtggcggg cgcctgtagt cccagctact cgggaggctg 37500
   aggcaggaga atggcgtgaa cccaggaggc agagcttgca gtgagcggag atcgagccac 37560
   tgcactccag cctgggcgac agagcaagac tgtctcaaaa aaaaaaaaaa attatatata 37620
   tatatacaca cacacacaca aacactgctt tactagcccc cgactatata atactgcact 37680
   gatttagaaa cagaaagcaa gtcatagcct gcagtgaggg aaagaaaagg gaagcactta 37740
   cccattgagg tggggtccac acctgactgt agctggcgtc tccagcccag ccagaggtcg 37800
   gtgtggaaag ccccagggct cttccatcgt tgccgtagag agacttggaa gagctgttta 37860
   gtgtgcgccc tctccagggt ggttgtgctg tggctgccac atcacaggcg cttaacgtaa 37920
   cacacagaag gtttcggtgt gggtgggcac agcacgccca gtgaggtgat gcaaagcaac 37980
   tgggtgacaa aggaatgctt tgagcggcct ttgtttggtg accaaccaca cattgcattg 38040
   cctggtcatg ccttagatta tggtggtggc ctctggtggc acctggcttg ggttgtagac 38100
   agcagtttct ggcacagtga cacacggggc tccttgaatg tctaaatagt cctgttcttc 38160
   cagagtggtg aaacaaacac acatgtgcca gtgacacatg gagagaaaag catattttaa 38220
   gaagtgttgg ggctgggcgc agtggctgac acctgtaatc ccagcacttt gggaggccaa 38280
   ggtgggtgga tcatgaggtc aggagttcga gaccagcctg gccaagatga tgaaacccca 38340
   tctctactaa aaatacaaaa attagccggg cacagtggaa ggcggctgta attccagcta 38400
   ctcggaggct gaggcaggag aatagcttga acccgggagg cagaggttgc agtgagctga 38460
   gatcgcaccg ctgcattcca gcctgggtga cagagcaaga ctccatatca aaaaaaaaaa 38520
   aaagaagaag tattgggctg ggtgcggtgg ctcacacctg taatcccagc actttgggga 38580
   gccgaggcgg gcagatcacc tgaggtcagg agtttgagac cagcctggct aacatggtga 38640
   aaccctgttt ctactaaaaa tacaaaaaat tagccgggca tggtggtgtg cgcctgtaat 38700
   cccagctact cgggaggctg aggcaggaga atcgcttgaa cctgggaggt ggaggttgca 38760
   gtgagccgag atcgtgccat tgcactccag cttgggcaac aagagcgaaa ctccgtctca 38820
   aaaaaaaaaa aaaaaaaaag tgtttttgct tcctttcaca gtgtggtgtg tgtgctgatt 38880
   gttgggtatt ttctatttat attggccata agctgattag gggcctgctt acttgattgt 38940
   tttagaagag agctttggtg gggcaggggg cggtaagtgc agaaggatgg atggttggca 39000
   agcactgggg aagaggttgt aaaattgcat tttcttcttt ggaagcagca gtatcttagt 39060
   ttgctttggt atgtcttagt ttggtttggt atgtcttgtt ttccgggaaa gacaaaggaa 39120
   gaggaaaatg acagctttgc aggtaaagaa actgagaggc ttctctccct ctataccaag 39180
   agtaggctga agacccatgt ccagaaggct gacagaaagg tgcctgggtg aagagccact 39240
   gagcttgatg ctcaggaatc tgagggcaga gctccatgcc agagaagtgg gataccacat 39300
   tggtcctggg agccaagagg accaggatag ggagatgtta aagtggccta gactgtgtct 39360
   ttatgggcag acgccagttc catggacaga ctgcatatct gtcgtccatg aagcattgtc 39420
   tgttaacacc attttccatt aaaaaagaaa aaaaattcag cacctactta gaaaataaaa 39480
   aaaaatagcc gggtgcagtg gctcacgcct gtaatcccag cactttggga ggccaaggcg 39540
   ggcggatcac aaggtcagga gatcgagacc gtcctggcta acacggtgaa accccatctc 39600
   tactaaaaat acaaaaaatt agccaggcgt ggtggcgggc gcccgtagac ccagctactc 39660
   gggaggctga ggcaggagaa tggcgtgaac ctgggaggtg gagcttgcag tgagccgaga 39720
   tcgcgccact gcactccagc ctgggcgaca gagcgagact ccgtctcaaa taaataaata 39780
   aataaataaa taaaaaagaa aataaaaaaa ataaaaatcc catcagtcac acttccccca 39840
   tgcagtcagt ctttcctaag taattgctcc ctcctctaac ctaccagaat atgagtctgc 39900
   gtctcacaaa tgtaatgctt cattttcttg ttattttttt cttctttaat gaggtaagat 39960
   taatatataa cgtagaagta accatttaaa agtgtaccat tttgtggcat gtagtacagt 40020
   cacagcgttg cacagccacc acctctacct agttccacta catgtatcac ccccaaagga 40080
   aaacccttaa agcagtcgct gtctagaccc ccttgcccca gcccctggca accacaaatc 40140
   tgctgtctct cttttcctat ttttggattt acctattcac ctctccggga tatttcatat 40200
   aaacggactt atacagtgtg taaccttcta tgtctggctt ccttcactta gcatcatgtt 40260
   tcaaggttca tttgtgttgt agaatgtatc agtatttcat tcctttttaa ggctgaataa 40320
   tatttcattg tatggatata ccaccatttg tttatcctca tccattgata gacatttggg 40380
   ttgtctccag cctttggctc ttgcaaatag tgctgctatg aacatttgtg taaaagtatt 40440
   taaacacctg ttttcagtta ctttgggtct acacttggga gtgggattgt tggtcatgtg 40500
   gtaattccat gtttaacttt ttgagggatg ccagaatgtt ttctacagct gccgcccatt 40560
   tgacattccc actggcaatg tataaggttt aagtttccat tctttttcat tttttttctt 40620
   tggtttagtt tatattcttt ccatcatgaa gtttaaaaat taaacatgtt aatttgtttt 40680
   tgtttcttaa atactcatcc tagtgggagt gacgtggtat ttcattgcgg ttttgatttg 40740
   tatttcccta atgtcaaata ccattggaca ccttttcatg tgcttgctgg ccatttttat 40800
   attttcttgg gagcaatgtc tattcatgtt ctttgcccat tttttaattg ggttgtttgt 40860
   gtttttgttg ttgagttgga agagttcttt ttttcttttt cttttttttt tttttttttt 40920
   gagatggagt ctccctctgt tgcccaggct ggagtgcagt ggtgcgatct cagcctactg 40980
   caacctccac ctcccagatt caagcaattc tcttgcctca gcctcccaaa tagctgggac 41040
   tacaggcgtg caccatcacg cctggctaat tttttttgta tttttagtag agacggggtt 41100
   tcaccatgtt agccaggatg gtctcgatct cctgacctca tgatctgcct gccttggcct 41160
   cccaaagtgc tgggattaca ggcgtgagcc accgcgccca accagaagag ttctttatgt 41220
   attctggatg ctaggtcata tcagatgata catgtatcat tggatatatg atttgcagat 41280
   attcttgtgt tagtccattg catcactata cagaaatacc caaggctagg tactttatga 41340
   agaaaagagg tttaggccga gcacagtggc tcacgcctgt aatcccacca ctttgggagg 41400
   ccaaggtggg cagatcactt gaggtcagga gatcgagacc agcctggcca acatggcaaa 41460
   accctgtctc tactaaaaat acaaaaaaat tagccagccg tggtggcgca cacctgaaat 41520
   cccagctact caggaggtgg aggcttcagt gagccgagag cgtgcctctg cactccagcc 41580
   tgggtgacaa agcgagactc cgtctcaaaa attaaaatta aaattaactg ggcatagtgg 41640
   catgcacctg tagtcccagc tacttgggtg gctaaggtga gaggattgct tgagcccagg 41700
   agtttgaggc tgcagtgagc catgattgca ccactgcact ccagcctgag tgacagaatg 41760
   agactctgtc tcaaaatata tatatatatt tatagtaaaa catatatggc aaaaacactg 41820
   gccacttgtt ttgcgatggt gatacaaata caaatatata tcatcactaa gcaagtggcc 41880
   agtgtttttg ccatatatgt tttactataa atacaaacta cagcatactt tactgtttgg 41940
   catggcagtg atttttatga attaggcacc tgtgaggtag atgttactct gcccatttta 42000
   cactcaaaga acctgggctt aggagggttc agatttgcct acgactgcac agctggtagg 42060
   tggtagagcc agaattcaag ttcagatttg tctgggtcca cggtgggcgc ctgacctcca 42120
   ctccacaact tcccttcctg gggacataaa tcgtgtctgt tcccaggcct attcatcagt 42180
   tcaccagggt ggtctaacgt ccatggagcc cctgacatgt gctagtgctg aactaaatga 42240
   gcttgaagaa gcttgaaagt gaacaggaga aggctcctct agagtatgaa caatagtgga 42300
   ttgtttttac catgcttacc tttgtggaag agagctgaaa ttttcacctc ttctttctta 42360
   taggctgcca gggcacagtg ccccccaagg actgaaatag actagatttc cttctctttt 42420
   taatgtttaa actttaaaag aaatgcgtgc tagctgtaaa aattcaaaaa aaaagcccgt 42480
   ctgcctcagt cctgcagccc tctccagagg gagtgacctc gtacagtttg gcacatatta 42540
   ttccagtctt tatccatttg taagcataaa aagtgcatac ttttatgagg ctataaatgc 42600
   ataaagacgg atatataaaa tatataagtg tatatataat tttagggctt ttttagtggg 42660
   ggaaaatcca caaatggata atactttgga tattttctgc agctcgcttt tttccacctg 42720
   acgtacattt acgtctcaca ttgccattcc tcctttgcag agatcctaca gtaaggaatt 42780
   gtttgaagaa gttatttcaa agatgcggaa ggcagggatc aaatccacaa tagcaataga 42840
   aaaatttaag ctgctcgccg agaaagtgga ggagatagtg gccaagaacg cacgcgcaga 42900
   aatcgactac agcgacgctc ctgatgagtt cagaggcaag tggactcgtc gttttcatgc 42960
   tgatttcttt tgagttaact ggaaatcgat aacattacaa gaggacgtcc tgtgcaatta 43020
   attttttaat ggtgtgctag agctgctttt tgagacaatc tgtcttaaat tacatttgcc 43080
   tatttttagc tcatattgta aattgagagg aagaaacatc cttagcttag cctgacttaa 43140
   agccagataa caaccctgaa gattctaagg gcagggctgc ctttgcaagt ggggattcac 43200
   attttcaaag aattcccaga agtcctaaga caaagaattc cagcctgccg tcctcgccac 43260
   ggagcctgtg gggcctgctg ctgggtctgt cccaggcttt cttctctgtt ccctcccccc 43320
   agcagtagct gacaaagaag cgccttcccc ttacaaaagg caatgtatat gcctttattg 43380
   aaggctgatt tccttctaaa cctgggggcc tcacaggggc cctttgattg ctgttccttt 43440
   ggatagattt ttcgctggta gaacgggctt tgttttcttt ttcagtcgtg ggctctcagt 43500
   agattagagt ttgctttgct ttgtccccgc agaccctctg atggacaccc tcatgacaga 43560
   ccccgtgcgg ctgccctctg gcaccatcat ggaccgctcc atcatcctgc ggcacctgct 43620
   caactccccc acggacccct tcaaccggca gacgctgaca gagagcatgc tggaaccagg 43680
   tagaggaggg tgcagtttga ggtgcagcgc tggcgtcagt accaagagat aaagcccaag 43740
   tcacatattg gagatgaagc agaagggaaa tttatcaaat gcagaaacta cctactttat 43800
   gacactctgt agcaaagacc caaaacactc ttggcccttt ttccctgcag acatccagtc 43860
   cctccctctc atgtcccagt ccttctggag tcttcggctc aatgagggaa agaatggcct 43920
   tttcttccca tttatacaga gcgacaagca tcctctctca ggaagagccc tcccatctgg 43980
   ggcattaatc ctcctttttt tcttttctca gtgccagaac tgaaagagca gattcaggcg 44040
   tggatgagag agaaacagaa cagcgatcac taaaccgttc cgccgcccac cctctgctag 44100
   acacagccaa ggccaacgag gcaagcagaa gcagcggccg cagcgaagct gccgttcatg 44160
   tgttggaggc caaatgtggc aaaccaaccc caggcccacc cagagcgagc aaacgctgag 44220
   acctgaaagg acatggatga gaagaggagc ccgcttcctg tacatatatt taagtgacaa 44280
   acacggtcaa aagcttaagg gacaggtttt atggttgctt gtgtaataaa gcatgtcctt 44340
   cgtatgtcac agtttggggc aacggaagtc ttttagtgat ggctaatggg tctgggcagc 44400
   atcccttcat gaattttttt ttaatccaat atccgttgat ttgattgtga ttagagaacc 44460
   ttggacattt tgctgctaaa gaatcttttt tcccctctcc cccttcctga ccctacttgc 44520
   actgctgtgg atttttttaa gagaagcaaa aacaaaagta aactcctttc cctggccctc 44580
   caaacatata ttctgtgaga taactgtgcc tgctaccaag tgttaatcct gggatcgtat 44640
   ttttatatca tattcacata tttgtttttt taattggtgt tagatgacat gattaataaa 44700
   aaaggcaaga tattttcaga atttgaattt cagttttttt tttcttttga aatgtccctt 44760
   taagattttt ttattctaaa caaaataaag aaaatcctgc tgctctggtc cttgtgataa 44820
   gcctctcttc ggcatctgag gagcagctgc agcaaaatct aggggtgtaa gtgtatcagg 44880
   acttatgtga cttatatttt ggggagatga gggttgggtt ttttttttaa tgctacgtga 44940
   cagtttgaaa ccttcacagt tatcctctgg gggtaaatca attcttcaac ccttgggtgt 45000
   gtgagtttga ggcagggtca tttgtgtgat gtgtttggcc ttaccaaagc aaaagagggt 45060
   gcaagaatgt gggagtatgt ctgccggttt caacacacac agacaaacac agccacacgc 45120
   gcacacaagt ataagacttt ttgtattact gctccctact taacatactt gaattctcaa 45180
   atttcctttg gggtaaaaaa aaaaaaagga tttgaaacca taaagtgttc tgaagaaatt 45240
   aagtctataa aaagcatact ttcttttttc ttttcctttt ttccctccac agacaatgtc 45300
   ctctgttcaa ttcctaacgc aaactacaat aaatggtgac acacgttcag aagaaatctt 45360
   ctttgagtct cttctctttt gtggaaaaaa aaaaagggca gggcgggtgg gtaaacaaga 45420
   gccaagtgag tttgcatgtg gtgaatgatc tttcctggat ccaattagga gctgctattt 45480
   ttaaaagaaa actttcttat tcatgtcagt ctcgacattt ggttctcatt tcactgtttg 45540
   ctgatttgag ggggtaagct ttgtcatatt cagtcttctg cctttcttga gtttcttctg 45600
   ctttatctca agtatcttcc ctgaccttca agattcttcc tcaggccggg cccggtggct 45660
   cacgcctgta atcccagcac tttgggaggc tgaggcggat ggatcacctg acgtcaggaa 45720
   ttcgagacca gcctggccag catggtgaaa ccccatctct actaaaaata caaaaaatta 45780
   gccgggcgta gtggcagcgc ctgtagtccc agctactcgg gaggctgaga caggagaatt 45840
   gcttgaacct gggaggtgga ggttgcagtg agccgagatg acgtcatcac actccagcct 45900
   gggtgacagc gagactccgt ctcaaaaaag aaaaaatata tatttatata tatttacata 45960
   tatatttatt tatatatatt tatatatata tatataacat ggccaggtgc ggtggctcac 46020
   gcctgtaatc ccagcacttt gggaggccaa ggcaggtgta tcgtctgagg tcaggagttc 46080
   tagaccagcc tggccaacat ggtgaaaccc catctcaact gaaaatacaa aaattagctg 46140
   ggcgtggtgg tgggtgccta taatcccagc tactcggggg gccgaggcag gagaattgct 46200
   tgaacccagg aggtggaggt tgcagtgagc tgagatcgcg ccatcgcact ccagcctagg 46260
   ggacaagagt gagacttcgt ctcaaaaaat agatagatag atagacacaa taaataagga 46320
   aaaagttcat gatagcaaaa attttaggca atataagcaa aaagaaaaaa aatccatatt 46380
   ctcaccatgc agaagattct gtttaattgg gtttactttt tactaaactt acatatctaa 46440
   gcttagtgcc tgtagaatta ggatactgac tggcatcttg cctaattttg tttgtgtggt 46500
   tatctgacca gtgtgtactg cttgtctcct ctccatccca gattccaaac tcttatttta 46560
   atcctccagt tgtccccgtg aatgtaataa aatgctgctt tataccgcca tatgcacttg 46620
   tcgttttgaa taattcagag gtttcaagga cctgccggat aggacagaga cacagtcagt 46680
   ctcctgcctg tactatctat agggggaaga tgatttgggt ttccattttt agagtctcaa 46740
   gagttgctgt tttgtgtttg tctgcagtct cttaattgtg gatattgaaa taaatcccaa 46800
   aatatattaa aagtaggtct tggctaaaag gaagattgtc aaataagaga atcagattgg 46860
   cagaagagaa atgatccttg agagaaagat gattaaaatc tgccttgtgt tgtgactcag 46920
   tgttgtgtag attttaaaaa gatattgatt gaatatcttt tgaaaaaaaa gctgagcaca 46980
   ccgttcactt tttgtaacct ctgggtttgg tacatcgagt agtgaatttc acagcaattt 47040
   gattggagtg ctaaagtgac atgaataggc ctcatttgag taggtacttt tttttgtttt 47100
   ttggtttttt tcagacggag tctcgctccg tcacccaggc tggagtgcag tggcgcaatt 47160
   ttggctcatt gcaacctcca ccttccaggt tcaagtgatt ctcctgcctt agcctcccga 47220
   gtagctggga ctagagcaca tgccaccatg tccagctaat tttttttttt tttttttttt 47280
   ttttttagta gagatggggt ttcaccatgt tggccaggct ggtcttgaac tccagacctc 47340
   aagtgatcca cctgcctcag cctcccgaag tgctgggatt acaggcatga gccaccacac 47400
   caggcctcaa ggtacgtttt tgattgaagg agacacagta gcctatgagc ttgatgctgg 47460
   tgtcagatag tgttggtgaa gaaagtcatt ctgaattggt gatgatcatt tgcatttcat 47520
   gagcatattc tatgtgcaag ccactcttac atgtattaat tttttttttt tttttgagac 47580
   agagtctcgc tctgtcgccc aggctggagt gcagtggcgc gatctcggct cactgcaagc 47640
   tccgcctccc aagttcacgc cattctcctg cctcagcctc ccgagtagct gggactacag 47700
   gtgtccgcca ccatgcccag ctaatttttt gtatttttag tagagacagg gtttcaccgt 47760
   gttagccagg gtggtctcga tctcctgacc tcgtgatcca cctgccttgg cctcccaaag 47820
   tgctgggatt acagacaaga gccaccgcac ccggccacat atattaatta atttaaccct 47880
   ccacaaccat aatgtagtgg aactggcaca tgaatctagg ccatctggcc tttccagcct 47940
   catctcttcc cgactcctgc tgtatggcag agttccagaa tgctagaagt tcagaatagc 48000
   tcagaatgcc caaggagaag cagcatagca ttttggaagg gactttggcc ttgagatcag 48060
   ctctagacct aacccccatt ttaaacaatt agataacttc aatatcacag ctttcattcg 48120
   ttactagcca ctcacgttga ccccagatgt atatctcaaa cctccacttc tctcctgtac 48180
   tgtacacact ggccatctca gatgactaaa aggtcctcag acttaacata tcccaacctg 48240
   aactctgtac atttcacccc acgcccgctc ctcttagtct cctcatcaca gtaaatgaca 48300
   actccatcct tccaagtcgc tcaggccaca aaccttcgag tcatccttga ctccgctgtt 48360
   gtgctctcct cctatgtcca atctgtccac aaatctctct gctcagctgg gtgcagtggc 48420
   tcacgcctgc aatcctagca ctttgggaga ccaaggtggg tggatcacct gagggcagga 48480
   gttcgagacc agcctggcca acatggtgaa accccgtctc tactaaaaat acaaaaatta 48540
   gctgggcatg gtggcgggct tctgtaatcc cagctgcttg ggaggctgag gcacaagaat 48600
   cacttgaacc cgggaggcgg aggttgcagt gagctgagat cgggccactg tactccagcc 48660
   tgggctacaa agtgagactc tgtctaaaca aacaaaaaaa tccctctgtt ctacctacaa 48720
   aatgtatccc agaactcgac gactactccc caggtcccct cctgtcactt tggtttgagt 48780
   tgctgctgct ggctctctcc tggattattc caacagcatc actggtcctc ctgcctctcc 48840
   ccttaacctc ctttctcaga aaagcaggta ggatgatctt gttaaaactt tggctgggcg 48900
   tggtggctca cacctgtaat cccagcactt tgggaggctg aggtgggcgg atcacctgag 48960
   gtcaagagtt tgagaccagc ctggccaaca tggtgaaacc ccgtctctat taaaaataca 49020
   aaaatcagct ggatgtggtg gtgtacgcct ataatcccag ctactcagaa ggctgaggca 49080
   agagaatcgc ttgaacccag gaggcggaga ttgcagtgag ccaagattgt gccactgtac 49140
   tccagcctgg gtgacagagt gacactctgt ctcaaaaaaa aaaaaaaaaa aaaccaaaaa 49200
   aaaaacaact atagctaggc gtggtggcat gcacctgtgg acccagctac tcaggggctt 49260
   tggcaggagg attgcttgag tccaggaagt tgaggctgca gtgagccgag attacaccac 49320
   tgtactccaa cctgggtgac aaagtgggga ccctatctca agaaaaagat gctgggtgcg 49380
   gtgactcacg cctgtaatcc caacactttg ggaggccgag atgggcagat cacgtgaggt 49440
   caggagttcg agaccagcct ggtcaacatg gtgaaaccca gtctctacta aaaatacaaa 49500
   attagccaag catggtgatg cgtgcctgta caagtcccag ctacttggga ggctgaggca 49560
   gaattgcttg aacctgggag gcagaggttg cagtgacccg agattgtgtg ccattgaact 49620
   tcagcgtggg caaaaaaaga gcaaaaattg cgtctcaaaa aaaaaaaaaa gaaaaaagaa 49680
   agaaaggggc tggaaaaaaa accctaaagc caggtgtggt gttgcacgcc tgtagtccca 49740
   gcacttccag agatcgaggt gggaggatcg cctgagccca gaaattcaag gctgctggag 49800
   cagaagctcc agcaaatgca aggggctatg atctagccac cacactccag cctgggtgac 49860
   agactgagac tccatctcaa aaaaaaaaaa aaaagaaaga aagaaatcct tacaataacc 49920
   tggaaggcaa aacgctcacc tcgcattctc tctgatctca tctcctacaa cactcccctt 49980
   actccaaaaa ctcagccatt ctggcctcat tgctctccac ccctgctagg gcactagact 50040
   acttcctcta tgaagaatcc tcttcccaca gatatacata tgggtcagtc actcccttat 50100
   ttgctttaga tctttactca aatatcacct tggtgagtcc ttccctgtcc accctattta 50160
   aaaatgtaat ttattcacct aaccttggta tcccttgcta ttccctttct ttgcattttc 50220
   tccttggcat ttatcactat ctaacatgct atatctatta tgtactttac agtccacttc 50280
   cccccactag aatgtaagcc cggggtgggg gggggcagag atttttttct gtatccttgg 50340
   cacctataag tgtgcctggc aaatatatgt gttgaatgaa ttaatctctt catatatttg 50400
   ttactctagt tgtatcagtt atttattgct gcataacaaa atcaccccaa aatggagtgg 50460
   cttggttcaa taacagtgat tttatccccc acagttctgg aagatccaag agcaagcttc 50520
   caagaggacc agacagaagc tgtgtattgc ccttttacag cctaggcttg gaagtcacat 50580
   agtgtcactt cagcagcagt cacaagcctg cctggattga aggggaagaa atgcagaacc 50640
   cgcctcatgc tgggagggag gtcaacgtca ctctgtgaga agagcttctg ggatgggaga 50700
   cgtcgttctg accatcttgc aaataacatt tgccacgttc ctcctctggc aacaatttac 50760
   attcttcata tctgcaaaat gatgccttat ttccttcccg tgagattccc aaaccgcatc 50820
   ccatcagggc atccgcttca agtccagcat ctcatcattg aaatcaagtc cagctgcaga 50880
   tgaggcttct cagatggatt ctttgaacgt agttccttga gtgttaatct tacggattta 50940
   aagatctgtt aatgaaagat acaagttacc tacacacaca tgcacacaca cacacaccac 51000
   gtgcacacat acaccacaca ccacatagac acacacgtgc acacacacac cacatgcaca 51060
   cacaacatgt gcacacacac catgcatgca atgatgggac aggcatggga aaaccattct 51120
   ggatgatcct gttcaaaaag gtggaaaatg gtaccccaca ggagtcactg gtccatagta 51180
   ttaataattc tgagatccaa ccaggcatat gtcaccagtt ccttgttcag ggctcagtcc 51240
   tacagcccag gaattgttca ccaagcctct cagctccacc ctcttggctc tttttgtcct 51300
   ctgaatcttt tcttcctttt ccataaaaca aaaaacaaaa actgagcttg caattaagcc 51360
   ttctcagtac atttcctgct tattaaaaaa atatatacat atgttgcagt aacgccgggt 51420
   gcggtggctc acgcctataa tcccagcact ttgggaggcc gaggtgggtg gatc 51474

<210> 61
<211> 3530
<212> DNA
<213> Homo sapiens

<400> 61
agatctcacc actgcagtcc agcctgggtg acagagcgag actctgtctc aaaaaaaaaa 60
aaaaaaaggt gcaggataat aggtgcggga cagtggcatt tatacataaa gtctgaagta 120
tgaatatgag cactatactg tttaggacta aatttatgta tagtcaagtg tgaaaacatg 180
gatggcaatg acaatcatca aattcaggat gagattaacc caggaaagaa agagagaaaa 240
acaaaacaaa caaacaaaaa aagggaggat gacatgagga tctgcaactt tatgtgtaat 300
attttatttc ttgaaaaaca aaataacagg aagatggaaa tgttgagttg ggaggaaaaa 360
ccagagaatt tattgatttg tcttgaactc cttattgtta ttattatttt ttgtttgttt 420
tttgagatgg agtctcgttc tgtcgcccag gctggagttc ggaggtgcta tttctgccgt 480
tgatgatttt ccgccttcca ttttcttctc tggaaatgtc tattattgat tgattgattg 540
accgattaat tgatttagag acagagtctc cccttctcac ccaggctgga gtgcagtgac 600
tcgatctcgg ctcactgcaa cctctgcccc ccaggttcaa gcggagcgat tctcctgtct 660
cagcctccca agtatctggg attacaggtg cccaccgcca tgcccagcta atttttgtgt 720
ttttagtaga cacggggttt cactgtgttg gccaggctgg tctcgaactc ctgacctgag 780
gtgatctgcc cgcttcggcc tcccaaagtg ctgggattac aggcgtgaac caccacgcct 840
ggccctgtca ttattattat tgcttttttg ttttgttttg ttgggaaaga gtctcactct 900
gatgctcagg cttgagtgca gtaatgcgat ctcagctcat tgcaacctct gcctcctgag 960
ttcaagcaat tttcgtgcct cagcctccta atgtagctgg gattacaggc atgtgccacc 1020
acacctggct aatttttgta attttagtag agatggccag gcacagtggc tcgcgctcgt 1080
aatcccagca ctttgggagg ctgaggcaag cagatcgcat gaggtcagga gttcaagacc 1140
agactgacta acatggagaa acttcgtctc tactaaaaat acaaacttag tcgggcatgg 1200

tggcacatgc ctgtaatccc agctgctcgg gaagctgagg caggagaatc gattgaaccc 1260
gggaggcaga tgctgcagtg agctgagatc gcgccattgc aatccagcct aggcaacaag 1320
agcaaaactc tgtctcaaaa aaaaaaaaat tgtagtagag atggggtttt gccatgttgg 1380
ccaggctggt cttgaactcc taacctcaag ggatccaccc acctcggcct cggcctccca 1440
aagtgctgga attacatgca tgagccacta cgcccagcct attgtcatta tttaatacgg 1500
tgtttaatac agtgtatgtg ggggggaatc atgttcgttt tttttttttt ttgagacaga 1560
gtctcactgt gttgcccagg ctggagtgca gtggcacgag ctcagctcac tgcaagctcc 1620
ctgtcccggg ttcacgtgat tctcctgcct cagcctcccg agtagctagg attacaggct 1680
cacactacca cgtcgagcta attttttgtg tattttgagt agagacgggg tttcactatg 1740
ttggccagac tggtctcgaa ctcctgacct tgcaatctgc ccgccttggc ctcccaaagt 1800
gctgggatta caggtgtgag ccactgacca ggccatcatg ttagttttta tactttacta 1860
cctgtttaaa aaatacatat gtaatttatt ctgggcaagg tgggatgcac ctgtagtccc 1920
agctacctgg gaggctgagg tgggtggatc tcttgagccc aggagctcta ggctacagtg 1980
cacttttatt gtacctgtaa atagccactg cactccagcc caggcaacag agcaagaccc 2040
cacctgtaaa aaagtaaata acttcataac ttaaaaaatg ataggtcagg ctcagtggct 2100
cacacctgta accccagcac tttgggaggc tgaggcaggt ggttcacctg aggtcaagag 2160
tttgagacca gcctgaccaa catggtgaaa ccccatcttt actaaaaata caaaattagc 2220
cgggcgtggt ggcacagttc tatactccta gcttctcagg aggctgaggc aagagaatct 2280
cttgaactca ggaggcagag gttgcagtga gctgagatcg tgccattgta ctccagcctg 2340
ggcaacaata gagaaactcc atctcaaaaa aaaaaaaaaa gatatttgtc cagaggcatc 2400
ccagctgtga tgccaccagt aatcctctgc agactgggtc ggcagctaca tcagagggca 2460
tgggaccgat ccccagcagc tgagccatgt gtgaggagcc gagtgggtgg gagacagcaa 2520
gccataatcc tctctctctg acctggtttt cagatgccct agtctcacag cctgcactgt 2580
aaaaggccac cgcgtctcgg ccacacattt ggtaaatgtg ttatggaaat cccttcctga 2640
ctgagtccat attcactcaa aactgccaca tgccgactct tgcactgaca aaaatgtggg 2700
aagagcccaa tttcatttct ggcaaaatcc tctacaggac catccatgcc cataaagaga 2760
attactttcc agttatttta accaaggtca ggtaacacat ggacttgagt cctagatcat 2820
gttgcatgag taaaaatagg aactgcttac attgttggac ttaagagact aggcagctct 2880
ctaccccatt aaatctagaa aatatatgct ttcctatgca aaatctttat aaatattaat 2940
taatgtctat tttatgtaag tatgtatcta ttaaacactg aattaaataa tgacaataag 3000
gagcttaaat caataaattt tctgtttttt gccaatgagt actttaacaa acaggatgca 3060
atctggttta actcattaat attaatgtac agtggcacca cttatgccag ggttatttgg 3120
aatcagaaaa tcaaaactgg aagatgcttt agaattggtc tgctacctag tcaggaaata 3180
agcacagcaa gtctctagtt tgctgaggac cgaatttttt tttttctttt gagactgtgt 3240
ctggctctgt cgcccaagct ggagtgcagt ggcgcaatct cggctcactg caacttccac 3300
ctcccaggtt caagcgattc tcgtgcctca gcctcccagc ctccctagta gctgggacta 3360
caggcacctg ccaccacacc tgactaattt ttgtattagt cagagatggg gtttagtaga 3420
gatggggttt caccatgttg gtcaggctgg tctcaatctc aagtgatatg cccgctttgg 3480
cctccaaaag tgctgggatt acaggtgtaa gccaccatgc ctggcctagg 3530

<210> 62
<211> 2607

<212> DNA
<213> Homo sapiens

<400> 62
tctagaggtc aaggctgcag tgaaccgtga ttgtgccact gcacaccagc ctgggcaaca 60
gagtgagacg ctgtctcaaa aataaataaa ataaaacaaa ataaatgtaa aagactcttg 120
gaaaagagca gtgctggagg agagacacag gaaggtttct tgaacggcag ggtgacgtgt 180
ggggacggca catggggaat tatggcctac aatttaatgc cacaaactct gtccacctcc 240
agctcaccag gcgtgtccac acacacgctc accaggaggg cacagcttag ggcctgagcc 300
ttgaccttgc tcagtacaca ctgctgttgc catccaggtg ggttgtaaca cccaggggat 360
tagcagcccc aggaaaataa ccagacatct gaggagcaaa tggccagaaa ttggagacaa 420
taaactgagc agtttatatc aggggaagca gaatgaatgg actggacaca acagaagttt 480
taaataagca taataaatat cttcaaagag ataagagatg ctattagtaa caagtaatag 540
aataagcctt tttttttttt ttttgatacg gagttttgct ctttcaccca ggctggaatg 600
cagtggcgct atctcggttc cctgtaagct tcgcctcccg ggttcacgcc attctcctac 660
ctcagcctcc cgagtagctg ggactacagg cgcctgccac tacgcccggc taattttttg 720
taattttagt agagacgggg tttcaccatg ttagccagga tggtctcgat ctcctgacct 780
tgtgatccac ccgccctggc ctcccaaagt gctgagatta caggcatgag ccaccacacc 840
cagcctgggt gacagagtct tactctgtca cccaggctgg agtgcaatgg cgcaatctca 900
gctcactgca acctccactt cccaagttca agcaattctc ttgcctcagc ctctggagta 960
gctaggatta caggcgtgca ccaccacacc ctgcaaattt ttgtgttttt agtagagacg 1020
gggtttaacc atgttggcca ggctggtctc gaactcctgc cctcatgtga tccgcctgcc 1080
tctgcctccc aaagtgctgg gattacaggc atgagccact gtgcctcaca tataataatt 1140
taccgaattg tacatctatg tttcattcat tttgctgact aagtgttgaa atagcttgga 1200
tatttgtccc tgccgaaacc tcatattgaa atgtaatctc cagtgttgga ggtgaggctt 1260
ggtgggaggt gtttgggcca tggagggatc cctcgtggct tggtgccatc tctacttgtg 1320
atagtgagtg agttcttacc agatctggtt gttttaagag taagtggcgc ctctccgcac 1380
cttgctcctg ctgtttctct gtgaagtgtc tgctccccct ttacctccca ccatgagtaa 1440
aagctccccg agggctcccc aaacaccaag cagacaccgg tgccaccctt gtacagcctg 1500
cagaaccatg agccaattca atctcttttc tttataaatc acccagcctc atatattcct 1560
ttacactgca gaggtgtcca atcttttggc tttcctggac cacattggaa gaagaagaat 1620
tgtcttgggc cacacataaa atacactaac actaacgata gctgatgagc taaaaaaaaa 1680
aaaaaaaatc gcaaaaaaaa tctcataatg ttttaaggaa gttcatgaat ttacgctggg 1740
ccacattcag agctgtcctg ggctgcctgc agcccacggg ccccaggttg gacaagcttg 1800
ctttatagca atgcgagaat ggcctaacat aggtgttata ctttatttat tattattatt 1860
attataatta ttattatttg agatggagtc ttgctctatc accaggctgg agtgcaatgg 1920
cgcgatctca gctcactgca acctccgcct cctgggttca agcgattctc ctgcctcagc 1980
ctcctgagta gctgggacta caggcgcacg ccaccatgcc cagctaattt ttgtattttt 2040
agtagagatg gggtttcacc atgttggcca ggatggtctc gatcacttga cctcgtgatc 2100
tgcctgcttc ggcctctgaa agtgctagga ttacaggtgt gagccactgc tcccagccag 2160
gtgttacact ttataataaa aagatggggc ccaaagataa acaacaacaa aacaaacaaa 2220
caaacaacaa caacaacaaa acaaagaaaa ggtgggccag gcacaagggc tcacacctat 2280

aatcctagca ctttgggagg ccgaggcagg aggattgctt gagcccagga gtttgagacc 2340
agagtgggca acatagtgaa actccatctc cacaaaaaat acaaaaaatt agccaggcgt 2400
ggtggtgcac acctgtagtg ccagctactc aggaggctga ggtgggagga tcgctggagc 2460
ctgggaagtt gaggctgcag tgatctgtga tcatgccact gcactccagc ctgggcaaca 2520
gagtgagacc ctgtctctaa ataaataaat aaataaataa ataaataaaa taattaaaat 2580
aaaactgact aggaagtaag ttctaga 2607

<210> 63
<211> 4276
<212> DNA
<213> Homo sapiens

<400> 63
actagtatta tttcattctt gtttatcact ttactgaaag atcaatttga gtcttctcct 60
tagattcctt ataatccgaa gtgcctttga gggcaagtgg gaattctaca agatcaaaat 120
taagcaattg ggcaaaaatt gagaaaaatt caaatgaatt agacctaaat gttttattgt 180
ctctttaatg ttagtgaatg cttctggaat ttttgctcca ttccactttc ctaacgcttc 240
taattgtatg tcgcaaggaa aagtggtcag aagaacagtt tagaactcag gctgtgtaag 300
tgatttagag tatgtatggt taccttttct acctttctgc tctggctttc aaaacttgga 360
agatgtattt ttttgctaat tattaagaaa tgttgttttc ctataacttc ttaaaaataa 420
ctctgtaggc tgggtgtggt ggctcacgcc tgtaatccca gcactttggg aggccgaggc 480
aggcgggtca cgaggtcagg agttcaagac cagcctggcc aacatagtga aactctgtct 540
ctactaaaaa tacaaaaatt agccaggtgt ggtggcacgc acctgtagtc ccagctactc 600
gggaggctga ggcaggagaa tcgcttgaac ctgggaggca gaggttgcag taaaccgaga 660
tggctattgc acttcatcta gcctgggtga cagagcaaga ctctgtctca aaaacaaaac 720
aaaacaaaac aacaaaacaa aaagcacctc tgtaaaatta ttgtttcttg ctggatttta 780
aacttgattt cagacttaca taagtaattc tttaaaggag cagggagttt tgaaactaag 840
tctttgatgg atgtctatta taatggatac tgtttatagt agtagagaag tgacagaata 900
actttctgta gtccacttaa ctgtagcttt gaatgactgg ataacatttt cactaaattt 960
tgaagatggt tgcaaggtgg tttttttttt tgtttggttt ggtttttttt tttttttttt 1020
tagacggagt cttactctgt caacaggctg gagtgcagtg gtgcgatctt ggctcactgc 1080
aacctctgcc tcccgggttt aagcaattat cctgcctcag cttcccgagt agctaggact 1140
acaggcacgt gccaccacac cagctaattt ttgtatttct agtagaaacg ggatttcacc 1200
acgttggcca ggatggtctt gatgtcttga cctcatgatc cgcccgcctc ggcctcccaa 1260
agtgctggga ttacaggcgt gagccactgt gccggcctga agtttttacc atgagacagt 1320
aaaatcagaa aaaatattta ggtttaggtt gttttgagaa agtaattaaa tataaacttc 1380
taaaaaggat agatgttatt agcacacaac aatgcccggc taatttttgt gtttttttgc 1440
agaaataggg tttcacatgt tgcctaggct ggtcttgaac tcctgagctc aagccatctg 1500
cctgccttgg cctctgaaag tgccagaatt acagcaggtg tgagtcagag tgtacagccc 1560
tgaccgtggt ttttgtttgt ttgtttgaga tggagtctcg ctctgttgcc caggctggag 1620
tgcagtagtg cgatatccca gctcactgca acctccgcct cccgggttta agcggtctcc 1680
tgcctcagcc tcccgagtag ctgggtttac aggtgcgtgt caccacgcct ggctaatttt 1740

ttgtattttt agtagagatg gggtttcacc atgttagcca ggatggtctc gatttcctga 1800
ccttgtgatc cgcccgcctc agcctcccaa agtgctggga ttacaagcgt gagctgccgt 1860
gcccagcctg ttttgtgtgt gtgttttttt gagatgttgt tggctctgtt gtccaaactg 1920
gagtgcagtg atggaatcat ggctaattgc agcctccaac tcctgggctc aagtgatcta 1980
cccacctcag cctctcaagt agctgggatt accgatgcat gccaccacac ccagctaatt 2040
taaacaaaaa tgttttttgg tagagagtgt ctgaatatgt tgcccaggct ggtcttgaac 2100
ttctggcctc aagcaattct ttcactttag cctcccaaag tgttgggatt ataggcgtga 2160
ggaaccatgc ctggccccaa tttatgtttt ttatttaatg ctgtttaaat gttagtttgt 2220
tgacatccag ttaaatagga attcaaccaa aaaaaataac tattgtattc aagacccatc 2280
ctgtgttgta gggcagctgt ccccattctt tttagcccca gggactggtt ttgtggaaga 2340
cggtttttcc acagacaggg ttggggtgat ggtctcagga tgaaactgtt ccacctcaga 2400
tcatcaggca ttagattctc ataaggagtg tgcaacctag atcccttgca tgcacagttc 2460
acagtaatgt ttgcacttct atgaaactct gatgccatcg ctgatctcac aggaggagga 2520
gctcaggcaa taatgctcac ttgcccaccg ctcacctcct gctgtgtggc ctggttttta 2580
acaggccaca gacctgtacc actctgtggc ctaggggttg aggacccctc ttgtagagga 2640
ttagaaactt gactaaggct gggtgtggtg ggacatgcct gtagtcccag ctacttgagg 2700
ctgaggctgc agtgagctga gatggttcca ctgcactcca gtctgggtgg acagagtgag 2760
gccttgtctt aaacaaacaa acaaaaaatt agccgggcat ggtggtgcac ccctgtagct 2820
ccagctgctc gggaaactga gacaagagag tcacttgagc ctgggaggtt gaggctgcag 2880
tgagccatga ttcccttgac agagcaagac cttgtctgaa aaaaacaaca aaacaataaa 2940
aaaagtaatt tattctgcct tgggtaaggt caatgatttg aaaagtgtta aatagaggag 3000
caattgtatt tggttttggg gtggagagaa tccatcaaga ctttagacag gagcaagtga 3060
gaaggtcctg gaagaataaa ttggaattta aaaagaacaa gacaaaggac acagtgggga 3120
aagggaaagt tggagcaaaa acacaggggc agaaaaactt gggatgcatt ctggaagtcc 3180
tctggtgggt ggttgtaaaa gaatgtggct ggaaggcagt gttcatgtgg gcttgtaaaa 3240
ataggtggtt tatgttgcag aacactttga atgccattgt ggggagttca gtcattgctt 3300
cgtagacagg aggggaactt ggaggatttt gaagtggaca gtgatagaaa tagatctgta 3360
cttcaggaag gtgaatcttg gcaatattat gtggtacaga tagggcagat gaaaaaggat 3420
aattcaccta attggataac tgtggtaaag ataataaaaa atatttttaa aaaagaaaat 3480
ggggatattg tgtagtatgg atagggtagg aggaagagag agaaagctgg ttaagaaggc 3540
cttgggtgag aagtattgaa agcctaaaag gtaatggtgg tggtagaaat ggagagattt 3600
tatagatata agctgattag atggatagac aaagcaagct tttagcctgt gtgagaacag 3660
cagaatggtg agaacattaa aaagaaatag aaaggaggcc aaagagctgg tattgttttg 3720
gggatagagg tttcggcttt agttgtgtga gattgaatga aatgctggaa ggatagctag 3780
gtagaggagt cgagtagtct atagaaaaat gtatgtgtgt atgtatgggg acattaggta 3840
ttagagatgt taatgttttg gttcatagtt acaaatacat agtactttat atctaaagcc 3900
caccaaggag gtttcagcct ttcttgtctt gtttgtaaca ttcaacactt tggtaggtcc 3960
taggtagaac acaaaagaaa aaagttttgt tttgtttttt aaaatttaaa tatcaacagt 4020
attcaacaac tatcaacatt ttggcaatct tgttctgtca tcccctaaca aattccttaa 4080
tatcatctaa tacccagcct atcttcagac tacctgatta tctaaaaaat gtatttttag 4140
gccgagtgtg gtggttcaca cctgtaatcc cagcactcga aggctgaggc tggaagatta 4200
catgagtcca ggagttcgag accagcctgg gcaacatagc gaaaccccgt ccctactaaa 4260

aatacaaaaa gctagg 4276

<210> 64
<211> 6405
<212> DNA
<213> Homo sapiens

<400> 64
actagtaagt aaccactatg ctgagtttcc tttgtaacta ccgttaactg tgaaatttct 60
tgaattgagc tcaagcaaac attggcttca ttctgttcca gttgtttagt gttatggcca 120
catttttaac ttgtcatcat gttaaggtct caaattcacc ttcaagtctt aaactgtgat 180
aattgatagc tttcactttc ataatcactt tatgtgtgtt ctcccactct aagtctccta 240
aaccgtcggc ctcttccaga ttttacttct tttccttcct tttctatact acgacctctg 300
ggttgcattg cttgcagttt ttattctttc ttattattct tcaggactta ctatgcagaa 360
gccagcctca catcatccct accatccata ccctccattc ttactccgtg tcatggagaa 420
acctgcatag tgctggggat tagtgccaca attaatgtgt agtccgaggt ctcccttaaa 480
tctgtctttg gtccctgtcg gagcagttct gcttagctcc ctccccattt ctccacagtt 540
gctattataa aatctggacc ccttgactca aacctgtcac tgccccatgc ttggcagaat 600
accttgcctg ccacctcata ggaaaaaagg gcatgaactc cctgagcttt cccttcctct 660
acttacaaac ttctctgccc tctttcttct gttctttccc ccagtgtgag aaagaggtta 720
ctctgtcttg gcctctgtta gggatagcac tgttctaatc tgtaggaact cgcagtattg 780
gttactcttc ttttccctgc agtacttgcc cattttcttt tttttttttt tttttttttt 840
tttttttttt gagacagagt cttgttctgt caccaagact ggagtgcagt ggcctgatct 900
tggctcacta caacctctgc ctcccggatt caagtgattc tcctgcctca gcctcccaag 960
tagctgggat tacaaacgtg tgccaccacg cccagctaat tttgtatttt ttagtagaga 1020
tggggtttca ccgtgttggc caggctggtc tcaaactcct gacctcaggt gatccgcccg 1080
cctctgcctt ccaaaatgtt gggattacgg gcgtgaccca ttgtgcccag ccttttctac 1140
tgttcttgaa cacaccaggc atagtcctac catagggcac ttgctcttac tctttctttt 1200
tctttttttt ttttttttga gacagccttg ctgtgtctcc caggctgggg tgcagaggca 1260
caatctcctg ggttcaagca attctcctgc ctcggccacc tgagcagctg ggattatagg 1320
cacccaccac caagcccagc taattttgta tttttagtag agacggtgtt tcaccatgtt 1380
ggccaggctg gtcttgaact tctggcctca ggtgatccac ctgcctcagc ctcccagagt 1440
gctgaggtta caggcgtgag ccaccgcacc cagctgctct tactctttct tctgcctgga 1500
atacttttcc cctgaaatct gcttggctaa ctcctttacc tctttcaagt ctttgcttaa 1560
atctcagcac atcagcaggc ccaccctgac ctcatttatg cacctgcaca gcaatccttc 1620
ccaccaccac aatcttgaac cccctgactt tgctgtactc ccccgtgatg ctgattacat 1680
tctcgattct ctatacctga tttattaatt ttaatgtcct ccctctccct actagaatgt 1740
aaattcctcc atgacaagga ggtttgttta tcttttttgt tcacggatta tttttcccag 1800
gaactgcctg ttgcattgta ggcacccagt aaatagctat tgaataaagt atagaaatgt 1860
gcaagtcttt cctattacaa ataaagggct gggggaggga aaatccctcc ttcgtttctg 1920
ttccttccaa taaaatcctt cattcctatt tgctctctcc tgctcatcat agccaggctc 1980
tgacagtgtc atggatactg gcatttccca cttccgtgcc aacccactac aggctggctt 2040

cagtgtcccc tcctgccccc cacttcatgg atactgccct tgctgtggtt tccagccttt 2100
attgctgaaa ccaggaggca cacttcacac ttttgcagta ctaagtatgt tactcatttt 2160
gtttttttct tccttgaact tttggctact tctgtttgtg ggctggtttc agtcttctga 2220
aactgctgtt tttctggcta ctcacatggc tattaaattc attcaacata aatctttgtg 2280
caactttttt tttttttgag acagagtctc gctctgtcat ccaggctaga gtgcagtggt 2340
gtgaccttgg ctcactgcaa ccttcatctc ccgggttcaa gtgattctcc tgcctcagcc 2400
tcccgagtag ctgggattac agtcatgtgc catcacaccc agctaatttt tgtattttta 2460
gtagagacag cgtttcatca tgttggccag gctggtcttg aactcctgac ctcaagtgat 2520
ccacccgcct tggcctccca aagtgttggg attatacgcg tgagccacga tgcccagccg 2580
atctttatgt aacttttaaa agcttgctgt gtcgtttggt tttatttgtt tttaggaact 2640
agaactttga ctacacaaca tatttacagt gttaaatagc agaataggct attacaggaa 2700
gtttttattt atttataatt tataccagtt actacttcta gaaaggattt gagcagctta 2760
ccacaaatat atacagtgaa gctattaaaa tataaataag taaaaattta tgtaacaaga 2820
aaaactaaat gtataggttg cctcggctaa tgtggttatt gtaatttaat ttggaacatg 2880
gaatttaatg caaaaaggga aagataaatt gtacaatttt cactctggta aaagaaagcc 2940
taatagtttg tcgagagaaa ccttttttcc tagtattaaa ttctaaagat gttctaagag 3000
aaaattgtta taaacatcct gatataaaga tacgtacggc cgggcgcggt ggctcatgcc 3060
tgtaatccca gcactttggg aggccgaggc tggtgcatca ctgaggttgg gagtttgaga 3120
ccagcctaac caacatggag aaaccccatc tctactaaaa atacaaattt agccgggcat 3180
ggtggtgcat gcctgtaatc ccagctactt gggagactga ggcaagagaa tcacttgaac 3240
ctgggaggcg gaggttgcag tgagccgaga tcatgccatt gcactccagc ttgggcaaca 3300
agagcaaaac tccatcttaa aagtaaaaaa taggccaggc cttatggctc acgcctgtaa 3360
tcccagcact tcgggaggcc gaggcaggcg gatcacctga ggtcgggagt ttgagactag 3420
cctgaccaac atggagaacc ccatctctac taaaaataca aaaaaatcag ctgagtgtgg 3480
tggtgcatgc ctgtaatccc agctactcgg gaggctgagg caggagaatc acttgaactc 3540
aggaggcaga ggttgttgta agctgagatc gtgccattgc actccagcct gggcaacaag 3600
agtgaaactc acctcaaaaa aataaaaaat aaagacttac tagggctggg gacagtggct 3660
cacacctgta atcccagcac tttgggaggc ctaggtgaga ggattgcttg cgcccaatag 3720
ttcggtatca gcctgggcag tgaagcagga tctcatctct attaaagata aatttagtgg 3780
atgggcgcgg tggttcatgc ttgtaatccc agcactttgg gaggctgagg caggcggatc 3840
acctgaggtc aagagttcga gaccagcctg accaacatgg agaaactccg tctctactaa 3900
aaatacaaaa ttagttgggt gtggtggcac atgcctgtaa tcccagctac tcgggaggct 3960
gaagtaggag aatcacttga acccgggagg tggaggttac ggtgagccga gatcgcgagc 4020
cattgcactc cagcctgggc aacaaaagcg aaactccgtc tcaaaaaaaa aaaaaaaaat 4080
taggtaggtg tggtggcatg tgcctgtagt cccaataact ccagaggctg agggaggagg 4140
attgcttgag cctgggagat tgaggctgca gtgaactgtg attgttccac tgtactccag 4200
cctgggcaac agagtgagac cctgtttcaa tagatagata gatagataga tagatgatag 4260
atagatagat agatagatag atagatagat agatatgtac taaatgtttt caacagcttc 4320
tttaagtaaa aggaagaaat gttcttcaaa tacctttaca aaataaaata aaaaataaac 4380
agggcagtga tgcatgccta tagtacccag ctactcctga ggctgaggta cagccaagga 4440
gttttgaggc tgccatgagc ttcaatcaca ccactcactc caacctgggc ggtgaaaaag 4500
tgagacccct tttctttgtt tcttttcttt tttgagacag agttttgctc atgtcgccca 4560

ggctgtagcg caatggtatc atcttggctc actgcaacct ccgcctccca ggttcaagct 4620
attctcctgc ctcagcttcc tgagtagctg ggattacagg tgcctgccgc caagcccagc 4680
tgatttttgt gcttttaata gagacggagt ttcaccatgc tggccaggct ggtcttgaac 4740
tcctgacctc aagtgatcca cccaccttgg cctcccagag tgctgggatt acaggcatgt 4800
gccactgtgc ctagccagat agtagttaaa acatttgggg agcatgactg agcctggtta 4860
gccatattgg agattaaaaa tctgatatgt caggagacta ctcgtgtata gtctgattct 4920
tggattgggt tagaatacag aaagaagctt tttttttttt tttttttttg acatgggagt 4980
ctcgctctgt cactgaggct ggagtgtaat ggtgcgatct tggctcactg caacctccac 5040
ctccgaggtt caagcaattc tcctgcctca gccttctgag tagctgggat tacaggcatg 5100
tgccaccaca cccagctaat tttgtatttt ttagcagaga tggcatttca ccatgttggc 5160
caggctggtc tcgaactcct gacctcaaat gatctgtccg cttcggcctc ccaaagggct 5220
gggattacag gcatgagcta ttgcgcccgg cctccttttc taatatatga ttcattttga 5280
acttccacag cgtggcccat ggttaacttt tattcagagt caaaaatcaa gaataaatag 5340
tgactacata cagacccaca cacacaccaa ttcttaaatt ttggggggcc attttatcag 5400
tatatccatt gtcagcagca cagtgttagg tccgaaaacc tgcttggtct tcatggaaag 5460
tattggactg agagatgcaa aatgtgatat aaggaggact ttttcatagc atctcaaaag 5520
gggatctcta gctataaaaa tttatttttg gatacatatt cattctttaa attataattc 5580
acactttaaa atgtaattca gaggaaaatg ttatatttta aatagctact tgtttcaaat 5640
ttgtttttgt ataacaaact gtgctaatat tgaattaatt aattaattta gacagtgtct 5700
tgctctgtcg cccaggttgg agtgcagtgg tgcaatcttg gctcactgca acctgtgcct 5760
cccaggttca agcgattctc atgcctcagc ctcctaagta gctgggacta caggcatgtg 5820
ccaccatgcc tggctaattc tagtatggaa tttaaaagac tgcatttctg tttaattcta 5880
acaaatgtac tgaataaagt attagtatat gagaggtaag ggaactactg actgcaacat 5940
aggggcagaa tgaaataaat gggaaagtga ggaatcagtc gaataatttt atggagcaaa 6000
ggaaaaaaaa ggtatgccct tttactcaga aaagcagacg gatattaaga aatgaattaa 6060
cgcatcctgc tccatggtct catttgacct tgtccttgaa agatgtgaac ttgtaatagg 6120
ttgagaaggg agattgggct cctaggaatc catgtgtata gtgtaggaag gtggtggcta 6180
gtggagaaga aggcttagag ttgggcaagg aaagctgaga caggggaggt aggttggatt 6240
gtggaggccc ttgaactttt aggttatgct ctgaggctgt ggcaaccaac tgaaggtttt 6300
taaggagagg caatggcatg aactgatcat ttttttagga atataatcct ggctgttttc 6360
tatggaaaag gaatgctaca aacacaaacc aaccaaaaaa ctagt 6405

<210> 65
<211> 4067
<212> DNA
<213> Homo sapiens

<400> 65
ccatggtgac cagactggtt tcaaactccc gacctcaggt gatccgtcca ccttggcctc 60
ccaaagttct gggattacag gcatgagcca ccacgcctag cctagtacca tctttataag 120
gttattgaga agattgtaat aattcatgtt taacattaag aacacttcct gatacaaaat 180
aagcttaaaa accggggtag ataggtagga gagaacattc tagtagagag aacagccagt 240

gctgtgcctt tataaatatg gggcatattt tgaactagtt aggatttttt tttttttttt 300
tttttgagat ggagtctcgc tctgtcgccc aggctggagt gcagtggcgc aatctcggct 360
cactgcaagc tccgcctcct gggttcacac cattctcctg cctcaggctc ccgagtagct 420
gggactacag gtgcccacca ccacacccgg ctaatttttt gtagttttag tagagacggg 480
gtttcaccgt gttagccagg atggtcttga tctcctgacc tcgtgatctg cccaccttgg 540
cctcccaaag tgttgagatt acaggcgtga gccaccgtgc ttggccatta ggatttctta 600
taaacataaa tgtctactga aaaaattatt attattatta ttattactga gacagagttt 660
cactcttgtt gcccaggccg gagtgcagtg gcgagatctt ggctcactgc aacctctgcc 720
tcctgggttc aagcagttct cctgctcagc cttccgagta gctgggatta cggcatgtgc 780
caccatgcct agctaatttt tgtatcatta gtagagatgg ggtttcacca gtcagccagg 840
ctggtctcga actcctaact cacccgcctc ggcctcccaa agtgctggga ttgcaggcat 900
gagccaccgc gtccagccaa aatatattat tttttaatgt gccttccgta attagttttt 960
gtcacaaaat gaacagaacg atagtatgtg aatctcatta attagccatt tgttgtggga 1020
aatggtgatg atatacagtg tcctttttaa aagctcgtaa gtagcctggt ttggggaggg 1080
ggccaattca atagatgatg ttgatatttt gactatcaat tgtggtcaaa atgcagttaa 1140
agttttttgt ttgtttgttt ttgttgttgt ttttgagatg gggtctcgct gtgtcctcca 1200
ggctggagtg gagtggcaca gtcttggctc actgcaacct ccacttcctg ggctcaagtg 1260
atcctcccac cttagcgtcc cgagtaactg ggactacata tgccctccac taagtccggc 1320
taatttttgt gtttttagca gaaacagggt ctcactatgt tgcccggact ggtctcaaac 1380
tcctgagctc aagtgatcca cccgcctctg cctcccagag tgctgggatt acaggcatgt 1440
gccaccacac ctggcccagt taaacttttt agattcatag ggctatggga aatcttttca 1500
tgttagtttt ggttactaaa gcacagccac agctgttagg gaaggtcatc ctcatacctt 1560
aactgtcaga gtctgagaat tttgtattca ttggtagttt gaccagcctg tcaacttatt 1620
cttgaagaaa tcctcaagat aaagtattaa catttttttt ctacctgatg atttttaggt 1680
aaagcagcag ctttctcttt ttgctataat aattccatgt aatgtatagt ttttccccga 1740
ctccccagct ttttgagaca tttgaaacag agaagctgaa aaaaaaacta taatgaactt 1800
ttgtataccc atacttatat atttttcctg aattatgtga aaattataga tatcatggca 1860
gtttacccca aatacttcca tgcttatctc ttaaaatcaa gaatattctt tttttttttt 1920
tttttttttt ttaagacgga gtctcactct gttgcccagg ctagagcaat ggtgcaatct 1980
cagctcactg caaactacac ctcccgggtt gaagtgattc tcccgcctca gcctcccaag 2040
tagctgggac tacaggcacc tgctatcatg cccagctaat tttgtgtttt tgtagagatg 2100
gggtttcacc atgttggcca ggctggtctt gaactcctga cctcaggtga tctgcctgcc 2160
tcggcctccc aaagtgctgg gattacaggc atgagccact gcatccagcc aggaatattc 2220
tcctaatacc actatcccat ctaggaaatt taatactgat tcaatattga ttcaatatta 2280
tatcatgtac agtccatttt caaatttcgt taattaaatc aaaaattagg aaagagagtg 2340
ttttaatcca agatccagtc aaggtttata tatttcattt ggttatgatt ttttaaatct 2400
ttcattatac tggtattttt gaattgtcta ggccacattc tgatttgtct gattatttcc 2460
tcagtattag attcaagtta aatctttttg ttaagaatgc tataaagggg ccgggtgcag 2520
tggctcacgc ctctaatccc agcgctttgg gaggccgaga cgggtggatc acctgaggtc 2580
aggagttcaa gaccagcctg gccaacatgg taaaaccccc tctctattaa aaatacaaaa 2640
tttaactggg catggtggta cacacctgta gtcccagcta cttgggaggc tgaggcagga 2700
gaattgcttg aacctgggag gcagaggtta tagtgagctg agattgtgcc actgcactcc 2760

agcctgggtg acagagcaaa actccatctc aaaaaaaaaa aaaaaaaaat attttaaggg 2820
ggggccaggc acgttggctc actcttgtag tcccagctac ttgggaggct gaggcaggag 2880
gatcacttga gcctgagagg tcaaggttat agtgagctat gattgtacca ttgcacccca 2940
gcctgggtga cagagggaga ccctttctcc aaaaaaaagg aaaaagattg ctataatggt 3000
gatgttgtgt acttcctgtt gcatcacatc agtagacata aaatgtcagt ttgtcctatt 3060
atcaataatg ttaaagtttg gtaaatttgg ttaatgtgat gtttcccagc tctctgcatt 3120
gtaaatatat catttccctt cttaaataga tatctgggca tggactttgg atcgtatgca 3180
tatcctgttt cccatgcatc ttttactcaa ttattttagc tttttgttgt ttgttgctat 3240
tgttgttgtt tttaagatgg ggtctcttgc tctgtagccc aggctggagt acagtggcac 3300
gatctcggct cactgcaccc tccgcctccc gggctcaagt gatcctccta cctcagcccc 3360
tctagtagct gggactacag gtgtgtgcca tcacacctgg ataatttttg tattcttttg 3420
tagacatggg gtttggccat gttgcccagg ccagtctgga attcctgggc tcaagcagtt 3480
tgcctgcctc agcctcccaa agtgctggga ttataggcat tgagtcactg tgcttggcct 3540
attttagtat ttattatcct ttcttgattc agttattaca tctggggttg caatatggtg 3600
attttccaat tgccatttct tctgcattta ttagccacct tttttttgtt tttttgtttt 3660
tcagatggga tcttgctgtg ttgcccaggc tggagtggct attcacaggc ctgatcatag 3720
cgcactacag ccttcaactc ctgggctcca gggatcccag gggctgggca tggtggctca 3780
cacctgtaat cccagcactt tgggagataa tttaactttc tcagaattag aaggctagta 3840
agaaaaagtg ccaggacccc aaacccaagc ctctttagcc tcgagtagct gaaatgaaca 3900
ggtgcatgcc ccatgcctgg taattagcta gcttttaaaa aatttttatt taaagaagag 3960
tttccccttt ttctctcctt ctttcagtat caccatggaa tcatgaattc tttctatatt 4020
caatgtactg tgctattttt ctgttgattt attggccagt gggatcc 4067

<210> 66
<211> 3262
<212> DNA
<213> Homo sapiens

<400> 66
gctagctccc aggcctgttc cagttgagtg gtcatgcgca aagctccttt gtctgtcgtg 60
tagagtgaat ctgcgccaca tagtgggcaa tttcactgtc aggattaggg gtaaaggtgg 120
tcagattcgc ggtggtgctg acgacctgac ggaaatcatc cacattgttg agttcatcca 180
gcgtcattaa ctggatgccg atattgccga gcgtcgaggc ttcaacaggc ccggcgatca 240
cccgaatacc gcaggcatcg gcgcatagct ggttgagcag cgtgttctgg cagcctccgc 300
cgacaatatg cagttgcgag aaatcttcac cgcgcagctg cgccagctca tgcaacacat 360
cggcatacag cagcgccaga ctgtcgaaaa tgcagcgcgc cagttcagca tcactttccg 420
ggatcggttg cgccgtttcc cgacacgcag cctgaatttc gctgcacatc gtctcaggat 480
taataaagcg atcgtcattg ggattgataa tgaagcggca agccggaagt gcctgtgtcg 540
cggagataag cgccggaaga tcgttgattt gctgctcctg aagcactcgc tgaagcagcc 600
ataagcccat aatatttttc agcacccgat agcgaccttc cgccccgcct tcattggtga 660
tgttggctgc cagtgccgtg tcattggtaa atggcgtctg gctttcgaag cccatcaatg 720
accaggtgcc agaagagaga taagcagcac gtgagccgtt taacggcgag gcgataaccg 780

cgctggcggt atcatggctg gcaacggcga ccactggaat ctcattaccc tgcgggcaaa 840
tccagtgacc tatgacatta cccggatgcg tcgggcgacc aaaccaggct ttgttggccc 900
cgctccacgc cagtagcgac tcgtcccagt cgtcgctatt gatattgacc agttgcgtgg 960
tcgtggcgtt ggtatattcc cagttcatct tgccggtcag gcgataactg aagtaatccg 1020
gcatcagcag agcgtgagca atgtgtggaa taagttcagg ttgttgctcc gtcagcgcac 1080
gcaactgata aagcgtattg aagggcagaa actggatgcc gctacgttga taaatatcgc 1140
gtttgccgag ttgttgttgt gcctgcgcca ttaggccatt ggtgcggcta tcgcgataag 1200
caacgggcag gcccacacgc tgaccctgtt ggtcgagcag cacaaagtcc acgccccagg 1260
tatcaatccc aatgctatcg atacgaatcc cttcctcgca caccttgttt aatccaaggc 1320
gaatggcact ttcaaggcta tccacatccc aggtgacata gccgttctga ctatgcagcc 1380
cattgttaaa acgatggatt tcgcgcagcg tcaggctgcg gcattcacgc tcgtaacgcg 1440
ccagcatcac gcgcccactg gatgcgccga gatcgacggc gacacaattg cgaaaggtca 1500
taatgtgatc ctgctgaatt tcattacgac cagtctaaaa agcgcctgaa ttcgcgacct 1560
tctcgttact gacaggaaaa tgggccattg gcaaccaggg aaagatgaac gtgatgatgt 1620
tcacaatttg ctgaattgtg gtgatgtgat gctcaccgca tttcctgaaa attcacgctg 1680
tatcttgaaa aatcgacgtt ttttacgtgg ttttccgtcg aaaatttaag gtaagaacct 1740
gacctcgtga ttactatttc gccgtgttga cgacatcagg aggccagtat gaccgtatta 1800
catagtgtgg atttttttcc gtctggtaac gcgtccgtgg cgatagaacc ccggctcccg 1860
caggcggatt ttcctgaaca tcatcatgat tttcatgaaa ttgtgattgt cgaacatggc 1920
acgggtattc atgtgtttaa tgggcagccc tataccatca ccggtggcac ggtctgtttc 1980
gtacgcgatc atgatcggca tctgtatgaa cataccgata atctgtgtct gaccaatgtg 2040
ctgtatcgct cgccggatcg atttcagttt ctcgccgggc tgaatcagtt gctgccacaa 2100
gagctggatg ggcagtatcc gtctcactgg cgcgttaacc acagcgtatt gcagcaggtg 2160
cgacagctgg ttgcacagat ggaacagcag gaaggggaaa atgatttacc ctcgaccgcc 2220
agtcgcgaga tcttgtttat gcaattactg ctcttgctgc gtaaaagcag tttgcaggag 2280
aacctggaaa acagcgcatc acgtctcaac ttgcttctgg cctggctgga ggaccatttt 2340
gccgatgagg tgaattggga tgccgtggcg gatcaatttt ctctttcact gcgtacgcta 2400
catcggcagc ttaagcagca aacgggactg acgcctcagc gatacctgaa ccgcctgcga 2460
ctgatgaaag cccgacatct gctacgccac agcgaggcca gcgttactga catcgcctat 2520
cgctgtggat tcagcgacag taaccacttt tcgacgcttt ttcgccgaga gtttaactgg 2580
tcaccgcgtg atattcgcca gggacgggat ggctttctgc aataacgcga atcttctcaa 2640
cgtatttgta cgccatattg cgaataatca acttcgttct ctggccgagg tagccacggt 2700
ggcgcatcag ttaaaacttc tcaaagatga tttttttgcc agcgaccagc aggcagtcgc 2760
tgtggctgac cgttatccgc aagatgtctt tgctgaacat acacatgatt tttgtgagct 2820
ggtgattgtc tggcgcggta atggcctgca tgtactcaac gatcgccctt atcgcattac 2880
ccgtggcgat ctcttttaca ttcatgctga cgataaacac tcctacgctt ccgttaacga 2940
tctggttttg cagaatatta tttattgccc ggagcgtctg aagctgaatc ttgactggca 3000
gggggcgatt ccgggattta acgccagcgc agggcaacca cactggcgct taggtagcat 3060
ggggatggcg caggcgcggc aggttatcgg tcagcttgag catgaaagta gtcagcatgt 3120
gccgtttgct aacgaaatgg ctgagttgct gttcgggcag ttggtgatgt tgctgaatcg 3180
ccatcgttac accagtgatt cgttgccgcc aacatccagc gaaacgttgc tggataagct 3240
gattacccgg ctggcggcta gc 3262

<210> 67
<211> 13927
<212> DNA
<213> Homo sapiens

<400> 67
actagtgggt aggcctggcg gccgcctggc cgtcgacatt taggtgacac tatagaagga 60
tcatctgtgg aatgttgctt gtagggtcca ggtctggagt atgctgtgtg tccagagcaa 120
cctagattga agaaaagacc tcaggcacag aagctgtaaa agaggccttg aaaagcagct 180
tgaaaaggtg gattatagga agtaaaacaa tatgaagtct ataatacttt taaaagagtt 240
attttctgac actagtcctc aaaaatattc ctggagtcca ttctttgcag gaaaagtggc 300
gacttgtcct agacaaagcc tacgtgctga ttccatgtga ccctgggggt gggtactaaa 360
ttcgtaacag cttgtgggtg aaaatatggt ttatatgttg aatgtgttc[?] ttaattactg 420
taattctgtt tttatgtgca tggtttctgt gaaacaaatt agaagtccaa cacactattt 480
tagctcagtt gaccttgata caagaactgc tgtgttaatt atgcccagtc tttcagtagt 540
acaatcctct tttttttttc tgtgaatgtt agtaaaatct actggtattt tatgtatata 600
ttgcacagct atacactgtt tttcttttta tgctcttgaa aatggaattt aatcagagtt 660
gtacatgaca catggagggc attcatttga gtccttggta tagggcagta tagaggatcc 720
ataagacatg gtccaagcac tttgaggata tatgatgttg ggggagcatc agagatttgt 780
tgacacattt aagtaaggac ttgaatgtcg tactttgact gttggcaaga aataggctct 840
tggctgggtc cagtggccca cacttgtaat cctagcactt tgggaggcca aaacaggaga 900
attgcttcag cccaggagtt cgagaccagc tggagcaacc tagggagacc ctgtctctac 960
aaaaaaataa acaaattagc cgtgcatggt ggcacgtgcc tgtggtccca gtcactcagg 1020
aggctgaggt gggaggatca cttgagccca ggaggctgag gatgcgtgag ctgtaatctc 1080
accactgcac tccagcctag ggcgacagag caagaccctg tctctaaaag agaaaagaaa 1140
cagaattctt acattgtgct ttagtttatc agttagtgaa cttttctttt tttgttgttg 1200
ctttttattt tttgtggaga tggggtctca ctatgttgcc caggctagtc tcgaacgcgt 1260
aggctcagat gatcctccca ccttggcctc ctaaagtgtt gagattacag gcatgagcca 1320
ctgaacctgg gcctggtgtg tttttcttac cttggtttaa agatcaaatg gaatattatc 1380
atacctactg aatattgaga gtttaaaaca gggtaaaata cgtatttaag aaaaattaac 1440
gtatgtgtaa gtgtttcact cctttaattc tgtaaactag gtattacctc aattttagag 1500
acgacacaga cgtgcagtca tacccaaggt cgtaaaaaac acctaaatac agtatttctt 1560
aacttttttt tttttttgga aacggagcct cgctctgttg cccagggtgg ggtgcagtgg 1620
tgcaatctca ggtcattgca acctctgcct tctggtttca agcagatctc ttgcctcagc 1680
ctcccaagta gctggggtta caggtgccca ccaccacgcc cagctaattt ttgtaaatac 1740
agtattttta aattgttctt tcatatgttt agaatattaa gccaggcatg gtagcccatg 1800
actttgggag ggcaaggaaa gaggattgct tgagccaaga gtctcagtcc agcctaggca 1860
acatggcaaa gccccatctc tgcaaaaaaa attaaaattt ggcttggcat ggtagtgcac 1920
acctataggc ccaattactt gggaggcaga ggtgagaggt tcacttgagc ccaggagttg 1980
caggctgcag taagccatga ttgagccact gtactccagc ctgggtgaca gaccgtgacg 2040
ctgtctgggg aaaacaaatg gattagttgt tgatagaagc agttattcta aaagcagctt 2100

gggggcgggt gcagtggctc acacgtgtaa tcctagcaat ttgggatgcc gaggcgggtg 2160
gatcacctga ggtaaggatc ctgttcaagg ccagcctggc caacatggtg aaaccctgtc 2220
tctagtaaaa tacaaaaatc agccaggcat gatggcgggt gcctgtaatc ctagctactg 2280
gggaggctga gacaggagaa tcgcttgaac ctaggagaca gcggttgcag tgagccgaga 2340
tcgtgccact gcactccagc ctgggcagct gagctagact gtctcaaaaa aataaataaa 2400
taaaataaaa gcagcttggt tttgaagata taagttagga aaatctatta gtttcagaag 2460
ctttaaaagt ctgggagaaa ggaatattga agaagaggaa aaggatttca tgtgaattga 2520
ggaagttaac tcattggtgt agtttttcct ttcttttttt ttttttttac taaggaaaag 2580
agaacaaagc gataaaatct gagtagtctt tcagtcattc ttggtagaat tggtagctaa 2640
aaggctgagt gaaatatggc ttaaaagctt tattggctgg gcgcggtggc tcacccctgt 2700
aatcccagca ctttgggtgg cttaggtggg tggataacct gaggtcagga gtttgagaac 2760
agcctgacca acatggtgaa accctgtctc tactagacat acaaaattag ccaggtgtgg 2820
tgatgcatgc ctgtaatcct agatacgtgg caggctgagg caggagaatt acttgaaccc 2880
aggaggcgga ggttgcagtg agctgagatc ctaccattgc actccagcct gggcaacaag 2940
agcaaaactc cgtctcaaaa aaaaaaaagc tttatcattt attttttggc cctgtcttat 3000
ggtgcagagg cttaaaagtt ttttgacagc aaattttcta gaggctagga gtgtttatta 3060
taaccatgtt tttgagcggt gaggactacc tcagagggca tgccttgtgt catcattgtt 3120
cttattgctg agctaccgaa acctagaatc tgactcacac aatatgacac ttatttccgt 3180
tttcttggta gagtttgtgt ggtcattcat cttttagatc ttttagaaac tacagtcctc 3240
ctagttccca cttttatatt tatttattta tttatttttg agatggagtc ttgctctgtt 3300
gcccaggctg gagtgcagtg gtgcaatctc ggctcactgc aacctctgcc tcccaggttc 3360
aagcaattct cccgcctcag cctcccaagt agctgggatt acaggtgcgt gccaccatgc 3420
ctcgctaact tttgtatttt ttgtagagac ggggtttcac cacgttggcc aggctggtcg 3480
caaactcctg acctcaagtg atcaaccccc ctcagcctcc caaagtgctg ggattacagg 3540
cgtgagtgac cgtgcccagc catcagttcc cacttttaaa tgagaggttc tttgtttttg 3600
tggggttttt gttgttgttg ttgttgttgt tgttgttgct gttgttactt gagacagggt 3660
cttagtctgt catccaggat ggagtgcagt ggcacaatct tggctaactg cagcctggac 3720
ctccctgggc tcaggtgatc ttcctaccta agccccctga gtagctggga gtgcaggcac 3780
acaccaccat gcctagctaa cttttgtatt ttttgtggag atgagatttc accatgttgc 3840
ccaggctggt ctcaaattcc tgtactccag cgatcctcct gcctgagctt cccaaagtgc 3900
tgggattata agtgtgagtc accgagccag gtcataaaga cagttttttt ttgttttgag 3960
atggagtttt gctcttttgt acccaggctg gagtacagtg gtgtgatctt ggctcactgc 4020
aacctccacc tctcgggttc aagcaattct cctgtcttac ttagcctccc gagtagctgg 4080
gattacaggc gtgccccacc acgctgcact gattttttat atttttaaca gagatggggt 4140
ttcaccatgt tggacaggct ggtctgcaac tcctgacctc aggtgatctg cccacctcag 4200
cctcccaaag tactgggatt acagatgtga gccactgtgc ctggccaata aagagaattt 4260
taaacaatgc ctacagtgtg attttagtgg ggtggccaca tacatgtagg tgtgcacatg 4320
tatttccatg aaaatgcctt cctgccttta gccttgacaa tgctgcatat agggaataag 4380
catttaaatg atggtgtctg ggctaagagt gtgaactctg gagccaaacc ctccaaaatt 4440
gcatcctgaa tctgccacta gctagctctg gagccatgaa taacttattt aacctctctg 4500
tgatggttta tctatctgta aaatgggggt aatgaaacat ctggctcatt gagtgcttgg 4560
gagcatcaaa taggttagta ggtctggtgt ggtggctcac acctgtaatc ccagcacttt 4620

gggaagctga ggcatgagga tggcttgagc ccaggagttt gagaccagcc tgggcaacat 4680
agtgagactc ccatctctac aaaaaagaaa aataattagc cgggtgtagt ggcatgaacc 4740
tgtggtccca tctactccag aggctgaggt gggaggatca ctggagccca ggaggttgag 4800
tctgtagtga attgtggttg taccactgta ctccagtctg ggcctcagag ttagatcctg 4860
tctcaggaaa aaaaaaggtt cgtagacaga aagtacttat ggctcagagt aagtgttctg 4920
tgaatgttgg ctgtttttta aatcttgcat ttaattcctc tttggttaga agtatacttc 4980
tttgcttgga ttaccatttg catgtggaga gatgttcctt ctagaaccat gtctccttgt 5040
cacatgatta tgtacagtgt gagctttatt aatactctgt cagcatttga ggttggaaca 5100
taaatgcgtg gctgtaccca gtggaatcca gcaagagaga gaactaagca ccgtagcaac 5160
tgttgctatt tgtgtactga gcaacagctg tgcgagacag atttattttt taatcctgat 5220
ctcactgata gcctgggatg tggccttgcc acatctcaaa ggagtagatt ggttgagata 5280
tccagtcttt gaggagattt catgaccaca tgccataaag attctgtttc atttcaaata 5340
tgaggagcta aagctttgtg tgtatgagtg agggagaaag attaagcttt aaagcattca 5400
tagtcagtcc ctgaaatata gtgtgactgg attaatgaca tgataaatca cttttaatgt 5460
gggtggtgag acttcatgca tttcagtcaa aaaagtaatt ttgctgtaat gtggttcatt 5520
tccaaatttt ggctgctaac ttacctagga tggataaaca ttatcacaga gtatttactg 5580
agcatctact ttataccagg gtcttttctg aatactggta acatgcagtt aattacactc 5640
caaaatgact tagtctgtga ttccactata tactacatta cataaataca cacacactaa 5700
tcatttacat acttgtcaac cttagatttc agaaataaca ctgtatttgt aatgaatgct 5760
tcattaccaa atgcttttct gaagtacata gtaagatctt taaaaagagc tttcatggcc 5820
aggcgcggtg gctcacgctt gtaatcccag cactttggga ggccgaggcg ggcggatcac 5880
aaggtcagga gatcaagacc atcctggcta acacggtgaa accccgtctc tactaaaaat 5940
acaaaaaaat tagctgggcg tggtggcggg cgcctgtagt cctagctact caggaggctg 6000
aggcaggaga atggcgtgaa cctgggaggt ggagcttgca gtgagccgag atcgtgccac 6060
tccccttcag ctagggcgac agagggagac tctgtctcaa aaaaaaaaaa aaaaaaaaaa 6120
aagctgcttt caaatagtgg ttggattcat tcaataactt tctaattttc atttctcttt 6180
tttttcttga gatgaagtct tactctgtca cccaggctgg agtgcagtgc attaatatca 6240
gctcagtgca acctctgcct cccgggttca agtgattctc ctgcctcaac ctccccagta 6300
gctgggatta taggcacaca ccactatttc tggctaattt ttgtgttttt ggtagagatg 6360
gggtttcacc atgttcgcca ggctagtctc gaattcctga cctcaagtga tacacctgcc 6420
ttggcctccc aaagtgctgg gattacaagt gggagccacc gcgcccagct ccatttctga 6480
tgtgttccaa tatttcaggg cttaacgggt gccttgaaat agtaatgtgg gccaggtgca 6540
gtggctcacg cctgtaatcc cagcagttag ggaggcggag atgcgcagat cacctgaggt 6600
caggagttcg agaccagcct ggccaacatg gtgaaaccct gtctctacta aaaataaaaa 6660
aattagccag ttgtagtggt ggatgccagt aatcccagct actcaggagg ctgaggcggg 6720
agaatttctt gaacccggga tgaggtggtt gcagtgagcc gagatcacac cactgcactc 6780
cagcttgggg aatagagcaa gactctgtct tcaaaaagaa aagaaataga aatgtcgttc 6840
atttccaaat tttggctggt aacttaccta aggaggataa acattgtcac agaatattta 6900
ctgagcatct actttatacc agggtctgtt ctgaatactg gtaacacgca gttaatgctg 6960
ttggtctggt ttttcatgtt ccatgtataa attacctctt aaaactatga ttgttttcat 7020
tcattcattc atttttgaga tcgggtctca aaataggctg ggtgcagtgg gctcgataac 7080
agctaactac atcctcaaac ctccaggctc aagccatcct ccgacctcag cttcctaagt 7140

agctgggact gcaggcatgt gccaccatgc ccagctagtt gttgttgttt tttttttttt 7200
tggtatttgt tgtagagaca gggtttcacc atgttgccca gggtggtctt gaactcctga 7260
cctctagtga tccacccagc tcggactctc aaaatgctga aattacaggc atgagccact 7320
gagcccagcc tattaatttt tgattgccct attcagattt agtggtatga atttactgat 7380
tgggatgttt tgaacacaca gaataacatg tttaactaaa gaacttgtaa ttgagtgact 7440
tataaaatga aacattttta tcttctaggt attattaacc caaagaatcc aaaggaagct 7500
ccaaagtcct tcagcttcga ctattcctac tggtctcata cctcagtgag taccctcatg 7560
ccacagcact gccagctcct gcctcctttc ctctttcctt gccgatttgt cttttctctt 7620
agctgagcag atccaagttt tgcttcttaa ctgttgtgta gcatgagttc attctttgat 7680
acagtgatat agtattttca ttcataaagt atttgggttg atggaaagtt tatgtacaca 7740
tatacataaa tatatacaca tatatacata cagaacatat ttagtttaca ccaaatttat 7800
actgaatgta cactaaattt atactgattt agacatttct tgaggattta tttatcagta 7860
tcttaggatc ctcattacca acattattgc ccattgatct taggaaatgc caaataataa 7920
taattattat tattattatt atttttcttc tgagacggag tctcactctg tcgcccaggc 7980
tggagtacag tggtgccatc ttggctcact gcaacctccg cctccgaggt tcaagcgatt 8040
ctcccgcctc agcctcccga gtagctggga ttacaggcgc gtggcactat gcccagctaa 8100
tttttgtatt tttagtagag acaggatttg accatgttgg ccaggctggt ctcgagcttc 8160
tgaccttaag tgatccacct gtgtttgcct cccaaagtgc tgggattaca ggtgtgagcc 8220
actgtgcctg gctggaaatg ccaaattaaa attgcttagg gtactgttaa ttttaagttg 8280
aattttgagg gaatggccag caagattggg aatgaccaca gagaaattgk tttttttttg 8340
gtttgtttgt ttgtttttgg gtaggntgat tttgaactcc tgacctcctg atccacctgc 8400
ctcgacctnt caaaatgttg ggattaaagg agtgagcccc cgngnctggc caattttttt 8460
tttttaattg tagtananca angtgggttt tgaacatttt gaaagatgat accgtgcctc 8520
tactaaaaat tccaaaaaaa aaaaaaatta ggtgggcttg gtggcgggtg cctgtaatcc 8580
cagctacttg ggaggctgag gcaggtgaac tgcttgaacc cagggaggtg gaggttgcag 8640
tgagccgaga tcgcaccact gcactccagc ctgggtgatg gaacatgact gtgtctcaaa 8700
aaaaaaaaaa ggaaagaggc tgcagatcca caagggaaac aaaagattac cagtattacg 8760
aaaatgcaga gtagggccct tcctggaccc ttactcagtg tggaacccca aatgttgctg 8820
cttcttaggg attgttcata gagtttactt ctgcatcatt catctctttg ttacatgttt 8880
ttggttttgt ttttcctgtt ttgtattttt ttttaaagac tgagtctcgc tctgttgcct 8940
aggctggagt gcagtggcat gatctcagct cactgcaacc tacacctcct gggttcaagc 9000
agttctcctg cttcagcctc ctgagtatct gagattacag gcaggtgcca ccacacccgg 9060
ctgatttttt taatttttag tagagatggg gtttcgctgt gttggccagg ctggtctcga 9120
attcctatcc tcaagtgatc tgcccgcctt ggcctcccag agtgatggga ttacaggcat 9180
gagccactgt gccctgccct ggggaatcga tgttttaaac tattgacaaa gcctaattgt 9240
caagggaagg gactgagaag gggattggaa atgctgtttc ctgaaagctg tgtttattga 9300
gaaatagtcc cagtagctgg agtgagttca aagaagtatt tttatatgtg gacttgacct 9360
ttggtctttt attctcattt tccacttaag aaaatcttgg ctgtgtgaat gaaagagtga 9420
tacttttaag gttatagaaa gtgaaatgta atcatgccag ataattttat atagatattt 9480
ttatgtatgg ctgacctgga tgactctaac agtgcatgtg tttgtgagtg tgtgtgtgtg 9540
catgtgcgtg tatttaatga gaaaagtaaa cttgtgtata ggaggcttaa aaaatgtgta 9600
gggaatttta ggtgactgtt ctgattccag acacttttat tatggaagca atcaagtaag 9660

tataggaaga aatattaata aaaggttatt tatttctcct tttactcttt acagcccgaa 9720
gatccctgtt ttgcatctca aaaccgtgtg tacaatgaca ttggcaagga aatgctctta 9780
cacgcctttg agggatataa tgtctgtatt tttgcctatg ggcagactgg tgctggaaaa 9840
tcttatacaa tgatgggtaa acaagaagaa agccaggctg gcatcattcc acaggtgaaa 9900
aacaaaacaa aacaaaaatc ttctcttcat tattagtgtt agtcttaaat tgctttaaca 9960
gttattttta tttggcgaac atttatgcgg ggattgtttt atgtcaggca caaagatgaa 10020
caacccatta ttttccctca gaggagctca caattgaatg ggaaggattg acatgtacac 10080
atgtctgtca ttaaaggtgg gaaagtcagt gttttgtaat gattttgcca tatatccaat 10140
gccatattat tttgtcattt gaaaagtgtt accagcttgt taaagctctg ttctaagtcc 10200
tggagatggg gggatattgt tgatctgatt ttttttcaaa ttccatgcat agattacctc 10260
tgaagaatgt gattattttt ggtttttttg atagccttat tcggagatac tttcatttta 10320
ttttcctaat ttaatactat gactttaaac ttcaaacact ctgagattct ccctcttttt 10380
tttttttttt agaacagtta gtattattgg gtttgatcct ttattgtttg ggaatgaaga 10440
agcgttctga catagatatt tatttatttt tattttcatt attcgtttca cttaggattt 10500
aatggtgaaa gacataagta tttaaaaaga acttcattat ttacttattt atttgagaca 10560
gggtctcact ctgttgccca tgctggagtg cagtggtgtg atcttggctc actgaggcct 10620
tgtcctccca ggctcaagtg atcctcctgc cttagcctcc agagtagctg ggactaaagc 10680
catgtgccac cacacctagc tcatttttaa attaaaaaaa aaaaaaattt tttttttttt 10740
ttgaggtgga gtctctctct gttgcccagg ctggagtgca atggcgccat cttggctcac 10800
tgcaacctcc gcctcctggg ttcaagtgat tctcctgcct cagcctccca agtagctggg 10860
acaataggca cccaccacta tgcctggcta atttttgtat ttttagtaga ggcggggttt 10920
caccatgttg gtcctggtct ggatctcctg acctcaggca atccgcccac ctccgcctcc 10980
caaagtgcag ggattgcagg catgagtcac catgcccggc ctttaaaatt ttttttttag 11040
agacagggtc tcaccatgtt gtccaggctg gtctcgaatg gacctgggct ccatcctggg 11100
cttgggatga tctgcctgcc tcgacttcca gaagtactgg gattacaggc atgagccact 11160
gtgcggggcc taaaaactta agaaaaaaaa aagattaagt gagaagattg tatttaaaat 11220
tttcctttta tagtcatgca tcacttaagg gtgggggtac attttgagaa atgcatcatt 11280
agtcgttagg tgcttttgtc attgtacaga tatcatagag tgtacttaca caaaccctgg 11340
aaggtgtaaa ctgctccaca cctagactgc gttgtatagc ctgttgctcc aaggctataa 11400
acttgtgtag catgctactg tactgaatac tacaggcaat tataatagga tggtaagtat 11460
ttgtatatct aaacatagaa aagtacatta aaaatatgat ataaaagatt tgaaatggca 11520
cacctgcata gtgctcttac actggagctt gcagggctgg aagttgctct gggtgagtca 11580
gtgagcaagt ggtgagtaaa tgtgaaggcc taggacattg ctttgcagta ttgtagactt 11640
tataaatgct gcacacttag gctacactaa atttagtttt aaaaacagtt tcctttcaat 11700
aattaacctt agttaaatta ctataacttt tttactttat aaactttaaa attttttaaa 11760
ctttctgatc cttttataat aacctttagt ttaaaacaca aacagcccca gcatggtggc 11820
tcatgcctgt aatcccatca ctttgggagg ccaaggcggg tggatcactg gaggtcagga 11880
gttcgagacc agcctgacca acatggtgaa accccgtgtc tactaaaaat acaaaaaaat 11940
gagccaggca tggtggcgca tgcctgtaat cccagctact tgggaggctg aggcaggaga 12000
attgcttgaa cccaggagac ggaggttgca gtgagctgag atcgcgccat tgcactctag 12060
catgagcaac aagagtgaaa ctcggtctca aaaaaaaaaa aaagaaacca cacacacact 12120
ttgtacagct gtacaaaaat attttctttc ttcatatctt tattctataa gcttttttgt 12180

attttttaat tttttttttc tttcttgaga cagagtcttg ctttgtcacc caggctggag 12240
tacagtggcg ccctatcagc tcactgcaac ctccgcctcc tgggttcaat taattcttgt 12300
gccttagcct cctgagtagc tgggactaca ggcacgtacc accacgtctg gctaattttt 12360
gtatttttag tagagacggg gtttcacaat gttggccagg ctggtcttga actcctggcc 12420
tcaagtgatc tgcctgcctt ggcctcccaa agtgctgaga tttacaggcg tgagccactg 12480
cacctggcca aatttttttt tttttttttt agacagagtt tcactcctct tgcccaggct 12540
ggaatgcagt ggcacgatct tggctcactg caacctccgc ctcctgggtt caagcgattc 12600
tcctgcctca gcctccagaa tagctgggac tatttttgta tttttagtag agatggggtt 12660
tcaccatgtt ggccaagatg ttctcgatct cctgaccttg tgatctgcct gcctcgtcct 12720
cccaaagtgc tgggattaca ggcatgagcc accttgccca gcccaaattt ttaatttttg 12780
aaaaaaaatt taaacttttt tgttaaaaac ttaggcacac atttattagc ctaggcctac 12840
acagggtcag gatcatcaag atatcactag gtgacaggaa ttttccagct ttcttataat 12900
cttatgggga tcactatcat atatgcagtc catcgttgac cgaatcatgg ttatgcagcg 12960
catgactgac tttattcaaa gcattaatcg tatcttgatg tttatgacat aatatatttt 13020
gagatggagc aagaaaggac gtctggctaa ttcattgagc acgcgtggat gacttagtac 13080
tcctctcatt tgtgctcttc atgcctctct cattctactt ccctagttat gtgaagaact 13140
ttttgagaaa atcaatgaca actgtaatga agaaatgtct tactctgtag aggtgagtac 13200
agccgtgagt tgacaccgta agcccttgtt ttccattctc tcaagcatca cttaaatggc 13260
tccaaattat gactgtggta cacatcactt caccattcct tcatttttgt cctttcaggc 13320
tatttctggg ttttgggtat aatgaatagt gtcctgcttt aatctttttt ttggggggtg 13380
cgcggtgggg acagggtctc actctgttgc ccaggccctg gagtgcagtg gcgtgatcac 13440
agctcactgc agccttgacc ttctgggcca aagcgatctt accccctcag cttctcaagt 13500
agctgggaca acaggtgtgt gccaccacca cccctagata attgattttt ttgtagagat 13560
ggagtttccc tatgttgccc aggttggtgt tgaactcttg ggctcaagca atcttgcctc 13620
ctccacctct gaaagtgctg gggttgcagt gtgaatcact gagcccagcc acaattaaat 13680
ctttgttcat aagtctgtgc atttatttgc agttattatc ttttgtatgt ttcttggttt 13740
ttaaataata catttgaatt gccaaatgcc tttttggaaa gtaatgctgg gtaacagttt 13800
tcagtcacac taggagggtg taagcaccca ttgtgccctt gccaacattt ttctattttt 13860
taatctttgc taatttgata atgtaagtat tctcttactt aaattttgaa tttctttgat 13920
tactagt 13927

<210> 68
<211> 34488
<212> DNA
<213> Homo sapiens

<400> 68
   tctagattcc tttatgtaat gtgtaccaga ctatccattt gaactcagga aattagtttt 60
   aaatcgcaaa tatgtcacta gtatttttgt tagaattctg ttttagatca gcaatgaagt 120
   ttatttggaa cgttaaacct gttatcactg agcaattaat tacttcatag aaatgttatt 180
   tgaaattttg agttttgttt catgggttgc tctggattag tgtcgtggca gtaatataag 240
   aaacgcacca ttcaagccta ccttcagata agattcacct ttgaatggcc ccctcctcct 300
   gtaaaatgct ggagtgcccc gtgctgccta tgaacatatg aaggggaaaa gttcaattcc 360
   tatgaagttc aagaactaca cttccagaga gtattgtttt acaatgttgt attaaaattt 420
   atcttaaaaa atctccgtga tgtgcctatc cagtggctta ttcagaagtt ctcttaagtg 480
   agtaaaatgg attcatatag gaatagaata tggtagaata tgggttagtt tagtaaagca 540
   ttttttttta gtgtaaagtt aaacttatat tccttgaagt tctaataaga ttttcaaggc 600
   ctcttctagt ttaattcttc ttattgatta gagtccgagt ttagatgaat accgtttgta 660
   aaaatgatac cgtattctaa gtattaactt ttccatctcc ccaaatgcca aaaactgctc 720
   tgttccctct ctgtgtcact gctgaacctg aactttgacc cttctcgtat tttccctcct 780
   gcttaccttc agttgatcca ttgatcgatg attactctgg aagtggaagc aaatgtgtgt 840
   atttcacata ttggttattt ccagtactct gacttgctaa tctgcctctg ctggatcagc 900
   cttaaaataa gctttgcatg ctggcaatcc ttacagggaa atcccagaca gagcatagta 960
   tagaatcaga aatgatccta tttttgccct tcttcattta gcaaactaat ctttgctgaa 1020
   tttcagtttg tggcttgagt gaagttgaaa ggcttaagaa tctcttgctg cttctactct 1080
   tcagittttc ccattgtgat tcaattaaat ccaaaaatca tagatgatag caactgacac 1140
   ttactgattt tgcataatat gccaggccct attctaggca ccttgaatat attgtccctt 1200
   ttaattctgt taatggctct gaaaatatct tattatgttc cttttaccaa taatcatttt 1260
   tagtcagcaa aactatctat gatttaagag ctctgtagat gttgggctaa gactcatgag 1320
   tgcttgtgct aagttgcttt tctccataac ttgggcctcg gaatgattag ataaacgttt 1380
   tttccttttt tttttttttt tttttttttt taaacagtct cactcttgtc acccaagctg 1440
   gagtgtaatg acatgatcgt ggcccactgt aaccttgaac tcttgggctc aagtgatctt 1500
   tccagctcag cctcctgagt atctgagact acagttgttc gctgccacac ctggctaatt 1560
   ttttaatttt ttatttgtag aggtgagggg tctcgctgtg tttcccaggc tggtctggaa 1620
   cccctgacct caagcagtcc tcctgcctcg gccttccaaa gtgctaggat tatagatgtc 1680
   agccaccaca cccaaccatt cctcttttca taagtcacaa attaaaataa cttaggtgtc 1740
   taaaatgact caaacatttt taaagagtca catgaacctc attattctct ccacatatat 1800
   cttaagttgg tgaggaaatt atttcttacc cttttaaggt aattcatcaa gaaattgtta 1860
   tcttagaaga aaaatgtctt gattttgggg ctaattttta gatttgtact gagttatttc 1920
   tttttttttc tttttctttt tcttttttga gatggagtct tgctttgtcg ctaggctgga 1980
   gttcagtggc ggaatctcgg ctcactgcaa cttccgctgc ctgggttcaa gcgatttctc 2040
   ctgcctcagc ctcccgagta gctgggacta taggtgcgca ccaccacgcc cagcttattt 2100
   ttatattttt aatagagatg gggtttcatc atgttagcca ggatggtctc aatctcttga 2160
   cctcgtgatc ctcctgcctt ggcctcccaa agtgctggca ttacaggtgt gagccatcaa 2220
   gcccagccac ctgagttgct tatttctaca gtaaggcatg taatgaggct tggcagacag 2280
   tgagaaaatg cataggagaa accaaacata gccctggctg tggttatgag gttagggctt 2340
   aaaggcacta taatgtcctg atacagaagt gactcttgga ctgcaacagc tctgtctgag 2400
   atttccaaag ctcaacgata ctggtgtgga aaaggtgcct tcttcttttt gcttttgtgc 2460
   tcaaggtgtt gctgctgcca gcttgttgcc tacctctttt cttagcagct gcggcatttc 2520
   ctgaaagacc atgcatgact aagttttaat tctgtattcc tgagggaaag cagccctgac 2580
   tccttctcag tttccatttt gatctatttc tctttattcc ttttagggct tgcatgtgac 2640
   cattggacct aaactctgtc catcctggtc ttttctccca atggctaatt ttttgctcta 2700
   atccctatcc cccttttaaa agatgcctct ttgctgttgc cctatgttct gtatttgcag 2760
   atggtggttt tcaaactgtc ttgaagcaac cactggcttt aagggagtta aaaaactggc 2820
   tttaattaaa tagtagagat gagtaagccc agggagaact aatagtattt ttttccagct 2880
   atgtgtattg actcatctcc tttgaggaaa actgactagt tttgatatga attttagttt 2940
   ctcaggttta aaaataagct aattttaaag ttggattgca ccaatactta agatttacct 3000
   ttggtatctt tttgcaaagg atgactaatg aaagaaagta atgaaagaaa gtacaaacag 3060
   caaattaatt aattttgttg tttgtggctt aatttttttt tcttttacca atactgagtg 3120
   taaaagcaaa attaacagtt ctattaaaga cttagtaaat ggaagtatgt tcctaaaact 3180
   tgcattttat actaggaagc aggcttagca gtgacccatg gttgtactgt taggctaatt 3240
   aagggcctta aattagcaaa taatcagcct tattttttgc ttgctctgac agaagaatgg 3300
   aacctgcact aaaactcatt aatctgctaa ttgttcttcc ctgttgaggc aatcaattag 3360
   tcctgagttt ttaaccagga cttatacaaa attcctctca tttctataat tacccctttt 3420
   ttatttaaga catattaaaa atccttttag catgttgaaa tgttgccata caaagcatac 3480
   tactcagtgc acaaattttg ttttaactgt aacattttct tggaaatttt tgctgaatta 3540
   cacgtaacac tgagccagac caatatggat tattattgat aaataaatga tcattgtttt 3600
   cccttactgt tacaaactac tggaaaagtg taaatctgaa gacattgtag actaggaaca 3660
   ctagaaagac taaatggaag gtagtttggc ttttttaggg agagtgccta agcagtatcc 3720
   tttgaggctc tggtttatcc ccctgcgaga tacagcgtta acctacattg gaatctttta 3780
   gtttgtaaat gcagaaatga ttctgaacct gtaggatacc actattaaag acagacatgt 3840
   tttagaactg ccatctagaa cagttgtaac ctgtgtttgt attatagcgt gtcttggtct 3900
   tggcgcttta atgctgtcct ttcttacaac gatattcctt cctgtctttt tttcttcctc 3960
   tgcttctttc ccttttccct acttttcctg ccttctcttc tttctatctc ccagatctga 4020
   aagattttca gaacaataag catagatact tgctagcctc tgagaatcaa cgccctggcc 4080
   atttttccac agcatccatg gggtccctca cttcatcccc atcttcctgc tcactcagta 4140
   gtcaggtggg cttgacgtct gtgaccagta ttcaagagag gatcatgtct acacctggag 4200
   gagaggaagc tattgaacgt ttaaaggtaa gtaatagttc agactgaata caaggtattc 4260
   tatgtagctc cacaaggaag aactaggagt aaaaatcact aagatttcga ctcagcatat 4320
   ggagaactct ttgactctta gaagtgtcct gaaaattgaa ttttgtgctt tgtaagttaa 4380
   tttcttttca ttagaatgcc tgagtctgac atggccaggc agagttggga gaagtgcatg 4440
   ggcagttcat aggctggcaa ggcagagtaa cagattaata gatgtgtacg ttaattctgg 4500
   gatagtacat caagttacag tgtaattgtt ttgttagaaa tttatttgga aacgtggttt 4560
   cattgaaaga tggaattctg tgtagagcca tgcttctgtt aataaagatg aagtttgggc 4620
   cgggtgcagt ggctcacgcc tgtaatccca gcactttggg aggccgaggc ggggtggatc 4680
   acgaggtcag gagatcaaga ccatcctggc taacacggtg agaccctgtc tctactaaaa 4740
   atacaaaaaa ttagccgggc gtggtggcgg gcacctgtag tcccagctac tcctgaggct 4800
   gaggcaggag aatggcatga acccaggagg cggagcttgc agtgagccga gatcgtgcca 4860
   ctgcattcca gcctggggga cacagtgaga ctccgtctca aaaaaaaaaa aaaaaaaaaa 4920
   aaaagataaa gttagaacat ttgagtagct gttccaggtt tattcagata gttattaaat 4980
   tatatcttct agaagttcac agaaagcctt gatgccctat gattactgta caggaaggtt 5040
   atctcctcct atgctggtac ccatggggtc acagggccat agtgctgcat gtatgcagat 5100
   tactggccta tggctcccag ctttgctgtt ggaggtattt cccagcccct tgctggctct 5160
   gtcaggttcg atcttatctc tttgtatgaa tatgaaagtt aagagcagca gagccttctg 5220
   actcatagct gcttctcttc ctcctcaatt aattcagtgc acagacagtt gtcacctgca 5280
   gtgctgaaag ctgttcagga atcctgccac ccatcagatg actgtgacag catcatctgc 5340
   tttgaagcaa atattttttc tttccctact tgtcacctcc aggaagatag tttgacccat 5400
   accttttagc cattgtgttc aagagtgcta gggtagggga cacagggagg acaaaaaaca 5460
   ctttttaaga gtctgagaag ttgcagtcat gctgccagcc agcagtcttc ttcacagtca 5520
   cttggtattc cccttgactg ccattcatgc aaggacgata gagaacagtg acacattgac 5580
   acatttaata ggtaaaaggc ttgccgaaaa actctttaac actaaagtga attaagcttg 5640
   ataattgaat atgtcagatg ctttcatttt aattttcatg tgaggctgaa aaagatttta 5700
   tatgaagaaa gaggttctgc tttttctttc ttaatcagcc taccttttcc cttgacattt 5760
   tacttacttc aaaagtacag tgtgggccgg gcatggtggc tcacacctgt aaacccagta 5820
   ctttgggagg ctgaggcagg aggatcattt tagctcagga gtttaaggct gcagtgagct 5880
   gtgattgtac tgctgcactc cagcctgagt gacagagcaa gactctatct caaaaaaaca 5940
   tgctttagaa acaaaaataa ggccgggcac ggtagctcac acctgtaatt ccagcacttt 6000
   gggaggccaa ggtgggcgga tcatctgagg tcaggagttc aagacccgct tggccaacag 6060
   tgaaacccca tctctactaa aaatacaaaa aaattaagcc gggcttagtg gctcatgcct 6120
   gtaatcccag cactttggga ggctgaggca ggcagatcac ttaaggtcag gagttcaaga 6180
   ccagcctggc caacatggtg aaaccccatc tcaactaaaa ataagaaaat tagccaagca 6240
   tggtggcaca cgcctgtaat cccagctgct tggagggtga ggcacaagaa tcacttgaac 6300
   ttgggaggcg gaggttgcac agcctggaga ttgcgccatt gcattccagt ctgggcggca 6360
   gagtgagact ctgtctcaaa aaataaataa ataaaaatag aaaaattagc cggccatggt 6420
   gacgtgcatc tatagtccca gccactcggg aagctgaggc aggagaatcc cttgaaccca 6480
   ggaggcggag gttacagtga gctaagattg cgccaatgcc ctccagcctg ggcaacagag 6540
   tgagactcca tctcaaaaaa aaaacaaaaa caaaaataat gtgcttgttt cttccttcag 6600
   aacatgtaag tggaaagtga gtgaagttct gcaacaccta gcaaagggag aaggagtgag 6660
   aaatctttcc attaaatgtt gtaggctgca gggtaacttt tactgttggt aaaattgact 6720
   ttgaaatttt actaaagacc ttagtgaaga ggagtggtaa gactccattc acacattttc 6780
   tcacaatagg cgtcagtgac catgttgctt tgcagggctc ttttcagagc cagagtgaga 6840
   aatagggagc tccttgggaa tgagaagtaa aactcccact tactgagtcc ttaatataaa 6900
   atgttctttt tctttgggga tcttaagtct taactatttg cttattaaag ccttagctct 6960
   tacacttgag attacccttt cctttgacct tcctcttgat gtaaaggaga aatttctccc 7020
   taactgccaa gtgggaccaa ttgaaaataa tgtcacatgt taggagtcaa gacattaacc 7080
   agactcttca agattcaggt aacccagtgt cttccaaata atttctaggg aataacactg 7140
   tcattattca gggaactctc cttccgactt ccctatgtcc cactaaagca agacaaaact 7200
   gtattacttc aatcagaaaa caaatttctc tgttttaatt cttccttacg ccaaaataaa 7260
   gaactcttct gttttcccta gttttattct agaaagaaaa cagcttcctt agtgttttgt 7320
   gttgtcttga gtttttaatg cagtgtggta gttcactgaa gcataagcac cttagttctg 7380
   ctcttatttt gtgaatggtg agaatttctt tcggcagacc ctgcatttta atgctgcacg 7440
   cagataacat ctttcatccg tagcgccagg gcaatggttg tgcagtagaa atgcttgaga 7500
   atatctagga actgggttcc taaggcatgg tgctgcagga gaaaaatatt ataatggaaa 7560
   acagcaatag aggaggttag tttattttcc taacatttta tttccctttg tttctttttg 7620
   actttaaaaa aggtcagttg attggatact gtttttacac atgatgttgc tgtggctttg 7680
   ttcactgaat agcctttttc catatgttag gtactcatgt cacaccattg taaagatgtt 7740
   tttttttttt gagacagagt cttgctctgt tgcccaggct ggagtgcagt gacgcgatct 7800
   cggctcactg caacctccgc ctcccaggtt caagcaactc tcctgcctca gccttctgag 7860
   aagctgggat tacaggcatg taccaccaca tctggctaat ttttgtattt ttagtagaga 7920
   cggggtttcg ccatgttggc caggctggtc tcaaacttct gactacaggt gatctgcctg 7980
   cctcggcctc ccaaagtgct gggattacaa gcatgagcca ccatgccagg tcttcctgtc 8040
   aaataaggtt ttaaacatgc agctgtagcc acagctctcg tgtttattag tctggcatca 8100
   cagtcagtac tgtgagtgcc gttgcttagg cttagcctat aaatatccac aaatatccat 8160
   tacagaagca ggcaagggca aatgctgtgc taagcagccc aagggccaca tttggaaccc 8220
   acttaggaag catgctatgt tcagtacagt aaaaccttgg tgattcaatg gcttcaacaa 8280
   atgttctgtg tagtaagtgt tttcatggaa aatatttctg acttgtcctg gtgatttctg 8340
   tgtttcctta actgaatcat catcaagaag agattaaatt gtttagctat atgaacataa 8400
   tttattaacc agtgctatac aaataactag agatgcagac agatatgacg tgagaccaaa 8460
   gagtgaatga cacctgagcc tggcattcct cggagcttaa agaagagttc ctggtgtccc 8520
   tctgtttttt ggaatttttt ttttttcaaa gaaagggagg aggattaaag atttatctcc 8580
   tcaacccctc atttctgaac catgaatcca catttcaagt tcatcttaca ttaggcccct 8640
   agagtggggt atgggaagcc ctggacacca gcatgcctgc ctgccagcag aatgagttga 8700
   tgctcctgag actgaatgct gttttgcact tggcttccct atttatacta agtttgctac 8760
   tcaggcttga acagaatatc cttcactttt tcttaactta gggtgaactg tttcatagaa 8820
   catttacttg aaagcctatt gatcctgttt tatttgaaga aagaacagtg agtcagcaaa 8880
   tagctctaga cctgtctcag tgagtagtgt gggctcactt agcttctctg gtctgttgga 8940
   acacagtggc accatcttgg ctcagtgcaa cctctgcctt ccaggttcaa gcgaatctcc 9000
   tgcttcagcc tcccaagtaa ctgggactac aggtgcgtgc cactgtgccc ggctaatttt 9060
   cgtattttta gtagagatgg ggtttcacca tgtgagccag ctgatcttga actcttgtct 9120
   ctggtccagt tttcttatct cgtgaatggt tccagaataa acgattttgc aggctttttt 9180
   cagccctgaa gttgtgctaa tgtgactcac agttattatt ttctctaagt ttcccccaaa 9240
   gtttttaaaa atagctttct agacaaccta aaaattgcag ctgaaaactg taataaaaat 9300
   aaaagttgta ccttactcat taaatctctt ttctgcaaag agatcatgtt ctgatttacc 9360
   cttcagttgg taagtaaaat tagataagtc atattagagt tggctgcaaa atggtaagag 9420
   gattcctaga cagtaatttt gctttcttag agtgagttct acagttgccc caaaatagga 9480
   tggctttaat atgactagat aatcctctgc tctgaaataa cctttttctg aaggtaatca 9540
   actaggaaga aatattttta aaatatctca tatgtcagtt tgtattccat aatcaacagt 9600
   ggtttattgt ggttagtact ataataagta ctagtcaaga tgccttgttt aggtcgggtg 9660
   catcctgttg tcctgtctgt tgaaggtttt cactttgaga agattgtata gatttctacc 9720
   cagctatctt taatagacct taaaattatg taggttatat catcagcttg tcttgtattt 9780
   gcctatttaa aatcatatgc agggtggggc tgggaggaaa agaaaaaaaa ggaaaaaagc 9840
   agtcatatgc aaaagcattt tcttttttta aagaggggct cactgtgttg ctcaggctgg 9900
   agtgtagtgg tgcagtcata gctcacttat gcagaagcgt ttaacaccac tcttagtggt 9960
   tgttgaataa cttttgtggt gctttataga taaactattg aaaacttggt tcttggcctt 10020
   ctcactcctt agggaattgt gtggatttgt cttgctcgct ggccctctag tatgacactt 10080
   ttgttctcca tttgtggggc ggcatgtgca ctgcatggta atcatttctg ctgaggactg 10140
   tgtagagatg ccattcaggt agctgtttta gcctttgagc ctctgtgtgt gttctgaagc 10200
   ctaatgagag ccatttcagg tggtttttgt cacttgccac ccccaactgc agacttgttc 10260
   tagcttcttc caccttgtaa gtataactct tttctgaaaa agtacctcct acatggctgc 10320
   cttactgatg cccaggttcc ttctttaatc tgttttgctc tttgcctacc tttgctggtt 10380
   gttgttgttc ttcatctttc tctttttttt aaattttatt ttaaatcata ccctcctatt 10440
   tgttccacca aactcatata ctactttgct tttgcagctg ctgctttgaa tctttactct 10500
   tgcttttttc acgtgttccc ttgccttgcc tttggtatgt tttttgtttt gaggtgacat 10560
   ccagtcatgt aatattctgg aagtccaaga gatccagaaa gtcaaacact agacaactta 10620
   aggtttatta ctcacaagtc ctttctgtgt agatatttac gttttggggt cacaggcttc 10680
   gttagtaacc tgttatcata tgaatcatac attgaagtgt ttgatgaact ataacacttt 10740
   tccttgttaa tcactactag catcattact atatttcatt atcatcatct gactttctta 10800
   cttcatctcc atgaccagca ttttcacctg ttttggcatg tgctcgtccc ccttttttgt 10860
   actccttttc cttccattct ttgcctaatt gctacttgtc cttcatttct ctgtctgggc 10920
   ctcactcagc cttgattcac ccatgttaca gagacctgtg aatactccag ttctagctct 10980
   tagtacactg ttctcttatc tcctactgta ttcgttctca gtcctcagac atgctgctct 11040
   agcaacacat tattcctaga aaactccagg tactttccca ctctcagcat acccacatgg 11100
   cagcttctgc tcttctttga tctctgctca catatcactg agtctgtgag gcctgtcctg 11160
   acaacctcac tttagattgc tccctcacct acccttcccc atgtgttctt tcctcttgtt 11220
   gctttatttt tgtctggagc acttgccact atctgacata ctgtgtactt attgattaaa 11280
   atggattata tcatggcggg gcacggtggc tcacacctgt aatcccagca ctttgggagg 11340
   ctgaagcggg ccgatcacct gaggtcagga gttcaagacc agcctgacca atatggtgaa 11400
   acctcatatc tactaaaaat acaaaaactt gccggacata gtggtgtgca cctgcagtgc 11460
   cagctactca ggaggctggg acaggagaat cgcttgaacc tgggaggtgg aagttgcagt 11520
   gagccgagat cgcaccactg tacgctagcc tggatgacag aatgagactc tgtctcaaac 11580
   agacaaacaa acaaacaaac aaaataaata ataggattat atcagtctcc ttactacaat 11640
   gtaaatctta tgaaggcaga gattttgttc tctgacatat ccataggtgc cttgaagaag 11700
   gcttgatatg taatggggtt gtatataagt attgaatgaa taaatcatca cttacttctc 11760
   tctctctctc tctggtatac agtaacctcc ttgagagcag gaaacatgtt ttcgttcttt 11820
   ttaaattaaa ttaaatttaa tttaattaat ttattttttt aattttactt tacgttccag 11880
   gatgcatgtg cagaatgtgt aggcttgtta cataggaata catgtgccat ggtggtttgc 11940
   tgcccctatc aacccgttat ctgggtttta aaccctgtat gcattaggtt cttatattta 12000
   tataagtgta aacactcagc aagtgattgg taaattgtca cttcagtgtg tgtcttcaga 12060
   tgcgatttaa tagtaaacct ttctatgttg actgctgggt tgtgttcagg aggttagtgt 12120
   gttatggcga aattgtagtg aaacaaaagc agtaaatacg tttgggaact ttattctttt 12180
   ttttcttttt ttgagatgga gttttgctct gttgctcagg ctgcagtgca gtggcgcgat 12240
   ctcggctcgc tgcaagctct gcctcccggg ttcatgccat tctccttcct cagcctcctg 12300
   agtagctggg attacaggcg cctgccacca tgcccacgtg gatcacgagg tcaagagctt 12360
   gagaccatcc tggccaacat ggtgaaaccc cgtctctact aaaaatacaa aaattaactg 12420
   ggcgtggtgg cacgtgcctg tagtcccagc tgctcgggag gctgaggcag gagaatcact 12480
   tgatccccag aggtgaggtt gcagtgatct gagatcgcgc cactgcagcc tggtgacaaa 12540
   ggaagactcc gtctcaaaaa aaaaaaagac ttggaggatt tatttgtttg gggttgattt 12600
   agttttaggt ctaatcttta tgattcttat cttatgtatt aataaaagaa aaatctcttt 12660
   acatttaatt ctggcctgta tcctcaaagg gcatgatttg cttcttgctt ttctagcatg 12720
   gtatgttaaa ttataagact ctttgcctct ttaactgtgc gcttccttcc ttaggaatca 12780
   gagaagatca ttgctgagtt gaatgaaact tgggaagaga agcttcgtaa aacagaggcc 12840
   atcagaatgg agaggtcagg aggttaaaat ctggaaatgt ttctaagttt tctaggggat 12900
   gtggattact ttatcgtaag aaaagataaa atataaatta aaatcaaaga gtctgccctt 12960
   ctaaaacaca aaacgaaacc aaaaaaatgg acagggattt atggccagtt gaatttatgc 13020
   tttgaaatga catctacata tttaaatatt atctagggag tcacaggttt attagttacc 13080
   atattacatg ttgctaggga ttcataaaaa tggaacatat tggccaggcg cggtggctca 13140
   cgcctgtaat gccagcactt tgggaggctg aggtgggcag atcacgaggt cgggagatcg 13200
   agaccatcct ggctagcatg gtgaaacccc atctctacta aaaatacaaa acaaattagc 13260
   cgggcatggt ggtgggcacc tgtagtccca gctactcggg aggctgaggc aggagaatgg 13320
   cgtgaacctg ggaggcagag tttgcagtga gccgagatgg tgccactgca ctgcagcctg 13380
   ggtgacagca agactctgtc tcaaaaaaaa aaaaaaggaa tagcctcttc ttttcttttt 13440
   tatattaata tgcatatcat ttatgaatta ttgactatcc caaagtcagt tgagacaaag 13500
   ttcttatctc agtgaatata actttaacaa agcaagattt cattgatttt gaatgtgatg 13560
   ttgcttttag aaataacttc tcattaatga tgagataagc acattatttt atgtttgcat 13620
   tattggctat aaacatggcc agttgttttt gctttgcagg ctttattttc caattcatat 13680
   tagactgatt taattgacat tttggtatta gtgttctgat atacctgttt ttttcctaga 13740
   gaggctttgt tggctgagat gggagttgcc attcgggaag atggaggaac cctaggggtt 13800
   ttctcaccta aaaaggtagg aaacaatgct gtgaaaccta atcagacaga gacacttttt 13860
   gtttgtcttt gttactgggg cacttcatgt ctttcaggac ctactctccc ccttattatc 13920
   ttgatacttg ccttaatttc agatatttct gatttcatct tagagcatgg tcttatagta 13980
   caaaggcagc ctatttctaa aatgcctatc ttgtttactg tttacaaaag aaaaaaccta 14040
   aggcaatatg taacctttgg ccatgattca gtttgtttag tcatctttat ttgttgcttt 14100
   ctcttaaatt cccagcctaa cctagcgtac tttcagtttt ggctactctt tcatgtagat 14160
   tttcccacag aagtcgtcta ccctgcttta taactttttg gaaccaattt cagctgtaca 14220
   ggtttgtgga gaataatact gcttccattt gcttctgcaa tttgtattat agacagccat 14280
   tgtactaagt aaagcaattc ctgtccctat ccttctagac tcctctgctt ccttcttcat 14340
   tttttaaaaa gtgtctccaa tagttacctt cctttttaca taatcatagt tatgaagtag 14400
   ttacgtagct tttagctaat tagagtagta agggtttttt tgtttttttc ttaaactaag 14460
   ttgcaacgtc ttgttggata ctagaaaatg actatcaaat ggttattttg tgtgcttatg 14520
   tatcagaaaa taggagaaca tatatcgatg tacaaagcat gtattttatg ccactagtaa 14580
   attgtccttt atttcaagtg acctcacatt ggctattgga gacttaaaca tctttattca 14640
   taggttctgt tgatgatctc tctaagttag gatacaaggg gtggtgagtt ttgaattggt 14700
   acttttatgt gatatgaaat attcctgtgg ttttcgctac aattcttctg gtcaggtttt 14760
   tctatcctgg ccacataact tttttttttt ttttcttccc aaattccaat gtgttatttt 14820
   caacagaaaa gactgatgta aagacatttt agatgcatat tgtagaatag aacagttaag 14880
   tattttatat gtttatatta tgaaaggtta gtggttttca cagttttaat agtttttcct 14940
   tcagtaagac atgtattcat tcagatatgt tacttaggat tatatagatt ttagcagtta 15000
   cagttttggt ggaatttaat tgtgcctatg catatgtcta ataaaggtat tttataacat 15060
   cttacagtat taaagactgt gaggaaattt tttttttttt tttttttgaa gacagagtct 15120
   cactctgtcg ccaggctgga ctgcagtggt gtgatcttgg ctcactgcaa cctccgcctc 15180
   ccgggttcaa gtgattctcc tgcctcagcc tcccaagtag ctggactgca ggcacatgcc 15240
   accacgccca gctaagtttt gtatttttag tagagacggt ttcaccatgt tggccaaggg 15300
   tggtcttgat ctcttgactt tgtgatccac ccacttcagc ctctcgaagt gctgggatta 15360
   caggcatgag ccactgtgcc tggctggaaa atttttatta taacaactag tgaaaatatt 15420
   atgcatttaa cataaatcat aaataacttg aacaaatgtt gagatgtaca ctgcatggaa 15480
   ttccaaagtg agatctctgc ttccaaagct ttttcttact ttaattttac ccatcttgct 15540
   ttagctttct agacttcttc atgaaaggaa gggatatacc agtgaccaca tccaaactca 15600
   gtcttaggac ctgtttctga gattccattt ttcaaattat gatgtgcttt aaagaatgct 15660
   actcacaaag ggctttgact ttctcaaacc agtcaagttc ttcaactaca aagggggatg 15720
   ctagttgaga agatgtggag gtttttcaga tttctcttta accatctttt atagttcctt 15780
   ttcctacccc ctcatctgac tcttctttca ttcttccaca tttgctggca ctctcttgag 15840
   tagattttgg cctcaagacc atgtgactta tttttggatt ccagtgggtt attttcacta 15900
   aagaaggttg gttttttaaa aagattttag aaatattaga gttagatgaa gaggattgtt 15960
   agaagatgtt tatcttatcg aatatcagta attttacttt tttctttaag gtaaagacta 16020
   ataaattaaa atcttttaag gtaaagacaa attaataact gggatcagaa gaactgataa 16080
   aaattatagt gggaagtggt gggggggtat ggggacattt taataaaagt aaatatttct 16140
   tgagattttt acccaggcat ttacaaaaat tataattatt ccgttcagag aattggcctg 16200
   tcattttttc cttgtgcttt gaccttatca aaaattaggt cttgtttttt tatcccagaa 16260
   ctcatgtttt aaaatggaat gccaggcaca gtggctcact cctatagtcc caacactttg 16320
   ggaggccaag gagggtgggt cagttgaggt cagtagttcg agaccagcct ggccaacatg 16380
   gtgaaaccct gcctccacta aaaatagaaa aattagccag gcgtggtggc gaacacctgt 16440
   aatcccagct acttgagagg ctgaggcagg agaatcactt gaacctggaa ggcagaggtt 16500
   gcagtgagtc aaaatcatgc cgctgtactc tagacagagc gagactctgt ctcaaattaa 16560
   ttaattaatt aaaataaaat agaatgcact tggttccgtt tggtgaggcc tgaagagagt 16620
   gttttctctt tattatggaa ccgcaactag ggataaaaag aaatccctct ctaggctctg 16680
   tggagagttg gagaagcctg gcctcttgta ggcccctgtt gttaccacag ctctctcatg 16740
   gtgccctgct ccaaattgat catctgtaat ctttttcaga ccccacatct tgttaacctc 16800
   aatgaagacc cactaatgtc tgagtgccta ctttattaca tcaaagatgg aattacaagg 16860
   tatatttatt tcctgttttg gtcacttcgt gtgttttccc cctcttagat aattgaataa 16920
   ctaaagggaa ggggttgaaa aaattaacgt aatgatttgc tgtatttttt gtctgaaata 16980
   gttacaaact atgctctctt tccaaataat gtgtttttgc cactggagcc agttactatg 17040
   tagtttttct ctgaagaccc taaataattt ttttttcctt taacaaatat acattcctta 17100
   gggattttat ttgactcatg tctttataat actgtatgag ttacattggt atatcagtgc 17160
   tccttgtttc ttattctgat gtagagaggt tgatcatatg tgaggataga attcatagtt 17220
   gaaagcttct gatgcaacaa ggcaatacct tttttgtacg taccaaagga tattctttgg 17280
   aataaagagg ttcattgttt gtagtgcttt ctgtgtctga atttccctgg gaaacacttt 17340
   ctcttgtgtt cagggttggc caagcagatg ctgagcggcg ccaggacata gtgctgagcg 17400
   gggctcacat taaagaagag cattgtatct tccggagtga gagaagcaac agcggggaag 17460
   gtgagcattc ctggctggag cttcagcaac aacattttca ttttatatta tgagaaatcc 17520
   ttaagacttt gtattctctg tctatcagta gtactttctt atacaatcta attctgaaaa 17580
   atggagagac ctgggctgct tatgaatgca gagatggaca aggctgcttt acatgaaaat 17640
   agcttggaca aaagaagccc ttttttactg ccaagaactg agaaggacat aggcaattag 17700
   gcttggtctg gaatgttaat tatttaatag aaaagtaaga aaatagcaga tatcctgggt 17760
   aataggagat ttgaaggaca ttaagtcaac ccagcagaat ttatttttat ctaaagggga 17820
   agaaaaagtc agtcttgatt tttgcctggg ttattaacaa aacaacaatt taatggtttt 17880
   tttctgttat ataagtcact cattccctta tcaaaatatt agcttctcag tctttagttt 17940
   ctggttatta cctatatctc atccttacaa tttctgatgg ttctgagttt tattgactga 18000
   accgtcagag atccctgaaa ctaatatttc ctatcatctt cttaggttta tcaaatagag 18060
   ttaaatgttc ttgtgttagc catggccaca gatagcctct tctattgggg ctagttctgg 18120
   taccccaaaa tgaactactg tatagacaac ttcagccact tgattgattg cagggattta 18180
   ttctacttac tgcaaatcct gataagcaac tgctttccat tatttgattc caatagtttg 18240
   taatgataac attagtttgt gtttgttcct cttagttatc gtgaccttag agccctgtga 18300
   gcgctcagaa acctacgtaa atggcaagag ggtgtcccag cctgttcagc tgcgctcagg 18360
   tgagactggg agaggtttgc catcttcagc aatgtgcaca tggcttctgt gacaactcta 18420
   atttttggct gtttaaaggc tgaagtaata gtcagcatta ggatttttgt tcttgtaaaa 18480
   acaacagctc tgaaagctgt cttttcacat tagggagggg tgaggttgtt aaatagatac 18540
   tatatattaa aaaaattatt tcttaaccct atttttctgt tttgtgctag gaaaccgtat 18600
   catcatgggt aaaaaccatg ttttccgctt taaccacccg gaacaagcac gagctgagcg 18660
   agagaagact ccttctgctg agaccccctc tgagcctgtg gactggacat ttgcccagag 18720
   ggagcttctg gaaaaacaag gaattgatat gaaacaagag atggagaaaa ggtaatgccc 18780
   agttacgcag cccatatgac tgtttcttct tttaaacatg taatactaat agcattcttg 18840
   aatttttttt ttttttttta cttctaggct acaggaaatg gagatcttat acaaaaagga 18900
   gaaggaagaa gcagatcttc ttttggagca gcagagactg gtaggagtcc tgaatctgct 18960
   aaactgttgg gaaaagggca gcttgttccc atactttccc tgttccacag agcagtactc 19020
   acccaaattg cttctgtctc aatgatacca agcactattc tttaatttcc ttaatggaga 19080
   atgaacttaa atctcccggt agccttagcc tgaaaaaata gtccacagag gtactctttt 19140
   gggcttttta tgtcttaaag ccaaatctta acttctgtta caaccaaata ctttttaagg 19200
   aaatagagct tttctggtag cctttgcctc ttgatagtgg ttttggaatt tgcttcagtg 19260
   gtggttcttt aaatgataat tactctgaat attgaatttg gtgagagttt gccttggttt 19320
   tgtttctgat cacttgatag tactaattct ctgctcttgg gctgactttg ggattgttct 19380
   tacgctgggc agactttttt tttttaagtt aaactgtgtc taaaagtgtt gctgcacagt 19440
   tgcatgtgtt actcctttcc ttatcccctg catggagtct gaattctcaa tcaggttctc 19500
   agtggcatgt gtggtagcgg tgggagcaaa ggctgcatac ccagcccgga caggacagaa 19560
   ggcttgcttc ctttgaggga aggaggattt gagtgagcag ctggaaagtc tgtttaaggt 19620
   cccagctatt gatacaatac taatggcttc agcgttttat aatggtaatt gatttcccag 19680
   atttgataga aactgaatct catgaataaa taagttgatc cattagttag attttcattt 19740
   ttaaagccaa ccaaacaacc aattttggtt aacaaagctg tggttataag aaccttctca 19800
   gcattcattt gatatgtaag aataaagtat aactaacctt tggatactgt ggatgcagtt 19860
   taggaagttc tcatttttta agattcagtt gtatctttct tttaaggtaa gggggcatta 19920
   gggagaaagt tcaagcactg gcttctttcc ttagtataaa atacagcatg taaactacag 19980
   ccttctagtc atgctctgtg ctttggagaa gaaggggctt agtgctaagt gggccagaaa 20040
   cgtgaaggac ttgagcacaa gcaacctttt ccagaatacc tcagttctca ggcatgggca 20100
   tggattaaac cagttagttt gttatcttgt aaattatagt aaagccctaa aaagaatgga 20160
   ttgtaagaat aaatcaaggg tttggggcaa gataatagca gtgttaccaa cagaggctct 20220
   ttaggaggcc tttttagcat taagttttta atgaaaacgg aaagaactag ctacaatgtg 20280
   attagtggct tgaaaggttt gtgacacaag aagatgtctt ttatcttaat aatgtagtac 20340
   attctaataa catgcctctg atggtctgtg agagatcaaa tgaggacagg tgtaattaag 20400
   ctctattatt aacaataatg ccttgtattt gtatagtgct ttataattta cagagagcgt 20460
   taacatccat caatttattt gaccctcaaa acaaccctct ttgtttactt atgcatatgt 20520
   ataagtaaaa tatgtaatat tacctccagt tgaggaaacc aaaacttggc tgggcgcggt 20580
   gggtcacgcc tgtaatccca gcactttggg aagcctaggc aggtggatcc tgggacctca 20640
   cttagaataa ctaagcacgg tgctcgcttc ggcagcacat atactaaaat tggaacgata 20700
   cagagaagat tagcatggcc cctgcgcaag gatgacacgc aaattcgtga agcgttccat 20760
   atttaaaaaa aaaaaaaaag aaacaaaaac cacaaaaaca aaacaaaaca agacaaacaa 20820
   aaaaaaagaa taactaagca cggcagaata aggatgcctg gtgctatcca taaccaagag 20880
   ttggtgatca ttctgtgccc taaaataaag atggccacta aataaagaga aagtgaatgt 20940
   ctaagacgta tttagtttct agggaatgta cataccctgc agataatcag attctggttg 21000
   ttgtttgctc tgtgtatctg aaagcaagcc aggatttcaa gagcagcttc ttaaagcaag 21060
   gaagttgctt tcctctcaaa ggtctgtctg ttccactttc atttcttgac ttaagggagg 21120
   aactgatttc taacacttca gcctgaagaa tatctaccag tagtaatact gaatggaaaa 21180
   tgtctttata tattaccctg tattggtcaa ttaacagatg tatttctaat gcaccctgtg 21240
   aataaacatt gagagaatac aaaggaatta ttaatcagta aatatttatt taatatttac 21300
   catatgtaat cactttttgc ataatgtcag cattaaacaa tggaattcag gctcatgagt 21360
   tttctattct gatagttccc aaatgcatca cattgttagg gtgctgagta ttttcttcag 21420
   ttagtgctct aggataccct ggtcctttct gtgcttgctt tttacacaca gttttgggat 21480
   cttctctctt cttatttgca taagttcctt gcagagtctc tttaggaggc ctactttgca 21540
   tttggcattt aatgaaagcc gaaggatcat tttgtgacat gtgaattgac cagacctcag 21600
   tagcttttcc aactccttaa taaatgaatc taaatatttt aaaaatctgg ccaggcgcgg 21660
   tggctcacgc ctgtaatccc agcactttgg gaggccaggg ctggcagatc acttgaggtc 21720
   aggagttcga gaccagcctg gccaacatag tgaaacccca tctctactga aaatacaaaa 21780
   attagcagga cgtcttggtg ggcacctgta atcccagctg cttgggaggc tgaggcagga 21840
   gaattgctta aacctagcag gcggaggttg cagtgagcca agatcgcgca actgcactca 21900
   agcctgggtg ataaagctag actcagtgtc aaataataat aataataata ataataataa 21960
   tatagataaa tataaaaaat tctttttttg acatggagat aggatttata tcacataaac 22020
   tttgcataca gtttttagta tcaccatcaa tatgtgacag ggtgatacat ttgaacttta 22080
   aagaggacat actctacaga gtcaaaatgg cagtatttta aaaagaaaga aaatgacagc 22140
   tgctagattt tacttgagtt gagttctttt ggatttatat agacttattt atattttatt 22200
   ttagagttta aagataactt ttgtctctta agcatgatat taaaacaatt cttagaatac 22260
   ttgtcatttc ctgaattagg taatattcat ttcatcctta agcaatttga agaggaaaag 22320
   aaaaacagcc agaaaaaaag tgatagttgt tggttatgtg ggaaagaatc aaagataatt 22380
   tgtttatgtt ctgtaaaatt cagtttatac atatttgcaa aaaatcctct tcaggagtca 22440
   gacatagttt tttctctaat atttatgaat gtcatattta tcacttaaaa ttagccatta 22500
   tgattttaaa aactaaaaat gaaaagaaaa attaatgtag agtctgtgta aatttagcaa 22560
   acttttgttg gtaagttatc aaataggtgg gagttataaa tgggcttagt actcatttgt 22620
   aggtcaatag aaataataca aatcagtggc aagattttta aattcagagg ccaaattatt 22680
   attattattg tggaaagtta cttttttgtt ccttagaaag tgactataca ggcaaatctt 22740
   tggttttgtc aggtgtgatg atgtgtgcct gtagtcccgg ctaaggcagg aagatcactt 22800
   gagcgcaagt gtttgagacc agtttgggtc cctgtctcaa aaaacaaagc aaacaaagaa 22860
   acaaacaaaa acaacaaact ttggtctagg aggcaaggta ctatttatca gtttcaagca 22920
   aacaaactct tctagagata tttttggtaa ccacagtgaa taaaagattg actgttagta 22980
   actattttca tttactagac tgccctgaac tactcatctg tgagttgagt attttttttt 23040
   taagtgaaag catgtttatt aagaaaataa aggaataggc tgggtgcggt ggctcacccc 23100
   tgtaatccca gcactttggg aggccaaggt gggtggatca cctgaagtca ggagttcaag 23160
   accagccggg ccaacatggt gaaaccccat ctctaccaaa aatgcaaaaa ttaggtaggc 23220
   gtggtggcag gtgcctgtag tcccagctac tcgggaggct gaggcaggag aattacttga 23280
   acccaagagg tggaggttgc agtgagccga gatcgggcac tccagcctgg gcgacagagc 23340
   aagattctgt ctcaaaaaaa aaagaaacaa agaaagaaag taaagcaata aagaatggct 23400
   acttcatagg cagagcagct gagtattttc ttttcccagc aacttttatc tcccgaatag 23460
   aaattctttc taatcccact tactatttgg aattctaaat ccaacattgc tttcccagag 23520
   atccatgtta tcaagatttg cttgtattag ttaagagcga ttgcttatta tctatacagc 23580
   ccttgaaatt acattgttgt gaagtcaggc tgctgtttta gggctaagta aggttttctg 23640
   aaaaagaggc aatggtgcaa agacagctgg aaagacagag ttaaacatac cttttaaagt 23700
   ggatcaattt tatgtaacca gagtatgatc tttctgtttt atgtgtttat tggaagagtt 23760
   gtcacttaaa gtgatattcc tcaaatagct aaaaatgtcc tgggttttct agatagaaaa 23820
   tatctctaag ttgtacttat taaaatgtga cttggggaat tttaaattat attcagtgga 23880
   agtatatttg cccagcttta tttttgttcg ggtaatttat ttctcaaaat tttggaggtg 23940
   aggtctttat tgatttgtgt gtgtgaaaaa caagacaaaa cacacccact atgaggatcc 24000
   tttaaaaaaa aagctttctt gttatccaga taacacactt taatcgtacg acggaaatgg 24060
   atacatttaa catgacaaaa aatagcaaat cctctgctca tggaaagatg atttccaaac 24120
   cctcttgtct tacagaagaa aacatttggt tgactaagaa ctgacatggt ctgctcatag 24180
   ggattcttag catttttaac atcatccctg tttgactgct tctttttcta cattctcatc 24240
   ttctaacttt tttctagggc tgctcttttt agtaaagcta aacaaaacct ggcgtcctac 24300
   agccttacta acctttgtta atccgcctgc tcgtcacatc tttgtatggc atctgtgctc 24360
   actcatgggg gaaagagatt ctggaagaag caacagcaat aaccaaggca aatgtgggga 24420
   agtgtctgca tgggttgtta gcatgatgtg tggggcctag gttttacttc attttcttcg 24480
   aatagtgagg tcgttaagtc acgccatttg aataattttg tatgaccata gggtgacata 24540
   ctcatccctg ttgtcaattc tcctaagaaa tttggtggca aatttcatca cttaagccag 24600
   aaagtggaaa tttggattag ggatgccaaa taagcaaatc tttatattga tagaaatgta 24660
   cataggcgta tgtcttaata gacttccaca ttattgaggg gttttttttg gttttgtttt 24720
   gttttttagt ttttttggtc ttatttttaa atttggttaa ggcttttttg cttgcttttt 24780
   cttcgattta attttccttt tttacatttt aattttggtt attttggggc cattttttga 24840
   ttttgttttt ccaaaggacg cggattctga tagcggggac gattctgaca agaggtcgtg 24900
   tgaagagagc tggaaactga ttacttctct gagagaaaag ctacctccca gcaagttgca 24960
   aaccattgtt aaaaaatgtg gcctcccaag cagtgggaag aaacgtgaac caattaaaat 25020
   gtatcagata ccccaaagaa ggcgcttgag taaagattcc aagtgggtca caatctcaga 25080
   tcttaaaatt caggctgtca aagagatttg ctatgaggtt gctctcaatg acttcaggca 25140
   cagtcggcag gagattgaag ccctggccat tgtcaagatg aaggagcttt gtgccatgta 25200
   tggcaagaaa gaccccaatg agcgggactc ctggagggca gtggccaggg acgtctggga 25260
   taccgtcggt gttggggatg agaagatcga agacgtcatg gccactggga aaggcagcca 25320
   ctgatgtaga tgacctcaag gttcatatag acaagctgga agatattttg caagaagtca 25380
   aaaagcaaaa taacatgaaa gacgaggaga taaaagtctt aagaaataaa atgctcaaaa 25440
   tggaaaaagt cttgccactg atcggatctc aggaacagaa aagcccagga agccacaaag 25500
   caaaggagcc tgttggtgct ggtgttagta gcacctctga gaataatgta agtaaaggag 25560
   acaatggaga acttgcaaaa gaagaacgtg tttcccagct gatgaatggg gatccagctt 25620
   ttagacgtgg acgtctgcgc tggatgaggc aagagcaaat tcggtttaag aacttgcaac 25680
   agcaggagat aacaaagcag cttcgtcggc agaatgtacc tcataggttc atccctcctg 25740
   agaaccggaa gccccgcttc ccctttaaga gcaaccctaa acacagaaac tcttggagtc 25800
   ctgggacaca tatcatcata acagaagatg aggttataga gcttaggatt ccaaaagacg 25860
   atgaagcaag gaaagggaat aaagaagaga gccaagaaaa agggggtaaa ggagctttta 25920
   aggatcccca gtttccatgg ggctctcaag gaatgagaag tcaagatcac atccaagtta 25980
   gcaagcagca cattaataat cagcaacagc cacctcaata cgttggagaa gcaattctct 26040
   caataatggc cagccgaaaa gtacgcgctg ccaggcatct gcctccgcgg agtcattaaa 26100
   ctcccacagt ggtcacccca ctgctgatgt acagactttc caggcaaagc gccatattca 26160
   tcaacaccgt cagtcttact gtaattataa cactggaggt cagttagagg gcaatgcagc 26220
   cacttcctat cagaagcaga ctgacaaacc cagccactgt agccagtttg tgacacctcc 26280
   gcggatgagg agacagttct cagcacccaa tctcaagctg gtcgagaaac cacagtataa 26340
   atcagttact ggacaaactt gaaatcatgg tggaagaaac agacagtgtt agctcatgat 26400
   ttgatttggt tctacctttg gccttgagtt cttattattt acattataaa tattaactgg 26460
   ttttatattg ttaagacaaa acactggtaa aagtttcaac acctcccttt tgcttgtata 26520
   ccataaatgg gcagtttctg aaattttgga taaagcatca agaactcctt tttctgaaac 26580
   gttcctcctt ttttagtgcc taattaatat acttacttac acagacttgt cccatcttga 26640
   tgtaagtttg ttatgttttt ataatgccta taaattaatc tgacatccag aaagacctgc 26700
   ctcttagttt atgcagacat tcaaaaggag aatttgataa gcagaattaa atgtctgtta 26760
   ttcataaatg ttagaaattg gtttaaaaat aataacatgc aactgggtgg gcacagcttt 26820
   ggatttctca tccacgtctg ttcccttctt taaatatgat tagaattttc gtaagccaaa 26880
   actaaaggtc ttcaaacata tcttgcaata catagctttg catcttccac acaacttcca 26940
   cgtcttcttt tattctgtat tttacaaaaa actcatataa tggttttttc ttttgaatgt 27000
   atcagaatat gtatgaaatt tgtcatatca gaatctataa attggcactg tgtctgggat 27060
   tctgtttgtg gtttgcattt tgaagttcaa tgtatcattt gacacacatg cacacaaagt 27120
   gaactattca ccaaaatcat gccaacagaa ataccaacaa aaaacttgtg tagcttgtaa 27180
   ggaggttatt tggcttgaga gggaagaggt gggacagaca tgggggacaa agctacagca 27240
   tatggctctg tggtgctgct tttttccaat agttgatgaa atagtgaaat aaaagttaac 27300
   tttggttggg cttggaataa aggagatgat acataataaa ctattctttg gttagcatta 27360
   gtaatgcttg tagacactca aacaaggaag gcgaagtcct gatgatgatt tattgtatca 27420
   atacctcatt ttatttggtt gtggtctcag tttgcctcac tggagaatcc actaagaaag 27480
   atggatttta atgggaagaa aagaattatt ttctacatcg ttcctcatct cttgagtttg 27540
   tcaaaaagtt ttctaagagg cagtgtgaag agccagagat gtccgaaatt gcctgacttc 27600
   atgttttgga agattcctaa gtcacatgaa atcatctatc aaacacttgt cttccaaacg 27660
   gtgtgaaaaa ctacagccaa gatagcagtt agcattcaag gaagccttaa agattgtgat 27720
   tatgtttttt tttcctttgc gtgtgggcac tatgctttat tagaacacat tatttttaat 27780
   catagtattt tttctttgcc tctaagaaaa tactttctta atgctcagaa agttgcttcc 27840
   aattaatgtt tttttcccct taaaaagaga agctttgaga gatatttttg ctttcatagc 27900
   tagaacagtt gaagtcttca actgaggttt tatagcagat tagacatggg taaatgatgt 27960
   ctgtaatggg ttgagttact gagatgacaa tctcctgtgc catttggttt gaatgtactt 28020
   gataggctgc ttcaaatcag tcacttcaat gcattttgtg taaacccagt tgtccttttt 28080
   tattcctctt tagacataaa tgtgctaact attcctttct atacacaatt tattttctaa 28140
   gattaaaaat aataattgct aggtttggtg gcacatgcgt acagccccag ctacttagga 28200
   ggctgaagca ggaggatccc tagagcccag gagttctggg ctgtggtaca ctatgccagt 28260
   tgggtgtaag tttggcatca atatggtgat gtccctgaag caggaaacca caaacttgca 28320
   tcaggacggg tgaacccagc ccaggttgga aatggagcag gccaaaactc ccaggctgat 28380
   cagtaatggg atcttgccta tgaatagcca gtgcactgca gccaggacaa cagagtgaga 28440
   acctgtcttt aaaaaaaaaa aaaaaaaaaa aaaaaaaggt aatatttatt atattattca 28500
   ggtttcaact ctgtagcaaa aatgggctct catttcccta acttgagata acatagggta 28560
   ggtccatata ttttcattct tacagtggtc ttttcatggg agtgaatgag ttactctcca 28620
   ctggtgatta ggtaatactg tagaatgaag agttgtataa tatattcatt tacagctgtg 28680
   gattgtggta aggactatgt ccacagtgat attccaaaga attgggttta tatttgtgct 28740
   tcatctgtta atcccaggtg tcctcatgtt gctgaaatat ttagatagct aaaatatccc 28800
   ttaatttcac agatgaccag gaagaaatta accaaggttt tattgactgc catgtatgtc 28860
   ccatgatgca tttctgagca aatgcttatc ctagagaata actctgtatg aataaaattg 28920
   cttaattgag tctcttacta aataagtaac tagtgccatg cttttgtgag ctcttggtat 28980
   ggcccatatt actttgtttt ttgtttttgt tattgttgtt ttgtgatagt cttgctctgt 29040
   cgcccaggct gcagtgcagt ggcacaatct cagctcactg caacctctgc ctcctgggtt 29100
   caagcaattc tcctgtctca gcctcctggg tagctgggac tacaggtgca tgccaccatg 29160
   cctggctaac ttttgtattt ttagtagaga cagggtttca ccacgttggt caggctggtc 29220
   tcgaattcct aacctcaggt gatccacctg ccttggcctc ccaaagtgct gagattacag 29280
   gcgtgagcca ccgcgcctgg cctgtttgtt tttttaacat gatttttctc taagcttaaa 29340
   taccacaagg ccaaagagaa atggtcataa tttaaaccat tattatattg ttgaggtatc 29400
   cctagctatt attatagcaa agtgggaaaa aagtgtttat tctattgaag ttatgtaatg 29460
   atccgacatt aatgggaata tagaggagtc ctaattaatt ggtataattt cacaaagcgg 29520
   aatggtattc cttggagagt taaagacatt ctctttagta agtgtaaacc aaagggcatt 29580
   ttctttattc ctgcttctaa ttccttctag cccagtgaat tatttctctt ttcactggta 29640
   atgtgatgaa tgggaattgt ttattacatt gaagtgactt gaagtgacct tttgtgcttt 29700
   aggtgcaggt tgacactgaa aaaaaaacaa aacactgaat ttttcacacc tatgtctgca 29760
   ttaaaggctg ttttactacc ggaagttaca tagacttcct gcagtcagct gctgtgcccc 29820
   agtgccttac tggtcctttg tagatttgcc ttaatgattt gtacaaatga ctgggaggcg 29880
   gggatgctgc ctgtgtcctg gtgaacctta atgaaggggc cgtcttaggc acagtgcaaa 29940
   acaagcattt gtcctgtact gttagagcca aaattgtgat gagcaatact gataattgtc 30000
   cagtttatgt catctttccc agattttaaa atctgttcta gatattctta gcttgaacca 30060
   cttttgattg tgaaatgtat taggtgttgt cccattatta ctgtaaaatg aagttttgaa 30120
   tcttcttgtt aataaactgt ggatttcccc tctcaatttc ttaaacaaca acaaaaaaat 30180
   gcttgaagat tgtctttgag tgtaagatct gccttttcag aaagggagtg ttagtttgta 30240
   atgttaaaaa ataaagacct cattcaataa aagttgaagt catcttttaa gagtgtgatt 30300
   tctctctatg tgggaagagg gaaaaggaaa gcaaggtaat gctaactaaa cctggttttg 30360
   acttttattt attgcttttt catctgaaga tggttgtaaa ctaaatcttc tttttgatat 30420
   ttctatgtga acttgattag ttttaaagct tttgctttag ctaccctttt ctgtttaaca 30480
   aaattctatc ttaagtgaga tctttcagcc ctattttatg ccacatttca cttacataaa 30540
   atcttttctc acaaaagatg aataaggata ttactttctg atttgagttt agtttaacca 30600
   tgttttcagt tttttctcta catactcttg gtttgaagtt cttttaaatg aaatgattta 30660
   ttttccttta tatgggcaga aactaaattt ctaaaaagtt ttgagtgact ctgcaggttt 30720
   tctttctcct ttaacctctg cttttcttca cccagagtat tgatggtaca ctagagagga 30780
   aacaggacaa agcagaatat ttttctggaa tggaccacta gtctcattga aaatttggac 30840
   ctgacattat tttttaatgt ttaaaaacaa cataatcacg catcatggga tagaatacaa 30900
   aatccacatg agtttttaat atgaaatctg aaactttaga gaagtgttat gcctgaatcg 30960
   gtctgcttcc agcctcatac ccttttcctt caggtacttt gtaatccttt gctgtcggag 31020
   aaactgttgg aaaagttgca atgtccctac cgtttatagg gtgacacaga agattacagt 31080
   gtgatatgtg tagtgacact gtgctgatga gactgtggcc acacagtgct gctggtggtg 31140
   agaagcgaag ttaagtgttg tcaacaactg gggtgtcatc ttgggtgtgg atattttcct 31200
   tccagctatt gcttgctaat tataatctgc cattaggagc caagccttta atgtctaaaa 31260
   cagcctaacc ggaaaactca gaatggtgat attgatttta ctgtgaatat caccttctca 31320
   gaggtgcctt aattgtacca ccatcatctc tcatcgagag taccctagct tgtctctttg 31380
   cttctattct tgacccctat aatccaatgt ccacatagca accaagatca tcttttaaaa 31440
   acataaatca tatcttgtca gtgtcctcag gggctttcca ttgcatgaaa gaatcttagt 31500
   cttactgtag tctccaaggc tttacatgtt ttggctgctt acctaccact cttaacactg 31560
   tcactctcac accacagaca ctctgccctt ttttttgttc cttgtatatg ccaagtttct 31620
   tcccacctct gggctcttgc acttctcaag cctggagccc atttccatca tgcttacctc 31680
   cccttccaga cttgagctcc tcagctcttt ctgcccagcc caccacaagt tgactcactg 31740
   ttttaataat gatggcctgt aattatcttt tctttgtatt tgtttggttc ttccacgaga 31800
   atgtaaactc acctagaata aatgccgtat ctgtcttatt cacttcgcca gtcatataaa 31860
   tccataacct ggaagagtgg ttctcaaact ttaatgtcag ttagaatttt ggggtcttat 31920
   gagaacacag actattgggc ccaccttcag agtttctgat tcactaggtc tggggcgaga 31980
   cctgataaat ttgcatacct cataaattcc caggtgattc tgatgctgct ggtccagtaa 32040
   acacctttga taaccagtga tctggaatgg gggcttggta agcattttct gtaaaacact 32100
   agatagtaaa tattttagac ttagtggacc atgtggtctt ttttgtgaaa ctgttcaact 32160
   ctattgttgt agtacgaaag cagccataga caatatggaa atgaacaagg gtggccgtgt 32220
   tccagtcaaa ctgtgtttac agaaacaggc agtgggctgg atctagcccg aaggccatag 32280
   tttgccgacc ccgatctaga agatagctac ttctgataaa tatttatcta aaagatcagt 32340
   atgtgactgt gtatgtaatc tagaagacag atacatgcca gttgctggga tgagtggtaa 32400
   gtgacaaatg tgtggtccct ggcctcataa agttctcaga gttatggagg gagacagctg 32460
   taataaccac accagtagtg agtcagtaca tttgtgacaa gtgccatgat ggaaaaacac 32520
   aaggtgctct gaaagggttt aacgggtgcc tttgggaatg aaaagtggcc tctcattgct 32580
   cttagctgac aatgtttttg tttcactgag gaaaaaaagc aatcagaaga aactttgttt 32640
   tcttcctttg tctagttttg cctgcatcat ccatccatac ctgtactggg ctttccctcc 32700
   tgttagtgga taaactgtcc ttgatcctct cctagcccag cccttgctcc ctgcattcca 32760
   tgccctctca agaacttgtt gaacatttac ccccttctct tttacatcat cagtttctct 32820
   cctggattat tcctgccagc atgcaaacat gtcaccagtt gcaaaagttt tctgtactta 32880
   ttacgtccta cttgcctacc tcccattctt tttttttttt tttttgagat agagtctcac 32940
   tctgtcaccc aggctggagt gcaggggcac gatctgagct tactgcaacc tctaccttcc 33000
   gggttcaagt aattctcctg cctcctgagt agctgggact acaggtgaac gccaccacac 33060
   cgagctaatt tttgtatttt tagtagagac ggacggggtc tcaccatgtt ggctaggctg 33120
   gtcttgaact cctgacctca ggtgatccac ctgtctcagc ctcccaaagt gctgggatta 33180
   cgggtgtgag ccaccgctcc aggccccatt ttttcttttc attatttcct atcctcattc 33240
   ttgctggaac tcatctagtc agactcttgt tctcccctca ccaccaaagc tgctcttatc 33300
   agagtcacaa aagctcttct tgatgataga ttcagtgcat tttctcagtc ctcatcttac 33360
   tgaatgtctc tggcatttga cacagttgtc tgctcttcat gggacacttt cttctcttgg 33420
   cttcttttcc ttgaaccttt ccagctgctc catcatggtc tgtggacaca gaccttggct 33480
   atttctatat gtttctctgt tcccccttcc atttttgtac tgatgatgaa gataatgata 33540
   actcatatat tgagcatgta ttacatgtat taatacacat aaacctttta catgttttga 33600
   ctcattgagt cctcacaaca cccctcttct gtgaggtagg tattgcttat tcccttttca 33660
   catactttcc cagtgtaaca cggctactca gcgggagagc cagagccagt cccagggagt 33720
   cattctgtct gcaaacctct gccctgcctc gccttctgct gactctcagg tgtcctctct 33780
   ctccagcctg ggcccctacc ctgagctcta gatgctgatg atacacaact actgcctgac 33840
   atccaaatca ctggcaggca ccttacattt aaatcatttg aactggagca gctagctcag 33900
   gccacaaatc caagtcacct ttagtttctc tttttcttca catcccatga tcagatcatc 33960
   agtagatcct gtttgctctc cctgtaaaac atatcctgac tccatctctt ttcaccacct 34020
   ttgctgctaa taacctggtc caagccacca cagtgctgga cagtgctgtg acagcctcat 34080
   gaccattctt cttatttcca tagttcctcc tgagtcccac agagaataat gtttttaaaa 34140
   catatcccag ctctaaacca tccagcagtt tccattagaa taatatcccc agaccttacc 34200
   atggtctata aggccatctg tactccattt gctacctgat tacatctgat gcatctccta 34260
   cccgtctccc tactctgcca ttccatccac acaagttttc ttgcttttcc tgaaatatgt 34320
   ggtgcccctt tccatttcag gaccttggca cttgctgtta ctatgaattt gagtagaata 34380
   acccctgcca aagacctctg cctgcttaac ctactcagtc tagaagtctg tctcccattc 34440
   ttctccctgg tagtctctaa ccctatcttt atctttcttc atactagt 34488

<210> 69
<211> 9629
<212> DNA
<213> Homo sapiens

<400> 69
agatctttat ggctctttat ttctacctgt tattcacttt acacttgaga gcccaaaggc 60
ttcactttaa gttgcttgtc atagtgacag agatggccta ggacccaagg cttctcctga 120
caattaggtc aggcttctcc tcaccatgtt acgtaagatg aagctgccta ctcttcactg 180
caatggtata gaacacaagc gaacagtttt agaaactgtg taattagcac tgggacaaag 240
tttatggcca gaaactgggt tttaacttcc aactccctta gaacaggatg gaaacgtgct 300
gaagaacaca ggcagccatc aaatgaagca agcattattg tcccattatt tgtacatacc 360
tttctcaatt ttaataaaaa tcagcttctc tgaaattcta cgttatctat tcatggcgat 420
atgccagtag aaaacaatat tcatggccac ataatccctt tatgtttatt catggaaaaa 480
ctgaattcta aatgctcctt tgccactgga aaacccagag actgtctaat actatgacct 540
acagttacac ttgaggcttt catttctatc aaaaaagctt ttcttttccc cctttccata 600
agaatgatca tcttctaatt tagttatgag gttccgacca tgtgaccaat accatactac 660

aaattctgtt ttattttatt ttatattttt tggtagactt ggggtcttgc tctgtcccca 720
ggctggagtg cagtggtgca atcatggctc actgcagcct caaactcttg ggctcaaatt 780
atcctcctgc cttagccttc caaggagctg ggactacagg catgcaccac aacacccagc 840
taattgtttt ttaaattttc aaagagacag ggtctcacta tgttgcccac tctggtctca 900
aacttctggc ctcagcctcc agagttgctg agattacagg tgtgagccac tgcacctggt 960
gagataaatt ttagaaccta tgtctctact aggtcaggag actcgctatc ctttaatttt 1020
tagataactg gtactcatcc atttaacact gacataattc ccttatttat aaaggattca 1080
acaatatgtg attctaatgt accattaaat ttaaatttaa tacttttaaa cctggacata 1140
ctttttggtt aattcaatgc atgctctact gttgaacttg taatttaaat tacctcagca 1200
tgaaggctgg agatgaacaa agacaccagt attaggatca tgtgcataga gagttctgca 1260
tcactgaaag atggatgcac aaactgagag ccagagacaa tgcctcagtt tggtgaccta 1320
gtcttaacac aaagcagttt catatagcac caaacaaata ttcatttttt tttttttttt 1380
ttgagacgga gtttcgctct tgtcacccag gctggagtgc agttgtgcaa tcttgtctca 1440
ctgcaacctc tgccttccgg gttcaagtga ttctcctgcc tcagcctccc aagtagctgg 1500
gattacaggc acccgccacc atgcccggct aattttttgt atttttagta gagacggggt 1560
ttcaccgtgt tgggcaggct ggtctcaaac tcctgacctc aggtgacccg gccgccttgg 1620
cctcccaaac tgctggatta cagacgtgag ccaccacacc cagcctaatt tttgtatttt 1680
tagtagagac gggtttcatg ggtcacgttg gtctcaaact cctgacctca ggttatccac 1740
ccacctcagc ctcccaaagt gctgggatta caggtgtgag ccaaagtgct aggattacag 1800
gggtgagcca ccgtgcctgg ccaatattca ctgctgatgg ggtcaataga tgattttctg 1860
agctttatca ttataaaccc tataggttaa aatttagcca gatgccaaag atccgtagat 1920
aggctcagtg aaactggcca aagacaggca tccgttttag gtaaaggaga agcttaactt 1980
tcctgattct ttttaggatt cagtagacta agcaaaaaat aaatttaact ctactcctga 2040
atctactttt gtcctctgac tcctgcagta ccacaaattc ttcagccaaa ccttggcctc 2100
actctgcccc ctctctcatg ttacctccat atgctttttc aatcacaatc ctttaaaaag 2160
gaaacatcta gaccataata ctattcactg aacccactac gtttgactct tagctttact 2220
ttctcctttg ggttcccttt cttcagaaca aaattactgg gcaatttaag acaaggtgtc 2280
ttccatgcag tcttcatata ttgctacgta ttgtgctgag tagactgagg acctcacaca 2340
caatgcctga gaaccatctc agccagacca gcactgacta agagcttgaa gtattggaga 2400
acttgaaaaa gagcattcat gagttgaatt ggctaatgca aggttatcca aaattgtgca 2460
agcagggagg ccttcagagc aagagctggt cccttttcgc ctttctcaca ataacctcac 2520
caaccttccc actaaaatga accaagggga tcggtcgtaa aacgggtcat gcccgtcact 2580
gaagagatct gatccctcct ccgtccctgc gtcagagccg gcgtcatcca cgaatgcctc 2640
atcatcaaaa tcctccatct catcttgctg ctcgtcagcc agctcagtga tgtcggaatc 2700
ggccgtggaa aaagtggggg agggtgtgcg gtcggcaagg cgctcgttca cacagccgtg 2760
gaaaatgggg gagctagaca ccaggtaaga cgccaattaa cacagccagg gagagaggaa 2820
tagttggtta aacaggaagc aataggtgct aacaggacag aatccactgg ggaatcaaca 2880
caataaaagc aaatggaaaa gcaaaaggat aaaataagca taaatcattt gggtcaggtg 2940
aaagggaaca agggcaagtt gaaaaagtta tgtaaggact aatccttaga ataagaaaag 3000
caaaggaaac acgtgagtag accatcctag gtaaatactg tgctataaca cgtttacatt 3060
ctccagttca cataaaagat cagcatccag gcctcaaagg acccactcaa catcaaagtg 3120
aaacaagaag gctttcagtc tttactgaaa ttctcacgtc attttggagc agaaaccaaa 3180

atggatgaaa tggcatatgg caaagcagca tgtgctttgc tagggtaaga tgtgtttggc 3240
caattcatct ctttgaaaaa ctactttgct atcagagcag agtggaaaag atttacttca 3300
tttccacaac atactatgac ttcagcaact cctttttacc cagcctcttt aaaaaacatc 3360
acaggttttt ctgcaattga aaaagcaatt caaattctga aatcataagc tcaaagttaa 3420
atttccctgc tttgaaaatt tgaaaacctc taagaacact tgactcaata tggactcata 3480
gaggcactat gtgttttcat taagtgctgg aaataatgta taaaaaaaaa atgaggctga 3540
aggggactac caaggtgcaa ttaatttacc cttgcaatga ctaaagtggg atgaattgct 3600
ttttaaaaaa aagaaaaaca aatcagatca cgaagagatt gtgtctgata agctgtacta 3660
tgaatttaaa ctgtgtagga tacaggcgta agttttcttt tccttctaaa tttatcttaa 3720
tcaattacag aacactttgt aagtttaaag tcagggaaga tttaaatact ttggctgaaa 3780
gtttgagagt cgaaacaaaa atgcctgctg aatataactg aagggaaata taccaaatga 3840
cgcagtggtt gtgagtggat aagattatag ataaggcctt tttacacctt tttttttcct 3900
cttaactttt tatattatct gctcaggttt taaacactta aaactaatca ttattttaaa 3960
agaagacatc aacaaaatca tcacatacct ccccacaagt ttgaaccagt ggaaccgatc 4020
atagaaggga tcgctgccag tcacagtggt ttcgctttcg tcttgggcac tggaggccat 4080
ctcccctgcc ctatcataca tctctcgcat caaatccagc ctctgcctat ttagtaaaca 4140
caaagcacat tgggttaata taatgagatt gcattaaact ttcactttgg aagcatcttc 4200
cgttcaagat tttcttcaaa aaaaaaaaaa ggagttgctc ccactgttta atgttactaa 4260
aaagaatgaa gcccaaaaat acagaaacca acactcagta tctaaagtgt cttgattcta 4320
gagataagcc atcaaaagtc aagaatggcc acctatcctc taccgggtga aaagtcataa 4380
gggggaatat tgtaggtcaa ctgtatgtga catctcaaat acactgagac gggggaaata 4440
cctcattata ctacatgtaa aagaagccag attataactt ccatgtttat tattgattat 4500
cctgaggtcc aggatagaaa aaataaataa gttccaaatc tgtaacagaa atatgatgaa 4560
actggaatat ttaaaattta taagagggtc tatggagggt aaacttaaat atctacaaat 4620
gtcagagtgt gtgaggagtg catttattca cttaaaagaa aacagatgga tggtaatacc 4680
attggccaac aacagcattt agaaagtaca gggaagtaag caacagtgtc ctcagagaga 4740
ggagagctgg agggagctca cttgagcagg tcattgttat ttcctaaata aaacaaccag 4800
cctttatgaa tgttttcata cttgagtttc tccaaagacc aatagtgtgt tgctccattc 4860
ttcaaatcct ggacttctac tgctaccact gtgcgaggga aaggcctgtc ctcatgagtt 4920
ttttccatct cagtgggaag taattcagga ggcaaagggg agtacagtgt gtcagtcagc 4980
agaacaaact gaaactgcac ctgaatagaa agaataaacc attgacaaat ggttttcagc 5040
aagcaagctt cagagatgag acgatacaat catacccaat gggaaagagg tcgaatgtgc 5100
tcaacaaata tcttgtgact aactttctac tgaaaaatat ggccacctgt tagcagtcat 5160
agggaataac cctaacttca aattaagcta tttaaggact ttgttttcct agcctggcag 5220
tgtttttgca catacgaaaa tcttcttgag gggaggcacg gtggggacaa ggttacactc 5280
tgtcacccag gctgggtgca gtgatgcaat cacagagcac tgtagcctcc acctcctagg 5340
ctcaggtgat cctaccatct cagcctcctg agtacctggg actacaggca tgcatcacca 5400
gcctagctga tttttaaaaa ttttttgtag agatggggtt taaccacatt gcccaggctg 5460
gtcttgaact cctgagctca aatgatccac ccacctcggc ggctcagatc acaggcacaa 5520
cccaccacac ccggcataaa atctttataa atacattttg attgaatgaa tgaagacttt 5580
tatagaattt ataccagaga acaaaaggtc gaccacaaaa tgcatatcta gtccctcact 5640
aatgagagag gatgaaaaca cagcaaggtt tcccatttta aattcagagg attgcaacta 5700

ctgctcttaa aagcatttat ttggctgggc acggaagttc acacctgtaa tcccagcaca 5760
ctagaaggcc gaggcggaag gatcacttga gcccaggagt ttgagaccag cctaggcaac 5820
acagtgagac actgtctcta caaactgagc ccagcccaga aggtggaggc agtagtgagc 5880
cataattgcg ccactgtact ccagcctggg cgacagagcc agatcctatc tcataaaaaa 5940
aagtgtttaa taaatatggt tttataaaag tgtttaataa atatggtaag tacttcacga 6000
cattgcatca tttatttctc cctaccacgt tatagtttca ccaacaatta ttaaacaatt 6060
atttaatatt aattaaatgc tatgcacagt agtaagaaaa aaagacagtt actgccttta 6120
tttagtctac ttgaggagag gagtggctaa taaataatta taaaatgaga aaggctgcca 6180
agattatgaa tgaagaacat atcgtattcc ctaaaactag aatttagttc taggagaaga 6240
taattaattt ccccaaagtt aggtggtcca tgctcttacc tactacacaa tatggtcttc 6300
cactgtaatc ttagcagtga agaaactgat aaacacgggg gcatacagaa gactgaatct 6360
aaggtttggt gtgaagtttt ggtaatactt acaaaaatga ataacattta ttgcctttta 6420
ccatgccaga cactataagt gctttatgca tatgcccatt ttcattcctc acaaaatccc 6480
ttggtgccag gtactgctgc caaccccatt atgtagatgg gggaatggag gcacagggct 6540
agggggatca acaccactcg tggagctggg actggaacct ggactgtctg ggtccagggt 6600
gcatgtgctt gtccactaca cagcagtgcc tctcagaccc tagaggagaa gcagatcaat 6660
ggcaaagggg cataccaaac ttagaggtgc aaacttaata acagaaaagt ggaagaaaat 6720
taaaattatt cagaaaccca agagaagtat ttttctatta ctatgtttct ccagtttttg 6780
taggaatata gtctatttgt aaaataagct agaaaacatg ttttaaaaat tttatttaac 6840
aaaaatttaa ttagcaccta ctatgttcca gccacatata ttaaataaag acatatataa 6900
agctgtttta tagcacaaag cagctgagtt cccccaaagg aaaggttaat tcaaggtgag 6960
aacccagatg cacccacagc aggctcggct ttgcctccag ctctcctgtg tcctcctgct 7020
ccataccttc tttttcagtt ccacactgat ggcattggcc tcctttaggt acacggcatt 7080
gccccagagt aagtcccgta atgaagtaaa ctgatgagac ttccatttcc ggaaggccca 7140
ttgggccaac tcaaattcat gctgtgtcca aggaactgaa agaatgaatg atgcatattt 7200
aatggcaatc ttacaactat ataaaaatta cacctctctt ctagcttatc tcttttacca 7260
aggtgaggtg ttttccagtt tctctccatt cagctgcttt tgtttctttt tagtaaagct 7320
taccaaattc tgttgatatt atgaaagcaa tatatacatt tcagaaaatc tttaaaaatc 7380
ctacttttgt atttatttaa aatagtacaa caaagaaata aatttttctt ttttttaaaa 7440
aaaggtataa catgtggcca caataaaaaa aaatactcct ggctaggcac agtggctcac 7500
gtctataatc ccagcacttt ggaaggcaga ggtaggcgga tcacctgagg tcaggggttt 7560
gaggccagcc tggccaacat ggagaaacca catctctact aaaattacaa aaattagcca 7620
ggcgtggtgg cacacacctg tagtcccagc tactcaggag gctgaggcat gagaattgct 7680
tgaacccagg aagcagaggt tgcagtgagc caagactgca ccactgcact aaagcctggg 7740
tgacaaagtg agactctgtc tcaaaaaaaa aaaaagacaa actcctgacc tctgttacat 7800
tgtatgactt catatatttc tttttttttt tctgttacag agagaaaaaa ggtaccatct 7860
cacttattta aaaataagaa attggctggg cgcagtggct catgcctgta atcccattac 7920
tttgagaggc catggcaggc agatcacctg aggtcaggag tttgagacca gtctgaccaa 7980
catggtgaaa ccccatctcc actaaaaaca caaaattagc tgggcatggt ggcgcatccc 8040
tgtaatccca actacttagg aggcaggagg cggaggttgc agtgagctaa gactgcacca 8100
ttgcactcca gcgtgggcga caagagcaaa actctgtctc aaaaataaat aagtaaataa 8160
ataaataaga aatgtaatac ataaattaag agttccttca aagttagttt gaagccttga 8220

gattgccaag aggttgggta gaaaaacgaa ttgtggtaag tgttcatact gcattataag 8280
gcaataagta ataatgaaag ccctaactca actaagaaat gctggaaaac ctaagatagg 8340
caaacatgac taagcaatat gattagaaat ttgccagaaa tggcttcaaa ttgaacagac 8400
tgtcttaaca aagattcagc tctatatttg taatggatat ctaaaagtca catctacagc 8460
aatatgagag acagtgcagg tcaacttcct aagggattta aatgaacgtg ctcagaaacc 8520
gtgcgaaggt ctaagaacac catcactccc atccccacct cataaaatac agcctggatt 8580
ctcaacactg cacatctatt caatcatctg taaactggct cagtaaaatg tcaataaatg 8640
atagggccaa ctaagaggaa tccagctttg attatgcata aatgactaat ttgataatat 8700
aacttcaaaa agatatacac aggaaacttt cggtctctag atttcacctt cttcctcttc 8760
ttcctcctct tcagttgttt ctgcagccag agatcgggtt tcaacctgct tctgcaaggc 8820
ctgcaattta ctctcatagt cctgaatgaa aagaaaggag ataacatgta ggagagggaa 8880
gaaataagag ggcagggaaa gagaaatgga aggaatattg ggggaagtag agaaagcaaa 8940
ggacaaggaa aagagacaaa ggcaggaaac aataataaaa agacatacag ggaaatgttt 9000
aggaaaaaaa cccacaaagc acacaagatg gtctgtaatg ggtaaatgtt aaaaagaaaa 9060
aactcactgc agtatgtttc tactccctca aagactcaag agttcttcat ctggggtagt 9120
ggtaggagaa aggggaattt tttaggtgaa ataacacgtg tataccttaa gcgttagaat 9180
atattcaagt aatatcttat atatcaataa attccagaga aaaacaatat tgaaaaagat 9240
taaattcacc agtatcatca aaaatctcaa ttatctaact ctttggaagg aaaaaaaatt 9300
aagattgtag actttgatgc ttcctctttg tgaaaaatta ctatctttga tcagtaggat 9360
gagtttagcc ttcaggtaac caaaagctca tagtgctatt aggactgaaa taatttatag 9420
actgcattta gttattcaag taagacactt tattttcaat atgcaactat aagatggatt 9480
actgaccggg tgcagtggct cacacctgta atcccagcac tttgggaggc cgaggtgggt 9540
gatcacctga ggtcgggagt tcgagaccag cctgaccaac atggagaaac cctgtctcta 9600
ctaaaaatac aaaaattagc tggccatgg 9629

<210> 70
<211> 10097
<212> DNA
<213> Homo sapiens

<400> 70
ccatggatgt tctgacattt gtctcctggt ttctgtcttt agggcatttg tttacctgag 60
caatctgctg tatcccgtgc ccctgatcca cagggtggcc atcgtcagtg agaaaggtga 120
agtgcgggga tttctgcgtg tggctgtaca ggccatcgca ggtaggtgac cctcttctga 180
aatgagagct gtgagttctt tgtctagaga caaagcaggc tgattctgcg tgggtcacgc 240
ttatattaca catacaaact gtggggagtt tgtatgtgta atatacagac acatactttg 300
gagaaagcct ttattgctcc tttctagcaa ttgttcccaa gtatcacaaa tagatcctgc 360
ttagtccaga ctactgctac tttgcaataa tggggtaaag agatttcctt aaacatgtta 420
aagaacagct cttgcattaa ctatataaaa gtcattggtg tgttgttttt tttttttttg 480
agacagggtc tcactttgtc gtccaggctg gagtgcaatg gtgcaatcat atctcactgc 540
atccttcacc tccaggctca gccatcctcc cacctcagcc acctgaatag ctggaactac 600
agatgcacac caccatccca gctaattttt aagtttataa atttatagaa gttggggtct 660

cattatgctg cccagcatag tctcgatcga actcctgagt tcaagtgatc ttcctgccct 720
ggcctcccaa aatgttagga ttacgggtgt gggccattgc acccggccag tcattggcct 780
tttattttgt tttgagacgg agtgtcgctc tattgcccag gctggagcgc agtggcacaa 840
tctcaactca ctgcaatctc tgcttcctgg gttcaagcga ttctcatgcc tcagcctccc 900
tagtaaatgg gattacaggt gcccaccatc acacccaggt aatttttata tttttagtag 960
aaacgaagtt tcaccatttt ggccaggctg gtctgaaact cctgacctca gatgatctgc 1020
ccacctcggc ctcccaaagt gctggattac aggcgtcagc cactgcaccc agcctggcct 1080
aaataatata tcttaactgc ttcattatta acaaaggatt gttaggcctt tgtcttgctt 1140
agttgttgtt tttttgtttg tttgtttttt tctcagagta cttgtaatag acatttgttt 1200
ggctgttgtt ttgtaatgca cgggtcaagc tgatataagc tgattgtggt ggctcgtgcc 1260
tgtagtccca gctacttggg aggctgaggc aggattgctt gagcccagga gtttgaggcc 1320
aacctgggca acatagtgag accccatctc ttaaaaaaca attgaaaaaa aaaaaagatt 1380
ttggtatttt ccatttttcc atgatccagc ctatgttctg aggaaagaat gtggtcattt 1440
gctgtaatct ttctgtctta attcccaaat gctgggctgt gtccagtgac ctttcagcct 1500
ttcccctatg gaaacttgtg acacaatcac cagtgttgag tatatggaag tctcctccac 1560
aagtacaata taatattttc aaatacttgt tgggtattat tgactattat cttgccagca 1620
tggagacagt caggatttct aggtatgaca tttgaacaat ctcaagaata aaactgattc 1680
tttttttttg agacggagtc tcgctctgtt gcccaggctg gagtgcagtg gtgcaatctc 1740
ggctcactgc aacctccgcc tcccgggttc aagcaattct cctgcctcag cctcctgagt 1800
agctgggatt acaggtgccc gccaccacgc ccggctaatt tttgtatttt tagtagagac 1860
ggggtttcac catgttggtc aggctggtct cgaactcctg acctcgtgat ctgcccacct 1920
cagcccctca aagtgctggg atgacaggcg tgagccaccg cacccggcct gaaaccgatt 1980
ctttattgac tagtctcatt tgagctgagg aagttgataa atctacatga atagagtgcg 2040
aagcagccca tgccgtgtta ctactttggt atcattctct catgccaaag acttgagttt 2100
ataatccagc aagagctttt tccctccctc cccctgtgta gtctcactca attcttgcta 2160
attttttttt ctgctttagc ggatgaagaa gctcctgatt atggctctgg aattcgacag 2220
tcaggaacag ctaaaatatc ttttgataat gaatacttta atcaggtgag aaaccgtcag 2280
gaagaaggaa aaccctgtgg aaagagaaag aatgttcagc attggagatc agttctcctg 2340
taaaactgaa aaatacaggg tgctggttgc cacagagtag ttcaatagaa tatgggacat 2400
gtctaacaat tatgaaggta aaggtgtata gactcaatat tcatatttta ttcattgcct 2460
ttcctcagac agccctcagt ccatataatt ttccagtcac tattatttgt tggacagata 2520
aacagaagta aggcaactgg ggaaaaatac gtttttatac tactttacca tgtatctgcc 2580
cctgcctttt tttcagagtg acttttcgtc tgttgcaatg actcgttctg gtctgtcctt 2640
ggaggagttg aggattgtgg aaggacaggg tcagagttct gaggtcatca ctcctccaga 2700
agaaatcagt cgaattaatg acttgggtat gtagacatag tttactgtgc ttggggacat 2760
tttcgacaaa gggaaaatat tgaccattat caagggacat agtggccttc atcaactagg 2820
aatggaaagc atgcccaact ccctcctctt tgcattattt gaaccatctg tgtcttcatt 2880
tgaccctctt tagatttgaa gtcaagcact ttgctggatg gtaagatggt aatggaaggg 2940
ttttctgaag agattggcaa ccacctgaaa ctgggcagtg ccttcacttt ccgagtaaca 3000
gtgttgcagg ccagtggaat cctcccagag tatgcagata tcttctgtca gttcaagtaa 3060
gctgcccctt tgctctgcct cccagctagc tgctaaccga ggtgacatct cttgtgcact 3120
gtagagtgct ttacatgtgt gagggattaa cactcttgag acaaagactg atcagtctct 3180

gaaggaaaat actttcatca ctcctatggg agtgagaacc agaaacctgt cctggaatgc 3240
aacagggttc cctggcagga ggagcatgct gcacatcctg ctgcagggct ctgctgtgag 3300
cggtccccct ttaccaggaa tgtagggtct gatcacataa gcctgttgat gagtctttct 3360
agtcctcttc atgttgaaat gtcttattaa taagcctgaa agcaatgact tatctagcta 3420
gcaattacgc ctatttaata ctcgggaaaa ggaaaattta aacaatgtaa agaaggataa 3480
attctacatc aactgaaaaa agctagtatg tagggaagat gtgatacctc tcatttctca 3540
tctacatagg acttgctctt tcccgcggcc ctctcaaacc ctgtgctctg gatgctttct 3600
tctcaagact gccccaggaa aacatttttt tgtgaatctt cagttttaat acctccaaac 3660
ttcaatttgt cttttcagta cttggaaagg gccacagtac aagagactga agtgtgaccc 3720
ctccatacct accgccctca gtttgccact ggtgccacca agaggctgct ttgggttctg 3780
ccatccctct cccctcagaa tgtgccttgg ccgtcccatg ggtcctagtg cagtttataa 3840
agcttttgta taggttttat cacagaaaat tgagtcaaat gtgatttaaa ttaaatgtga 3900
tcactatata gtaagccact aatatcattt gctacaagtc atagttgttg gaatattacc 3960
cattgagcat tgatttcacc tatttaatgt gttttcactc ataccctcaa acttagggca 4020
ggatagtgtt caccagatga atctgttttt tggaactgtt gctattattc taatcaggca 4080
actgatacgt tttatggaaa acatctattt ggttaactga tgtctgaagt gacaataata 4140
gtgagttgac ctttgtccta caatctccta aaaagagcag catctctacc tggtgttagc 4200
tctggggaaa agggcatgtt gcttctgttg aaagcagcct caaaggcgtc ctgtgttttt 4260
atagtcgctc ctctggcctg tgccgggaag gtatggctgc agagcactgt gaatgagttc 4320
acctgtcgtc agggaacccc aggaatgctg cggtgccagc aatacaggaa tcagggattt 4380
ggagcacttg gcaaaagcat gctagtgttc tcagacgggt gaactaattt ggaagacaac 4440
aagttcatat taccagtctg tctgtccccc cagttgaaaa tagtttcttt ttctccattt 4500
tttcttgatg gaaggggtgt ccttctgatg gatttttcta tctggcagta aatctttgtg 4560
ttgggtcctc tttaactgtg cagttctttt tatttcagac taataatagg gagagagaga 4620
gagagaacga taccatctca ctatctgtga aaataactaa atatagccca ttgttttaac 4680
aaatctttcc agagtatctt ctgtgtgtcg agacctgtgg gaaaacgctt gctgtctggg 4740
ggagagaggc atataaatac attattacag tgaaatcatt gctattaagg aaaaaaacat 4800
tactagtaat gtgggaacac aaagctgcca gggtaagtca ggaaagactg cctggaagag 4860
gtgacatttg aatccacaac agactcttat gtaactatgc catccacagg ctctcgcact 4920
aactgtggaa gcttgagtct gctaaatttt gtctgtacaa aatgacattg tttttatttt 4980
tagtaaaaag cttttacaaa ggaagaggag ttaattgaat gtacttcttc actctctcta 5040
aagcattctt gcatatacct ccatatagct aagtgatcag ggtcagttaa tttttcagtc 5100
atttttgaaa taaaatctaa aatgccctct gtgcaatatg gaaaaggttc ttgacaagga 5160
aagtaaataa cttaaagaat ctgcttaagt ggaatgcttc tttctgtagg atccacattg 5220
ggctcttgaa agagattctg ataaaaccgt ttaatgcatt gttcacgagt ctttttattt 5280
tttttatgaa gctttttgca tcgccatgat gaagcattct ccacggagcc cctcaaaaac 5340
aatggcagag gaagtcccct ggccttttat catgtgcaga atgtaagtga catggacctt 5400
tttgccaaac atatgttttt ctggtacctt ggctttctca gcccgggaag ggtgataaga 5460
agaggtacaa gtcactggct gtgcaggggg agagtagaca atagagggcg tggctagagt 5520
ggtgaggtca tccacagcgg cctgagcagc aagccttctt gtctgtgtta cagtgggcct 5580
agtgaatgtg ggttcgtctg ccaataaaat cttccatcag tttgaatgca atgagttgtt 5640
ttagtgtttc tgtcctagaa aaataagcat gcttaataag agttttatta aagtgaatgc 5700

attgtccttt aaaccctaag atacttatgt tgctcgattg caaataacat aaaaaatatc 5760
aaataccttc tgtagctaaa ttaagattta gatctgtttt ccttgcaact gatagtataa 5820
ttaacacatt aatgcccttt acaactgtat tcctacgtgg agtaagaaaa cagacccttc 5880
cctctcttac gtgaaatttc attcaaactc tattccaaag tcattatcct catcacaata 5940
gctaatattc actgagagtt tattttgtgt tagtcactgt tctgagagtt tttgtgtaat 6000
ttaatcttta cctttatacc atagattaag ataatccctg tgttagctga gatgactgaa 6060
gcatagagca attaaagtca ctcatcaggc tgggcgcagt ggctcatacc tgtcccggca 6120
ctctgggagg ccgaggcagg tggatcactt gaggtcagga gtttgtgacc agcctggcca 6180
acatggtgaa atcccatctg agacacgaga attgcttgaa cccaggatgg gggggttgca 6240
gtgagttgag attgtgccac tgcactccag cctgggcaag aaagcgagac tcccatctca 6300
aaaaaaataa aaaagtaata ataaaaataa agttactcat cagtgttaca agaggcagag 6360
ataggactta agctcagaga aatttagctc tggtattcag gatattcact actacatgaa 6420
actgtctctc attctcttgc taaccatgtc tgctctctaa agtaagagag gaaaggagaa 6480
cttcaaagat ggaacaagca aacacaaaat atgccatctt ctttttcatg ttggtataaa 6540
tataagcaag catgtatctg ataaactttg atcagctgct gtgcacaagg attgtgcctg 6600
acaactgggg aatgcagatg aatctcagat gctcccagtg agcttcgaat tacgataggg 6660
gaaataaaat gcatacataa acaccagtca tgcaaagatg aacatgagaa aatattcact 6720
aaatgcagtg ggagtggaga aagggaaagt tatttctatt aatggaaagc tattttaagt 6780
tactttttgt tggggaggat agttgggtgg agaaagggga taggaagctc cctgaaggat 6840
gtagtataag caagaaaagg tgtgtcaaac ttggcattgt gcctggcaca taggccatag 6900
tcagtacatg catggatgtg ctggaagggc cttccaggca gggttgcagc ctgggccagt 6960
ccttggcaca gctgaggaga gagcaggtga aaggagcagc aggtgagtga gtgagccaga 7020
atagtaagtg tgggaaggac aacaggggcc aagatcagtt gaggcaaagt atggaagatc 7080
ttgaatacct gaagctggga aaagttactt tagaattaaa aacaacatag gggatcccct 7140
gttttttgtt gcaagagtcc tgcatagtaa aatacccagc agatactgga cactcagcag 7200
cagtaaaatt gggccaggca tagtggctca tgcctgtaat ccagcacttt gggagctgag 7260
gcagaaggat cagttcagca catgagttca agaccagtct gggaaacata gtgagatact 7320
gtctctacaa aaaattgttt taaattagcc aggcatggtg gtgcatgtct gtagtcctag 7380
ctactccgga ggctgaggca ggaggattgc ttgagcccgg gagggtgagg ccgcatgagc 7440
cttgtttgcg tgctacagag caagaccttg cctcaaaaaa aaaaaaaaaa acccaaaata 7500
cgtactaaag ttctgattga agcaaaaatg tgtattttct tgtaatcttt tcctaatctt 7560
gcttggcttt agattgcagt ggagatcact gaatcatttg tggattacat caaaaccaag 7620
cctattgtat ttgaagtctt tgggcattat cagcagcacc cacttcatct gcaaggacag 7680
gagcttaaca ggtttggacc agataagcaa acatttttga tggtattgtt ttgattttta 7740
gtttctcaat tgctggaaag gaagaaatct tttgagggat taagataact aaataagtat 7800
tctatattac cctgtttcct ctttagaaac ttcactatca tcttatagcc acctaatcac 7860
tgattttaga tagagcaaat taaattttga ccaggtgacc tcaaatacca tctttttgta 7920
ggaagagact ttgaatatgg tagaaatttg aggtaatagg aatataacat gaaaattgtt 7980
aaaaattgtt cctaatgccc acagctttat gaaacagaag ctctaagtac agtcatcaca 8040
actgctgagg aaagcaacag tgggcctttc tgatgatggc agagtgttgg tgttggcaga 8100
agaatttttt tatttattag ctaagactta gagggagaca ttttttaatg agatttagcc 8160
tgtttcacta tctaggatta tctctattat ctggtattat gccagagttg aaaatggaaa 8220

agtctgcttc cttaaaggga attaaaacat gctattgata ccaacatcca actctttgta 8280
gtctttttct ttctcttgaa aaaacagggt ttattgattt ctacactgca aatagtcatt 8340
acaaaaagtt ttttttcttt taaataaatt cacacaaaga gaattagaaa gcgatggtaa 8400
tgaccagcaa gaggaataat aattacgttc atcttaatgt ctgtgtgcca gttctgttta 8460
cgttaatgtt ggaaaactcc aacttggaat ccagaacttc aaatctgcaa atagaatgtc 8520
ttgagatagg cacgtggaag tcaaacagtt tctctctttt tccctgcttt cagaagctat 8580
acataaaatg ttgttgcctt ctgtactgtc acagaacttt tacgtacatt ctcactccta 8640
cttgtgaaac ccctaaagag aaggaaattc aatttgcagt ccagcacaaa ggggagaatt 8700
tctaaaataa ataactcttt atagtcttga tctttgtctt cctttctttg cagtccgcct 8760
cagccgtgcc gccgattctt ccctccaccc atgccactgt ccaagccagg tgagcactcg 8820
ctccgctttt tgcatgatga tctctttgtg aatacatgtc ttattctgaa tgacttttca 8880
aatagaagtc atgtttgaat tcctattctt ctatatatcc acatgtattc ctgctcctct 8940
tgtatgtatc tagtaggaac tagaattcct agttcctgat ttaaaataag aagtaaaggc 9000
tgggcgcggt ggctcatgcc tgtaatccca gcactttggg aggccgaggc aggtggatca 9060
cctgaggtca ggagttcaaa aacagccaga ccaacatggt gaaaccctcg tctctactaa 9120
aaatagaaaa ttagccgggc atggtggcac acgcctgtaa tcccagctac ttgggaggct 9180
gaggtaggag aatcgcttga accggggagg tggaggttgc agtgagctga gattgcacct 9240
ttgcactcca gcctgggcaa caagagcgaa actccgtctc aaaacaatat aaaataaaat 9300
aaaataaagt aataaaataa gacaagaagt aagaaacaat agggggcctt atctacaaac 9360
tctctggtct tctatttgca gattttgata atcactctgt tcctaaatct ttctgctttg 9420
catgtaactt cccataactg tcaggggact aggcatttct ttgcatagta aatttattct 9480
gtatgttcag tgatcttgtt ttgatttttc tttatttctg tcctttttcc tcattgccta 9540
ttcgccaaat tcccacccca ccctgtgctc actctccacc ctctgtattc cccatcttca 9600
tctgccttca gaccatgaga gaaagattga gctgattcgg tttctcgtgt ctggctatgt 9660
gaatgtgcat atgttggaca cattttctga gcacgccagt gtgcttgcat cctccgctat 9720
tgccaccttc caggatgagg ctgacccatt gccgcctttt ctctttctgc cagttcccag 9780
ctgccagagc gagagggtac ccaaacgaga tggttagtag ctgaattagc tccaccttag 9840
atgtagaacc atcctcaggc gcttgtcctg aagcctggct ctctgcatat gaaacactga 9900
catttccctt tgcaacttga gctgaatgta gagctcagca cattttcaag gttgtatcac 9960
attttcttcc tagagatttg ggaagcagct tctctacatg tgcctctttc acatcaccat 10020
cgcagtgggg gaggcctttg catttgtttt tatggattct ttgcaatagt atttttaatt 10080
ttgaagattt tactagt 10097

<210> 71
<211> 13646
<212> DNA
<213> Homo sapiens

<400> 71
agatcttact cttaggacag aggcaagggg ctgtatcagt taatataaag ggtaggagta 60
aatgttatcc taatgttctc ctatatgtaa ttctgagaac caccatcatg gtcatcattg 120
ggaaacactt attaagttcc tactatgtgt aggatactgt gttaagtgtg gtatgtagtt 180

gttagtagtg acagtgcttt gaggaatttg acttttcata gacagactta tgatgtgatt 240
acaagttgca ttatgaggga gaggtagtta cagtaattgt ttctactgaa ttaattgctt 300
tctgaatctt aaaaaaaact atagtctgaa attatctgtg tttcttatct cctgtcaatt 360
gccattgcac ttctacatta tttattgaat acttaatatc aaggggctgg atgcaaccaa 420
aagatgttac tgtcctttga ctactcaagt ctagagagga ttgagacata gatgtgtgga 480
aaggatgagt aaatgcaaat gactgtatga ttaaatgcca gcatgggtga gaaagacatg 540
ggatcagaat ttatcaaaac aaagaccacc aggggctaag ccttgcagat gaagtagcac 600
ttagtttttt cttttgcgtt tctatttttt aggtatatcc cagctgtggt tgaccacaca 660
gcaggcttgc cttgccaggg gacatttttg cttcatcagg tactaatgag ggaccaaaac 720
aggcatcgga gggaacactg aaaagaattt agaataagag acggaattct ttcttattct 780
ctgttttcat ctctatttca gagggtgggc ggggcattgt cctgttgtca tttttttttt 840
tttctgtgtc tgtctgtaga tatttcttcc tcagaccttg gcttatattt atccttgctt 900
ttatatgtat ttagaatagc tattttgaaa aatattttgt ttcattcaat tactctaatt 960
tgtgtagctc ctatgtcact tttaatactt ttcatttcgt gatgtttgga agctctgtgc 1020
tttcacacca gaagttctta ataggaatag aggatgaagg tttcaatgga ctaaaaatct 1080
ggttatgaaa cataggtccg cttttggtaa atcaaaagta ggttcagtca gtgttcaaaa 1140
gtacttaaga cccaagtggg cataggggct agggatacca tacatttggg acatacaatc 1200
agggcatggt tgaatactta ttatacaagg ggctgggatg cagccacata atctcctgtg 1260
acagattatg gttattctta aaaccataga gaaaaagaat tttttaaaaa agaaaagaaa 1320
accatatcag aacctgactg ttccttagtc cctgacacat ctccctttgt tttgaataac 1380
aatgtggata gcattttccg tctttccaca cctctccttc ctgctcatcc cccatgccat 1440
gccatacagg ccagaaaaca gtagacgaga ggagataata gattgcttca gctaaattgc 1500
aaccctgctt cattacctag ggcatccagc gaaggatcac agtgaccatt atccatgaga 1560
aggggagcga gctccattgg aaagatgttc gtgaactggt ggtaggtgag tacgtttcat 1620
cagccaagga tagaaccagg acttacagag attttttttt tttttgagat agggtcttgc 1680
tctgtcaccc aggctggagt acagtgatga gatcatagct ccctgtagcc tcaaactcct 1740
gggctcaagc aatcctcctg cctcaggttc ccgagtagct gggaccacaa gtgtgaggca 1800
ctgtgccctg ttagagatgt tattcttccc tgtttgcggt acatggaatg tgtagaagac 1860
catattcgga aacacagact atggagatgc agaggccttg agtttgaaac tctgtattcc 1920
tgtttcttcg ctctgtcatt agtgatatgt tatccaacta actaactcag ttaccaggga 1980
atggttctgt ggtagagtct gtgagagggc ataaaagaaa tctctgctgg ctgggtgtgg 2040
tggctcatgc ctgtaatccc agcactttgg gaggctgaga tggtggatta cttgagccca 2100
ggggtttgtg cctggccaag gtagcaaaat cccatctcta caaaaaaata tgaaaattag 2160
ctgggcatgg tggcacgcac ctgtagtctt ggctactcgg gaggctgagg tgggaggatc 2220
gcttgaactt gggaggtgga ggttgcagtg agctgagatg gcaccactgc attcaagcct 2280
ggacaacaga gtgggactcc gtctcaaaaa aaaaaagaaa agaaaaatct ctgctaattg 2340
tgagcttgca ttttagttag ggagacagca tatatatgtg cagaatgctt tgatgaataa 2400
ataccaaaat gaggacaaag gaaaatctgt gctaagagag ttaacagtga aaacaagcca 2460
ggaggactga atagattcaa gaaaaattag agggtttttt tttttttccc agacacaagc 2520
attttgagta agagagtcag gaatatgtct aacatgcaaa tgacaagaaa ccccatggcc 2580
agagtggaga aagtaaatta aggagtattg gctagatagt gtaaacttta gaatgacagt 2640
ccgaggcaag agtttggaac ttttgttgcc atggacctct ttggcagtaa cgaagcctta 2700

gaattatgtt ttcaaatgtg agaaataaag tatttaggat tacaaaggaa accgataata 2760
ttgaaataca gttgttaaaa cattaagctc ttctttactg atgcatgaaa taacaagatt 2820
tagcagttat tctaattacc ataatttcca agtagtgatc aatgaaaaag atatcttgtg 2880
tccaggcacg gtggctcacg cctgtaatcc cagcacttta ggaggctgag atggccggat 2940
cacaaggtca agagattgag accatcctgg ccaacatggt gaaaccccgt ctctactaaa 3000
aatacaaaaa ttagctggtt gtggtggcgg gcgcctgtag tcccaggtac tcggaaggct 3060
gagacaggag aatcatttga acctgggaga cagaggttgc agtgagccaa gacagcagag 3120
tgagactctg tttaaaaata aataaatagg ccgggcgtgg tggctcatgc ctgtaatccc 3180
agcactttgg gaggccaaga tgggcggatc acgaggtcag gagatcgaga ccatcctggc 3240
taacacggtg aaaccccgtc tctactaaaa atacaaaaaa attagccagg cgtggtggtg 3300
ggcgcctgta gtcccaactg ctcgggaggc tgaggcagga gaatggcatg aacctggagg 3360
cggagcttgc agtgagccac gattgtgcca ctgcactcca gcctgggcaa cagagcaata 3420
ctccatctca aaaaaatata taaataaata aataagatat tttgcacatg gaaacatctg 3480
tggtttctac tggggacaga gtcacgggta ctggccaatt cttctgtggt ttattgcctt 3540
cctttgtgat tgaaggagat gccaattttt agttaaaggt ttgtgaaaat aaagatgtaa 3600
tttttttccc catccaagct caaggaccct ttgaattctg tccacagagt gcttgtgtct 3660
ctctggacca gattaagaac cttggggcca ggcacagtgg ctcacgcctg taatcccagc 3720
actttgggag gccaaggcag gcggatcact taagttcagg agtttgagac tatcctagac 3780
aacatggcaa aaccctgtct ctctacaaat aagctgggtg tggtggctca tgcctatagt 3840
ctcaactact caggaggctg aggctggaag cttgcttgaa cctgagaagt cgaggctgca 3900
gtgaaccatg atcatgccac tgcatgacag aacaagaccc tgtcaaaaaa aaaaaaaaaa 3960
agaatcgttg gaagggagat tatgttctct cttgtctggg gcagcagtgg tagtagcttt 4020
tcaatatttt tcataataaa tttttctttt tttaatttag tttttttttt aactttattt 4080
acaaagtact gaatgtcctt tgacaaaata atgtttatat ggaaccctaa taccgaaaca 4140
gaagagatgc tttctgacca gagaaggaga gggaggatgc agaggcagca gttactatgg 4200
cttctctgaa ggatcctatg atatattttt agtttgtttt attaacaaac tctgatgtag 4260
tatttaccac atgccaggtg cttttctgaa tgctccaaaa atactagttt gtttaatcct 4320
catagcaacc atatatggaa ggaattgtta ttctcgcttg acatatgagg gaactgaggc 4380
tcagagaggt tgagccttag gctcaaagcc tcacaggtgg taggagttgg aacccaggca 4440
gtccagcact ggaggccaag tccttaacgt ctatgcaatg ctgcatccgc tagtgttaac 4500
ttgaaaacag agtgtaggat tttgagcaag aaaggggtgt aataaagtca gcatggccag 4560
gtatggtggc tcatgcctgt aatcccagca ctttgggagg ccaaggcagg aggatcgttt 4620
gagctcagga gttcgagacc agcctgagcg acatagtggg gccccgtctc tacaaaaaaa 4680
taaaataaaa taaaatagct ggatgtggtg ctgcaggcct aaagtcccag ttactcaggt 4740
ggcttaggtg ggaggatcac ttcagcccag gaattcaagg ctgcagtgag ctatgatcaa 4800
accactgctc tccagcctgg gagacagacc aagacattgt ttcaaaaaaa aaaacacaaa 4860
aaaacaagaa actgtcagtc agtggcttca gaagttgctg ttaggtgaga gtggaatgga 4920
ttggaacggg gaggtgtgaa tagcaggtca gaagttgctg ttaggtgaga gtggaatgga 4980
ttggaacagg ggaggtgtga atagcaggtc agaagttgct gttaggtgag agtggaatgg 5040
attggaacgg gggaggtgtg aatagcaggt cagaagttgc tgttaggtga gagtggaatg 5100
gattggaacg gggaggtgtg aatagcaggt cagaagttgc tgttaggtga gagtggaatg 5160
gattggaacg ggggaggtgt gaatagcagg gagaccagtt aaatggctgt tgcaggttcc 5220

agaccagaga ggaggtggcc ttttgtgatc agggcaatga tattgtgaat aaaagttggg 5280
atgaatgtgg gaaactcttt gaataaagaa tcaatatggt ttcatagtta actcaatgtg 5340
ggaagtaatg gagtcagacc gtggaggacc ctacaggtaa ccaaatgacc cttccagctc 5400
ctgaacttac acagagattt aaaagtctct tttgggctta atgaattcat gttttgtttt 5460
caaatgtgtc cgtgctctgt tttttttatc ctttctttta ggtcgtattc ggaataagcc 5520
tgaggtggat gaagctgcag ttgatgccat cctctcccta aatattattt ctgccaagta 5580
cctgaagtct tcccacaact ctagcaggtg ggacacccag agcagtgtga agaagtccac 5640
acttgcaggc gttaattggt acaccgttag gtgccttatt cattaatgac tcccagttcg 5700
gacaaagaaa ttaactccct tctcccttct agcttcaaaa atctctttat ttcttcacct 5760
gcctgctgta ctctccaaaa agaaagaaag aaagtattgc agatatttgt atgtgatcag 5820
ttactcttag agaatggaag tgatcctgtc ccatgtgaag tttgaataga tgtaacaagt 5880
gataaatgaa aattggagaa agaaaacagt atattcccca aggatttaaa agtacgaatt 5940
aattattgcc aagattaaat tttttcctgt gaaatggttg ctgtggagag aaaggtttct 6000
ctcactcccc aagtatcatg gaaatgtgcc ctctgagata aaatgaagcc tcttgtgtta 6060
agtctttgct gctgctgact ataggctcca ttatccttag tatttgtttt tcatttatgc 6120
acaaaagcga taaataatat gaactcttat gaccaagatt ggctgccata aataatagat 6180
tttactttgt tttttatttt aaatgtttca cttaaaatta tcttttataa tcaggtgata 6240
acagacagtt cgtaaagtac atagccattc tgctttcctt tgtaagacta gaactaaacc 6300
agctgggatc ctcttacctc acccaggaag ggagttctca ttgtctcaga ggctccgtct 6360
ctatatccta ggggctggga aacaggagcg gctgcctctt ctaatgtggg ctgagcacac 6420
tgccttccca ccacaggact ttgctgtgct tcagtctgct cctgaaaaac ttgtttacat 6480
ccatgttgag aggagggagg gaaggaataa gacgataggc agaaatggaa tgaaaggttt 6540
ggaccaggat taatttcttt ctgttgtttt cttgtattat agtttaccat aggaaagaga 6600
aaaaagaaac aaacttctgt ttgctggcag ggctgtggga gaaagaggga aggagtgaga 6660
gggtacatat gtatgtctgg ggagggggag tggcagaaat aaagaggggt gctttgacta 6720
tccatgggca aaatcacgag caatactatt tccctgaagt cagcagtaat tctgcatagc 6780
tttctgtttt ctgccaatgt tcagccgggt tttaccacca cattttcttt gtagctatga 6840
aaaatgtatt gtaaatttcc ctagggtact tagtaaaata tacagggaaa gatgggcttt 6900
ggttgaaata ctggttattc taattttgcc tccattagag taactttaag aagtccttta 6960
attcacgccc aacaacaggt caggtgctac aagaaatgaa tttggctttg ttagaagcag 7020
cttatcttgt gagttagaat gagttcatca aaaaaggctg aagtcaagaa taacaacaac 7080
aacaaaaaac accttaaata tttctgttct catttaaact atataaatct acattaattt 7140
atcaaacttt tgctggaaag ccatggcctt ttctttaatt ataagatcat ttactggagt 7200
tctgattgtc tttcttgcaa aaccacaccc ataattcatc atctatgact ttcagtttgg 7260
tggagcaaga attagaaacc tgtgaagaaa atctaagttc aaaatccttt aaatttttta 7320
tcatttatta acattaatca tttaaacatt ttatcatttt cccctttctt ttacatttgt 7380
ctcacccaaa cttaacagtt atttttaaac aactcaccat tttctaacct tttgaaaaca 7440
ttcaacttag ttttcacagg aacaaaaatt atttatgttt tcatatttca ttgatttgcc 7500
taatgtttaa tgaagttaga taataataat aagtacaact ggcataaaca gatttgggac 7560
cagatactac tgacatttga taagcactca actcttcgga gcgaagtgtc tgggcaaatg 7620
agacatcttt gtagtttaga atgctcaagg agaactagcc atcaatctgt atattttagg 7680
gatatggaaa acataggtta atatggtaag tggcaaaatg gagtttggtt aagagaactt 7740

tcgttctgtt tgtctaaggt ttaatgttta aagttgaact tacctgcaat ttgggtatat 7800
tatgtgtgta tcttagaggg ctttgctaat acattgatag tggggtataa ttgacaaggg 7860
accattcatt attccttttc ttcccagtcc tacatttatg ttaatgcatt gacttggttt 7920
tcccttaact ccacagcaac ttcctattat tctcaatact aagtcaccac tgaaaggggt 7980
actgatggtg gtggtctcca ctttccctct tatgtctggc attttcctta gttggaagtc 8040
tcaggagagg gacccatgtt attgtaagtc ctctatggaa ttgtgttcca tgggattctg 8100
tgttaattgt ctgtccactt gaattcactt tgcttatcag caaattttgt cttctttata 8160
tatatatttt ttctgattct ctcccttatt tcttttgttt ttttgctgca ctaactaaag 8220
ccttttctat gtttttgcag gctctttctt gataaggata ttcctaggta ttgtctgttt 8280
gcttttttgc acgtaggatt ctagtttatc agaatgcttt ctgtcttatg ttgctttgcc 8340
attttaattt tgtagtcccc tccccatgta aacctactgt acctaggata gagtagaccc 8400
tgttctgtac cctaagaagc gtatagcttc ttaaatgatt tttagagttt caaattattt 8460
tttcattcca tttgctaaac ttgtcagttt tattcttaga ctctttcaag ttttcctctt 8520
ctacattaca aagtagaatg tttctccctt catttcatac agaatctcag gttttgtttt 8580
ctgttttcct ctttatctgt cttctctccg tcaaaagcat gaagggttta ttgctttata 8640
aaacctgagt ccattgcagt cttgcaaaac tgttcacttt cttctctgtt ttgtcctgtg 8700
tggtaataga gacaaatttg gctatttgga tagctgagtc tatgagaaat attttatata 8760
tattttttaa catttatctt tctcctgtca gggctgcttg ttttatctgt ttattttctt 8820
cttttggaat aggaaaaatc ctggtttggt attaaaggaa gttttttaaa aaatctgtct 8880
catttaggga agaaaattga aggaagtgat acatgacaat ataacctaaa atgttaaaaa 8940
aaatacaaca taaacgggct gggctcagtg gctcacacct gtaatcccag cactttggga 9000
ggccaaggcg ggtggatcac gaggtcagga gttcaagacc agcctggcca agatggtgaa 9060
accccatctt tactaaaaat acaaaaatta gcctggcatg atggtgggcg cctgtaatcc 9120
cagctactca ggaggctgag gcagaggatc gcttgaaccc gggaggcgga ggttgcagtg 9180
agccgagatc gcgccactgc attccagcct gggcgacaga gcgagactct gtctcgaaaa 9240
aaaaatacat aaacatattt atatatttga ctgatgttac tgatataatg attttatccc 9300
tcaatttgga ccccattcag tttacatttc agagattctc ctaatgaact caaaagggtt 9360
catcagatgt gaccatgata taagaaggtg gcttctgttt tgttttcttg agacggggtg 9420
ttgctttgtt gcccaggcta gagtgtagta gctatacctg cagatgcttt aaaaaaagaa 9480
aaaaaaatgc ggccgggcac ggtggctcac acccgtaatc ccagcacttt gggagaccga 9540
ggcgggcgga tcatgaggtc aagagatcga gaccatcctg gccaatatgg tgaagcccca 9600
tgtctactaa aaatagaaaa attagctggg cgtggtagca tgtgcctgta gtcccagcta 9660
ctcaggaggc tgaggcagga gaatcgcttg aacctgggag gcggaggttg cagtgagcca 9720
agatcgtgcc actacactcc agcctggcga cagagctaga ctccgtctct aaataaataa 9780
ataaataaat aaataaataa agtgcggtag catgatcata gctcactgca accttgaatt 9840
cctgggctca agcagtcctc cctcctcagc ctcccgaata ggtgggactc cagacacgtg 9900
ccaccatgcc tcgctaattt aaaatttttt tgtagagaca gggtctcgct atattgccca 9960
cgctggcctc aaattcctgg cctcaagtga tcttcccact tttatctccc atagtgctgg 10020
gattacaggt gtgagctacc acaactgacc agaagttggt ttttaattgt tcataaataa 10080
taaattcaaa ggaatatatc cttcaaagca gtcaccttga aacctgtttt tcctctgatg 10140
ctgctgctgt tcagaacatc ttgagaaatc catttggaat tacttctcac caccttagga 10200
ggcatacagg aaaatatgtc ttgggattca gccctgtttg taaccctaaa ttctattgcc 10260

agcttgatta aattgacatt tctccccagt cttggttgta tttcttacaa ccacctctca 10320
agaattgcca gtgtaatggc tcacacctgt aatactagca ctttcctact aaggtaggaa 10380
gatcacttga gcccaagagt tcaagacctg cctgggcaat gtagaacttg tctctaaaga 10440
aaaaaaaaaa ttaattagcc gggtgtggtg gctcgtgcct atattcccag ctactcagga 10500
ggctgaggtg ggaagatcac ttgagcccag gagttagatt gcaccagtgc actccagcct 10560
gggcaacaga gcaagaccct tgtctaaaaa taaaataggc caggcgcagt ggctcacacc 10620
tgtaatccca gcactttggg aggccgaggc gggtggatca cctgaggtca ggagttcgag 10680
accagcctgg ccaacatggt gaaaccccat ctctactaaa aataaaaaaa ttagccagac 10740
ttggtggtgg gcgcctgtaa tcccagctac ttgggaggct gaggcaggag aatcgcttaa 10800
acccgggagg cggaggttgc agtgagccaa gatcgcacca ttgcactcca gcctgggtga 10860
caagagtgag actctgtctc agaaaaataa ataaataaaa tatatatttt tttaaattga 10920
attaccacct ttggtgggta ttctaacaga acttgctgca gcaagtccca agccaaaaaa 10980
aaaaaaaaag aaagaaactt tttttgacaa aagaaaaaaa tgaaagaaaa atttttccct 11040
caatacagga agactttagt ggagtcaaaa tttcctttgt gttaaccagg tttctgcaag 11100
tgaaattttc tctttatgtt aagtgaaagc ttagcctgaa aacagaaatg tgaagaggct 11160
tttttagaca agttcaaaac atttgcctca gttgaaagag tcagtgtcat gaaacctcag 11220
cagtggtcct gggaggtgcc acgttatggt ttggaactta atgaaggcag cagcattgat 11280
agaaatttct gtcaaatatc accaaagtca taaatgccgt cactctaaga agaggggttt 11340
atttaacttt agcatggatg tatccagcct ctgaaatggg acaaataagc cccgcccctt 11400
ctaggtactt tgacaaaaac aggaacacaa taaaagagca gacctctgga ggctgaggca 11460
ggagaatcac ttgaacccgg catgaggagg ttgcagtgag ccaagatcat gccaccctac 11520
tccagcctgg gtgacaaaag caagactctg tctcaaaaaa aaaaaaaaaa aacagatctc 11580
acaatgatac agtcagttct caagtggcat ctgttgaagc agtggatgtc gtcacccagt 11640
attagaatat gtcttaaacc aaattaaaac tttactttac acagagtttt atggacacat 11700
gaagggtttc ctcctgtgcc cagggagccc aagaactata gaaaggcctt cattgtccat 11760
catttctgtt cctgtttggg tcacttggtg gctttgtctt tgacttgtta gttgtttctc 11820
tatcttctgt gtctttgtag agaattttct tgacccacac tcactgcctc aaagactaga 11880
catttgagag agtattaagt acttgaagaa gacttagatc tagaagcagc tatgttagat 11940
gatcagacct catacactgc cacttggaag atgaggtgtg gtcatggggt tcagtttcag 12000
aaatgtgttc tctctgttga tcgtttctgg aggaagaaac atggggatgt gtttagaggg 12060
aggatatgcc agctacttat cataggaagt ctagaaaagg cagccatagt gaggtcacta 12120
tttgaaaagg agtaagaagg aatgtattgg gcaggaaaga gtgtgcatag actcttctag 12180
gtgaatcagg tgagctgtgt tctatctgcg gagaactagt gatggtgggg cagaccaggc 12240
atcttcccct ccacttgtca taggggtctg agaagggaaa catggggcag aattgggaat 12300
caatctgtaa cactgctttt aaactaagtg ctgtatttca cattagactt gcagaactgg 12360
aagatttaat gtcaattaga gttaatgttg ttttaccagt agaaaaaaaa cgatattaga 12420
acaataacaa caaaatagtt tgtgggtatc tattaaatta agtttccatt tgttacataa 12480
atgtaagatt aatttttcaa atggcttttt tttttaagct aacaaatggt atttgtctgg 12540
ctctagtgtt aacaatttat tctcttttac tgaaacatca gttaagaata accttgtatt 12600
cttttattta aaataaggct ctcggccggc acagtggctc acacctataa tcccagcact 12660
ttgggaggcc aaggtgggca gagcgcttga ggtcaggagt ttgagaccag cctggccaac 12720
atggtgaaac ccctcctcta ctaaaaatag aaaaattagc tggcgtagtg gtgcgtgcct 12780

ataatcccag ctactcagga ggctgagtca agagaatcgc ttgaacccaa gaggcagagg 12840
ttgcagtgag ctgagattgc gccactgcac tccagcctgg gtgacagagt gaaactccgt 12900
ctcaaaaata aaataaaata aaaaaataat aaaataaaat aaaataatgc tctctatact 12960
taaacccatc tacccagttt ctaccattaa tcccttcaga tatatctgtg gtgtttctga 13020
ggcccagcag cactggattg aatgatgttt agccctcttg tgatgtgaag attgaagctg 13080
acaagttacc aagcattcac ttcctgctgt catcattaac tgacaggaga gcttacatag 13140
tctttctttc ttctttcagt ttctgtccat actcacttat ccatttcatc ttaatccctt 13200
tgattgacct tactattttt atgtttatag gcagaggcag gcttgtcaaa acaaaattaa 13260
aaaaagcaca actggccagg cacagtggtt cacgcctgta atcccagcac tttgggaggc 13320
cgaggtgggc agatcatctg aggtcaggag ttcgagacca gcctgaccaa catggtgaaa 13380
ccctgtctct actaaaaata caaaaattag ccaggcgtgg tggcgggcgc ctgtaatccc 13440
agctactcgg aaggctgagg caggagaatc acttgaaccc gggaggcaga ggttgcagtg 13500
atccgagatt gtgccactgc actccagtat gggcaacaga gcaaaattct gtctcaaaaa 13560
aaaaaaaaaa aaagcacaac taagtgttcc ttaatgatgg gtggggttgg gagcaagagg 13620
atggggacct atcttttcct cctagg 13646

<210> 72
<211> 10709
<212> DNA
<213> Homo sapiens

<400> 72
   cctaggagga aaagataggt ccccatcctc ttgctcccaa ccccacccat cattaaggaa 60
   cacttagttg tgcttttttt tttttttttt tgagacagaa ttttgctctg ttgcccatac 120
   tggagtgcag tggcacaatc tcggatcact gcaacctctg cctcccgggt tcaagtgatt 180
   ctcctgcctc agccttccga gtagctggga ttacaggcgc ccgccaccac gcctggctaa 240
   tttttgtatt tttagtagag acagggtttc accatgttgg tcaggctggt ctcgaactcc 300
   tgacctcaga tgatctgccc acctcggcct cccaaagtgc tgggattaca ggcgtgaacc 360
   actgtgcctg gccagttgtg ctttttttaa ttttgttttg acaagcctgc ctctgcctat 420
   aaacataaaa atagtaaggt caatcaaagg gattaagatg aaatggataa gtgagtatgg 480
   acagaaactg aaagaagaaa gaaagactat gtaagctctc ctgtcagtta atgatgacag 540
   caggaagtga atgcttggta acttgtcagc ttcaatcttc acatcacaag agggctaaac 600
   atcattcaat ccagtgctgc tgggcctcag aaacaccaca gatatatctg aagggattaa 660
   tggtagaaac tgggtagatg ggtttaagta tagagagcat tattttattt tattttatta 720
   tttttttatt ttattttatt tttgagacgg agtttcactc tgtcacccag gctggagtgc 780
   agtggcgcaa tctcagctca ctgcaacctc tgcctcttgg gttcaagcga ttctcttgac 840
   tcagcctcct gagtagctgg gattataggc acgcaccact acgccagcta atttttctat 900
   ttttagtaga ggaggggttt caccatgttg gccaggctgg tctcaaactc ctgacctcaa 960
   gcgctctgcc caccttggcc tcccaaagtg ctgggattat aggtgtgagc cactgtgccg 1020
   gccgagagcc ttattttaaa taaaagaata caaggttatt cttaactgat gtttcagtaa 1080
   aagagaataa attgttaaca ctagagccag acaaatacca tttgttagct taaaaaaaaa 1140
   agccatttga aaaattaatc ttacatttat gtaacaaatg gaaacttaat ttaatagata 1200
   cccacaaact attttgttgt tattgttcta atatcgtttt ttttctactg gtaaaacaac 1260
   attaactcta attgacatta aatcttccag ttctgcaagt ctaatgtgaa atacagcact 1320
   tagtttaaaa gcagtgttac agattgattc ccaattctgc cccatgtttc ccttctcaga 1380
   cccctatgac aagtggaggg gaagatgcct ggtctgcccc accatcacta gttctccgca 1440
   gatagaacac agctcacctg attcacctag aagagtctat gcacactctt tcctgcccaa 1500
   tacattcctt cttactcctt ttcaaatagt gacctcacta tggctgcctt ttctagactt 1560
   cctatgataa gtagctggca tatcctccct ctaaacacat ccccatgttt cttcctccag 1620
   aaacgatcaa cagagagaac acatttctga aactgaaccc catgaccaca cctcatcttc 1680
   caagtggcag tgtatgaggt ctgatcatct aacatagctg cttctagatc taagtcttct 1740
   tcaagtactt aatactctct caaatgtcta gtctttgagg cagtgagtgt gggtcaagaa 1800
   aattctctac aaagacacag aagatagaga aacaactaac aagtcaaaga caaagccacc 1860
   aagtgaccca aacaggaaca gaaatgatgg acaatgaagg cctttctata gttcttgggc 1920
   tccctgggca caggaggaaa cccttcatgt gtccataaaa ctctgtgtaa agtaaagttt 1980
   taatttggtt taagacatat tctaatactg ggtgacgaca tccactgctt caacagatgc 2040
   cacttgagaa ctgactgtat cattgtgaga tctgtttttt tttttttttt ttgagacaga 2100
   gtcttgcttt tgtcacccag gctggagtag ggtggcatga tcttggctca ctgcaacctc 2160
   ctcatgccgg gttcaagtga ttctcctgcc tcagcctcca gaggtctgct cttttattgt 2220
   gttcctgttt ttgtcaaagt acctagaagg ggcggggctt atttgtccca tttcagaggc 2280
   tggatacatc catgctaaag ttaaataaac ccctcttctt agagtgacgg catttatgac 2340
   tttggtgata tttgacagaa atttctatca atgctgctgc cttcattaag ttccaaacca 2400
   taacgtggca cctcccagga ccactgctga ggtttcatga cactgactct ttcaactgag 2460
   gcaaatgttt tgaacttgtc taaaaaagcc tcttcacatt tctgttttca ggctaagctt 2520
   tcacttaaca taaagagaaa atttcacttg cagaaacctg gttaacacaa aggaaatttt 2580
   gactccacta aagtcttcct gtattgaggg aaaaattttt ctttcatttt tttcttttgt 2640
   caaaaaaagt ttctttcttt tttttttttt ttggcttggg acttgctgca gcaagttctg 2700
   ttagaatacc caccaaaggt ggtaattcaa tttaaaaaaa tatatatttt atttatttat 2760
   ttttctgaga cagagtctca ctcttgtcac ccaggctgga gtgcaatggt gcgatcttgg 2820
   ctcactgcaa cctccgcctc ccgggtttaa gcgattctcc tgcctcagcc tcccaagtag 2880
   ctgggattac aggcgcccac caccaagtct ggctaatttt tttattttta gtagagatgg 2940
   ggtttcacca tgttggccag gctggtctcg aactcctgac ctcaggtgat ccacccgcct 3000
   cggcctccca aagtgctggg attacaggtg tgagccactg cgcctggcct attttatttt 3060
   tagacaaggg tcttgctctg ttgcccaggc tggagtgcac tggtgcaatc taactcctgg 3120
   gctcaagtga tcttcccacc tcagcctcct gagtagctgg gaatataggc acgagccacc 3180
   acacccggct aattaatttt tttttttctt tagagacaag ttctacattg cccaggcagg 3240
   tcttgaactc ttgggctcaa gtgatcttcc taccttagta ggaaagtgct agtattacag 3300
   gtgtgagcca ttacactggc aattcttgag aggtggttgt aagaaataca accaagactg 3360
   gggagaaatg tcaatttaat caagctggca atagaattta gggttacaaa cagggctgaa 3420
   tcccaagaca tattttcctg tatgcctcct aaggtggtga gaagtaattc caaatggatt 3480
   tctcaagatg ttctgaacag cagcagcatc agaggaaaaa caggtttcaa ggtgactgct 3540
   ttgaaggata tattcctttg aatttattat ttatgaacaa ttaaaaacca acttctggtc 3600
   agttgtggta gctcacacct gtaatcccag cactatggga gataaaagtg ggaagatcac 3660
   ttgaggccag gaatttgagg ccagcgtggg caatatagcg agaccctgtc tctacaaaaa 3720
   aattttaaat tagcgaggca tggtggcacg tgtctggagt cccacctatt cgggaggctg 3780
   aggagggagg actgcttgag cccaggaatt caaggttgca gtgagctatg atcatgctac 3840
   cgcactttat ttatttattt atttatttat ttatttagag acggagtcta gctctgtcgc 3900
   caggctggag tgtagtggca cgatcttggc tcactgcaac ctccgcctcc caggttcaag 3960
   cgattctcct gcctcagcct cctgagtagc tgggactaca ggcacatgct accacgccca 4020
   gctaattttt ctatttttag tagacatggg gcttcaccat attggccagg atggtctcga 4080
   tctcttgacc tcatgatccg cccgcctcgg tctcccaaag tgctgggatt acgggtgtga 4140
   gccaccgtgc ccggccgcat ttttttttct ttttttaaag catctgcagg tatagctact 4200
   acactctagc ctgggcaaca aagcaacacc ccgtctcaag aaaacaaaac agaagccacc 4260
   ttcttatatc atggtcacat ctgatgaacc cttttgagtt cattaggaga atctctgaaa 4320
   tgtaaactga atggggtcca aattgaggga taaaatcatt atatcagtaa catcagtcaa 4380
   atatataaat atgtttatgt attttttttt cgagacagag tctcgctctg tcgcccaggc 4440
   tggaatgcag tggcgcgatc tcggctcact gcaacctccg cctcccgggt tcaagcgatc 4500
   ctctgcctca gcctcctgag tagctgggat tacaggcgcc caccatcatg ccaggctaat 4560
   ttttgtattt ttagtaaaga tggggtttca ccatcttggc caggctggtc ttgaactcct 4620
   gacctcgtga tccacccgcc ttggcctccc aaagtgctgg gattacaggt gtgagccact 4680
   gagcccagcc cgtttatgtt gtattttttt taacatttta ggttatattg tcatgtatca 4740
   cttccttcaa ttttcttccc taaatgagac agatttttta aaaaacttcc tttaatacca 4800
   aaccaggatt tttcctattc caaaagaaga aaataaacag ataaaacaag cagccctgac 4860
   aggagaaaga taaatgttaa aaaatatata taaaatattt ctcatagact cagctatcca 4920
   aatagccaaa tttgtctcta ttaccacaca ggacaaaaca gagaagaaag tgaacagttt 4980
   tgcaagactg caatggactc aggttttata aagcaataaa cccttcatgc ttttgacgga 5040
   gagaagacag ataaagagga aaacagaaaa caaaacctga gattctgtat gaaatgaagg 5100
   gagaaacatt ctactttgta atgtagaaga ggaaaacttg aaagagtcta agaataaaac 5160
   tgacaagttt agcaaatgga atgaaaaaat aatttgaaac tctaaaaatc atttaagaag 5220
   ctatacgctt cttagggtac agaacagggt ctactctatc ctaggtacag taggtttaca 5280
   tggggagggg actacaaaat taaaatggca aagcaacata agacagaaag cattctgata 5340
   aactagaatc ctacgtgcaa aaaagcaaac agacaatacc taggaatatc cttatcaaga 5400
   aagagcctgc aaaaacatag aaaaggcttt agttagtgca gcaaaaaaac aaaagaaata 5460
   agggagagaa tcagaaaaaa tatatatata aagaagacaa aatttgctga taagcaaagt 5520
   gaattcaagt ggacagacaa ttaacacaga atcccatgga acacaattcc atagaggact 5580
   tacaataaca tgggtccctc tcctgagact tccaactaag gaaaatgcca gacataagag 5640
   ggaaagtgga gaccaccacc atcagtaccc ctttcagtgg tgacttagta ttgagaataa 5700
   taggaagttg ctgtggagtt aagggaaaac caagtcaatg cattaacata aatgtaggac 5760
   tgggaagaaa aggaataatg aatggtccct tgtcaattat accccactat caatgtatta 5820
   gcaaagccct ctaagataca cacataatat acccaaattg caggtaagtt caactttaaa 5880
   cattaaacct tagacaaaca gaacgaaagt tctcttaacc aaactccatt ttgccactta 5940
   ccatattaac ctatgttttc catatcccta aaatatacag attgatggct agttctcctt 6000
   gagcattcta aactacaaag atgtctcatt tgcccagaca cttcgctccg aagagttgag 6060
   tgcttatcaa atgtcagtag tatctggtcc caaatctgtt tatgccagtt gtacttatta 6120
   ttattatcta acttcattaa acattaggca aatcaatgaa atatgaaaac ataaataatt 6180
   tttgttcctg tgaaaactaa gttgaatgtt ttcaaaaggt tagaaaatgg tgagttgttt 6240
   aaaaataact gttaagtttg ggtgagacaa atgtaaaaga aaggggaaaa tgataaaatg 6300
   tttaaatgat taatgttaat aaatgataaa aaatttaaag gattttgaac ttagattttc 6360
   ttcacaggtt tctaattctt gctccaccaa actgaaagtc atagatgatg aattatgggt 6420
   gtggttttgc aagaaagaca atcagaactc cagtaaatga tcttataatt aaagaaaagg 6480
   ccatggcttt ccagcaaaag tttgataaat taatgtagat ttatatagtt taaatgagaa 6540
   cagaaatatt taaggtgttt tttgttgttg ttgttattct tgacttcagc cttttttgat 6600
   gaactcattc taactcacaa gataagctgc ttctaacaaa gccaaattca tttcttgtag 6660
   cacctgacct gttgttgggc gtgaattaaa ggacttctta aagttactct aatggaggca 6720
   aaattagaat aaccagtatt tcaaccaaag cccatctttc cctgtatatt ttactaagta 6780
   ccctagggaa atttacaata catttttcat agctacaaag aaaatgtggt ggtaaaaccc 6840
   ggctgaacat tggcagaaaa cagaaagcta tgcagaatta ctgctgactt cagggaaata 6900
   gtattgctcg tgattttgcc catggatagt caaagcaccc ctctttattt ctgccactcc 6960
   ccctccccag acatacatat gtaccctctc actccttccc tctttctcca cagcccctgc 7020
   cagcaaacag aagtttgttt cttttttctc tttcctatgg taaactataa tacaagaaaa 7080
   caacagaaag aaattaatcc tggtccaaac ctttcattcc atttctgcct atcgtcttat 7140
   tccttccctc cctcctctca acatggatgt aaacaagttt ttcaggagca gactgaagca 7200
   cagcaaagtc ctgtggtggg aaggcagtgt gctcagccca cattagaaga ggcagccgct 7260
   cctgtttccc agcccctagg atatagagac ggagcctctg agacaatgag aactcccttc 7320
   ctgggtgagg taagaggatc ccagctggtt tagttctagt cttacaaagg aaagcagaat 7380
   ggctatgtac tttacgaact gtctgttatc acctgattat aaaagataat tttaagtgaa 7440
   acatttaaaa taaaaaacaa agtaaaatct attatttatg gcagccaatc ttggtcataa 7500
   gagttcatat tatttatcgc ttttgtgcat aaatgaaaaa caaatactaa ggataatgga 7560
   gcctatagtc agcagcagca aagacttaac acaagaggct tcattttatc tcagagggca 7620
   catttccatg atacttgggg agtgagagaa acctttctct ccacagcaac catttcacag 7680
   gaaaaaattt aatcttggca ataattaatt cgtactttta aatccttggg gaatatactg 7740
   ttttctttct ccaattttca tttatcactt gttacatcta ttcaaacttc acatgggaca 7800
   ggatcacttc cattctctaa gagtaactga tcacatacaa atatctgcaa tactttcttt 7860
   ctttcttttt ggagagtaca gcaggcaggt gaagaaataa agagattttt gaagctagaa 7920
   gggagaaggg agttaatttc tttgtccgaa ctgggagtca ttaatgaata aggcacctaa 7980
   cggtgtacca attaacgcct gcaagtgtgg acttcttcac actgctctgg gtgtcccacc 8040
   tgctagagtt gtgggaagac ttcaggtact tggcagaaat aatatttagg gagaggatgg 8100
   catcaactgc agcttcatcc acctcaggct tattccgaat acgacctaaa agaaaggata 8160
   aaaaaaacag agcacggaca catttgaaaa caaaacatga attcattaag cccaaaagag 8220
   acttttaaat ctctgtgtaa gttcaggagc tggaagggtc atttggttac ctgtagggtc 8280
   ctccacggtc tgactccatt acttcccaca ttgagttaac tatgaaacca tattgattct 8340
   ttattcaaag agtttcccac attcatccca acttttattc acaatatcat tgccctgatc 8400
   acaaaaggcc acctcctctc tggtctggaa cctgcaacag ccatttaact ggtctccctg 8460
   ctattcacac ctcccccgtt ccaatccatt ccactctcac ctaacagcaa cttctgacct 8520
   gctattcaca cctccccgtt ccaatccatt ccactctcac ctaacagcaa cttctgacct 8580
   gctattcaca cctcccccgt tccaatccat tccactctca cctaacagca acttctgacc 8640
   tgctattcac acctcccctg ttccaatcca ttccactctc acctaacagc aacttctgac 8700
   ctgctattca cacctccccg ttccaatcca ttccactctc acctaacagc aacttctgaa 8760
   gccactgact gacagtttct tgtttttttg tgtttttttt tttgaaacaa tgtcttggtc 8820
   tgtctcccag gctggagagc agtggtttga tcatagctca ctgcagcctt gaattcctgg 8880
   gctgaagtga tcctcccacc taagccacct gagtaactgg gactttaggc ctgcagcacc 8940
   acatccagct attttatttt attttatttt tttgtagaga cggggcccca ctatgtcgct 9000
   caggctggtc tcgaactcct gagctcaaac gatcctcctg ccttggcctc ccaaagtgct 9060
   gggattacag gcatgagcca ccatacctgg ccatgctgac tttattacac ccctttcttg 9120
   ctcaaaatcc tacactctgt tttcaagtta acactagcgg atgcagcatt gcatagacgt 9180
   taaggacttg gcctccagtg ctggactgcc tgggttccaa ctcctaccac ctgtgaggct 9240
   ttgagcctaa ggctcaacct ctctgagcct cagttccctc atatgtcaag cgagaataac 9300
   aattccttcc atatatggtt gctatgagga ttaaacaaac tagtattttt ggagcattca 9360
   gaaaagcacc tggcatgtgg taaatactac atcagagttt gttaataaaa caaactaaaa 9420
   atatatcata ggatccttca gagaagccat agtaactgct gcctctgcat cctccctctc 9480
   cttctctggt cagaaagcat ctcttctgtt tcggtattag ggttccatat aaacattatt 9540
   ttgtcaaagg acaitcagta ctttgtaaat aaagttaaaa aaaaaactaa attaaaaaaa 9600
   gaaaaattta ttatgaaaaa tattgaaaag ctactaccac tgctgcccca gacaagagag 9660
   aacataatct cccttccaac gattcttttt tttttttttt ttgacagggt cttgttctgt 9720
   catgcagtgg catgatcatg gttcactgca gcctcgactt ctcaggttca agcaagcttc 9780
   cagcctcagc ctcctgagta gttgagacta taggcatgag ccaccacacc cagcttattt 9840
   gtagagagac agggttttgc catgttgtct aggatagtct caaactcctg aacttaagtg 9900
   atccgcctgc cttggcctcc caaagtgctg ggattacagg cgtgagccac tgtgcctggc 9960
   cccaaggttc ttaatctggt ccagagagac acaagcactc tggggacaga attcaaaggg 10020
   tccttgagct tggatgggga aaaaaattac atctttattt tcacaaacct ttaactaaaa 10080
   attggcatct ccttcaatca caaaggaagg caataaacca cagaagaatt ggccagtacc 10140
   cgtgactctg tccccagtag aaaccacaga tgtttccatg tgcaaaatat cttatttatt 10200
   tatttatata tttttttgag atggagtatt gctctgttgc ccaggctgga gtgcagtggc 10260
   acaatcgtgg ctcactgcaa gctccgcctc caggttcatg ccattctcct gcctcagcct 10320
   cccgagcagt tgggactaca ggcgcccacc accacgcctg gctaattttt ttgtattttt 10380
   agtagagacg gggtttcacc gtgttagcca ggatggtctc gatctcctga cctcgtgatc 10440
   cgcccatctt ggcctcccaa agtgctggga ttacaggcat gagccaccac gcccggccta 10500
   tttatttatt tttaaacaga gtctcactct gctgtcttgg ctcactgcaa cctctgtctc 10560
   ccaggttcaa atgattctcc tgtctcagcc ttccgagtac ctgggactac aggcgcccgc 10620
   caccacaacc agctaatttt tgtattttta gtagagacgg ggtttcacca tgttggccag 10680
   gatggtctca atctcttgac cttgtgatc 10709

<210> 73
<211> 6519
<212> DNA
<213> Homo sapiens

<400> 73
ggatccacct gcctcagcct cccaaagtgc tgggattaca ggtatgagcc atcacaccca 60
gccagaacca tggtgttttt agtaattcac tttggtaatc ttgagaaagg tttttttaag 120

ccacttgctt tatctttatt agacatacat agaacttccc ttactatata cttagttcta 180
atttctagag ctgtttgggc aacatcggag ctgcactgtg ggtttaggac atgttatttc 240
ccctcctgcc ttgtatccag ggcttcattt agtcattgag gtttttgttt gtttgttttt 300
aaatttactg atttattaga aaggacagtg caaaggagac caatgaacgg cagatggacg 360
aatgcatagg gagaggagat gtggagcttc catgccctcc ccgggaactc agcccttcag 420
gagcctccac gtggtcagct atcccaaggc tcctggctgt tgacatttta tgttcagctt 480
gcacttctct ttctcttctt tagtgcctgc aacttctcac ccacactttc aacagagaat 540
tcagccaggt gcacggcagc gtcagtgact gtaaggtgag cacattgact gtaattttta 600
gccagtatgt tgataactga tttctccaca gcagcccaga ttacctattc ctggtttttg 660
ctgcttttaa gcaacttgtc atgggcatag cattgtattt gaaaatttat gacatactgc 720
tctggtattc attctaattt ttcagagtcc gaacactgac ttctgaagat aaaagtactc 780
ctttgtgtct cttagagtga ttatcagatg ggaaacattt tggctttttc atgactcctt 840
tggaggagaa tattctatgg ggaggtggta tgttattctt tgccagggta caaggaaacc 900
ctgaggttcc tggtggcata aagttttatt gacttcaaca aagagtgaag taaacacttc 960
agagaatttc tgtgttattc actcaattct agtcagcttg accttaaacc ctcccggctc 1020
attcctgcct tggggtcttt gcacacctac tatttctttg cctggaattg cctttctcca 1080
ggtattgcca tggttggctc ccttacctct ttcatttttc tactccatta tcacctcctg 1140
tgagtcgact ctgttaacac ctgataataa atctgacagc tgtctgaaac tttctaccta 1200
cacctctttc ctgctttatt ttttgctata gcactaatta ctacctgatg ctttttaaag 1260
tgtttgttga gtggtttatt gtctgtctta agctccaggg aggcagatcc tctgccatat 1320
tattagaaca atgcctagta catggaaggt gcaccactgt aataccactt atattaattc 1380
aggggctggg tgtggtggtg catacctgta atcccagcac tttgggaggc caagatgagg 1440
ggatcgcttg aggccaatag gttgagacca gcctgggcaa tatagcacaa ataccctatc 1500
tctacaaaaa aatttttgaa aaattagcca aatgtggtgg tacacaccta tagtcctagc 1560
tactcaggag gccaaggcag gattgcctga gcccaggagt ttgaggctgc agtgaggtat 1620
gattgtgcca ttgcacccca gcctgtgtga cagtattttt ttgtctctta atgataacgg 1680
actggggggc tggctgcggt ggctcatgcc tgtaatccca acactttggg aggccaaggc 1740
gggaagatca cctgaggtcg ggagtttgcg accagcctga ccaacatgga gaaaccccat 1800
ctctactaaa aatacaaaat tagccggata tggtggcgca tgcctgtaat accagctact 1860
caggaggctg aggcaggaga atcgcttgaa cctgggaggt ggaggttgca gtgagccaag 1920
atcgtgccat tgcactccag cctgggcaac aagagtgaaa ctccgcctca aaaagaaaac 1980
aaaaaacaaa aaaaccccca aaataatggg ccaggcatgg cggctcatgc ctgtaatccc 2040
aacactttgg gaggccgagg tgggcggatc acctgaggtt gggagtttga gaccagcctg 2100
accaacatgg agaaaccctg tctctgctaa aaatacaaaa ttagccgggc gtggtggcac 2160
atgcctgtaa tcccagctac tcaggaggct gtggcagaat tgcttgaacc tgggaggtgg 2220
aggttgcggt gagccaagat cgcaccactg cactccagtc tgggtgacag agcgagactt 2280
cgtctctcca aaaataaata aagataatgc aggagaggcc agtgacagtg gctcacatct 2340
gtaatcctgc cagcttggga ggtcaaggtc gatggatctc ttgaggccaa gagttcaaga 2400
cgagcctggg caacatgaca aaaaccccat ctccacaaaa aaatacaaaa attagttggg 2460
tgtggtggtg tacacctgta gtcccagcta ctcaggaagc tgagatggga ggatcacttg 2520
agtcctggag gctgaggcta cagtgagcca acatcgcacc actgcactac agcctggtag 2580
acatagcaag accttatctc gaaaaagaaa ataataataa taataataat aagaagaaga 2640

agaagaagaa gacatagcaa gaccttatct cgagaaagaa aataataata ataataataa 2700
taataatgac aacaataata ataatgcagg agggcagtag gaacataaaa aaccaacctt 2760
gcccctttta taaatgtctt gattcctggt gtggcactta ggccgtaaaa tatttgtagg 2820
cttgtttttt ttcttttttg aaaatatttt caccagttgt ggtggtacac gcttgtagtc 2880
ccaggtactc aggaagccag ggcacaggag gatcgctgga gcccaggagt tggaggccac 2940
aggaagctag gattgcatca ctgccctcca gcctgggcaa tagagcaagg ccttgtctct 3000
taaaatttta cctatgtatt tatttattga taaattatgt ataggcttgt tttcttaaat 3060
gaccttctga aacaagatgt atcaaagtga ggttctggtt gttgaaactc cctattgtta 3120
ctgtagaggc agcttttttc agctgaatgt aacttgtagt gttcggtttg cttccagttg 3180
tctgatatct ctccaattgg acgggatccc tctgagtcca gtttcagcag tgccaccctc 3240
actccctcct ccacctgtcc ctctctggta gactctagga gcaactctct ggatcagaag 3300
taagtaccca gatttcactg agagaagtca atctaagaac caaggtaaat gtcaaccttc 3360
ctctagctca atggttcttg actgaaggca gcaccttctc cctaggcagc atttggaaat 3420
gtgtgggagc ctttttggct atcacagtga ctggagaggt gctagctact ggcatctctt 3480
cacatcatag cttactaacc ttctgcatca tcaactcatc atgactgtct tcttcatccc 3540
tttctgctag ttttatcctc cattcctacc cctgcttgtc tccccagtca taataccaga 3600
cataggaagt gctatcttgg gaaaaatatt ttgaaactgt tttggaaaaa aatattttga 3660
ttccacagca aattatgtag gtaagaaaaa taaagacaag gccaggtgag gtggctccca 3720
gcactttcgg atgccaaggc aggaggattg cttgaggctg ggagttcgag accaggctgg 3780
gcaacatagc gatatgctgt ctctacaaaa aataaataag atacccaggc gtggtggtct 3840
gtgcctgtgg tcccagctac tcgggaggct gaagtgggag gatcgcttga atccaggagt 3900
tcaaggttgc agtgaggttt tattattgca ccactgcatt ccaccctggg cgacagagca 3960
agaccctgtc cctaaaaaaa ataaaaaaaa gacaaatgta gtctagagtc catgattcat 4020
catcataatc cgttctttgc aacacaccct cttgtgcatc tccatcaatt atgctcacct 4080
ggttatcatc cacctgtcta tcttattgta ccaacactga cacaggcaaa ggtggctgga 4140
gaaaataaca caaccaggta atttcattaa atttaggatg ccacagactg tagggactat 4200
tctgtgccac taagagaaga agaaaaagca ctgacaacta acttaagcat gccatcaatt 4260
ataagatgaa tcccaattta agaaatggtg gctgagtgca gtggctcata cctgtaatcc 4320
cagcactttc aggggctgag gtgggaggat tgcttcagcc cacaagtttg agaccagcct 4380
gggcaacata gggagacccc atctctgcac caccaacaaa aaattagcca ggcatggtgg 4440
ctcacgccta tggtcccagc actttgggaa gctgaggtgt gaggattgct tgaggccagg 4500
agctcaacac cagcctgggc aacatagcaa gatcctgtcc ctacaaaaaa aagtttttaa 4560
ttagctgggt gtgatggcac acacctgtag ccccacctac ttgggaggct gaggcaggag 4620
gatcacctaa gtccaggagt ttgaggctgc agggagccgt gattgtgcca ctgcactcca 4680
gcctgggcaa cagagtgaga ccctgtctct aagaagacat ttcttctggt gctttggtga 4740
tgcttctgct cagcctttct gtctgtagtc tcagtccgat agtccagtct gctttgaggg 4800
cccaccagga cctggaggag gggccagttt cagtcaaggg tctgcagttg accttgagaa 4860
cttagcacat tcattatctt tttgtgggca cttccacctc ccattagaaa gaaaaagatg 4920
ggcccagtgc ggtggtggct tatgcctgta atcccagcac attgggtatc cacggtgggt 4980
ggatcacttg agctcaggag ttcgagacca atctgggcaa catggcaaaa ccccatctct 5040
acaaaatcca aaaaaatgag ctgggtgtgg tgtcacatac ctgtagtctt agccacttag 5100
ggggccgagg caggaagatt acttgaacct gggaggttga ggaggctgca gtgagctgag 5160

atcacaccac tgcattccag cctgggtgac aacgtgagac cctgtctcaa aaaaaaaaaa 5220
aaagaaagga aaagataggg gttggtgtct atgggatatg ttggtgtcta tgggtatggg 5280
ctctcagccc gtctcttctc attaactgaa ccacactgtt tctttacaca ggccccacat 5340
caagaccatc ttccaaggca ttgctgcaaa agtgggaact ggagaaccct gctgtgactg 5400
ggcaagttct gggctactct ttaaagccaa ttggcaacat gggcgtgtct aagtgatgaa 5460
gtgcgtggag aaaggccacc gtggtctccc aggggttagc ttggtgacat aaacgtctcg 5520
atctttgatt gctagaatag ccaacgccta gaatgcatgc ctgctgttca catctttgta 5580
tcttagctga ctgttcatag ccttgaggtt gatattaaaa gcagccttct ataattcatc 5640
taaaagatga attctttaaa aaggaatagg atgaagtgaa ataagatttt ttcgagccat 5700
attatttcat ggccaaagga aagaatgtca gaatcaaaag tggcgtttgc ttgcatgggt 5760
gaaaggtgga agattgaagg tactgttgct gtgtcaacat tccatgaacg tggaaaattc 5820
cccatcctgt agtgtgggat tcaggcattc tgtgcttgaa caaagtaacc tgtggacttg 5880
caccctcact gaatatgaag gaggcctaac ctagttcata gtacagtggt gcgatcttgg 5940
ctcaccgcaa cctccacctc ccaggctcaa gcaatcctcc tgcctcagcc tcctgagtag 6000
ctgggactgc aggtgcatgc caccacgcct agctaatatt tgtatttttt gtagagacgg 6060
ggtttcacca tgtcacccag gctggtctca aactcctgga ctctcaagtc atctgcctgc 6120
cttggcctca caaagtgctg aggttacagg catgcgccac catgcccggc ctcgtgttgc 6180
tgttaaagta aactttatct catattgtag ttcagccatg ctgtggatgg aaaaaagcat 6240
taggctcagg agacctgagt ggcagttctg gctttccata aacttaactg tgacatcagg 6300
caagccacgt cccttcatgg acttcagttt ccttatctgt aagatatggg gttggattac 6360
atgatctcca agctctcttc agctcctgat tccctttaat tttatatcag caattttaat 6420
gtgaatttta ttattcaatt ttattttata tgaaatcaat gctgaggttg aaataaggac 6480
tcaggccttt gaggaatccg ggaagccctc cctcctagg 6519

<210> 74
<211> 9799
<212> DNA
<213> Homo sapiens

<400> 74
aggcaaaatg ccgcaaaaaa gggaataagg gcgacccgga aatgttgaat actcatactc 60
ttcctttttc aatattattg aagcatttat cagggttatt gtctcatgag cggatacata 120
tttgaatgta tttagaaaaa taaacaaata ggggttccgc gcacatttcc ccgaaaagtg 180
ccacctgacg tctaagaaac cattattatc atgacattaa cctataaaaa taggcgtatc 240
acgaggccct ttcgtctcgc gcgtttcggt gatgacggtg aaaacctttg acacatgcag 300
ctcccggaga cggtcacagc ttgtctgtaa gcggatgccg ggagcagaca agcccgtcag 360
ggcgcgtcag cgggtgttgg cgggtgtcgg ggctggctta actatgcggc atcagagcag 420
attgtactga gagtgcacca tatgcggtgt gaaataccgc acagatgcgt aaggagaaaa 480
taccgcatca ggcgccattt gccattcagg ctgcgcaact gttgggaagg gcgatcggtg 540
cgggcctttt cgctattacg ccagctggcg aaagggggat gtgctgcaag gcgattaagt 600
tgggtaacgc cagggttttc ccagtcacga cgttgtaaaa cgacggccag tgaattggcc 660
cttatggccg gatccaagcg gtttggccgt gggccgtgat ggcagcaggc ggtgggatgc 720

ttgtagctcc tcacagcagg attcctgccc actgtttttt ctctgttggg agggaagctc 780
ttttctagga gtgtctcagt tctgcttttg gcattagtga tggtggtggt acagttggaa 840
ttagtgccat gtcatacaca aatgttccac aaggcgggag tgtttcactt tctggtgata 900
aacttgatgg tcattgttat gattaagata atgccgggca ggccgggcac agtggctcac 960
gcctgtaatc caagcacttg gggaggccga ggcgggcaga tcacgagatc aggagttcaa 1020
gaccagcctg gccaatatga tgaaaccccg tctctactaa aaatacaaaa ttagtcgggt 1080
atggtggcac atgcctgtaa ttccagctgc ttgggagcct gaggcaggag aactgcttga 1140
acccaggagg cagaggttgc agtgagccaa gatcgcgcta ttgcactcca gcctgggtga 1200
cagagcaaga ctctgcctca gaaaaaaaaa aaaataataa tgctgggtag tgaccttgtg 1260
attgttacag ctccctttga tcaaagaaat atagctttca ggcataaacc tggaagtctc 1320
cctctgaatc cagcagttgt tttcattgat gcttgtcagg ttgaagatgc tttcagtgat 1380
gctctctata ctcataaata agcaaatgtg gcaggctttg ctttctggat cccaggatta 1440
aaactaaccg tgaccactac tccaaacaaa acacaatatg cctaggggca cggatgaacg 1500
tccagggagc ccgggcccca ggctttgttg cgtgttccct gctcctctcc atctggtgtg 1560
gaaacactgc ccagggagaa aggaggaagc tcactgtgga cagtcttctt tccttctgac 1620
agaccaggtc atctggcttc cgagatcatc agagaagata agtctgtctc tttcagctgc 1680
cagtaagttt tccaggatga gaggggaaaa agaaagcctc cagtgacttc agttgctttg 1740
ccagttgtct tgggattgtt ttacaccatc ctttacttcc cttgctcaga cctctctgtt 1800
tcaccattgc tcaggcattc aggaaagtat ctgctcactc ccacttggtg agtcctcggc 1860
cttgaggttg ctgactctca ggcgttaggc agctggatga cttcccgctt cacgcagcaa 1920
aggccagggc ttgcgcgcct ctgcagagtt gttgctaggg agcttgtgtc atcatccaca 1980
accttgtttc tcacttcctg gttgggctca tctctgaaga acaggtctcc cagcttcgct 2040
ccttatcact gcattgtgaa gaggaggaaa agtgaatcac ggagagagaa aggaaaggat 2100
agaatcacag gctgcgtctg cacctgaaaa gtgacccgcg gaaactctat ggcggatttt 2160
ttttttaact ttcttcttcc tgttaaaaca taggtcacta actgtgatgt tatttgtttt 2220
ctaagtggta tgtgagattt tctaatgtag ttagaagttt cattgtctga tggacacaat 2280
atgcccttcc ggttctattc aaaccagcag gatctgtcgg tgcttagaga tggctgcctg 2340
gactggaatc aaatctaatt tcagggaaat gaagatggaa tttgaaggtc acttttaaaa 2400
ttaagtcatt gatgctgctg ttacagagtg tgacagagga tccatgtctg tgacacagga 2460
cggtgggaag cctgagagag agtgaaatta tgtgatacac tgaaatgact tttgtttttc 2520
ttctaactca tacaaaactg gtttggaaag tctttgcttt ggaagcgtca gacattagaa 2580
caggccaaac tggactgtct gttcatagcg tgcctgaata agaaggcctc ttagggagcc 2640
agagggagca gagtggtcgt gtcctgcgtg ctcttcaccc tctggggcgc ccctgctgcg 2700
gctggcaggt gcagacagcc tttgctggtc cccagcacgt ccagggtggg tgctcccttg 2760
cccgacagaa ccatccccac tgtgaggctg tgagagattt gtggcaggaa ctgtttatga 2820
ggctctagtt gttgctgttg tggcgggaaa gttaagaaac atagccctta aggaaaccac 2880
ctttatgtat tttcttaaag cacgccttta aataagcaaa aactttaaaa ggcaggaaag 2940
agaattctta ggcaaattca gagaaataag tgctagttaa tactaatcac ctcctcctct 3000
gtctctcatc ctcctttctc ccatcaaagc aaaatatggc ctcaccacca gccccaaatc 3060
agtgctcaga ccctctctgt gtctgtgtgc cctcctggga gtcagtcagc gctcaggcca 3120
ggactgtgca gggccagcca gcccatgcgc tagtcaggag cacaggcaag gggtgcttgt 3180
ggcagtggcc gggcacctga gccccagctc gttgttaaac gtgctgacgg caaggggcaa 3240

tggagtgagt ttcccaacta agaaaccact attatatatt ttttcccttc agtcacatag 3300
acttcagaca actctcctat tttttatgga tttttcagct catttcagat gaaggaacta 3360
agtcattgtg aactgtctct tgagatctaa aaacaagatg acttttcctg gcacatattc 3420
caaagcaaag actttgttgc ctgctgctta ttgtctaatt tacagggata tttaattttg 3480
tcaggtctat gtatatttat ccagctatac ttacttgcac agtggattgg agagaaagga 3540
ttctccagtg tgcacactca tcggtactct ttctgcattt ccctcgtgct gtgtcccgct 3600
cgggttccaa tggacagtat cagggcttgt ttgacttagg tctttcagtt ttcctttcgg 3660
ttccctttta aaaatgtgat tgttaacctg cctcttgaaa gattcaaccg ggtgtggtgg 3720
ctcacgcctg taatcccagc acattgggag gccaaggcag gtggatcacc tgaggtcaga 3780
agtttgagac cagcctggcc aacatggtga aaccccgtct ctactaaaaa tacaaaaagt 3840
agccaggcgt ggtggcgtgt gcctgtagtc ccagctactt gggaggctga ggcaggagaa 3900
tagcttgaac ctgggaggtg gaggctgcag tgagttgaga ctgcaccatt gcactccagc 3960
ctgggtgaca aagcaggact ccgtctcaaa aaaaaagaaa agattcatga tgctgctgct 4020
cccagaaggt ttgctggatg tgtttacata ggactctaac ttgtgtgcac tacagttgtt 4080
caccagggcc agtgattcac cccagtgtgt ggccagacca tgactgtgta gcaggaatgt 4140
tttaatttgt gcttccttag taaattgaaa tatcagctga gagattattt gctgctgtta 4200
ttcaaaaggc catttatgaa gttagtattt gagccccata agatctttaa aaagcctcca 4260
atcatttaaa ggaagaaatc agagttgcta taaaattcag taaaaagctc atagccaaac 4320
ggctgtgctc agatggaaag tctgagctga ggttggtctc ttgccaaacc gtggctgttg 4380
tgtgttgttc ttcatgtctt cgagttcatt ttttttcatt ctgcctattc tggcatcagc 4440
tcacttgagg agtccctcag ccttcttgta tttaaggcat cgtcttagac tttgtggctc 4500
taaagtacct gtctgttgag atttcaagtc tcttgtcacc atcctcacac atgacaacaa 4560
aacccataat gcataagtgg cctttttgaa ccaagacttt gcaaactgat ctctcccccg 4620
tgaaggagtt gagcacatta gcaacaatgt acattaattt tggattttca ttttcatgtt 4680
ttattttgta aatattatct gatgtttgga gcttgagtat acagactgta aatatagttc 4740
ttgtatttgt actaattctg attcttttgc tgtatagcct tagatgtgca atgcagacac 4800
tatctaactg tgtgtggtaa ccttgcgtca cggagctgtt agtgaacgag gtaaaaataa 4860
taaaggtaca gccagtgcat cagaaggttc tcgatgtgca tttattcttg caccccttga 4920
aaggtaattg cacaaagatt tcttttcttg atttgcaaag ataatacctt tctaagacag 4980
aagtcacgat atcatcgctt aagtattcct ttctgatatt caaaatcgtg gtttttatat 5040
aaccaagaaa gctaatatgg tgctgtttct ttgaaagtac ttttctccca aagatgtaag 5100
tggttatttt agctagaaga tttgttgttt ttctcctgaa gcctccttgt atgccttcca 5160
ataattctgt agtgttgtgt tggcttcttt tttttttttt ttttcttttt gagacagagt 5220
cttgctctgt cacccatgct ggagtgcagt gatgtgatct cgggtcactg caacctctgc 5280
ctcctgggtt caagcgattc tcctgcctca gactcctgag tagctgggat tacaggcgtg 5340
cgccaccatg cgtggctaat ttttgtatta ttattattat tattattatt attattttat 5400
ttatttattt atttattttg agatggagtc tcactctgtc gtccaggctg gagtacagtg 5460
gcatgatctc ggctcactgc aactttccac ctcccgggtt caagcgattc tccctgcctc 5520
agcctcccaa gtagctggga ttacaggcat ccatcacgcc tggctgattt tatttttagt 5580
agagaggggt ttcactgtgt tggccaggct ggtctcaagc tcctgacctt gtgatccacc 5640
cgcctcagcc tcccaaagtg ctaggattac aggcttgagc caccgtgccc aacctataat 5700
tatttttttg agatggagtt tgactcttgt cacccatgct ggtgtgcaat ggcaccatct 5760

cagctcactg caacctctgc ctccccagtt caaacaattc tctttcctca gcctcccagg 5820
tagttgcaat tacaggtgcc cgccaccaca cctggctaat ttttaaaaat atttttagta 5880
gagacggggt ttcagcacat tggccagact ggtctcaaac tcctgacctc aggtgatccg 5940
cccacctcgg cctcccaaag tgctgggatt acaggcgtga gccaccgtgc ctggccatgt 6000
tgggtttttt tgggtgggga agggtaaatg gggatttgaa aagttgaaca tgtcaattta 6060
atttaacaag ccctcatcac gagcagagca tgagcagggg ccactgtcct gggtgtttgg 6120
ggagaaacag aagaagggag ggaggagcag ccctcaggtt aaaagcaatg atttgttcca 6180
accaatattt gagtacccac cctgtgcctg gtgtgctgct gggtgcagta attcagcagc 6240
caacaaagca agcccatgca cttgtgccct cccattccag tggggaagac aatgtttaaa 6300
caaatttaca acatcggtcc taagtatttt ggaggaaagc agctaaataa aaggatgaaa 6360
gagggatggg aacgagggat gtttagatgg catagtcagg gaaggcctct cttcggagat 6420
cacatttgag cagactctag aatgaaagga tagggtaagc catgcaaacc cctgggcaaa 6480
gaggtctcca agtagagaaa tgaagccctt gaggcagcag tgtcctcagt aggttccaga 6540
aatagcaagg tcgctggtac agagtcaggg actgggagcg caatagaaga catcagaaag 6600
tagccaggga ccaggatatt tagggcttag gtgaggactt ggaatttcat tagagtgaat 6660
gggaagccat tgaaggcttt ggagcagagg agtcacaggg gcttatttac atttttaaaa 6720
gaccattctg gaaaacagtc tttgtactca ggcagggaca agtggaaaaa aggggcaaaa 6780
taagattgtt accccagtat tccaggggag gagacggtgg ctcagactaa ggtagtagag 6840
tataggtggg acgaaatggg cagattcaag gtacattttg aagttagaac agttttctga 6900
gagacttcat gcagagttgt tggagagaaa ggagaaaagt aactaaaagg gtaagtgcct 6960
gagtaaaaga acagcattgc agttttctaa ggtgaagaca attggtagaa caaaagctga 7020
gaatggtcat gcctgtaatc ccagcacttt gggaagccaa ggtgggagga tcactggagc 7080
ccaagacttc gagaccgacc agcctgggca atgtagtgag accccatctc tacaaacaat 7140
taaaaattag ctgggcatga tggtgcacgc cagtagtgcc agatacccag gagactaagg 7200
cagaaggatc gcttgagccc aggagttgga agctgcagtg agctgtgatc ttgccactgc 7260
actccagcct ggtcaacaga gttgagaccc tgactgttaa gaaatagcca ctttgggagg 7320
ccgaggcggg cagatcacct gaggtcagga gtttgagcca gcctggccaa catggtgaaa 7380
ccccgtctct actaaaatac aaaattagcg gggcgtggtg gcaggcgcct gtaatcccag 7440
ctatttggga ggctgagaca ggagaaccgc ttggacccag gaggcagagg ttgcagtgat 7500
ctgagatcac gccactgcac tccagcctga gcaacagaga gcgaactgtc tcaaaataat 7560
aataataatt tgatttgagt catgttcact ttcagatgcc aacacatgca cctaagtgta 7620
tagtcaacaa aacagtgggc tggactagcg ggattcaagg ggagtggttg gagctggaga 7680
cacacatttg ggagttggcg gcatgaagat agcagatcga gcctcaggca gggaaggaga 7740
cctggagcgt gtgtgtggct gacaggaacc aaggactgat cttggagctt ggtgcgctcc 7800
aacacatgga ggtcaggaag agagaggagt ccagccagga ggacaagcag tggccgtgga 7860
gggagagtgt ggaagactga ggaagtggtt gctttgtcca gggcagccaa taggttacag 7920
gaaggactga gcagggacca caggttttgt taactgaaag gtgtaagaag tccgagggag 7980
ccaggggata ccctaatttt tcccaatttc agaaccaatt aagaaacaaa aaataagacg 8040
atgggagatg ggagaccaaa ataccaccct tattactgat aaaactgctg ttagaaaatg 8100
tggttacatc tgggaacact ggatttcttt tcttttcttt tttttttgtg atggcatctc 8160
gctctgtcgc ccaggctgga gtgcagtggt gtcatcttgg ctcactgcaa gctccgcctc 8220
ccagattcac accattctcc tgcctcagcc tccccagtag ctgggactac aggcacccgc 8280

caccgcgccc ggctaatttt tttttttgta tttttagtag agacggggct tcaccatgtt 8340
agccaggatg gtctcgatct cctgacctca tggtctgccc gcctcagcct cccaaagtgc 8400
tggaattaca ggcgtgagcc accgcgcccg gcctggattt attttgtaag gctgaacctc 8460
agaatgaggc ggtttagagc agtgcttctt gaactttatt gtgcaccaga atcccctggg 8520
gatcgtacta aaatgcaggt tctgattgag tagggcttgg gtggagcctg agattctgct 8580
tttctaataa gcttccaagt gatgtcagta ttgccattgt ggggatcact ctttgagtag 8640
cagaagttaa gaggaacgca gcgcatatgt cccctgaagg cagccgaggt tctgagagag 8700
agggaggaag tggccaggca gtctatgcct aaccgctcct tccagactca caggaacatt 8760
ctagtaatgg ggctcacttc tcatggaggg aggcatgcag gagtgcttct acactagcaa 8820
ggtcattggt gaggggagca tttgtttgag tggtgcctaa ttggagtagg ttctagggaa 8880
agtgggagga gaggagggtg aaacagtaca aacaattctt tcatagattt ttctgtaaaa 8940
gggagaaaga caaatggagt aactggaagg ggatatgggg taaaggagag gctttgtgtt 9000
ttaaagaagg gaaatagcat agcttgtgca tgtactaatt ggaaaaaatg aactgggagg 9060
gaaaacctgg cagtggggaa gacagtgagg tggagagagt tgtggagtgg gcctgaggga 9120
ctgggggcca gttgcacagc agatgaggta ggtcctagat ccaactgagc accctggctt 9180
atatccacag tgctgccgac agctcagtga taggaacact gcaagtctct ggattgctag 9240
gactcttcag tcttaagtta atgtctggct gggcgtggtg gctcatgcct gtaatcccag 9300
cactttggga ggctaaggcg ggtggatcac ctgagctcag gagttcaaaa ccagcctggc 9360
caacatggtg aaaccctgtc tctactaaaa atacaaaaat tagctgggca tggtggtggg 9420
tacctgtaat cccagctact cgggaggctg aggctggaga atcacttgaa cccgggaggc 9480
agaggttgcg gtgagccggg atcacaccat tgcactccag cctgggctac agagcgagat 9540
ttttgtctca aaaaaaaaaa aaaaaaaaaa gttagttaat gtcagctcta gggcttatgt 9600
caggccacgt cttcctgaaa ctggacttgc agtttaaaat gggagatcat ccccgaatca 9660
gtgcatatta tgctggttgt gccctcaact agacctcacc caggagcatg taggattgaa 9720
tcctcactag ggttgcacgc ttagtaaatc cccatgtctc ttgcaaagct gatagtttgg 9780
gaagtcaggt gacccatgg 9799

<210> 75
<211> 13864
<212> DNA
<213> Homo sapiens

<400> 75
   cctaggctca agtgatctgc ctgccttggc ctcctaaaat gctgggatta cagccatggg 60
   ccactgtgcc cggccataat ttttgaattt tttttttttg gtagagatgg gtttccgcca 120
   tgttgcccag gctgttctcc aaccctgggc tcaagtgata ctcctgcctc ggccttccaa 180
   agtgctggga ctataagtgc aggtgtggac caccgtgctt ggcctttttt tttttctttt 240
   tttttttttg agacagggtt ttggctctgt cactcaggct ggagtgcagt ggtaccatca 300
   tgactcattg cagcctcaat gtcccagcct caagcagtct ttccacctaa ccctctcgag 360
   tagctgggac tacaggcatg caccaccaca cctggctagt cttattttta atttttgttt 420
   ttttaagtag agatgggggt ctcactatgt tgctcaggct ggtctccaac tcctgggctc 480
   aagtgattct cccacctcag cctcccaaaa tgctgggatt acaggcgtga accgccatgc 540
   ccagcagaaa aattgtcatt ggaataaagt ctggggtttt gttaaaagtt ttatatcggc 600
   cgggtgtggt ggcccatgcc tgtaatccta gcactttgag aagctgaggg gggagattcc 660
   ctgagctcag ggattcaaga ccagcctgga caatgcagtg aaaccctgtc tctactaaaa 720
   tacaaaaaat tagctgggcg tggtggcgtg cacctgtagt cctggctact tgggaggctg 780
   aggcaggaca atttcttgaa cccaggaggc agaggttgca gtgagccgag atcacgccac 840
   tgcactccag cctgggcgac agggcgagac ctggagtgca ggcacaatta tggctccctg 900
   cagccttgac ctccccaggt tcaggtgatc ctcccacctc agtttttcta tttttagtag 960
   agccctccac ctcccgggtt caagcaattc tcctgcctca gcctcctgag tagctgggat 1020
   tacaggcatg tgccaccacg acgggttaat ttttgtattt ttagtagaca cagggttata 1080
   ccatgttgcc caggctggac ttcaactcct gggctcaagt gatccacctg tcttggcccc 1140
   acaaagtgct aggattacag gtgtgagcca ccacacctgg ccagcagttt cttaaaaagt 1200
   tacacttacc ttaaaaccca gcaatccact cctaagtata tgtctaaaag aaatgaaaac 1260
   aacaggctgg gtgtggtggc tcacacctgt aatgccagca ctttgggagg ctaaggtggt 1320
   agctctccag accacccagg gcaacatggt aaaaccccgt ctccactaaa aatacaaaaa 1380
   aattaaccgg gtgtggtggg gcacacctct agtcccagct acttgggagg ctgaggcagg 1440
   agaatcactt gagccccaga ggcaaaggtt tcagtaagcc gagatcgtgc cactgcactc 1500
   tagcttgggc tacagagtga gactccatct cacacacaca cacacaaaag aaatgaaaac 1560
   aacaatttgt acatgagttc aaaacagaat tatttatttc ccaaactaga aacaatccaa 1620
   atgtctatca acagataaat ggataagcaa atgatgacac aattgcatta atggaatacc 1680
   actcagcaat gaaaaggaac aaaccactca tacctacaac acagatgagc ctcagaaacg 1740
   tgatgctaag tgaaagaagc cagaccaaaa aaattatatg ctattattcc atttctatga 1800
   aatttctgga aaaggcaaaa accatgcaaa gtagatcaga ggttggctgg gggtgagagc 1860
   agagactgac tacaaatagg cccaagggaa cgttttgggg tgatggaagt tttctataag 1920
   tggattatgg tgatggctac acaacagtat aaattaaagg tcatccaaat gtgtacttac 1980
   aatgtttgaa ttgtatgaca tgtaaattat ctcattaaat ctgtatttta aaaaaagaac 2040
   tgtgctgtga agtttgacat attttaaagt aggatgtctt agccgggcat ggtgttgcat 2100
   gcctgtagtc ctagctactc aggaagttga ggctagaaga ttgctcgacc tcaggagttc 2160
   aaggctgcag taagctatgg tcatgccact gcactccagc ctgggcaaca gagcaaagac 2220
   ctggtcttta taaaaattaa aaaccggccg ggcacagtgg ctcacacctc taatcccaga 2280
   actttgggag gctgaggtgg gtggatcact tgaggttagg agttcgagac cagcctggcc 2340
   aacatggtga aaccctgtct ctactaaaaa taaaaacatt agctgggcat ggtggcacac 2400
   acctgtagtc ccagctactt gggaggctga ggcaggagaa tcgcttgaac ctgggaggtg 2460
   gaggttgcag tgagcccaga tcgcgccact gccctccagc ctggagacac agccagactc 2520
   catctcaaca atgacatcaa aaaaaggccg ggcacggtgg ctcacgccgg taatctcagc 2580
   actttgggag gccaaggcgg gcggatcacg aggtcaggag tttgagacca gcctggccaa 2640
   gatggtgaaa ccccatctct actaaaaata caaaaattag ctgggcgtgg tggcgggcgc 2700
   ctgtaatccc agctacttgg gaggctgagg caggaaaatt gcttgaaccc gggaggcgga 2760
   aggtgcagtg agctgagatc acaccactgc attccagccc gggcgacagt gcgagactcc 2820
   gtctcaaaaa aaacaaaaaa aatctaaaac tcaaacttct gaataacaat ttcctcctag 2880
   caagaaggga ctcagtaaga taagaagcgc tgctgggcta ctttgcaaat gtctggctta 2940
   agatggctgg ttttttgtat ctgcctctgc atttaacctg gtccatctcg cgggtcacat 3000
   agcctctaca aaatgagaag gggttcaata ccgttaactc cgggcctggg taaaagactc 3060
   aagtcctgac cccatcatac tccaaccaat acataccccg cctgcgctgt ctgcgcctgg 3120
   gtctcttcaa ccgcagtgag cggtcgcacg gctccacatc agggtcgccc tgaggctcag 3180
   ccttccccgg ccccattccg agtgcgcaca ggactgaacg agcacgcccg gtccctgctc 3240
   cggagcggag gacacgacgc ggacccgcac ggtgcaccgc agtgacctga ggaaggagcg 3300
   acgggcgctc gcgccaaagg cgtccgagtg cctggtaaac tgtaaagcgc ggtcctgcgt 3360
   gagttgctat ggctacagct gcgccaggcg gacggcctgc aggaccgagg tgcagctggg 3420
   acgagcggaa agctctgcgc gtgggcgggg agcccggagc ctcgccgggg caatgtcatc 3480
   taggtgctcg gcgctccact gatccggccc ccgggggtgg gcgggcacgt ttgcctcgga 3540
   tggtctaaca ggcgtcgggg agagccaatg gtgtggcttc atggctccac ccccttccat 3600
   tcgattggcc ggcgccgaaa agccgggcgt gagcggccgc agtttctgga gggagccgct 3660
   gcgggtcttt ccctcactcg tcctccgcgc gtcgccgctc ttcggttctg ctctgtccgc 3720
   cgccatggcc cagtgagtga ctcgccaggg gcagcccggc tcggcctcag cgggcgggga 3780
   actctttggg ggtcgagatc tccctcgttc tctccgacgc ctcccaccct gggggtcgcc 3840
   tgagctcact tggggctctg tgaccctggc cctacggcgt ctcgggccca gagctccttc 3900
   cctgcgggcc cggccccctg ccctctcggc cgcgcaggca tggggcgggg cgggtccctt 3960
   cgagggccag ggaggaggcg cggccaggcc tcgccgagtc tgcagggaca cctgcgggac 4020
   gcgtgccagc gggagccggg cgcgagggcg gggctggggg ccgcctgccc ggccgaggtc 4080
   tgcagcgtgc gcgcccggct tctgggggcc cgcggggagg cgcggaggaa agtgcagact 4140
   cccagtcacg gccaaatgtg gaaggaccgg acccctgggt tgcagcgcgt cgagcggtgc 4200
   tgactctttc ctttgttctg tttctgcctc tctagagctg acatcgcgct gatcggattg 4260
   gccgtcatgg gccagaactt aattctgaac atgaatgacc acggctttgt ggtaagcggc 4320
   gtgggcgcgt tgtcttctct ctggttcccg ggcgctttag ccgaggccgg cgataggttt 4380
   gggagcttac gggtctcctg gccgtgcttt gctaatgtgc tctgttgctg ctcgtggcat 4440
   ttttgtatgg aaaggagaag caccctgtag gcgtgggcgg gccgatcccg aacttagtcc 4500
   tgcggagtgt gcctgtgggt ccgtgaggtt cacagcccga atgaacgaat tagtgtctta 4560
   agtagagaag aagggtgcgg gaggagaacc cttgctggct gtgtagagct ctaacttgat 4620
   tgcctgatga gatctgggga gagtgaaaat gtttcttcta aatgttaaag tggcttagac 4680
   gccaggatga tcaataacaa cagatagagc ctgttgaacc ggccagttcc tggatttgat 4740
   tttgagtcct aacacgttgg gtgtggattc tacctgacac accgggggta gttggccttc 4800
   gcctcggttg tccgccgttt tcatcctact gaggtgacat aactttacag tgagcctccc 4860
   acagctgggg gaagaaaagg caaaggcagg tctgacctcc ccagaacttg ggttgaatgg 4920
   aaaggtcatt gttactttgg ccacagtctg aaagtcttgt gtgtcctgga tctcctactc 4980
   aggacttttg tccttctagg tctgtgcttt taataggact gtctccaaag ttgatgattt 5040
   cttggccaat gaggcaaagg gaaccaaagt ggtgggtgcc cagtccctga aagagatggt 5100
   ctccaagctg aagaagcccc ggcggatcat cctcctggtg aaggctgggc aagctgtgga 5160
   tgatttcatc gagaaattgg tgaggccagc tgtgctctca gctgctacca cgatagcagc 5220
   tgtttttggt ttcttccttt agttctcctt cttttaactc tagagatttt tttttttcaa 5280
   tttctgctaa gctctgacca aatgattgct taactgttgc agtgttggtt aacttgataa 5340
   gcgcttatga agtaactttg tcctttggtg gtagtaattc tgagaatact aacagacatt 5400
   tcaataaaca ttaaggcggg gcacagtggc tcaggcctgt aatcccagca ctttttggga 5460
   ggctggttca gatggatcgt ttgagcccag gagttcgaga ccagcctggg caacatggcg 5520
   aaaccctgtc tctacataaa attaaaaaat tagctgggta tggtggtgta agcttatggt 5580
   cccagctaca agcttatggt cccaggttcg agaccagcct ggccaacatg gtgaaacccc 5640
   gtctctacta aaaatataaa aattagctgg gctttgtggc tcatgcctgt aatcccagtt 5700
   actcgggagg ctgaggaatg agaatcactt gaacctggga ggtggaggtt acagtgacct 5760
   gagattgtgc cactccagtt tggcgacaga gcgagactcc gtctcaaaaa aaaaatgaaa 5820
   ataaaatcaa gatctcctta gggaatccta gggaactgga tgcttatttt tgtaccaaac 5880
   aatttgttgg ctttagcagg tatatctttt ttggaagaaa aaagtagggg aaccagtgga 5940
   aataatgtgt ttagataccc aaaataattc tatagtaaca actctgaaaa aacccaggca 6000
   ctcccattta gaagaaatgt tttgctgtaa gcagtgcagt gatagctcag gggaggctga 6060
   gcattccttt ctagaggagt cgtgtcaaca gtggggttgt ttccacaggg agaaggattt 6120
   gtaaccagta tgccactgtt tttcaatcaa atgacctaga aaggaagaac actgagttta 6180
   aaaaaaggac atactaaatt ttttgtgtgg caagctgcct gtaatcagtt tcagacagag 6240
   aaaactgaaa aatggtggat tattcttcag tataggaaaa tactaagtaa tgttaactgg 6300
   agccttgtag ggggaagatt gaataatata atgaagccca ggctgggcgc ggtggctcac 6360
   gcctgtaatc ccagcacttt gggaggcaga ggtgggtgga tcatgaggtc aggagatcga 6420
   gaccatccta gctaacacgg tgaaacccca tctctactaa aaatacaaaa aattagccag 6480
   gcgtggtggt gggtgcctgt agtcccagct acttgggagg ctgaggcagg agagtggcgt 6540
   gaacccggga ggcagagctt gcagtgagcc gagatcacac cactgcattg cagcctgggt 6600
   gacagtgcga gactccgtct caaataaata aataaattaa ttaattaatt aataaagtaa 6660
   taataataat aaagcccagc ctggttggtg tgctgtaggt agatattcat gttcaaggct 6720
   ctgtctcttc ctgacctccg aactgttgtc ataaaatcat tcattcatac actaaaccat 6780
   ttgatatgta tttactgaat ccccactctc ttctagacac attatagtca gtgtttaatc 6840
   tgtatactta cttaaaaata gaaatggggt cttgctatgc tgtccatgct ggtctcaaat 6900
   tcctgggctc aagcgatcct cctgcctcaa cctcccaaag tgttgggatt acaggtgtga 6960
   gccactgagc ctggtcagcc ctaaactttt aaatagtgtg cagctctggc cacagagagg 7020
   attattgaag attaagtagg ctgggtgcgt tggcctcctg ccaagccagg aggcttttag 7080
   cccaggagtt tgagaccagc ctgggcgaca tggtgagact ccatctcaaa aaaaattttt 7140
   tttttttgag acggggtctc actctgtccc ccaggctgga gtgcagtgtt gcgatctcag 7200
   ctcactgcaa gctccacctc ccgggttcac gccattctcc tgcctcagcc tcccgagtag 7260
   ctgggactac aaggcgcctg ccacaacgcc cggctaattt ttcgtatttt tagtagagac 7320
   gaggtttcac catgttagcc aggatggtct tgatctcctg accttatgat ccacccacct 7380
   cggcctccca aagtgctggg ataacaggcg tgagccaccg cgcctggcct acaaaaaaaa 7440
   ttttaaaact agctgggcat tgtggttcaa ggagttcaaa gttgcagtga gtcatgttca 7500
   caccactgca ggagttcagg gtttcagtga gccatgttcg cactcctgca ttccaagctg 7560
   ggcaacagaa cgagactctt tttagactat gtttatttgg aataaattct tgtttgtttt 7620
   aatttgccaa ggtatgtttc attttggtcc atcttaatgc tggtgtctgg gtacaggtac 7680
   cattgttgga tactggtgac atcatcattg acggaggaaa ttctgaatat agggacacca 7740
   cagtaagtgt tcttcagtcc agattcttcc aggttccgag aacattctag aaaaaagtgt 7800
   cagcatcgca tatgtgacag tagggtgaac tgccctgtgg tactgatttt tttgagatta 7860
   ctactgatac aatagttctc agcctttttt tctgttaaga cacacttaac agaagggccg 7920
   ggtgtggtgg ctcacacctg taatcccagc actttgggag acggaggcag gtggaccaag 7980
   aggtcaagag atcaagacca tcctggctaa catggcgaaa acccgtctct actaaaaata 8040
   caaaaaatta gcctggcatg gtggcatgcg cctgtagtcc cagctacttg ggaggctgag 8100
   gcaggagaat tgtttgaacc tgggaggcag aagttgcagt gagccgagat catgccactg 8160
   cactccagcc tgggcgacag agtgaaactc catctgaaaa aaaaaaaaaa aaaaaagaca 8220
   cttggtgggt gagattctca gtcacacata caactcaaag gatgactaac tcagatacag 8280
   caggctgaga attacgtttt aactcctccc cttccccttc caagtaatcc atgatatgtc 8340
   agtctataac tggtttagaa ccactcctct aatggtatca aatgctgagg gaattcctgt 8400
   gtgctctgaa gtgctgaatg gcattaagtt gtattacacc tactcttcgt tgagtcttat 8460
   tcaaggtctt ttccagtaaa atgccaatgt gtgtttggct taattatttt actttgggga 8520
   tgaggtaggt atcgtgaatg tccatagttg tattgttttg ttttttaact cctacacata 8580
   ggggattaac tttcctgagt catcagttat tcctagatcc ttgggtcatt tcctgtaaat 8640
   gtaggctctc cattcctatc ttatacttcc tctcatttat aagggaaaca tttttgggta 8700
   gcataatgaa acatggaagc ataatgaaac atggaagcat aatgaaatta tttttctgtc 8760
   cttcagagac ggtgccgaga cctcaaggcc aagggaattt tatttgtggg gagcggagtc 8820
   agtggtggag aggaaggggc ccggtatggc ccatcgctca tgccaggagg gaacaaagaa 8880
   gcgtggtgag tgccatcagc actgtgccca tctctgtaca agggagctgg actttgagaa 8940
   cgaataagcc aggagcagtt ctgccaccct gatctctgtg gtgccctgag tgtgacagct 9000
   gggagatgct ggcaatttct tttatctaga cattttgttg ctttgatata gtatatgtgc 9060
   acaatcctct tttgcctttt ttcttctgtt atttctatca agactgatac ctgacgttct 9120
   aaataggctt tttttttttt tcccgcccag gcaggagtgc agtggtgaga tcttagctca 9180
   ctgcaccccc cgtctcctgg gctcaagcaa ttcttgtgcg tcacctcctg agtagctggg 9240
   actacaggca catgccacca tgcctggcta attttttgcc ttttagtaga gacagggttt 9300
   caccatgtta ccttgggtgg tctcgaactc ctgaccttag gcgatctgcc cgccttggcc 9360
   tcccaaagtg cagggattac aggcatgaac caccacactt ggcctctaaa taggctttaa 9420
   ggaagcagtt taggtaatgt tctgtgcaat gtctggatgt aggaataagg tctgcctctt 9480
   ttagctgata tataacccaa aagtaactta atgtgataat cgtgggtaac aggctgggca 9540
   tagtggccca tgcctgtaat cactgcccag cactttggga agctgcggtg aatgatcatt 9600
   tgagcccagg agttgaagac tagcctggac aacatagcga gatcctgttt ctacaaaaaa 9660
   gtaaaacaaa cattttttta attaaaaaat ataaattggc cgggcgtggt ggctcacgcc 9720
   tgtaatccca gcacttcggg aggctgaggc aggcagatca ccaggtcagg agatctagac 9780
   gatcctggcc aacatggtaa attcctgtct ctactaaaaa tacaaaaatt agccgggcat 9840
   ggtggcgtgc gcctgtagtc ccagctactt gggaggctga gacaggagaa ttgcttgaac 9900
   ctaggaggca gaggttagtg agccgagatc gcgccactgc acttcaccct ggcgacagag 9960
   tgagagtttg tttcaaaaac aaaacaaaac aaaacaaaca aaaaatacac acacacacac 10020
   acacacacac atatatataa ataattgtgg gcaatccgat atatatataa ataattgtgg 10080
   gcaatccgct tgccctggcc tcccaaagta ctgggattac aggcgtgagc caccgtgcct 10140
   ggtctgaaat atgattcttt aaggctgggc atggtggctc acgcctgtaa tcccagcact 10200
   ttgggagact gagatgggag gatcacttga ggtcaggagt tcaagaccag cctggccaac 10260
   atggtgaaac tctctactaa taatacaaaa attagccggg catggtggag ggcacctcta 10320
   atcccagcta catgggaggc tgaggcacaa gaatcacttg aacctgggag gcagaggttg 10380
   caatgagccg agatctcacc actgcactcc atcatgggtg acagagcgag actcggtctc 10440
   agtaaaaaag aaaaaatgat tctttttttg agacggagtc ttgctctgtt gcccaggttg 10500
   gagtgcagag tgcagtggcg cgatctctgc tcactgcaag ccccacctgc caggttcacg 10560
   ccattctctc gcctcagcct cccaagtggc tggaactaca ggcacccacg accacgccca 10620
   gctaattttt tgtattttta gtagagacgg ggtttcaccg tgttagccag gatggtcttg 10680
   atcccctgat ctcgtgatct gcctgcctca gcctcccaaa gtgctgggat tacaggtgtg 10740
   agccactgcg cccagccaaa aataattttt ttaattacct ttctggtgaa tgagtctggg 10800
   gattctcttc agtgtgttat taataaaaca cagaagtaga gaggcctttc tgcttctcag 10860
   aagtttaaac agctgtgtgt gcagtggctt gcacctgtag tgccagctat tcaggagact 10920
   gaggtgagag aactgcttga gcccaggtgt tcaaggccag cctggacaac atagcaagac 10980
   cctgtttctt aagaaaaaaa aaaaaggttt gaaaatactt atctcgaagc acctgcacag 11040
   tgagcagtta gaaaggcaac cagcagacaa tgcacgtaaa agtatttgac atatggagtg 11100
   ctccagttgt ttccatcagt tctctttcct catactgtgg tggcagaagt acaaatggca 11160
   cctttaccag aaggacactg taaaagttca gtgaagctct gcggtttccg gtgtggctgt 11220
   cttcatccct aatcctaaat gttaactttg caaccctgta agatttcatg tctgtaccag 11280
   aaactttttg tgaggaagcc ttgactggcc attagggagc agaacagtga gggaaagtct 11340
   caagagtaat atatccgcgt gctgagctgt ggaaggaatg aacagcggcc tttctctgaa 11400
   tggtaatgta gctctcaaaa acgctgcctg gttcttaatc caaatgtcca gaaccattct 11460
   gtgcctggca gcacacatcc agacaaatga aagagggtct cttcacttgc taaaagccat 11520
   tataaggaga aataaagaaa agggaaaaaa ttaaaagagg atctctaaag gaactttcgt 11580
   attgcctgct gtttgcaatc atactgtctg ctgctgctgc agtagaaatt tctgagccgg 11640
   gtgcgatggc ttatgcctct aatttcagtg actcaagagg ctgaggtggg aggagtgctt 11700
   gatccgagga gttcaagacc agccacatca acatagggag accccatctc tgcaccacca 11760
   acaaaaaatt agccaggcat ggtggctcac gcctatggtc ccagcacttt gggaagctga 11820
   ggtgtgagga ttgcttgagg ccaggagctc aacacccagc ctgggcaaca tagcaagatc 11880
   ctgtccctac aaaaaaaagt ttttaattag ctgggtgtga tggcacacac ctgtagcccc 11940
   acctacttgg gaggctgagg caggaggatc acctaagtcc aggagtttga ggctgcaggg 12000
   agccgtgatt gtgccactgc actccagcct gggcaacaag aagagaccct gtctctaaga 12060
   agacatttct tctggtgctt tggtgatgct tctgctcagc ctttctgtct gtagtctcag 12120
   tccgatagtc cagtctgctt tgagggccca ccaggacctg gaggaggggc cagtttcagt 12180
   caagggtctg cagttgacct tgagaactta gcacattcat tatctttttg tgggcacttc 12240
   cacctcccat tagaaagaaa aagatggccc cagtgcggtg gtggcttatg cctgtaatcc 12300
   cagcacattg ggtatccacg gtgggtggat cacttgagct caggagttcg agaccaatct 12360
   gggcaacatg gcaaaacccc atctctacaa aatccaaaaa aatgagctgg gtgtggtgtc 12420
   acatacctgt agtcttagcc acttaggggg ccgaggcagg aagattactt gaacctggga 12480
   ggttgaggag gctgcagtga gctgagatca caccactgca ttccagcctg ggtgacaacg 12540
   tgagaccctg tctcaaaaaa aaaaaaaaag aaaggaaaag ataggggttg gtgtctatgg 12600
   gtatgttggt gtctatgggt atgggctctc agcccgtctc ttctcattaa ctgaaccaca 12660
   ctgtttcttt acacaggccc cacatcaaga ccatcttcca aggcattgct gcaaaagtgg 12720
   gaactggaga accctgctgt gactggcaaa gttctgggct actctttaaa gccaattggc 12780
   aacatgggcg tgtctaagtg atgaagtgcg tggagaaagg ccaccgtggt ctcccagggg 12840
   ttagcttggt gacataaacg tctcgatctt tgattgctag aatagccaac gcctagaatg 12900
   catgcctgct gttcacatct ttgtatctta gctgactgtt catagccttg aggttgatat 12960
   taaaagcagc cttctataat tcatctaaaa gatgaattct ttaaaaagga ataggatgaa 13020
   gtgaaataag attttttcga gccatattat ttcatggcca aaggaaagaa tgtcagaatc 13080
   aaaagtggcg tttgcttgca tgggtgaaag gtggaagatt gaaggtactg ttgctgtgtc 13140
   aacattccat gaacgtggaa aattccccat cctgtagtgt gggattcagg cattctgtgc 13200
   ttgaacaaag taacctgtgg acttgcaccc tcactgaata tgaaggaggc ctaacctagt 13260
   tcatagtaca gtggtgcgat cttggctcac cgcaacctcc acctcccagg ctcaagcaat 13320
   cctcctgcct cagcctcctg agtagctggg actgcaggtg catgccacca cgcctagcta 13380
   atatttgtat tttttgtaga gacggggttt caccatgtca cccaggctgg tctcaaactc 13440
   ctggactctc aagtcatctg cctgccttgg cctcacaaag tgctgaggtt acaggcatgc 13500
   gccaccatgc ccggcctcgt gttgctgtta aagtaaactt tatctcatat tgtagttcag 13560
   ccatgctgtg gatggaaaaa agcattaggc tcaggagacc tgagtggcag ttctggcttt 13620
   ccataaactt aactgtgaca tcaggcaagc cacgtccctt catggacttc agtttcctta 13680
   tctgtaagat atggggttgg attacatgat ctccaagctc tcttcagctc ctgattccct 13740
   ttaattttat atcagcaatt ttaatgtgaa ttttattatt caattttatt ttatatgaaa 13800
   tcaatgctga ggttgaaata aggactcagg cctttgagga atccgggaag ccctccctcc 13860
   tagg 13864

<210> 76
<211> 22081
<212> DNA
<213> Homo sapiens

<400> 76
tctagaaact atcctttaac ttctgctaca gttaaggctt gtgaggtctt tcatttcccc 60
tctgaagtca agcaaatacc aatgctacaa gctcagctgt caaagaaact atgggtgata 120
taaaaacgtt ataaagcact gaaataaaga atttaatgtc taatgttcta gccatcaggg 180
cagtttaaaa caataaaata aaggcgttaa tgccccagtg ttagtgtaat aggaaaaaaa 240
tagttcttcc cataaaatat ccacatctca cagccaagaa gagaagtgtt tcacgaagaa 300
cgcatgagga tgataatatt cttggtctgc tggtcagact cgccacagct cccacccacg 360
cactgctccc aggtgaaatc aacctttggg gtttacagac atggctgggg aggccgaagc 420
ttcataccac actgtcggaa agtcagctcc gaaatggccg caatggtctg tttgctgaac 480
tgcatctctt tgtccaatgc aacttcctcg caaagacaac ccacagtata gtgaactgct 540
gcctttagcc tctggggaaa aacatcaaaa tatttcacgg gtaaggctgc aaataagagc 600
ttttcacgcc ttaaaaaatg aagaccaagg agagggtctg ctcagcccaa tccctctggc 660
gctttccata atggctgagt ggcttttcaa gtgtactaat aagttaatga gacccactgg 720
agtgcgactt gtggaaacat tacttccttg ttaatggtta tgatttatgt caggtaaaga 780
taagaaatta gatggcaagt cagtggggag acaggtcaga ctgatgcctg ccaaggccct 840
gagtggtttc actcacttat tccaaactat ctgcagagct ccaactcagt aagggcatat 900
gctaggtgtt tgggaaacaa tgaaaaaccg ggcagggaac tttgccctta aggcagttaa 960
tcaacaatac agaaggatcc catccctctg ctgcaagccc ctggcagccc caggcctgca 1020
cgtcacagag gacagaagta caacacggga aaggctcaag agggcacacc cacccctctc 1080
gatttggagg gttgggaggc cacctccacg gggccctcac tgctccttgg caaacctttt 1140
caatactgaa aaagatctct ccaccccttg cagaaaatta ccgagagggg aggatgagcc 1200
tcaattgcaa gacaagctaa gcaatggtct cagccacgtg tcgacagagt tctgttgagc 1260
ccacctgtgg gtactccgcc tcggggccga agacacagct gagctgcacg tgatgacagc 1320

tgaacccaca agggcataaa atctaccagc aagtgttcaa cgcataggca agaggaggag 1380
gaactgccaa aacccaagtc caggaactca ggatagaggc cagacagatg aggagggcat 1440
ctactttcct gaatttcaga cctcacaaga ctaggtaagc tgaaggcctc tggtaggaga 1500
agccaaagag gcatcaacct gggaggcacg aagatcacag gtgtgatcac tgcacactac 1560
agcctcgaac tcctgggctc aagtgaacct cctgcctcag cctcttgagt agctgggact 1620
acaggcccac aacaccctgc ccggctcaag gtcatctttt ttaggtatgg gtgggggtca 1680
gggtacagga catgaattct tctgaatgct cttggctcca ggaatttttc tcagatcttc 1740
ctcctcctcc tcctcccctc cccctggctg ggctggggta tcactgcact tagaggctaa 1800
atcactaaac tcctcaaggg caggaccctg tcaattttgt tcactgctgc ctaccaagtg 1860
cctagaacag ttggttacat gaatgggggg aaatcgtcta caagctaatc acagctgagg 1920
ggcaaatttc tcagcccatc tttgcagctt catcagtaag catttgttgg tctatttgag 1980
agccaggctc tgttctaggg actaagaaca ggccgggcac agtggttcac acctgtaatc 2040
tcagcatttt aggaggccaa ggcgggccag atcacttgag gtcaggagtt caaaaccagc 2100
ctggccaaca tggtgaaact gtctctacta aacatacaaa attagctggg cgtggtggca 2160
ggggcctgta atcccagctg ctcaggaggc tgaggcagga gaatctcctg aacctgggag 2220
gtggaggttg cagtgaggcg agatggagcc actgcactcc agcctggaca acaagagtga 2280
aactacgttt caaaaaaaac aaaaaccaac caaaaaaaaa cagggtaggc aggaccaaag 2340
atgactaaat gcaatgcacc atccttaact gaatcctgga tgaaggaaaa aagacagtta 2400
aaagaaacat ctttaggaca agtgggggca cttgacaatg cacattcaat agagttttat 2460
caaaattaat tgtcccactt cctgagtggg acaatttatt gaaatgatgt agtaggagaa 2520
tgcctttttt tttttttttt ctgagatgga gtctcgctct gtcacccagg ctggagtgca 2580
atggtgcgat ctcggctcac tgcaacctcc gcctcccggg ttcaagcgat tcccctgcct 2640
caacgtcccg agtagctggg attacaggca cctgccacca cactcggcta atttttgtac 2700
ttttagttga gaggggtttt ccatgttggc cagtctggtc tcgaactcct gacctcaagt 2760
gatctgccca cctcggcctc ccaaagtgct ggaattacag gcgtgagcca ccgcgcccag 2820
ccaatgcctt gtttttggga gatgtgtact aaagaattaa aatgtaaagg cgaagtgtca 2880
aaatatctgc aattaattct ccaatcgcgg ggctgggagg gaagcatggg agcaaacaga 2940
gcaaaatgct aactagtggt gaatcaaggt gacgggcatc tgggcattct gtaccgtgtt 3000
cttgcaactt ttctgaaggt ttgaacttct caaaagacaa ggggcagggt agacagctgc 3060
tgcagtgtaa ctttgtagag aggaaactcc cgcgtctgga gctcattccc gcgtttcttc 3120
agtccgtcct ctttaaggtc gccactccgg gtcttgcttt taagcctctt cacggcaacg 3180
ttctcagcct ctgacgcccc atatgcccgg cgctgatgaa aggaacgaat gggatgcccg 3240
cagctaccct gacaagtgat tattcttatg acgattttgc agagaagaaa attgcgtctg 3300
ggccgagtcg atacgatcac aagttggcgg ggcagagatt cgaacccaca tctaaggacg 3360
agctctgggc ctgactggtc cgcaccgcgt ccctgcatca gtaacgaggg gtcgacgcct 3420
ccagggccag cgttccagag ctccggcccg aaccgcccac cgcgtacggg cgtcacctgg 3480
aatgtctaat gccgacgttg taccctcacg aaaaccccat ctgagaaggg gaaaccgagg 3540
attcatcgca aggccagaag gtggcctctc gccgccggac cccggcgccc gcacgctccc 3600
ctagaaccgc ttcctcccca gccagcgcag gccagggtcc agccgcgttg ccgcggaaac 3660
cgcctcccag caccacccgc ggcagttaag ccaacgcatg cgcagccgcg gggggcgccc 3720
gggggcgggg tgccgatgag aagccgccgg gggctgccgg gtacttttct gtccagaaac 3780
tcgacgccgt cctgccccag cccagcccga cccccgtttt cctgtacctg ttggtaagag 3840

aatcgctgct gctcctcggt ctccgcctcc tcctccatca ctgcgggccg accctgggca 3900
aacgcaggag ggccgagaag gggcggtggt gcgcggccag gtcggatttt cccgccgcgg 3960
cgcgagcgac gggccgcgcg ggcggacaaa gtacgcagct ccgccctgcc tcgcgcgccc 4020
cctggcggcg ctccgttgag gggattccgc cgtcggcctc cagccagccc cctgctcagc 4080
ttgctctgcg gaggtggccg ggcgacgcgg ggacatgacc accgaggtca ctgctgcacc 4140
ccagagcctg attcagcgcc tggcacaacg ctgagcacca ctctccgcga aacacgtgtg 4200
cgcataggtg caaagcgtgt tccagtcggg tgtagaggat gcgtagccca cgatttacag 4260
atcatgtaac gcagtacaca ttatcaggag ctccatgcac ctgtacctca ggtgtatctc 4320
gcaaaatcga ccatcaactt tatcattcta tcaccaacaa tagcgtcaca aagactacaa 4380
tgagtgaacg acccgtgacc tcattcagcg agtggaacag gaactgcgtt gttgccattg 4440
gcaggagtat agttctgaca caagtgttac tgtttttcct ttatgttaac gatgaagtga 4500
gaccatcaaa cccaattttg gtttgttttt attgtagttt tttgagaggc agggtctcac 4560
tctgccgccc aggctggagt gcagtggcac caccaagact cactgtaacc tccacctcct 4620
gggctcaggt gatcctccca cttcagcctc ccaagtagct gggaccacaa gcctgcatca 4680
gcgcacctgg ctaattttta ttttttgtag agaccaggtc tcactatgtt gcccaggctg 4740
gtatcaaact cctggcttca aacaatcatc ccacctcagc ctcccaaagt gctggaatta 4800
caggtgtgag ccaccatgcc ctgcctgtat ttctaggttt ttgttttatt attttttatt 4860
gcctatattt ctgtattctg catttgtcac aagcaggtgc tactttcaga acgaaggaaa 4920
gcacataaag ccactccttg tcttcctttc ccagggacgt ccctcaagtt ccctaagctg 4980
gccaggggct gaaacccaga tcaaagcttt ttggaggaat cagccttctg aggactttcc 5040
tctgcatctg ctcagtgggt ggcatctgga aatgacaggc acttctcgcc tctgtccact 5100
gctctctgtg ggcatcccag tctacctgga cagtcctcct gcccaagcag ggcaagcctc 5160
tgacggattc ccctaatggg cagcatgggg ctgggcaggg caggccatcc agagtgagac 5220
caagcctgca gcaggagaga aggccggtcg ctggttggtg agcagcccca tttagagcac 5280
actgtggctg caaagcccag ctccctccct tgagcctggt gaccccaagc aagtcacagg 5340
acgtggccaa gcttcatgtc ccttatttat caactgggga cagtagttcc aacttcacag 5400
ggcaggtgtc aggttttaaa caattgcctc acataagagc taaagaaatt gtaacaattc 5460
gtcccaggaa gttcctgtgc tttgtacctg tgtgttccct cattttaaag cagttgcttc 5520
ccaggattaa agaacagtat tccagtgaca tgtgtgcaat acagaggcac ttaagagcct 5580
cagaggctag gagcagtggc tcatacctgt aattcctgca ctttgggagg ccgaggcagg 5640
tagatcactt gaggtgagga gttcaagacc agcctggcca acatgatgaa accctgtctc 5700
taccaaaaaa tacaaaaatt agccagacat ggtggcacat gcttgtaatc ctagcacttg 5760
ggaggctaag gcaggaggat tgcttgaacc cgggaggtga aggttgcagt gagctgagat 5820
cgcaccatag cactccagcc tgggcaacag agcgagactc tgtctcaaaa aaaaaggtct 5880
gagaaatagg tcgagggttg aggtattcat tacagaaaat ggaactgggt gtggtggttc 5940
aagcctgtaa tcccaacact tagggagacc aaggcaggag gatcacttga gcccagttca 6000
aggctatggt gagccgtgat tgccccactg ttttccagcc agggtgacag agggagacac 6060
tgtctctaaa aaaaataatg aaaaaaggat atattaatat ttggttgaaa cttacttgat 6120
gaagaagtag ccctataatc tggccactag atgtcagtgt tgcactacag cgtgaagcaa 6180
ggccagggat gcccgctggc aatgttgcca tgacactgag cactttgcac ttgtcctcgc 6240
tctaggcact catttaatat ctcatctgcc ctcaaaagta ggtgctgtta gccccatttt 6300
acatgaggaa acaggtgaaa gaactctgcc caaggctaaa ctactgggaa gtgatagaga 6360

ccagatttga ttattaggct ggtttccgag tttccggttt atccactgcc ctcaccataa 6420
ataagacaaa gaccttggtg ggtttcttta atccttttca tctgcattaa ggagggttcc 6480
ataaatattt agttcagccg ggcgtggtgg ctcacgccta taatcccagc actttgggag 6540
gccgaggcag gcggatcacc tgaggtcagg agttcgagac cagcctcaac atggagaaac 6600
cctgtctcta ctaaaaatac aaaattagcc aggcgtagtg gtgcatgcct gtaatcccag 6660
ctactcggga ggctgaggca ggagaatcgc ttgaacctgg gaggcggagg ttttgcggtg 6720
agctgagatc gtgcattgca ctccagcctg ggcaaacaag agcgaaactt gaaggggtgg 6780
gttgcccctc cacacctgtg ggtgtttctc gttaggtgga acgagagact tggaaaagaa 6840
aaagacacag agacaaagta tagagaaaga aataaggggg cccaggggac cagcgttcag 6900
catatggagg atcccgccgg cctgaattcc cttagtattt attgatcatt cttgggtgtt 6960
tctcggagag ggggatgtgg caggatcata ggataatagt ggagagaagg tcagcagata 7020
aacacttgaa caaaggtctc tgcaccatag ataaggtaaa gaattaagtg ctgtgcttta 7080
gatatgtgta cacataaaca tctcaatgcc ttgaagagca gtattgctgc ccgcctgtcc 7140
cacctccagc cctaaggcgg ttttccccta tctcagtaga tggaacatac aatcgggttt 7200
tatactgaga cattccattg cccagggacg ggcaggagac agatgccttc ctctcgtctc 7260
aactgcaaga ggcgttcctt cctcttttac taatcctcct cagcacagac cctttacggg 7320
tgtcgggctg ggggacggtc aggtctttcc ctcccacgag gccatatttc agactatcac 7380
atggggagaa accttggaca atacctggct ttcctaggca gaggtccctg cggccttccg 7440
cagtgtttct gtccctgggt acttgagatt agtgagaggt gacagcgtgc tggcagtcct 7500
aaagccctcg ctcgctctcg gcacctcctc tgcctgggct cctactttgg tggcacttga 7560
ggagcccttc agcccaccgc tgcactgtgg gagccccttt ctgggctggc caaggccaga 7620
gccggctccc tcagcttgca gggaggtgtg gagggagagg cgcaagcggg aaccagggct 7680
gcgcgcggcg cttgcgggcc agctggagtt ccgggtgggc gtgggcttgg cgggccccgc 7740
actcggagca gccggctggc cctgcccgcc cagggcaatg aggggcttag cacccaggcc 7800
agcggctgcg gagggtgtac tgggtccccc agcagtgcca gcccaccggc gctgcactcg 7860
atttctcact gggccttagc tgccttcctg tggggcaggg ctcgggacct gcagcccgcc 7920
atgcctgagc ctcccaccca ctccgtgggc tcctgtgtgg cccgagcctc cccgatgagc 7980
gtggccccct gctccacggc gcccagtccc atcgaccacc caagggctgg ggagtgtggg 8040
cgcatggcgc aggactggca ggcagctcca cctgcagcct ggtgcaggat ccactgggtg 8100
aagccagctg ggctcctgag tctggtgggg aggaggagaa cctttatgtc tagctcaggg 8160
attataaata caccaatcgg cactctgtat ctagctcaag gtttgtaaat acaccaatca 8220
gcaccctttg tttagcccag ggtttgtgaa tgcaccaatc gacactctgt atctagctac 8280
tctggtgggg ccttggagaa cctttgtgtc tagctcaggg attgtaaacg caccagtcag 8340
cgccctgtca aaacagacca ctgggctcta ccaatcagca ggacgtgggt ggggccagat 8400
aagagaataa aagcaggctg cccaagccag cagtggcaac ccgcttgggt tcccttccac 8460
gctgtggcag ttttgttctt tgcaataaat cttgttactg ctcactcttt gggtccacgc 8520
tgcttttatg agctgtaaca ctcaccgcga agatctgcag cttcactcct gagccagtga 8580
gaccacgagc ccacgagaag gaagaaactc cgaacacatc cgaacatcag aaggaacaaa 8640
ctccagatgc gccaccttaa gagctgtaac actcaccgcg aaggtccgcg gcttcattct 8700
tggagctagt gagaccaaga acccaccaat tccggacaca ttagggagtg gtgatgactc 8760
ttaacgagca tgctgccttc aagcgtctgt ttaacaaagc acatcttgca cagcccttaa 8820
tccatttaac cctgagttga cacagcacat gtttcggaga gcacagggtt gggggtaagg 8880

ttacagatta acagcatctc aaggcagaag aatttctctt agtacaaaac aaaatggagt 8940
ctcttatgtc tacttctttc tacacaaaca cagtaacaat ctgatctttc ttttccccac 9000
aaaactccgt ctcaaaaaaa aaaaaaaact gcactgtaaa atgtcaatac aaaaatcaat 9060
cttggctggg cgtggtggct catgcctgta atcccagcac tttgggaggc cgaggcgggt 9120
ggatcacccg aggtcaggag tttgtgacca gcctggccaa catggtgaaa ccccatctct 9180
actaaaaata caaaaattag ccaggcatgg tggtgcacgc ctgtaatctc agctactaag 9240
gaggctgagg caggagaatc gcttgcccag gaggcggagg tcgtagtgag ccaagatcat 9300
gccattgcac tccagcctgg gtgacagtga gactacatct caaaaaaaaa aaaaaaaatc 9360
aatctccatt tcataggttt attcaagaga agataaaaat ttaaaaataa ttatcgttaa 9420
atattatatg tatcctgtaa gcacatgagg tcaggacttt ggacttctct caccccgaca 9480
gagggtacct agcacaatgc ttggcacatg gtaggtgtac cataaacatg ccgaatgact 9540
gaatgagtgg ggcgatgaac tgtgcacgcc gacaggtcag tttcatgatt aagtgtatgg 9600
actctggcac cagatctctg gggttcaatt ccagttcttc agtagctctg accttggacg 9660
aattacttaa tatcagtgtg cttcatctct aaattaggga tagtaatagt gcttacttca 9720
gagggtgctg cgagcattca atgagttaca gagcctggca tatgctctca atgttagcta 9780
gtagtatata tactatatga gaccctcagg aaaagcccca aatagtgccg gttcccaact 9840
ctgctatgta acagattttc cttttcaggg gcagcatatg tggcagactg taatttacga 9900
agatggcaag aacaatgtct cctgatccat gtgcttttct agaacctcat actcccccat 9960
caagaggtag agtctaatcc tctgcccctt gagtctgggg gacttgcggc ttgcttggaa 10020
ccgattttgg atggctataa tcctgccatg taacattcac agctgggttt ttaaaagcca 10080
cgtgtgtgct tctgcctcct tagtgagaac cctgggccaa caaacaagat gtttctctgg 10140
cctgaggcca tgtgatgagg ccacatgaac tttttccagc tcatagccct agctgaggtc 10200
ccagccaaca gccagcatcc accgcgagat gaatgagaga ggacactcca cgcccccgag 10260
ccatggtgcc acccctgctc tcgagtcctc ctagctgagg ccccagacat tgtggagcag 10320
acaagctgcc tctgcctccc ccgtccccca ccatgctctt tccagattca tgacccacag 10380
aattagaaga aaatttaaat ggtgattatt ttacaccact gactttgggg tggtttgtta 10440
cacaccagtc cgcagcaact gtgacagcat acaagattta acagagtgag cagacgcatt 10500
cgcccgcatg ctgaacagcg tgctgaaggc tcccacccct tccaaaatga tgggctccac 10560
atctgcctca aagaacgtgt aacccctgca aatccttccc taaagtctct ttgcttggca 10620
atccttctgc ttggccaggc atggtggctc gtgcctgtaa tcccagcact ttgagaggct 10680
gaggcgggtg gatcatttga ggtcaggagt tcaagaccag cctggccaac acggggaaac 10740
cctgtctcta ctaaaataca aaaattagcc gggcgtagtg gtgggcacct gtaatcccag 10800
ctattcagga ggttgaagca ggagaactgc gtgaactagg gaggtagagg ttgcagtgag 10860
ccgagatcgc gccctgcact ccagctggga aacagagtga gactgtctca aaagtgaaaa 10920
caaaactcct gtttccttgt ctgacccttg tctgtataac acacctttgt gtccccagta 10980
aagcagagtt gtggcagaca tcattagggg cctataattt ctacctcaaa gaagccacca 11040
gatctgtccc ttttagtggt tagactgaac aggactgact tggctaaggg caaaacccct 11100
ctttccacct ccttccacac tcagaattgt ttggcagcct ctctttagga ggcaggtaat 11160
aaacagtaaa ccttacatag tgctcagtgt gtggtggctc tggtgcaagt gccttgtgtt 11220
tagtcgggaa gacggctctg tgaggtgggt gttatcatca tcccttttat atagaggaga 11280
aaatacgcac atagaggtta actaacttgt gaagtaaggc tgaatttgtg gcggagccaa 11340
gattggggcc cagggccagg cgtggtggct catgcctgtg gtctcagcac ttgggaggct 11400

gaggcgggag gatcgcttga gcccaggagt tgaagatcag cctgggcaac atagggagag 11460
ctcatctcta caaaaagttt agaaattagc caggcgtggc ggtgtgcacc tggagtccca 11520
gctagccagg aggctgagtt gggaggatca cctgagccca ggaggttgag gctgcagtga 11580
gccatgatag tgccacactg cactccagcc tgggccacag aatgagacct tgtctcaaaa 11640
aaaagataga aatgcagaac atggggctcc accccacgaa gagccatttt caggattcta 11700
ccgaatcaga atctacattt tttcaaaatc tcgggggtgg tggtgggagt gggggagttg 11760
taacaggtta gtttgagaag ctctgacctt tcttaacctc tacacctgct gcgcagcaca 11820
cagaggcacc cccaccagaa accttttgaa gactggggtg caaagcaaat tcaccactgg 11880
tgctgagagg taacatctgg tttctctcag tacaaacagc acgcactgct ggccaggggg 11940
tagtggcttc tgcctgtaat cccagcactt tgggaagctg aggtgggagc agcatcactt 12000
gagccctgga gttttgagac cagcctagac aacatggtga gaccttgtct ctacaaaaaa 12060
tacaaaaaat tagtggggcg tggtggcaca tgcccgtaga accagcgact cgggaggctg 12120
agacaggagg aaccacctga attcggggag ttcaaagttg cagtgagcag tgattgaatc 12180
actgcactcc agcctgggca agagcaagac ccagtctaaa aaaaaaccac cactgctcca 12240
ggggtccagg ggtcaggcac actcaatggt agtaattgct gccactattt tttttttttt 12300
ttgagacagg gttttgcttt atcaccgagg ctggagtaca gtggtgtggt catgggtcac 12360
tgcaacctcc atctcctggg cttaagcgat cctcccacct cagcctgcca cctagctggg 12420
atcataggtg tgcaccactg cgcccagcta atttttattt tttgtagaga tgggggtctc 12480
actatgttgc ccaggctggt ctcagactcc tgggctcaag cagtcctccc acctcggcct 12540
tccaaagtgc tgggcttacg gtgtgagcca gcgcacccag ccttgctgcc actggtgtag 12600
gtatttacta tgtgccaggc actgctctat cttcccttgc tgaatcccca aagtagcctc 12660
aaggacacag gtgctattat ttcccatttt acaaatgaga aaactgagaa cacagatagt 12720
aaataatttc cccaaagtct aacagctagt aagcaaggag ggctggattt gaaaccaaac 12780
tccaccactc acagcttttt ggaaggtaag cggaaagggg ctgagtggat tcccttctga 12840
tcattactgt ttgaaacatc gtgggccctt tgacacttag cctctgacct cttgctgttc 12900
tctctccatt ccagcagtgt ccatccctca gcctcagagc caggtgaaaa gatccccctc 12960
gaacagtgcc tttgacaagc cttaaagaaa aatcccgtca cgtgcggcta ctcgggcttc 13020
tcttaaatgt acagggaggg caggaggggt gcgtgggggc tgatctagcg aagtactcac 13080
atctaggcag agtttaaaaa gttggtagca gggcacggtg gctcctgcct ataatcccag 13140
caccttggga ggtcgaggca cgaggattgc ttgagtccag gtgtttgaca tcagcctggg 13200
catcatagca agaccacatc tctaaacaaa attttaaaat acggtggctc acgcctgtaa 13260
tcccagcact ttgggaggcc gaggtgggcg gatcacaagg tcaggagatc aagaccatcc 13320
tgaccaacat ggtgaaaccc cgtctctact aaaaatacaa aaaaataagc caggagtggt 13380
ggcgggcacc tgtagtccca gctactctgg aggctgaggt gggagaatgg tgtgaacccg 13440
ggaggtggag cttgcaatga gccaagacca cgccactgca ctccagcctg ggtgacagag 13500
cgagactctg tctcaaaaaa aagaaaaata aattaaaagt tggtgattta atgaaaattg 13560
tctccttcac tgacattagc cgagaggtca gaggtcacac tgtagacacc agctgtcgag 13620
gcacaggcgc tgctcgctcc tcttaggcaa ctcttaggac tttaacactc cagggaaatc 13680
atcatgaagt caaaccgctg cagggaacag tcacactggc cccaggccac acaacaatat 13740
cactggcagg ttttatcttc tctgagtaag taagataaag tgaaaagcct agtttaagga 13800
gccggcttag cttgttcaaa atccctgggc ctggcttaga gcaagtagag acaggtgtgg 13860
gaaaggaaat aaatcagacc aagctgagcc catcattaaa gtttaattct ctagaaagtt 13920

tagtatgaag tatttgacat acaaaaagcc ataaagaatg tcacgcacag cttgccagga 13980
gcaaaaaaaa aaaaaaaaaa aaaaagacga aagaaaaagc acgttacagt gaacgtccat 14040
gtacccattg cccaattgga gaaagaaaac atgcccagtg tattaaagcc cctggtgtgt 14100
cctccctgcc tgcctcctct gagtgaagca taatcctaaa tgtggtttct tgtgttattc 14160
tagtcttttt ttttaaagaa aaatctttct gccaatgggg ataatggctc catgtggccg 14220
aggtgagact aagaatctgg agaaagagca tcagtctgat gtagctttta ttgagtaaag 14280
gaaaaaggga attcagccgc atgatacaga ggttccagtt gatcagagtg cgcaaacacc 14340
cttcctgtct gcgtgatggg aaccgcacca gcacacgggg cacgcggaag ccactgccgc 14400
aaggagatgg ttcccactct cacgcacatg agcagctcct ggtcagtccc aagaggcaag 14460
ggcagagggc atggtggctc tcacagagct actttacaaa taaactgtgt gtcttcctca 14520
ggagtctctt acaacacttt taaaaaactg aacagaaaat agggcaaagg gccaggtggc 14580
agtgccatga ggcacatgga atgtcctggt ctgtggaatc gtggagggtg acaggagcag 14640
catgatcagg cattgtatga cgaggatgac acggtgccac catggcctgt ccagttggtg 14700
tggataaact gccctggttt ggccaagagt tcataggtgt gagccccgaa gtaatcccgc 14760
tgagcctagg aaacgagaga gtaggttggt caggacagtc catggcgcac gcccccgagg 14820
ggccagccag gttaatccct gctccacagg cttacctgga tgaggctggc tggaagcatc 14880
tcatgtctgt acccgtcata gaaggagagg gcagtggtaa aacagggcat gggaatgcca 14940
gcctggaccc cagtgctgac tgcccgccgc caggagtcct gagcacaaaa cgcacggcca 15000
gagattagtg atctgcccct ggtgacgcgg atagattttc attcatatca atgtgggtaa 15060
atgaaaaact gatggttagg ccaggcgcgg tggctcacgc ctgtcatccc agcactttgg 15120
aaggctgagg tgggcggatc acgaggtcag gagttcaaga ccagtctagc caacatggtg 15180
aaaccccatc tctactaaaa atacaaaaaa aaaaaaaaaa ttagccaggt atgttggcac 15240
acctgtagtc ccagctactt aggaaggctg gggcaggaga atcgcttgaa cccaggaggc 15300
ggaggttgca gtgagccaag atcatgccac tgtactccag cctgggtgac agagcgagac 15360
tctgtctcaa aaaaacaaaa aaccaaaaaa aaaaaacaaa aaaccaaaca tcacaactga 15420
tggccccagg gaggtagagt aaccatggca ccagccctag gctacatacc tggcagtttt 15480
caacagctga cttaaagaag tcgtccagta ggaggttctg aagttccggg tttcgatcaa 15540
atgcatcctt tatctttcct aggaatacac tgaaataatc aaggggggaa aaaagcaatg 15600
agtctttttt tttttttttt tgagacggag tcccgctttg tcaccaggct ggagtggagt 15660
ggcacgatct cagctcacag caacctccac ctcccaggtt caagcgattc tcctgcctca 15720
gcctcgccag tagctgggat tacaggcacg caccactaca accggctaat ttttgtattt 15780
ttagtagaga cagggtttca ccgtgttagc caggatggtc tggatctcct gacctcggga 15840
tccgcctgcc tcggcctccc aaaacgctgg gattacaggt gtgagccacc gcgcccggcc 15900
aaatgcaatg aatcttaact ctaacaggga agtaagctgg aagccgatgt ataccctagt 15960
gaaacaaagt tacatttctc atttcttcaa gctgttaacc cttttcctgt ttagaaaaaa 16020
agtgcagctc gctgccagcg ctcatttaat tttacataaa cacgcttttt gaggctgaag 16080
aaaatctgac gattttcaat atgaaaataa aatataaaaa ctattcttgg cgttatttct 16140
aaagagaact aatattagaa tcatctgaat caccagaatc atctacttca gaaaaatcag 16200
attcatcaaa tgaatcttca gccaacatga gaacgatgta acgtcatgtg tagaaatgct 16260
acgttttcta tgatttggca ttttcaatga aaatacagag caaaattccc aagaggcaag 16320
gccagagggc acgatagttc acagaagtac tttaaaaggt gtagtgtagt aggtgtaatg 16380
cggagccggg cgcagtggct cacgcctgta atcccagcac tttgggaggc tgaggcgggc 16440

agatcaccca aggcaaggag ttcgagacca gcctggctaa catggtgaaa ccctgtttcc 16500
actacaaata caaaagaatt agccggacat ggtggcgtgc ccttgtaatc ccagttactt 16560
aggacgctga ggcaggagaa tcacttgaac ccgggaggca gaggttgcag tgagtcaaga 16620
tcgcatcatg gcactccagt ttgggcaata agagcgaaac ttggtctcaa aagaaaaaaa 16680
aaggtgtagt gtggacaatc tagttacagc aactgaggta cagtgaagaa gggaaccaca 16740
taatctaagc ctatcttgtg ccagtgtcca gtgatggggc taagtgccca tccctggaaa 16800
ctgctcaagc taagtgtcct aagatcgcac ttctgcacag ccgtgtgagt ccttcccgtc 16860
ggaccctggg ctgcctctca cttaccttct aatgatgcag ccccctctcc acatcagggc 16920
gatgccacca taattgagag tccagccaaa ctcggtggct gcctgcctta gcagcataaa 16980
gccttgagcg taagagatga tcttggaagc gtagagtgcc taaagagtga cacagagacc 17040
tactaggatt gccattagga gagtgaccgc cttatgtcta ccagtcttat tctcatcagc 17100
agtggagatc ccagaagggc caatattcta ggaaaacatt gtaagtcaag atccaagtca 17160
aggctgaact tcattccacc tgccatcctg tggcctcgag tgttctccca ccacaacccc 17220
cacccccact gccttccgca ggaccaacaa agaccactgc cagggactgt gtcccacctt 17280
ccgaatgtcc tccaggaatg atttcttatc accatcaaac tggaacttct ggggaccctt 17340
cagctttttg ctagcttgaa ttctctcatc cttcagagat gataagcacc gagcaaagac 17400
agcttctcct acatggggag atcgggaaga ggaaacagat tactgtcagg cctctgagcc 17460
caagctaaac tatcatatcc cctgtgacct gcatgtatac atccagatgg cctgaagcaa 17520
gtgaagaatc acaaaagaag tgaaaatggc tggttcctgc ctttactgat gacattccac 17580
cattgtgatt tgttcctgcc ccaccttaac tgagcgatta accttgtgac attccttctc 17640
ctggctcaga acctccccaa ctgagcacct tgtgacccct cggccctgcc cgtaagagaa 17700
aaaccccctt tgactgtaat tttccactac ctgcccaaat cctataaaat ggccccaccc 17760
ctatctccct tcgctgactc ttcggactca acctgcctgc acccaggtga ttaaaaagct 17820
ttattgctca cacaaagcct gtttggtggt cttttcacac ggatgcgcat gaaaattacc 17880
aaagttcgca cagcaaccag aagaaaggtg gagcaggaaa gtctgtttat acaaagagct 17940
gaaagtttca accttaggcc acaaaagttg acatggatcg gaaggaactt ttataagaag 18000
ctctaaacag ttaatttgat aactaaaact cttaagacaa tcttccaata caaacatgag 18060
tattcaatca caaagattaa catgaaattc aaaagggagt tttgagggaa aatgaagaga 18120
tagaaaccac tgttatggct gggcaaggtg gctaacacct gtaatcccag cactttggca 18180
ggctgaggtg ggaggatcac ttgagcccag gagttcaagg ctacagtgag ccatgatcac 18240
accactgctc tccagcctgg gcaacacagt gagaccctgt ctctaaaacc agtaaaataa 18300
aataaaaacc actgttatgt agaatactca gtgttggtcc atgagtaaaa tttcaaattc 18360
aatttagagg accttaattt ttttttttga agattttaca ttgataagtc acatttacgg 18420
actcacctct caaaaaaatg ttaggttttt atattttacg gttttcagta tagccgtaat 18480
cattcaagtt taaccacttt acttcatttg aattaaaaag tcaatgtagt attttaatat 18540
taagacattt agtattttca gaaagtattc cttataaaag acttgctcaa ctgacaactt 18600
atagttagaa taaatgactc tttaaccact ttttccccca tagacaaaat gaagtaaaat 18660
ctcagggaac taacagagga ctttatttct gaggaaagag gagcagctat ggcttgagac 18720
tgctaaccct actccaaaca cacaaaagca gctacctctc tcctggccca aatgcacaca 18780
caggatgact tagcatttct agaaggaagc tttgtaagat gactggtgca acaggctatg 18840
aggcaaaccc ttcctgttag agggggagag ggcagctgga agacttccct gttagttcac 18900
aaataatgat ggctcccagc agggggtggg cccagccacc ctgggggaag aggtatatgg 18960

atccacagga ggagagtagg attctgagat cctaatactt acacatatac ttgagtccgt 19020
gatgaataaa ttgctcctag ctattggccc ctccctcctc acctatgtta tggctcagat 19080
cggctaaaag cagccaactt ccttctatac agcacggaac tgtagaaacc accgactttc 19140
ctccttactc tgccttacac cacacagttc aatagggcga ggcacaatga acagggaggt 19200
caaaagggga gaagagtcaa gggagagaaa ggcaacctgt atccagagga gcaaacagga 19260
aaaattctgt tttattattt ttgagatgct tctttttttt tttttaggcg gagtcttgct 19320
cggtcgccca gcctggagtg cagtggtgcg atctcagccc actgcaaatt ccgcctccca 19380
ggttcaagtg attctcctgc ctcagcctcc tgagtagctg ggattacagg cacgggtcac 19440
tatgcctggc taactttcgt atttttagta gagatggggt ttcgccatgt tggtcaggct 19500
ggactcaaac tcctgacctc gtgatccgcc cacctcggtc acccaaagtg ctgggattac 19560
aggcgtgagc caccgcgcct ggcccaattc tgttttaata tagtgagaac aatctgccag 19620
aagcaaagat cagcctctca ttaaccacga gaggcatcct tcgttctcct ttttcattcc 19680
tgccagaagt ctatgtattt tactcttaga accacctttg ctgggcatgg tggctcacgc 19740
ctgcaatccc agcacttttg gaagccgagg cgggcagatc ccctgaggtt aggagttcga 19800
gaccagcctg accaatatgg taaaaccccg tctccactaa taacacacaa aaattagcca 19860
ggcatggtag cgcatgcctg taatcccagc tactcgggag gctgaggcag gagaatcact 19920
tgaacccggg aggtggaggt tgcagtgacc cgagatcatg ccactgaact ccagcctggg 19980
tgacagagca caacaccgtc tcaaaaaaca aacaaacaca ctgaaaacca cccccccggc 20040
caagattttg atattgttaa tcccctctcg ttttatgttt atttctttat attatttcct 20100
tatctttctg agtttgatgc ttagctcact aatgtaagat ataaaacaag tgggcaggat 20160
acacaccaac ttcaggatag tggctttctt ctaaaagagg aaggagaagg aacttagcaa 20220
aacaacgagg actcaaatgg ataggtaatt tttatctttt taatatctag gggaaaaaag 20280
taaattttta ataatttctg agtacacaag catatgttat tcgcaaactt tttttggagg 20340
ggatggagtc tcattctgtt gcccaggctg gagtgcagtg gcatgatctt ggctcactgc 20400
aacctctgcc tcctgggttc aagcgattct catggctcaa cctcctgagt agctgggatt 20460
acaggcacct gctaattttt tttttttttt gagacgaagt ctcgctctgt cacccaggct 20520
ggagtgcagt ggtgccatct cagctcactg caaactctgc ctcccaggtt caagcaattc 20580
tcctgcctca gcctcctgag tggctgggac tacaggcacg tgccaccaca cctggctaat 20640
tttttgtatt tttagtagaa acagggtttc atcatgttag ccaggatggt ctcaatctcc 20700
tgacctcatg atccacccgc ctcggcctcc caaagtgctg gcattacagg cgtgaaccac 20760
tgtgcccggc cctgttattc atgaactttt ctatgaaaaa ttttactcaa gttacctgcc 20820
tcataaataa aatcttagat cactgactac aattacatca ctaatgctaa aatattccaa 20880
agataaaact ggataggaag gacagtcact tctgaattta cctcacagtt tgtacgaatg 20940
agataagaac tacaaggaag agaatcaaaa gcagcgtcag cctctctcac actgtcagtt 21000
tcttcaacgc aagaaagagc aaaaggcact ctgaaagcta aagagggctg aagagattct 21060
ctaaagacca gtctgtatct gtcctccaga catcaagatc agaatagtgg acgaaagggc 21120
ccatgtgaaa agcataacat taccaatgag ggtgacgggt acgccgtatt ccagggcgga 21180
gatggcggtc cacttccctg tgcccttctg ccccgcgctg tccctgatct ttggcagcag 21240
gtgtttgcca tcggtgtctt ggaacttgag aatattggct gtgatttcaa tcaggaatga 21300
gtctagctct gtcttattcc aatcctcaaa ggcctacatg cagaacacag gagagaacca 21360
tgttagagga atgagggctg agcaaggaag ccccttgtcc aatgtccacg ctgaccaatg 21420
ctcacggttg ccagcaggct ctgctcgcgt ctgcctgggg acttctgctc ctgctggttg 21480

agttttatgc taacccagag tcagttgtgt ttgttctgaa cctggttata ctgaatttct 21540
ggccaccaga agtgttgtct cctcagaggc aagggctcct cacttcactt aggtctctgt 21600
gcagtgtcgc ctcctcagaa gccttctgca atcaccggat cttgtcccag tttactcttc 21660
gttctcacag tcatcgctaa ctagtttgat attgtatatt tatttctgtc tgtcttccca 21720
gctccgggag ggcaggcact ttgctttctt cactagtata tccccagcat ttagaatcat 21780
gcttggcata tagtaaagca ctcgtgtgtt tgctgagtga acacgttaag tattatatat 21840
accaataaaa aatgaacaca aaaagaatag cactgtttct gtgaaagaaa actaaggtaa 21900
tgttttagaa agaacaatta acagcaatcc agtgaaacaa acaatagtca aagtaagtga 21960
ggatgacatg aataaaaaac taggaggaaa acaacaaaaa atctggaaag ctatactgta 22020
tgccttgcta gagtctgtaa atcacatccc attggaaata actgaaacta gaaattctag 22080
a 22081

<210> 77
<211> 11967
<212> DNA
<213> Homo sapiens

<400> 77
tctagaaacc aaaggcatag tcacaattaa cggaagactt gccatgaagg gtgaagggct 60
ctgggaagga caaggagatc gtcatgaata aaaagctaca ggctcttatg gaatggtggg 120
gctgctggaa atgcctcctt tcacctttaa gagggtttgt tatattagtc aagtcactgt 180
actggctgta gaaatcaagg cttcactttt tctgcttctg aaattagatg ggagggtgcc 240
taaagtgtgt atcttttttt tctctttcag aaaattttgc caaagacctt gaaatgtttg 300
caaggtgggt agagaacttg attatccgac actgcgtctg tgtagctttt tggggcctct 360
ccatctggtg ggttatttca ggagagcttt agctattcag ttttccgtgt gttttgtgga 420
ggcttgtctc taatcatggt tctgcaagaa catgacgcct ccttcaatca cggtgattca 480
cacttatttg aacatttttt gaagttttcc aagtattttt tgttaaagaa cccaagttat 540
tcagtcccaa gtgaaaaagg tgtgaatacc atctccagga ccctgaatgc tggtgcctgt 600
attaaaaacc atgtcttcgg gtggtttgat tgctcagggt ttgagaacat ggtgcctggg 660
ggtggccgcc cctctgcgga gggagctgca gcgtgtccac ggtggtaatt gcttgcaggt 720
gtacacagaa tggcaggcag gggttttcat ttgccgcatt tgttccgagt aagacgcact 780
ccttggaaag tgggtgactt gctttcagct gctaaagcct cagcttttac aggtgattta 840
tgctccgctc ttcaattagt accgcaggta attgaaaaca attgcaccaa attgctttta 900
tgaagcaatt tgaatgacga aggcctgaaa gggcgaaagt gttctaattt gctgccactt 960
ggattcggtg ccagcctctc ccacacccct tacttttcac cttgttcccg ttagctcttt 1020
gtttctcggg tgcgtgtttt gaatgaatta aagcagtggg ttctcggagg gcaagctctc 1080
aatctgtcct attcctgtgg atattcgtcc ctgaaaaccg cttagcatct tcagcttggc 1140
caaaagggat aaggtagtag gaagggatac cgatagctca tgtggcttag tcattgctga 1200
ttcaggcaga tgccccttat gtgtggccct cctccccggt gaaagatatt aataccttta 1260
cttgttattt attagctgtg ggaaaaaaaa atgaaaaatt taaatactta aaaataatat 1320
atatatatat acacacacac acacacacac atatacataa atatttatat tagagacaca 1380
gcgtcttggt atgttgccca ggctggtctt gaactcctgt gctcaagcaa tcctgccttg 1440

ggctcccaaa gtgctgggat tacaggtgtg agccaccatg cccagcctga aaatacttct 1500
gtaaaaattc tatttaaaaa atgaaattag aaagagcatg gtaacacatt aggctttcgg 1560
atattctgtg ttttttaaag tatataagag gttgattttt tttttttttt ggagacagag 1620
tctcactctc gcccaggcgg gagtgcagtg gtgcaatctt ggctcactgc aatctctgcc 1680
tcctgggttc aagtgattct cctgcctcag cctcctgagt agctgggatt acaggcatgt 1740
accaccacat ctggctaatt tttgtatttt tagtagagat ggcgtttcac catgttggcc 1800
agggcggtct tgaactcctg acctcaagtg atttgcctgc ctcagtctcc caaagtgcta 1860
ggattacagg cgttgagcca ccgcgcccgg caaacagttg attttctttt gctgggtact 1920
ggtggggaaa ggggtatgtg cgtgcatgtg agtgtgtgtg tgtatgtgtg tgtgacttag 1980
accataagct ccacaaggaa gggcgacatc ttttatgtaa aagtgtttgt ggaggccggg 2040
cgccgtggct cacgcctgta atcctagcac tttgggaggc tgaggcgggt ggatcacctg 2100
agctcaggag ttcgagacca gcctgggcaa cagggtgaaa ccctgtctcc taaaatacaa 2160
aaaaaaaaaa ttagctgggc gtggcagcat gcgcctgtaa tctcagctac tcaaaaggtt 2220
gaggcaggag aattgcttga accagggagg tggaggttgc agtgagtcga gatcgtgccc 2280
cttcactcca gcctgggtga cagagtgaga ctctgtctct ttaaaaaaaa aaaaagaaaa 2340
gaaaagaaaa aaaaaagttt gtggaaacgc tttgtctgag aactgagtga gtgcagagtc 2400
cagtgatgag gctccaggag tctggggtgc agccagaggc agggaagcac cccctcttct 2460
tagcacaacc cacaagtaca ggtttccttt gaagactcat cttgtgtgaa ttccagtttg 2520
ctttcataca aaaaagcatt gttttatccc tgaataaaat tgatattcct ggaaggtcag 2580
ctttggttat cttgtggatt taagttgtcc ctgtcacact tctctactcc caagactatc 2640
attaccccag tttgagaaag atggactgtt tcaagtcaac tatatgttac atagtctctg 2700
taggctggcc tgggaaccta atgagtattc gtaacattgt ttcattagga caaatttatt 2760
ttcttgtttc ttgtttcttt tttttttctg agacaggatc tctctgttac ccaggctgga 2820
gtgcagtggc aggatcatag ctcactgcaa cctagagctc ctgggctcaa gtgatcctcc 2880
caccttggcc tcccagagtg ttggtattac aggcatgagc tgagccactg tgcctggcat 2940
cttttttctt tttggtgagc ttgttatgag aaatttctat gagaatacac atttagattc 3000
ttatttctta tttctttttt tttttttgga gcaggaggga aacttgagag caggggctgt 3060
tttttttttg ttttttgttt tttgttttta gatggagtct cactctgtcg cccaggctgg 3120
attgccatgg cgcgatctca actcaccgca acctttgcct cctgggttca agtgattctc 3180
cagcctcagc ctcccaagta gctgggatta caggcgcctg ccactatgcc cagctaattt 3240
ttgtgttttt agtaaagacg gggtttcacc atgttggcca ggttggtctc aaacttatga 3300
cctcaggtga tccacccacc tcggccagcc aaagtgctaa gattactggc atgagccact 3360
gtgcctggcc aatttttttt tttttttttt ttttttgaga cagagtctct ctgtcgccca 3420
ggctggagag cagcagctca atctcggctc actgcagctt ctgccttgcg gattcaagtg 3480
attctcctgc ctcagcctcc cgagtagctg ggactacagg caaatgccac cacacccact 3540
aatttttgtg gttttaatag agatggggtt tcaccatgtt ggccaggctt gtctcaagct 3600
cctgacctca ggtgatccgc ctgcctcggc ctcccaaagt gctgggatta caggtgtgat 3660
ccaccatgcc tggccagaga atacacaatt aaattctaaa taatcaattc actagcagac 3720
tttggaaagc aactgccaat ctgaattttt gtttgttttt gttttgtttg ttttttgaga 3780
cggagtctca ctctgtcacc caggctggag tgcagtggtg tgatctcggc tcactgcagt 3840
ctccgtctcc tgggttcaag cgattcgcct ttctcagcct cccaagtagc tgggattaca 3900
ggtgcccacc accatgcccg ggtaattttt gtcgttgtag tagagacagg gtttcactgt 3960

gttggccagt ctggcctcga actcctgacc tcaaatgatc cacccacctc ggcctcccaa 4020
agtcctggga ttatagacat gagccgccac acctggcctg agttgtgaat ttctttattt 4080
agctttgaaa tctctagtct cctagtattg ttaacaatca aacattttga agaagataga 4140
tattctatag gaaatacatt ctggaaactt ttgggaatcg tctttaaagc ccgtgttaca 4200
aagcgtgtta tatgaaaaat tgcttttgat tttccctagc tagagtaatt ttctggagtt 4260
ggaggtttga cttaggagag tctgtgatat gcagtggcgg gcagaggatg ccctgcccct 4320
ccattgaggg ccttagtgca gagatagcag aggccaagct gaagtggtgg agaggactga 4380
gatcgttgtt tcctaaacgc tttctcgtaa acagataagt tcactatgta aacagttcat 4440
tgtgcttatt ccttttactg tactttaaga agaatgtaaa tacagacaga gcagcctggt 4500
tgtgaggttg agactaggcc accctgtcca tgtgcactta acattctcca cccagaacgt 4560
ctgtggactg acctcattct ttgtggctgc ttttatgtca cctctgtctt ttttctcccc 4620
tgtgtcctgg tccttgcagg ttgctaatta gaaaattatc tcttgaagtg ggtattaatg 4680
tcatgacaag cagatattgt caatcacgat gagatgacac gtgatatttg tctatttagc 4740
agaatgtaaa ggattaattt ggccaaatta tggagaataa tttctgtgtt gaatttatct 4800
tttaacttag cagttgttca gtgaggactc accatgtgtg ctggccagtt tgctagttat 4860
taaggatgca aagattccta tttattcctg cttacaaagt acctgtgtat tttgtgggga 4920
agttagaaat ggaagaaggg tgtaatcaga gtaatatatt attgaatcga gcttattatc 4980
tcaaattacc tttcctagca aagataaaag gactagaatg agaatttgag tccaacttgt 5040
gagcaagaag cagactccag tttaaagggt gttctttgtg gctgggcttg gtggctcacg 5100
cctgtaatcc cagcactttg ggaggcagct ggatcatgag gtgaggagtt caagaccagc 5160
ctggccaaga tggtgaaatc ctgtctctac taaaaataca aaaaaattag tggggcatgg 5220
tggcgggcgc ctgtaatccc agctacacag aaggctgagg cagagaattg ctttaacccg 5280
ggaggcggag gttgcagtga gccaagatcg tgccactgcc ctccagtctg ggcgacagaa 5340
cgagactctg tctcaaaaaa aaaaagaaga aagaaggatg ttctttgttc tacctgggtt 5400
attgcggagg gaatgcatta actacttgtt gggaatgggg ccttaggagg tgcctgcata 5460
tgctttttcc tgttaagtta gcccttggag aacatcagga tcttgaccta agtaaagcca 5520
tcctttgtgg aggttctctt tgttcttcta tttaatgtct atttaagcag gtgccctctg 5580
tatcatgagg cccattttct ctgcactaga gttgtacact gaccttcttg atttttctgt 5640
ggccttagct gtggccaagc aagccaggtt tggaagggcg ctaagcccca gcctcctgtc 5700
tgtgctctta tgcttcgggt acccttggcc ctaagcggag gtggagagat gggcctccgc 5760
attctccatc tctttactta attggggtct taggaacttc actaattttt gtgtcacttt 5820
tgcttaatgc cctttatcta aaaggatggg ctacttagag atccctgcta gtttgaagtc 5880
tgaccgtgaa cttctgtgtt tcatcaaaac tttaaataat accaaacatt ttggtgactt 5940
gcttctgccc tttctgcaga catgcgaaaa gaaccacaat taacactgaa gatgtgaagc 6000
tcttagccag gaggagtaat tcactggtga gagatgaatt tctttcctca ctcccctttc 6060
ccatcggaat acattattcc caatcaatca cttgaagcca tgaagttatt ccccagggca 6120
ccttgcatta gaaactagag gtggctgaac cattcagacc tgcctgtaat ttacagtctt 6180
gctttcatta tgatggatct tgctaagcca atttgaaatt ttctaatcag cactctctgg 6240
attttacaat ttgcctgttt ttgtaatacc tcaaaacttt ggtcccatgt ttcatttaca 6300
acatgatgtc agagactggt cacaggccaa tctgcaggga tagttttgtt attgtattca 6360
cagttctccg aataggccag gtatggacgg agtttccgta tggctggata tgcaggaaaa 6420
cacattttcc atattttcaa gtctagaaat catttgacca agcctccgtt gttcaggtga 6480

agacccagag atactctggt catcccaggc tagtggcttt ggccagtagc agagccagga 6540
cagcggggag ctttcctgac tgagtgtaat gctagacatt aaagcacgtg atggctgtga 6600
tagaatgtgg gccccacaag agcaggggct tttgtctctt tgttctctct ccatctgtgt 6660
tgcctagaac gcacctgtgt gcctggcaca caggaagggc tcaataaata gttgttgagt 6720
gaatgaattt gtatagcagt ttacaggggc ttttccagat actattttac gtatattatt 6780
taatgctcac aacttttttt ttcttttttt aaattttgga catgcagtct ctctctgttg 6840
cccaggctgg tcttgaactc ctgggctcag gcagtcctcc cgccttggtt tccctatatg 6900
ccaggattac aggcatgtgc cacaaccttt tttttttttt tttttttttg acggggtctc 6960
actttgtcac ccaggctgag ggcagtggcg catcattgct cactgcagtc cccgtctcct 7020
gggctccagt gatactttca ccttagcctc tcaagtagct gggactacag gtgcatgcca 7080
ccacacccgg ctatttttgt atgttttgta gagacggggt ttcaccatat tgcctgggct 7140
ggtctcaaac tcctgggctc aaatgatctg cttgccctgg actcccaaag tgctgggatt 7200
ccaggcatga gccaccgcac ctggctacaa ctcttttttt tttttttttt tttttgagat 7260
ggagtctctc tctgtggccc aggctggagt gcagtggcgt gatctcggct cactgcaaac 7320
tccgcctcct gggttcatgc cattctcctg cctcagcctc ccgagtagct gggcctacag 7380
gcacccacca ccacacctgg ctaatttttt gtatttttag tagagacggg gtttcaccgt 7440
gttagccagg atggtctcga tctcctgagc tcgtgatctg tctgcctcag cctcccaaag 7500
tgctgggatt acaggcgtga gccaccgcac ccgacctctg gccacaactc ttaataacac 7560
aacttattgg ctaggtgtag tggctcatgc caataatccc agaactttgg gaggccaagg 7620
caggatggct tgagcccagg agtttgagac cagccctgcc cacatagtga gaccctcata 7680
tctacaaaaa atttaaaaaa aaagaaaaaa aaaattgaag ctgcgtttaa ttcttagaaa 7740
tgtaacccag gctttggctt ttctacccaa attctttgag tcagaggtgg ttgtggaggg 7800
tttagtttcg tttgtttgtt tatttaggtt tataaaatgg ttacagccag atataaatac 7860
agattttttt ttatagctaa aatacatcac agacaaaagt gaagagattg ctcagattaa 7920
cctagaacga aaagcacaga agaaaaagaa gtcagaggat ggaagcaaaa attcaaggca 7980
gccagcagag gctggagtgg tggaaagtga gaattaaagt ccctcgccgc ttggaaagtg 8040
cagccttcta caggtagagc cacctagaaa tgcatatggc tgcaaaggaa actttgaagg 8100
gttaaataga gatttaaaaa aataaaataa aaaggctggg ctagggtgct ttttgtgctg 8160
aattctccac attgttaact gccaaagcta gttttagaga atgagaaagt cttaagcaaa 8220
atactcccag gtctcactcc agaacataaa aatggtgtgt gatcgaatgg tatatattag 8280
aaattacatc tgttgtaatt aaaattgtgt gagcaattaa acatggttga ctttttcaag 8340
caaaaatcag ttcatctttt gatgtaattt tctaggctaa atggcaatct ctgaaagatg 8400
aataaagcta tatttattta gcttttctgg ttactaatgt tgtttttaaa cacatgccgc 8460
ctttggtcac caggtaaaag ggcatatgtt tgggaatggc ccttttgact tttttttttt 8520
tttttttttt tttgagacgg agtcttgctc tgtcgcccag gctggagtgc agtggcacga 8580
tctcagctca ctacaacctc tgcctcccgg gtacaagcaa ttctctgtct cagcctcccg 8640
agtagctaag ttactggcac ctgccaccat gactggctaa tttttgtagt tttagtagag 8700
acagggtttc accatcttgg ccaggctggt cttgaactcc tgacctcatg atccacccac 8760
cttggcctcc caaagtgctg ggattacaga cgtgagccac tgcgcccagc ctttttattt 8820
ttttatttat tattttttac ttttttgtgt gtgagacaga gtcttgctct gttgccagga 8880
gtgcaatggc gtgatctcag ctcactgcaa cctccgcctc ccgggttcaa gcgatctcct 8940
gcctcagttt cccaagcagc tgggactaca ggcatgcacc accatgccca gctaatttgt 9000

gtgtttgtgc tttttttttt tttttttttt ttttttgaga cagagtcttg ctctgtcgcc 9060
caggctggag tgcagtggcg tgatcctctc cctatagtga gtcgtattag cggccgcaaa 9120
tttattagag caatatagtc ctacaatgtc aagctcgacc gatgcccttg agagccttca 9180
acccagtcag ctccttccgg tgggcgcggg gcatgactat cgtcgccgca cttatgactg 9240
tcttctttat catgcaactc gtaggacagg tgccggcagc gctctgggtc attttcggcg 9300
aggaccgctt tcgctggagc gcgacgatga tcggcctgtc gcttgcggta ttcggaatct 9360
tgcacgccct cgctcaagcc ttcgtcactg gtcccgccac caaacgtttc ggcgagaagc 9420
aggccattat cgccggcatg gcggccgacg cgctgggcta cgtcttgctg gcgttcgcga 9480
cgcgaggctg gatggccttc cccattatga ttcttctcgc ttccggcggc atcagggatg 9540
cccgcgttgc aggccatgct gtccaggcag gtagatgaca gaccatcagg gacagcttca 9600
aggatcgctc gcggctctta ccagcctaac ttcgatcatt ggaccgctga tcgtcacggc 9660
gatttatgcc gcctcggcga gcacatggaa cgggttggca tggattgtag gcgccgccct 9720
ataccttgtc tgcctccccg cgttgcgtcg cggtgcatgg agccgggcca cctcgacctg 9780
aatggaagcc ggcggcacct cgctaacgga ttcaccactc caagaattgg agccaatcaa 9840
ttcttgcgga gaactgtgaa tgcgcaaacc aacccttggc agaacatatc catcgcgtcc 9900
gccatctcca gcagccgcac gcggcgcatc tcgggcagcg ttgggtcctg gccacgggtg 9960
cgcatgatcg tgctcctgtc gttgaggacc cggctaggct ggcggggttg ccttactggt 10020
tagcagaatg aatcaccgat acgcgagcga acgtgaagcg actgctgctg caaaacgtct 10080
gcgacctgag caacaacatg aatggtcttc ggtttccgtg tttcgtaaag tctggaaacg 10140
cggaagtcag cgccctgcac cattatgttc cggatctgca tcgcaggatg ctgctggcta 10200
ccctgtggaa cacctacatc tgtattaacg aagcgctggc attgaccctg agtgattttt 10260
ctctggtccc gccgcatcca taccgccagt tgtttaccct cacaacgttc cagtaaccgg 10320
gcatgttcat catcagtaac ccgtatcgtg agcatcctct ctcgtttcat cggtatcatt 10380
acccccatga acagaaatcc cccttacacg gaggcatcag tgaccaaaca ggaaaaaacc 10440
gcccttaaca tggcccgctt tatcagaagc cagacattaa cgcttctgga gaaactcaac 10500
gagctggacg cggatgaaca ggcagacatc tgtgaatcgc ttcacgacca cgctgatgag 10560
ctttaccgca gctgcctcgc gcgtttcggt gatgacggtg aaaacctctg acacatgcag 10620
ctcccggaga cggtcacagc ttgtctgtaa gcggatgccg ggagcagaca agcccgtcag 10680
ggcgcgtcag cgggtgttgg cgggtgtcgg ggcgcagcca tgacccagtc acgtagcgat 10740
agcggagtgt atactggctt aactatgcgg catcagagca gattgtactg agagtgcacc 10800
atatcgacta cctggcacga caggtttccc gactggaaag cgggcagtga gcgcaacgca 10860
attaatgtga gttagctcac tcattaggca ccccaggctt tacactttat gcttccggct 10920
cgtatgttgt gtggaattgt gagcggataa caatttcaca caggaaacag ctatgaccat 10980
gattacgcca agcttgcatg cctgcaggtc gaaggccgtg gcggcggcac ttttgcccat 11040
gaattgctcg ctgtggagta cgcaggctgg atttctcccg cgtttcggct gaaggtaaac 11100
cagacattta tcgactatcg aaccggaaga ttacaacctg ctattccgca gagtctccct 11160
gaagctctcc gtttggctgc cgacctggca gagcaaaagc aacggctgga gcaaaaaatg 11220
cttatggatg cacctaaagt cgaattcgct gaacgcgttg ctaccgccag cggggttcta 11280
atcggcaact atgccaaagt gctcggcctg ggccaaaact atctcttcac ctggttgcgt 11340
gataacggaa ttctgatcgc aaccggtgaa cgcaggaacg tccccaaaca agaatacata 11400
tctcgtgggt atttcaccct taaagaaacc gtgatcgata caagcaatgg aagcaggatt 11460
tctttcacga ctcgtataac cggcaaaggt cagcagtggc tgatgaagcg attgcttgat 11520

gctggtgtgc tggtacctgt cgcggcaacg cgctaacaga cgtagtaaga accaccagca 11580
ttgtaatgct ggctaaagtc actttcctga gctgtataac gatgagcgat tttacttttt 11640
ctggctatga attggcctgc tttgtaacac actccggtct atcccgtagc gccgggcata 11700
tcctgtcgca atgtgcaaat ctcgcggcaa caaccagtga atacttcatt cacaagcctc 11760
accgcctgat cgcggcagaa actggttata gccaatcaac cgtcgttcgt gcattccgtg 11820
aagctgtaaa caaaggaatt ctgtctgtag agattgttat cggcgatcac cgtgaacgtc 11880
gcgctaacct gtaccggttt acaccatcct tttggccttt cgcacaacaa gccaaaaatg 11940
cgctgataga aagcaaatta aagatct 11967

<210> 78
<211> 1119
<212> DNA
<213> Homo sapiens

<400> 78
agatctcttt catttgtgtt tttacaaaag cattttagag atgattatac acaacaatta 60
cattaatcac ttactctaac tcagctcatc gagacagagt aagctgatgg tatcactttg 120
atctaggaca gtggttctca actgaggatg ccctccctgg gggatatttg gcaatgtctg 180
aagacatttt tggttgtcac aaatagagtg gagctgctac tggcatcttg tgggtagggg 240
ccaaggatgc tgcaaaatat tctaaatgca caggacagcc cctcacaaca gagaattatc 300
cagccccaaa tagtgctgag gttgagaaac cctggtctag gaaaatattc tgcccttcta 360
tgtcctcctt gacatcagta atgtaagtct ttaccatatt ctatgcaata tgctcctttt 420
gctttactac taataaaacc cattatgata aaacccttac aacaaggtgc tgctgggcac 480
agtggctcac gcctataatc ccagcacttt gggaggctga ggcgggtgga tcacctgagg 540
tcgggagttc gagaccagcc tggccaacat ggtgaaaccc tgcctctact aaaaatacaa 600
aaattagcct ggcgtggtgg cacgcacctg taatcccagc tacacaggag gctgaggcag 660
gagaatcgct tgaacctggg aggcagaggt tgcagtgagc tgagatcgcg ccattgcact 720
ccagcctggg caacagagca agcctccatc tcaaaaaaaa caaaaaacaa aaaaaacccc 780
aaaaaacaac aagatgcctg tctccatgat tttgctgttg ctaacgagaa ggcaaattat 840
tatgctcaac agtcagttct ctgataaaca tctgattcaa ataccaagac caccaaaaat 900
caattcaaaa ctggaattcg gccaggtggt ggtggctcat gcctgtaatc tcagcacttt 960
gggaggtcga agcaggagga ctgcttgagg ccaggagtgg tcaagaccaa cctggtcaac 1020
ataacaagac tgcatctcta ttaaaacaaa aacaaaaaca aaaccatccc tggaatttga 1080
cagtgacaat gattcatgaa cccaaataca aggaaagga 1119

<210> 79
<211> 937
<212> DNA
<213> Homo sapiens

<400> 79
ggatccaccc acctcggcca gccaaagtgc taagattact ggcatgagcc actgtgcctg 60

gccaattttt tttttttttt tttttttttg agacagagtc tctctgtcgc ccaggctgga 120
gagcagcagc tcaatctcgg ctcactgcag cttctgcctt gcggattcaa gtgattctcc 180
tgcctcagcc tcccgagtag ctgggactac aggcaaatgc caccacaccc actaattttt 240
gtggttttaa tagagatggg gtttcaccat gttggccagg cttgtctcaa gctcctgacc 300
tcaggtgatc cgcctgcctc ggcctcccaa agtgctggga ttacaggtgt gatcatctct 360
ccaccccttg cagaaaatta ccgagagggg aggatgagcc tcaattgcaa gacaagctaa 420
gcaatggtct cagccacgtg tcgacagagt tctgttgagc ccacctgtgg gtactccgcc 480
tcggggccga agacacagct gagctgcacg tgatgacagc tgaacccaca agggcataaa 540
atctaccagc aagtgttcaa cgcataggca agaggaggag gaactgccaa aacccaagtc 600
caggaactca ggatagaggc cagacagatg aggagggcat ctactttcct gaatttcaga 660
cctcacaaga ctaggtaagc tgaggcctct ggtaggagaa gccaaagagg catcaacctg 720
ggaggcacga agatcacagg tgtgatcact gcacactaca gcctcgaact cctgggctca 780
agtgaacctc ctgcctcagc ctcttgagta gctgggacta caggcccaca acaccctgcc 840
cggctcaagg tcatcttttt taggtatggg tgggggtcag ggtacaggac atgaattctt 900
ctgaatgctc ttggctccag gaatttttct cagatct 937

<210> 80
<211> 5301
<212> DNA
<213> Homo sapiens

<400> 80
ccatggaagc atgcatgagt ctggtgcgct gcttttcttt tgttgttgtt gttttgagac 60
ggagtcttgc tccgtcgccc aggctggagt gcagtggcac aatctcggct cactgcaagc 120
tccgcctcct gggttcacgc cattctcctg cctcagcttc ctgagtagct gggactacag 180
gcgcccgcca ccacgcctgg ctaatttttt gtatttctta gtagagacag ggtttcaccg 240
tgttagccag gatggtcttg atctcctgac cttgtgatct gcccacctcg gcctcccaaa 300
gtgctgggat tacaggtgtg ggccactgcg cctggcccgg gctcgtgcgc tgcttttcta 360
tctggttctt cttgttattc cacttgcggt tggatgggga gatattccac tttcccttag 420
gaactcttgt ccctgcacag accattttag ctattctgag cacatcacaa gcatactcct 480
ctggggtgag aatatatagc ctctgggctt tttcagaatt gctccttctg ctactctgga 540
agctaacgtg gaatgattgc ttgagtccag cagttcaagg ccagcatggg caacaaagca 600
agaacctgtt tctttaaaaa aaaaaaaaaa aaaaaaaaaa ggcgggggga gcaggcatgg 660
tggcttacac ctgtaatccc agcactttga gaggccgtgg caggaggatc acttgaagcc 720
agagctcaaa accagcctgg gcaacttagt gagcccccat ttctacaaaa aataaaaaca 780
attaactggg catggtggta cacacctgta gcctcagcta ctcaggaagc tgagatggga 840
ggatcccttg agcccaggag tttgagactg cagtgagcta tgatggtgcc actggcgcct 900
gggtaacaaa gcaagaccct gtctcaaaaa aaaaaaaaaa aaaaaaggag ctgggcatag 960
tggcatgtgc ctgtaaatga aaggcatcat ttcatgcaag ctcctctgag cccaggagtt 1020
ccagcctagc ccaggcaaca gggcaagacc acgtctcaaa aaaaaaaaaa gtactctttc 1080
cacctaaaat atattcaggt catttgagtt cagtttgagt tcagctacga gaattattta 1140
gttgagtgag tgtcagagct gggattttca aatctgccct tactggtatg ttgctttaca 1200

caccctctta cgtaaatcaa actaggattc caccctggga ggtttgcata gagggctgtt 1260
cttgtagaac ttgtgctcat gctttgattt gggatttggg gagttagggc aagccagaaa 1320
gtttttctgg tggataataa tgtgggttga ctttcttaag cattttaagc caagcacttg 1380
agtttctaac aactaaaaag ctaagtcagc ctgacacagc tctagcgcgc cctggcttga 1440
ttctgttcat ccccaggggg agacttgcct ttgttccagt cctgctctcc caagccagct 1500
tactgtagtt ttccagcaat tctgagaagc agtatttttt actgctgatt agaaccttaa 1560
catggaaatg gaaagtttgt gtagcatgta acattattag aagggaaaac atgactatat 1620
gattagatac atattgattt tatgcaatat gtttgcataa aatctcttca gtagtaactt 1680
ggttaaatta ttcacattga ctcagcataa tttctccctt gctaaatctc agactaaagc 1740
aatcaatttg ttcctgcaag aaagtctgat ggtaggcttt ccttgtaact gagccccata 1800
gaaagaggaa gcagctgcaa caaagttgaa aaggatgcct gtcaccaagg ttcttttaag 1860
ggagccattg tgtgaagttc tacttgtgaa atttctgcaa gtactgtttg ttctccaaaa 1920
tccagctttc cgttgaaagt caatgaaaca tctctaaaat gcagacatac ccaggttcat 1980
tgggaacaca gtcactgtga gtatacatga ttatgtcctg ctaaccctat tgggaaagaa 2040
tgctgggctt ggccctagta agtagacttg ggttaaagtt cacctccgcc actaactagc 2100
tgggaagttc aacttccctg agctgagttt gtcacgttag agaatattaa taaatatgaa 2160
aattgtggag cagtcttaag tttaaaagga actttcatgg acattagctc attttgctgt 2220
gcccaccctg ggaaggaagc aggacaggca ggcagtggca tccctgtcgt caagcagcct 2280
gaggactgct gtgagcctct ggagcaaggc aggagtttga ggaggctctc agtccttttg 2340
tccttttttt gagacagagc atcattctgt tgcccaggct ggagtgcaat ggcatgatct 2400
tggctcactg caacctctgt cccccaccct taccactggg ttcaagcaat tcttaggcct 2460
cagcctccta agtagctggg actacaggat gcaccaccac gcccagctaa ttttgggggg 2520
gttttttttg agacgagttt cactcttgtg gtccaggctg gagtgtagtt gcgtgatctc 2580
ggctcactgc aacctctgcc tcctgggttc aagtgattct cctgcctcag cctcccgagt 2640
agctgggatt acaggcgccc accaacacgc ctggctaatt ttttgtattt ttagtagaga 2700
cggggttttg ccatgttggt ggtctcaaac tcttgacctc aggtgatcca cccgcctcgg 2760
cctcacaaag tgctggagtt acaggtgtga gcctgtgtgt cctttctatt gaggcttctc 2820
tccacatttt ctgatcctca gtctccttat ttcctgtaag gctgaggtcc caaatccagc 2880
ctgtgaaaga gaaatgccag cagaggcagc acccagcaca ggatctatta ttgcagcatg 2940
ccctgtggtc agggccttca gtaaaagccc acagaccctg actactttct ggcggactct 3000
ggaggaaggc cttaaccaca gacaaaagga caaggacagt gctgtggagg gactgctggc 3060
tgctgcagcg tgaagagaat agggaatgca ctctacaaaa ataaaaaagc ataaaattgg 3120
aaaaaatata taccaaaaac atttcaaggt tctggacatc aaccaaaggc aggcaaatta 3180
agaagcattt attcttgaga aaaaactggc tgggcacggt agcttgcacc tgtaatctcc 3240
caacactttg ggaagcaaag gtgggaagtt ggctggaggc caggagttca aggccagcct 3300
atgcaaccaa gcaagaccct gtctctacaa aaaataataa tgaataataa aaagaaaaag 3360
agtggctcac gcctgtaatc cctgcacttt gggacgccta ggcaggtgga tcacctgagg 3420
tcaggagttc gagaccagcc tgaccaacct ggtgaaaccc atgtctctac gaaaaataca 3480
aaaaaattag ccaggcatgg tggctcatgc ctgtaatccc agcaacttgg gaggctgaga 3540
caggagaatc gcttgaacct gggaggcgga ggttgcggtg agccaagata gcaccactgc 3600
cctccagact gggcaacaag agcgaaactt agtatcaaaa gaaaagaaaa agataaagaa 3660
aaaaaaagtg ctagaacttt tttttttttg agacggagtt ttgctcttgt tgcccaggct 3720

ggagtgcagt ggcacgatct cggctcactg caacatctgc ctcccaggtt caagcaattc 3780
ttttgcctca gcctccccag tagctgggat tacaggcatg caccactgcg gctaattttt 3840
tatttttagt agagatgggg tttctccttg ttggtcaggc tggtctcaaa ctcccgacct 3900
caggtgatcc tccacctcgg cctcctaaat cggcctccca aagtgctggg tttacaggtg 3960
tgagccactg acccgggccg agaagtgtac cttttttttt tttttttttt tttaaatgga 4020
gtctcactct gtcgcccagg atggagtgca gtggcgcgat ctcagctcac tgcaagctcc 4080
gcttcccggg ttcacgccat tctcctgcct cagcctcccg agtagctggg actacaggcg 4140
cctgccacca tacctggcta atttttcaat ttttagtaga gacggggttt caccattttg 4200
gtcaggctgg tctcaaactc ctgaccttgt gatccgccct cctcagcctc ccaaagtgct 4260
gggattacag gcgtgagcca ccgcacccgg cctcaagaag tgtacttttt tatcagctaa 4320
aagtggcaaa gttcttgaga aataaacggc ttgagaaagc cgtttcttga gaaagaaacc 4380
cacagcttac tagcctgaga tctcaagtag cagactagct agaaatctag gagactctgg 4440
aaatgagaat gccacaaaag gattagacag gacctcagca catcactcgc tggctggaaa 4500
accagacacg tacacagatg agacttcaga aagcccatag aaattaaaag ccaagggaga 4560
gctgggcacc atggctcaca cctgtaatcc cagcactttg ggaggccaag gtaggcagat 4620
cacctgaggt caggagttca agactagcct ggctaacaca gcaaaacccc gtctctactg 4680
aaaatacaaa aattagccag gcgtggtggt gcatgcctgt aatcccagct acttgggagg 4740
atgaggcagg agaatcgctt gaacccagga ggcggaggtt gcagtgagct gagattgcat 4800
cattgcactc cagcctgggt gacagagtga aactccatct caaaaaaaaa aaaaagaaaa 4860
agaaaaatgt taaaagtcaa gggaggctgg ttgtggtggg ctcacgctgt aatcccagca 4920
ctctgggagg cagaggcggg tggatcacct gaggtcagga gttcaagacc agcctggcca 4980
acatggtgaa accccgtctc tactaaaaat acaaagaatt agccaggcat ggtagcaggt 5040
gcctgtaatc ccagctactt gggaggctga ggtaggagaa tcgcttgaac ccaggaggca 5100
gaggttgcag tgagccaaga tcccaccact gcactccagc ctggggagca agagcaaaat 5160
tctgtctcaa aaaaaaaaaa aaaaaaaagc caagggagat ttaagaagta ggtgatccct 5220
gaatgcaccg ccaacccacc cagataggcc taaatatctg agcacagcca aggtgtttga 5280
gcacaacctc ttcccagatc t 5301

<210> 81
<211> 13161
<212> DNA
<213> Homo sapiens

<400> 81
cctaggactt actcagtata ctgtgggaag ggctgacttt tctacctgtt gccaaagacc 60
tgctgtctgt ggcagccgca acgatatttg tcctgctatt aatcctctgg aatcgatcaa 120
atatcaaaaa taacttctct ttgtttttgt ttctaattta gcagagaggg attaaaagaa 180
aaaaaaaacc tttagattat atagatctaa aaatagacat catggatgct gattggaaac 240
cctcccagtc gttcatatga gagatctcaa tttgtgttac atagaggggc agctcacttc 300
tgtcttgttc gccagttata ctactcccca gagaagctca ataaatttta taggaaagtt 360
tcctccttat gtggaaggag ctaagctcaa tgactggtgt gtctgatata atttagggct 420
ccaagggagt acgtctgcct tgtggaataa cctatcaaaa gtaaatgtac agaaactttc 480

gatcttggtt gatttggcta tgtcattgaa actgctcagc actggacccc gtgaacccag 540
cctgtctgct ttggaattaa actgctcccc caaactcaca ttcgattcac tgttaaagcg 600
gccttttgtg tgtctcactg gtagtggctt ctgggctcgc ctgctgttgt tccacggttt 660
ccaccgctgg ccctttgaaa cctccaaact tggacgtggt tgagttttta catccctctt 720
tgcctcttct gttgacatta ggaaagcaga cctgccttct ctttaggctt cgagtggaac 780
aagcctgcca gtttcctcag tctttcagct agcagtgaaa tacattttgc actggctttt 840
ttactcagtg atttacactg ggagacgtgg ctgggtgatg tccagtgtcc ttttgtcttt 900
gtgtttattt ttcttccaca tttaatgtat ttaaaggata tattgtcttt atggttcact 960
gggataagga gggattggtg caagttttaa tgagaggtgc cccagctctc tgtgttgagc 1020
tgagaattat cttcttggtt agatatcttt ctttctttcc ttaggtggca ggaggcagtg 1080
ttttgctgct ttttgaaata atgtttctgc ctaacgattt ctagaaggat accgagagga 1140
cattcagctt cagggcatca tcagcaactt tttctcctgt tgtttgaact tccctcattt 1200
aaggagaggg gaaagtctca ggtcttgttc ttgaatttac atatcaatat taagatgatg 1260
ggaaacagcc ccctgccact ccccatcatc tgatcaattc ttataaaaac tcttgcattc 1320
agggtagtga catgtggaac tagttttctc agaaactgtt ttcactgctt gggaatagtt 1380
aatcagtgga ggaaaaagtg tttgatttat ctgcttaata ttttaaccaa gtgaattaga 1440
caggttgggg ggctggggga ggggataaga aagtgggtgg gggaggcccc ataaacaagg 1500
tgatatataa tatttttttt gttctccttt taaataaata ccgatcagct ttatgttcag 1560
agacaataga agccgttggc ttaaatttgc agtttactgt atttatggct gtaatatcaa 1620
ggtgctgccg tcgtaatttc atgccccaat gagaagagca aggtcgaagc aaatgcttcc 1680
atcggcatct gctaacacac taactcataa acaaggcccg gctggatcag gtggcacgga 1740
ataatacagg ctaatgaaat acagcacagc tttccattac tgttagtttt tacagtgtcg 1800
tcattacgtg taatttatgt ttaaaaaatt caattttata caaggcactg ggaaataggg 1860
gtctccaggt catcctacga cttttagagg ctctgagagt ccttattgta ctctactgtt 1920
ccaacaaaat gttcataaaa taaaataaaa atacttttcc ccttcccctc cctgaacctt 1980
ttctgagaga gttgattttt ttcttttaat ttgggaagta tttttagcca gctgtcttct 2040
ctgcctcatc tcctcccaga gtgtgccggg gcagataact tagttgttct gagagaggtg 2100
accccctctc agtgtccatg agtcccagat gaattggcca agtcctagaa atagaggggc 2160
tgcagagcgg ggaggaagag gtcccaatga ccagcccttg actgccaccg tttgtcactg 2220
gccccctggc cctgttcccg tgatcccagt cagatgccac atttttataa taaaactagg 2280
gctgagcagg aaggcgctgg caggagttca ttaccttgct tattttcaaa ctcttcctaa 2340
cccttctgtc ctggttgttg tttgacctga aagatgaaga gaacactgct gtcttctgtc 2400
atctgtcttt ctgcttcacc tgcacagggc caagccccgt ggcagtgccc agcggtcact 2460
ggggacagtg ctgaggttac ttatcctgag aaacccttgc acggcctgtc ccgcagagaa 2520
aagacagccc ttccgggtcc ctggtttggt ctgagtcgtg ggaaaggacc ctagggcatc 2580
acaatcgcag cctgtaccct gcagctcaca gagtcaatca gtttggtttt atttgcattt 2640
gaacagaaac cttgagaaaa agaaagccaa tttgtttatc ttctagggga taaaaatagg 2700
caagtgtggc agccctgtgg ccacgcaggt cagacactgc agcagtgaca ctggggtttt 2760
attaatcaat aggatgtagc tcacttgttc tgtcctttta aaaaaaaaag cccaccctgg 2820
agaagagatg cttggcccag ctcctacaca agggcagcag tcatctccgg tccagggagc 2880
tcttctggag gtttttgcaa gtagattcta gagaactgag agaacaagaa gtcttcccca 2940
ctctgggcac atggaatctt cattcaaaga gtttggtttg aattgagagc tctcagtttt 3000

gcatatcagg taactatgat gtgaaaagat gaagcggcct ctttacctct cagagtcagc 3060
ccagaccctc ttcgcctccc ctcctctcag agaagctccc tcgtcccttt ctcctccttt 3120
tgactagagc tagtgggtgg agagagtaac tgaacgggaa tttcttagtg ttattctcta 3180
tcaataatat attttaattg tcagtgctat agactgggtg gctgggggtt gggagctggt 3240
ggctcttgaa aatcaccaag tggcagaaag gaagttatag gaagaacata aaggcttagt 3300
gccagtggtg tcaggtaatg catattgata cctcaactaa aagaacattt ggggctttat 3360
taaaattgac taattcttcc aaagatagca gctaaaggag gacttagatg aatagaggag 3420
ggaggaggct tgcaggacgg tgaagccctt tgcttccagc tctctgcctg ctcagccatt 3480
gctccgtccc tctgcagtta gattattcct tgcagatagc aatcaactgg aaggaagtgt 3540
ctgtgtctaa tttgcatgag attatttaaa acaacaacaa aaaaaaggtc tgcattagtc 3600
agggtgatgg taaacaacgc aaagaagatg ccttttataa tctagcctgt gcctcagctt 3660
tcagagccaa atataattca cttacaattg gcagtttcct ttattattcc ttttaacctt 3720
ttggattctg caaggtctca atctaagatc agcagtttgt cttggcagta gtgggaaaga 3780
tggggctagg gcaccgggat gtcctatgtt ggctccatgg cagcactttg accctgaagg 3840
cagggtcatc acagcaccaa gagccaatga ctgggcagat aactagttgt gatctggaga 3900
caggtgggtg ttcccactca gtctccctaa gttccaaatg aatcacctga gtcctgtaaa 3960
tgcagtctta aagctggcca gttctgccta aggtggggac attccagagg aagatgacac 4020
tcaaggaagc ctatgacagg catttggact tgatttcggg ttagaatatt agaacgagaa 4080
caagatgagg acattctacc tttttctctc tagtattgtt caaaaagttg tccaggcgtc 4140
atttacagat aggagccagg ggttaagttc ctgtattgtt tcaatgtagc cccaaaacaa 4200
tattcactgg ctccttcctg tagatgagag tggcggggta gggagaagaa aatgtagtcc 4260
ttctcccaag ctctttaaca agtctgaaga cctaggacat agtttgagtc tgccatttga 4320
tgttttgtaa ctctggtgta gtggagagtc actgggctga agagggagag ccttgagtgc 4380
ccatcacatc accactctct acaagtagga tgtggggcat cgtttctacc ctccttgggc 4440
ctcaggtttc ttatctgtta aatgagggca gtgactggga taatggcccg gctctgaatg 4500
tgattcagtg agattgttaa aacgcttact tttaaaaaga agagaaagga ttcatttact 4560
gtaactaaat caaaatcctg cctctcccag agaaccaggg atatcagaat ttcagccaat 4620
ataaattttg agcctcaaat gataacgtct gtgaaaaaca agagcttgga aggaagtact 4680
gatgcccttt caactgtggc ctcactagtg ggctcggctg cgataatgca aggaacatgt 4740
tttattagga aaataggctt cataaaagct cagggccaga gggcagaact gcgggcaggg 4800
cgtcctgtcc ctttaaccag ctccggccct cagggccaca gcacagtctg taacttactg 4860
tatatttccc cagtgttaaa cattattgta cttgtttata tttgaaagag ccaaaaggga 4920
aaatgagaat gcaactgaat gctccttttg ggaaaattat cttcatttgc agccagcggc 4980
tgacaaggca cacgaaccac caggtttata aaattccgta gtgtaccagt gaccatcagc 5040
ctgtgctgac atcgcagatg ctgctttctt ctctaatggc tgattatgaa ctggttaaaa 5100
caatgtcttt cccaccaggg ctccgaaagg ggttcccctt cctgtttagt cggagcgatc 5160
ggcagtcact acacaggctg gaacctgaga ggcacaaact cagggcgggg gtattctctg 5220
ttgttgcctg gcttgcctgt tggtcctgtc cctgttcttc ttggaaggtg aaattatggg 5280
taaagcagtc cctttgcagc aagcaccccc tctctagctc cactctactg accttcagct 5340
tttgtctttg gaaggtggta cgtgatgcgc tgtgaaccct ggaagcattt tcatccagcc 5400
caccaatctg ttgagagcat ggtgctatca gggcaacccg gtggctcttc agacttccca 5460
gtggtgaggc tgtgcctgcc cttggccagc tccactgtgt gaagagggta aagaggggaa 5520

cagctgcagt tttaaatggg gatagtcctc aactcctcag cacagagaag ggaaaaaaga 5580
gtccagggct gtttagaaaa gttcgggcat tttcctttga ctagctgtgg tagggcatga 5640
aaagtttgag gggtggctgc agtcaactca tatgccagta tggtgcaggg cggagctgtg 5700
ctttaaagca ttaatgtgca ccatctatta gcccctaggc atggagtatg aggcaccatc 5760
cttttgaaga tgctgatcct cagaacctcc ttgtacttca tctaccccca ggggcaaata 5820
taccatgctg tgggcctcta atggagagct gccgaccaga tgtggaaaag ctctcggctg 5880
tagtttaaaa gctctgtgtt gcggctcgct tggatgttgg gattcctgga acagcaaggg 5940
ctggccgagt ggtcttttgc agactaacag tgacctccca cctgcttttc ccactctcag 6000
cagctggagt gcctttgtac acacagctac ttcctgagct ggccccgtgt gaccgctcct 6060
ttcatacttg agcgccacat ttcattctgt tctttgggtt ggatggggct ctcttcctcc 6120
tcccaagagt cagtgtccca aattggctca gacccggtgg aaatatccca gctgtgtccc 6180
cactggaaat gcggccttca tgggttttca tttgcctttt attcttctgt gggcaaatcc 6240
tctccacatg gcatagccac tgcagatggc gccctgccac ttcctgatgt cccatgtatg 6300
gtgtggttta gagtgtcggg aggatttcaa gaagggaaaa gaaaaactag ccaaaggcga 6360
tacaggcagc gtgtgatagg ctagaataat aatcatctga aagtttgggc ctggccttgt 6420
tttgatctgt tttcttgttt tattttcttc tttacattct atatcccatt catactaatc 6480
acagtcagtc ctgatttttc tttttatttc tgcatttgtc ataaaataaa gcaaattatt 6540
cccgtcttca gaaacgatct agactaatta gtcgtctcac ccaataatta gttttttgtt 6600
tccctgtctg ttttcgtgca ttattgttag tttttccccc tctttttttt ttaacaccat 6660
tacagattaa aatgagccac atttgcagtt gatggtatct gttttcgggg gaagaatgga 6720
gaattgcagt accgagcgac ttcaaaagca gcaataaact caaggataaa ttaaggaaat 6780
tgaatgagcc acatttggaa gcagtgttga ggctaatatt ctgtcgctta aggttaaatt 6840
gcaactgaga gaggttccgg agaatctgaa atcggggagg caacttacta ggatgcgagg 6900
cattctgtgg ctgtaaaggt ctttgctcag tgaagattct gttgcagcta tggacactga 6960
caaaaggtac tcacctgcaa tgatgtcctc ttctccccag ggctgacaga tgaagagatt 7020
gatatggcct tccagcagtc gggcactgct gccgatgagc cttcgtcctt gggcccagcc 7080
acacaggtgg ttcctgtcca gccccctcac ctcatatctc agccatacag taagtcaccc 7140
gctcaaactc ctgcttaacc ttgattggga ttcaggactc tggccaaagg gcagtgttct 7200
gtatctccca gacttagcaa accaggagaa actgggagca gcagaaagct cttgggtttt 7260
tcccaaggaa tgtgacacac cccagtcttc tagacaaact ttttaaagta tgtgacaaag 7320
gtttaagaag agacaaggga tcagaataaa tgattttatt gttgtttcag gtattttggt 7380
tacatgcaga aaacctgtac aactatttat tgattctgtt cccatctcct cataccagtg 7440
cttttgaatg ttactgtttc actaaaaaca gcgtgaccat ctgactcatg ggtgcattct 7500
tgtttgctca ctgcaataac ctgtctccca cttctatttt tttaagttaa tatttgtaag 7560
tttaggtcag cctccatttc cctccctccc ttgtcctggc tatactttgt acgtttatgt 7620
agtagcggtc tgttagggaa tttcaggtta aaacatgaag ttcctttttg aatgtttctt 7680
ttaattatac gagatcatga cccatgaggg gtctttcgtt gtcttaaatg aaagctacta 7740
taggaataaa gtttcttcaa acaagttata aatgcatacc tccaaaatgc agccagaagc 7800
cctccttgaa atcacttata aaaagtttaa aaaggctaaa attgactaca aagttatgga 7860
aaatagctcc attttgtttc tttaatgaaa ctcagcattg ggccgggcac gttggctcac 7920
gcctctaatc cctgcacttt gggaggctga ggcgggtgga tcacctgagg tcaggagttc 7980
gagaccagcc cggccaacat gatgaaaccc tgtctctact aaaaatacaa aaaattagcc 8040

agatgtggtg gcaggcgcct gtaatccctg ctactcagga ggctgaggaa ggagaattgc 8100
ttgaacccgg gaggtggagg ttgcagtgag ccgagatcgt gccactgcac tccagactgg 8160
gcaacaagag cgaaactctg cctcaaaaaa acaaacaggc agggtgcagt ggctcacatc 8220
tgtaatccca acactttgag aggccaaggc aggcggatca cttgaggtca ggagttcgag 8280
accagcctgg ccaacgtggt gaaaccgtgt ctctactaaa aatacaaaaa attagccggg 8340
cgtggtggca cacgcctgta atcccagctc cttagggggc tgaggcagga gaatcactta 8400
aacccaggag gtggaagttg cagtgagccg agatcacgcc gctgcactcc agcctaggtg 8460
acagagtgag actctgtctc aaaaaaaata aaataaaata aagaaactca atactggatc 8520
tgtgatctct caaaatgcct tttctataat tgttattctc agaagtttta tttttgcaaa 8580
aaatgattta aaactgttag atatcagatt ttaaggccgg gcgcagtggc tcccgcctgt 8640
aatcccagca ctttgggagg ccgaggcggg tggatcatga ggtcaggagt tcgagaccac 8700
cctgggcaac aagagcaaaa ctccatctca aaaaaacaga caggccgggc gcggtggctc 8760
acgcctgtaa tcccagcact ttgggaggcc aaggcgggcg gatcacttga ggtcaggagt 8820
tggagaccag cctggccaat gtggcaaaac cctgtctcta ataaaaatac aaaaattagc 8880
tgggcatggt ggcgggtgct tgtaatccca gctactcagg aggctgaggc aggagaatcg 8940
cttgaaccga ggaggtggag gttgcaatga gctgagatca tgccattgca ctccagcttg 9000
ggtgacaaga gcaagactct gtctcaaaaa aaaaaaaaaa aaaaaaagtg ttagatatca 9060
gattttaaaa ggcatatttt aaaatgacag attcgttttg atcccccagc acctggtctg 9120
aagctgcctt ctttccctca cctcagcact gttttcatcc agctcagctg cagctaggca 9180
ggtgtgatct tggcagtaga gccaaaaagg ccttctcttc tccggacttg ctgaagggcc 9240
agccagtggg ttggaatctg ctgtctgtgt tacggatctt atttagagat ctcctccttt 9300
ggctgtccac agaaggctga atgggggagg ccagggagtg tgtgctcaag tgtgtcaatc 9360
tgtcagtgcc ttgcatgggt gacccactgc aggttgcaga gcttcagtgg tgagccttgt 9420
cctgtatttg ggctactggg ttagcacctg tgacttgtca gcttggggct cctttctcct 9480
tcctggccag cattttttcc aatccattgt agttgctttg ggtcctaagc tgactttatg 9540
tgcttctttt taaccaaagg ctgagtctcc acactttttc tttagttgtg tccctgggcc 9600
cagccgtgta atgggatttg ttgaggatag gcagcggcag ctggggtgca gaattttcta 9660
agatgcacat ctgttctgtt ccagctgttt cctccagtgg cttaataccg aacaggtctc 9720
tcggctactt ggaaacttct tactcagaac aaattgttct ggttgatcac agagttggcc 9780
taaaacatca tatgttcacc tactttccat agacacaaag ggttaaactg ttagtctttc 9840
tgaaagaggc aaaagaatgg ggtggggagg ggcaattcag ttatttaaat catactcatg 9900
tccagatcgc tctgagaaaa agcaatacac gttgaggtat ttaggtagga atatttttaa 9960
gagccttcca gcccctaaaa ataaaataca cattcttaga cacagacttc ttcctgcttt 10020
gacactagtg taaaaacaat ggtaatgata attttaaata cctgaagttt ttagttgtca 10080
tcattgagct tcattcttct cataccgtta cattgctcct ctcactccgg gggtgactct 10140
acctcttcac ttctgtactt ttgccaagag gtgtcaaaac tgcttaaaaa aaaaaaaagc 10200
ttgattttga ttaatataga gaaacaagac tttgcctgac tgataataaa actgataaat 10260
gacatttgaa ttacttgtag cggtaattta ttacataaaa tatcttttat ttgacatgcg 10320
attagttgct ggaagcatca ctcaatctga ctagtttaaa catgctccaa aatgaacccc 10380
agtaaaaacc agggctgtct gctgctggca ttatgaaata tactaatcct ggagtgaggg 10440
aaaaatctga ctttaaatat ttaaatgatt aattgggttt atatattttt ttacttttct 10500
aaccccctta attacaagca aactcagggg agtttctgaa ctaggtgcac aatctgcatg 10560

cttatcactg aggcattgtc tgcgtcggga gggatttatg gcctctattc atgtgtgttc 10620
ctgccgattt tacatctaga tccccggcag ccttttcctt cccatttctt ggctttttac 10680
ggacattcca tctctgagcc caaggctaga aaatgggatc ccctgggcct agccccaaac 10740
actcaggtct cagggtaggg tagcttttcc aagaccaccc cagggagccc agtgctcttc 10800
acctttgaac ctgctggccc acctgctcag tggcaggtgt gccactgagt gcctcttttg 10860
gcatctctgt tctttggtga atgtggttga ccttgagatg ttagcaaatg ccagtgttgg 10920
cagagagaac gtagagacct gcccaaatag taacagttca atttctcagt atttttatta 10980
accatctact atacggtcag tactatatca tgcgagagat ccaaaacctg atctttaagc 11040
atcttgctaa gctctttggg aaagataaga taaactctta agaaaaggat aataatataa 11100
gatgttgtga tttaatgcca aaattaatta tacagacaag aaattctgaa attctgaaat 11160
atagaaaaga gaagaattaa tgtgggctag agtcagggaa ggcttttgtg aaagtgatag 11220
gaggttggca ccaaggggaa gcttggtgtc acctggtgtg agcataaacc agtgctctcg 11280
ggggctagaa gaaagttggt acagttcagg agaagtcagg ctgtgattcg agagaagatt 11340
tgaaattctc catttctcat tttggtttct tgtctggctt tacatcggga gtcagtgcat 11400
gtgtataggt gcaggcctag cccagtcctg agcagaggtc tgataaatgc tggttgattg 11460
attcacgcca agctgatttg tcttcgtcct cccagagaga ccaagaagct ttgagatata 11520
agcaggacca gtgtaaattt tgtaggataa taatttcttc ttagatttga acattttccc 11580
tccactgtct ttgataaaca atttaaaccc cttcaaagac tgacctagag cttcagttgc 11640
ttcttctcaa aataggatgt cagcatctga ggccggtgaa aggttttgaa ccatttgatt 11700
agaaatcctg agagagagtg agttattcct ctgtttattt cttttgtagc cctgtgtagt 11760
aacacaaact tatctgtggt tatcttaatt cactgagcaa gcattgttat tgaatgccta 11820
ctgtgtgcca gacccctggg ctaaagagac taacaagacc tggtcgctct ctctggttgc 11880
tcccgtctgg taggaaagac ggttaggcca acatagcagt gcctgtcagg tggtcccggt 11940
gaggacggtg gcacagaaga ggcgacggga agcttcagag agcagatgtc taactggatg 12000
tggcgggctg cttggttcag tgatcagaca ggatagacaa gagcattcca ggctaacaca 12060
aaagcatata gatgtgaaac tgatgaattt aagaaactcc aggttgttgg acgtggcgcc 12120
tgtaatccca actctttgag aggctgagac aggaggagca ctttgaagct aggagttcaa 12180
gaccagcctg ggcaacatag tgagatgcca tctctgtaaa aaaatttaaa aaacaaaact 12240
agccaggcat ggtggtgcgt gtacttggga ggctgaggtg ggaggattgc ttgagcccag 12300
gagatcgagt ctgcagtgaa ccatggttgt acccctgcac tccagcctgg gctccagagc 12360
aaggccctgc ctctaaaaaa ccaaaaagaa agaaactctg agttgttcag agtggctgga 12420
gcatgggatg agagtcagag gaaatggctg gagaaagggt ggctctgtca gctccgacag 12480
aggacagcca aaagcacagt gatgttttac tttgaaatgc atcatctaat tatttttgtg 12540
ccatacgacc tatttggcca ttttattctt agattaatat taaaattatt tgtatcatct 12600
cagcaggtgc catgaggtta gttaggggag acatgggtgg gggggcatca gccggaggcc 12660
attgttcttg tgccgagcct gtagtgccta ccctgtgtgc tcactgttgt tctccatggt 12720
tgggccagca ctcgctcctc cagggagtct ttccagtgcc tccattctga cttggaggga 12780
agctcccctc aggcccctag actccccacc ccgccccact gcacttaaac atttattcca 12840
gtgtctcccc tattaaactg tgagttctga ggcttaaaaa aatctagtgg cctgaggctc 12900
tgactgagca ggtgttctag aaatgcttgt ggaatggatt aacaggggtg gaaatgtgct 12960
tgagaatgga agggctatca tgtggacgag ctgccctggt ggtcggtcgg tgatacggtc 13020
tgtgagccca gctggtgcag actgctctgg gcaagctatg gaagaagtag gggatgggac 13080

ccaccttcag gagctgctgt tcatcgtaga gggaagccct ccattgcttg gagaaagaca 13140
gggaagacca tcccacgtca c 13161

<210> 82
<211> 42881
<212> DNA
<213> Homo sapiens

<400> 82
tgctcagagg aggcgtctgc acccatgtac ctcactaatg acaggttatg ggatggagag 60
gagataatag gatcttttca tgtgatgtct atatagcaag gtgctatgtc cctggtagct 120
tctagggggc agcagccgct tgttgttctg tctcttgctg gcggggagaa ggttggacaa 180
ttgagcaatt tgctctggac tccagctctt aaagtggtgc ctgcaggtct ccagggagtc 240
actggaatcc gcataagtaa agaaaataca atatccagga tcttttattt ctactttctc 300
tgccccctca ggtaaatcct ttatctgtgt ttctatcaac atccacaaat tcctcattgt 360
cagagttaag gcagcaaagt tcacctgctt tgaagataag agaatagaga tgtctttaag 420
agagtaaaat ctttctgtca gatcttactt gttgccaaga gctagacatt gataacccac 480
tcaaataatg tttagaggtt ttgaaacttt cccccatggg cacgtgtctt taattctgtt 540
gggaatacca ctgtcatatg gttgattacc tgttcttcct gtcagcattt ccccccaacc 600
ccactccctc cccctatcca aactagtctt acagaggttt ctcttcaatt ctaaatgact 660
tatttcagaa acacatgaag gggaacattg cagatgtgcc atttttttgt tttttgtttt 720
tgtttttgtt ttgagacgga gtctcgctct gtcacccagg ctggagtaca gtggtgcgat 780
ctcggctgtc tgcaagctcc gcctcccgga ttcatgccat tctcctgcct cagcctcccg 840
agtagctggg actacaggcg cccgccacca tgccctgcta atttttttgt atttttagta 900
gagacggggt ttcactgtgt tagccaggat ggtctcgatc tcctgacctc ggcctcccaa 960
agtgctgggg ttacaggcgt gagctgccgc gcccggcctt ggatgtgcca gatttttaga 1020
ttgggggccc aagactgaca tatgggcaac ttgtgccttg tcagagtgag tcccagctcc 1080
ctccccccca gaagtgcacc tcctactgtg ccctctgaat gctcctgcct gccagatctt 1140
ccagctgtgt ggctgaggca ggtgcccagg aggagaaagg ctggttgcag gcagaaccag 1200
atggttgcag tccacacggg gaagcaaatt ggccgtgaca tttcagccat gtgaggttta 1260
tttaggatta gctaagaatt ctcagccttg actccactga gggaggagcg ggtgacctag 1320
ggccactttc ttgtggcttt gcattctttc aggctttcat ctgaagatcc cagggtactg 1380
gggaagggtg ggagtttcca tccagctcca ctgcagagag tggcattcct gaaagacaaa 1440
ggcatgcctc cttttttttt tttgagacgg agtctcgctc tgtcgcccag gctagagtgc 1500
agtggtgcga tctcggctca ctgcaaacac cgcctcctag gttcacgcca ttctcctgcc 1560
tcagcctccc gagtagctgg gactacaggc gcccgccgcc acgtccggct aatttttttg 1620
taattttttt ttgttgttag tagagatggg gtttcaccat gtcagccagg atggtctcaa 1680
tctcctgacc tcatgatccg cctgcctcga cctcccaaag tgttgggatt acaggcgtga 1740
gccaccgcgc ccggcaaagg catgcctctt aacctcacct ctccagctct ctgttgtctg 1800
aaaaatggca gttaatagaa tttgttaata aacagaagaa ataacgagaa atagtggcac 1860
atcatttgac agtaaggtgg tttataattt ctgtttatca aatgattctc attattatta 1920
tttctattgg atttttgaag aattcagcct ggtccccaga gccccatttg taaggcattt 1980

tatttcttta taatatctag aaataacttc agttccagtg aaatccagat ctgtggtccc 2040
atcccactcc ccagccctgg ttctctcccc ttcctctctg cttgtcttct gtgacctcag 2100
cgggctccat caggtggggg tatcccctac tgaccagact ggcagagcta atgccggctg 2160
gaggaacgct gccgtgtttg tttttgcagt tcctggattt ggtttaatgt acaattgatt 2220
ttttaaatgt atttaatcat ttttttctta cctcttaagg attaacctgc acaaacctca 2280
ttttatccta agtgtggctt tgggaggaag ggaggagaaa attgagtttc tccagcttat 2340
tttattcttt tacgctttat tttccaaatg gcttttcctg ccgaagcagc tttgtataat 2400
acaatgtatt ttcgtggcca ttacttactc attactaact ggtctctcct gcttcccttt 2460
gtctgtgaac taaatacagg gggcctcaag ataattggta gctccatcac tgacccacca 2520
attatagatc agcagggaag gcagcccagg gatggtttgt ggttggcaca ggctgcccac 2580
ctagaaatgt gagttattct ctacctgcca ccaagttggg cttgactaga tcgatccttt 2640
cctaattttg gactttggca tctctaattg catcacacgg ctccctggtt tacaggtttc 2700
agcctcattt catccctaca cgtgctgctg gtgtctttct tagttcatgc ctccttggca 2760
tgtctacaaa gtattaattt gcagaggttt tgtttgtatt ttcctttgag gttccctggt 2820
gcctagaatg gttagatggc aggctgcaga taaataattc tgttgaaaca gcaggtttct 2880
gtgcattttt gtctggtgtg tccaagtaaa ggcaagtctt tgtgggcacc atattaagcc 2940
ccctttttga gcatttatct gatttgtgtt ccagcttatc tactttgctc tggcatggag 3000
aaggaaaagt cccatttcct gctaggctgc caggcactta tgtaagactt ttaggtgcca 3060
aagtaggaaa ggagtccctg tgttgtatgg ccgtatatat tttgcatgtg tgtttcctgg 3120
ctgtttggca cctaggcact ttgctaagcc ctgtatgggt tgggcatata ccatattgct 3180
cagctcctgg cagaaaatag gcactcatac atatttgtgg aatgagtgag tgtgtgtgct 3240
cacttatgtg cgatggagat gatgagccaa ggagttggat tgcacagttg ctgcctggcg 3300
tcctgctagg aacttagggt gcaacattcc cactgggcca cacatatgcc gatgttcagt 3360
ggatgactgc tacgcaagca acataaagag gccattttat gaaagggaac atcagaggaa 3420
acattataac tgagaccttt atagcctttc cattattatg gtttaaatct tgacagtctt 3480
tatttgcaat gacactggga ccagtaaaga caatgctcac tcccacctcc tcgttctcca 3540
catcctgttc cgtagctgtg gtgcaccgat aggcatcgtt aaatgtgcag gcaggctggt 3600
ttgtctagaa acagcccctt ttgactcaga agttggaacc cagatgactc accattctat 3660
ttgttttatt gcaactgtca tttcataaaa tgccctgaca gctttaaact gtaaatgcca 3720
aagtgaggtt tttgtctttt ttattttgtt atctcctgtt ttttctaatc ttttatattt 3780
ctattttctc ctttcttttc agacttattt tctctttcat tactgtctct ttctctgtcc 3840
ctcccttcct tcctgcctcc cccctttgat gactctaatg gcatgagtag ttgaagtggt 3900
ttttgctaac atcatggtgc gttctttcca gctttaatgc tatctgattg agcgtgtctc 3960
ccctcagatt tttgtgtagg gtttagaaat ggaaatgacc atgcacatca gagtttaaat 4020
tttcctagga aagtctgcca tgaactgtag gacctataac ttctagagat ggagggaccc 4080
ctgacatctg tggactatgg actcctgact tctgtggact ctagaattta tgacttctgt 4140
ggattgtggg gcctctgact tccatagacc gtgggactgt gacttctgtg gacccaggga 4200
cctctgaatt ccatggactg tgagacctct gacttctgtg gcctgtgaga tctctgacct 4260
ctgtggcccg tgggacctct gacttctgtg gtctctggga cctgtgacct ctttgacttc 4320
caagtcttct gtggactgca ggacctcatc aacacgtgta ttgccagaag agaggcttga 4380
ttgtgttaac ctgtttctct ccagaggtgt cacatactag aatttctgct cagtcttatg 4440
gcttaggtgg ggtgttgagt ctaaatatat atattttttg agacggagtc ttgctctgtc 4500

acccaggctg gagtgcagtg gcataatctt ggctcactgc acgctccgcc tcccgggttt 4560
acgccattct cctgcctcag cttcccgaat aactgggact acaggcgcct gcccccacgc 4620
tcggctaatt ttggtatttt tagtagagac agggtttcac catgttagcc aggatggtct 4680
cgacctcctg acctcgtgat ctgccctcct cggcctccca aagtgctgag attacagacg 4740
tgagccaccg tgcctggccc aagtctaaat ttttaaggac tagagctctc agaccaggac 4800
tttctggatc tttttttctg atacccaaca ctgtggcact tgactaagtc acctgattga 4860
tgacatgagc attgctgggt gttgaaacac ctggccaggg aaacagagac ttctgtggat 4920
tcatccaaag gatggcagac agattgtgga attttaatga aggtggggtg gatccctgat 4980
gttcgcctgc tttgaagcat agcagaatcc cagcattaac ttcaggggtg ttggttggaa 5040
acctgcactg gcgtcggtgg ttatattcca catttgtcaa atccttaaat ccaactgaac 5100
taattttaga agtagctagt tataatgaaa aagaacaata tattcagatg cataggatcg 5160
tttttaaatg aaatgctccc tgttcttttc cttctttttt tttttaactt ttatttttta 5220
tgatggagtc ttgctctgtc gccaggctgg agtgcagtgg cgtgatgtcg gctcactgca 5280
acctccgcct cacgggttca agcgattccc tgcctcagcc tcctgagtag ctgggactac 5340
aggcgtgtgc caccacgccc ggctaatttt ttgtattttt agtagagatg aggtttcatc 5400
gtgttagctg ggatggtctg gatctcgtga cctcgtgatc cacccacctt ggtctcccag 5460
aatgctggga ttacaggcgt aggccaccgt gcccggccct tttccttctt ttataaaagg 5520
cctttggtct cttttggcct tcagggtgag ccatcctttg acttatatta gaatacattt 5580
tttttcctgt atgcctccaa tgaaggagag tgtgagagtg ttttctatga tgtgagtggg 5640
agaaaatagc gtgttttcac aatagttaac aaattgatgg ctgtgagttt tgaaagtgag 5700
ttctgtatat acactggggt aggattccac atagaatgct gtttttcacc cacctggctg 5760
gttgtctgat tctaacatag ctgctatccc tgattctaga aatcagaaaa ccacaggctg 5820
ttcacataat actctaccct tctgaggcta aggtgggaac aggatggtgg cggggtttga 5880
gtcaacatgg tctcagttcc caccaaagac cagtgtgttc ccctgttcca gccgacagct 5940
tgaacaagga ttctaaagtt tctggccgtt ttaattgaac tgcagtgagg ggtggcccga 6000
tagcctgctt aggacatggg taattactcc tagggcctgg gagccaagtg tccctcgcag 6060
gttccctctt ggttctctac ctaaggatat cttccccttc cctgggcagg gcctctgagg 6120
aacagaagga tcatttagtg ctcatggcct gctcattcct tggcttctct gctgagctat 6180
ccagcagggg tgccaattct tccatttagc tgactattat atagccctta aaaggaaggg 6240
gtttatttta gctggctgct atattgattg ctatagggac gtttctattt tcactggctg 6300
ctatattgac tgggaaaggg aaaggggccg tattgccggg agctgtcata ttgactgtta 6360
cagaaaagat ctgtatttaa gcagtaccag tgcactggga tctcccaggc aggatattaa 6420
tgctaattca tccgctcacc ccctgctgtg agtgttattt gtttaaatct gtagattccc 6480
acagcgcgtt tatatcctca ctagagtgag ctccgctccc tgtaattatt taacattata 6540
tgctgatata aattgtaaca gttaaagaat ggtaatgagt agccaaaaaa gaaaaaaaga 6600
agaaagaaaa gaaggaaata gaaaaaaagg aaaaaatgta taaaataatc tatgcctctg 6660
tgtgtgtgtg tgtgtgtgtg tgtgtgtgtg tgtgtgtgta tggtgttaga aacggcagag 6720
tcaccttcta aaacagcagt gagaaacttc tgtcatcctt tctcccagtt caggttgatt 6780
tgtacggtgc tgctcagccc acgtattgtg catctgtatg tgtgtatgtg cgagcgcctg 6840
tgtacgcacg tggtcgtcac ccgccagaaa gatgccatag tgaggtgtgc tatactgtgt 6900
tgtttgtgca cgtgtgtaac aatatacagt gtttgttcaa tgtcctggca ggaaattggt 6960
ctattagcga ttcaccttct tggtgagtag ctaatacctt attttgttca aggttccacg 7020

ttcacacagg aactttgggc ctaagacgca actcaaggga aagccattgg ggaatgctca 7080
gttgggattg aactaatagg ccattgggtg tcactgtcgc tctactggct ataggagaaa 7140
gaggggttat ctgctgggga aactggattt caaaagaaag aagtttttct gcttgagttg 7200
gaggtgttgt ctctttgaga tgtttgtctc tcatttggag attcctttaa aaaagtagtt 7260
gtttgcttgt ttttgctaca ccaagcttct ggcattttta ttgttcttga aaattgtgta 7320
ctgattgcga tctgttctgc tatcacctgc agtgcatcta gatgagaact tctgtgtgca 7380
gaaaactgtc ccccttaaaa gcctgggtaa caaggagagc tgatttgggc aagaacttga 7440
aaaaggaggg agaaaatctc cttgggcaaa tgcttgccac tcccctgccg cctgcccctg 7500
tgtaggtggg aagagagcgc agagggagag ccaggctgct gcctggtcac agaagagtgg 7560
gctccgtcct ccagcgggct ctccagcagc atggatctgt gccaggcttc accacaattc 7620
atactgtgtc agtttaataa atgcactact taaattatga aattacaaaa gaaaaaagag 7680
aatgcactta ttcagactct taatacatta aaaataaggc attaaaactc tgactttttt 7740
ttttttcttc cagaatcaat aatgaacagg aagggttgta gacattaaca gtcatggaca 7800
ttcaaactct tatacataaa ttatcttaga aaaatgcaca taagacttga ataatgtaaa 7860
tacattaatg catttaataa tttaaacatt aaagcgcaag caggaatttg aggccgaggc 7920
tgttctgagg taagggattg gtcctagaac tcgtgtgtcc caggatcgat gggtgatttc 7980
cccttgagac accctggaaa gagagtgcct ggccggcttg tgtggccgag gatccctgga 8040
cattcctgat ctcagctgcc tcccagcctc ttatttttgt gtatcctgag cttcgttctc 8100
agtttcatgt tctctactcc tgtgcttcta attgtttgtt agaaaactaa atgtttccac 8160
ttcatagaac aataaacttt gagaatgtct tggttgaaaa accaaaccaa agtaaaaaat 8220
ccttcacccg gctctggctg cttgtctgct tgtgatttga gaaaaaggaa gtggctgctc 8280
cttgtgatca gcctgtcatg tctcagcctg gaactccact gtgtgctgtg gtcaggggat 8340
ccaggttgtg acatgctgac agatttgaag actggatcta atgggagagc agaaacgata 8400
tcctatatta gcctctgtgt gttgggcata acgatggcag gtagccatgc caggaaacaa 8460
gacaggtgcc atggagttag actcagtggt ctgtgatcat accttacttt gatttgaggg 8520
tttaaccatt gggtagactg atgctactgt tttttcccct ttcttccaaa attgtatttg 8580
caatagcaga aagtcactgg aaactccttt tctggcagaa taccgaatga tctaattcaa 8640
tttttgacat ctgcactgtg aaataccctg ggcagaacta agaagccagg cctatgtgtc 8700
ttaagttttc atttattaat gctaatgctg ttctctggtt gttccctagc cctgttttat 8760
agtccaaatc acagatgaga aactagaaga actgtagaaa ctgtgttact gagtacatag 8820
tctgagaact ttgtctctct ttgtagaaga gtgtgtctat ttggtcagta atgcaaatgt 8880
tgtcccactg ggtttgctgt gggacccact aggtttgccg tgggaccttg agaaacggtt 8940
atcagatggc ggtgactgaa ccaaacctgt tttcattatt gccaggtccg tgggagtctt 9000
ttacaggatc tcaaatctcc tgcctgaaac tttacctggg attgtcttga tttgacccaa 9060
gcctcgtctg tgctggcatc tgtgactttg tttgcaaccc tttctagtct ttgttgtggc 9120
caaggacagt ctggtctccc agctaacatg ggtacaaacc agcttctagc tacttgcagg 9180
agaatagcta tgggttgact tggcatctga agcagataca aacagattag gctgagctgc 9240
cccagaacag ccagaaggaa aaccagtact ttaaaaactg gtcgtcaggt attggtttag 9300
aaagaaagta gtttggagaa tgggtgagga cagcctggtg agtgatcccg aggtcacggg 9360
ctgtccttgg gatgtgctca gattcttctc tagacctgga gagcaacagc cgtgcctcac 9420
aagcccttct ccacatgttt tgttttaaga tggcagagcc tttctttgca gttttagttc 9480
agccttggga tgacggtcag acctcggttt gttcttttct tttccagttt tcagagccca 9540

agcctgctcc ctctgttgct tgtccagcct tgaccagtga ctgtcttcag cctgcactct 9600
gcatttcacc ccagcagtag gaagaagtga ggtcagagga gcacgtgaac cagggagaat 9660
cgaattctct ttggattttg ctggtcacac ccagcaaagc tgctttctct tcccttaagt 9720
tccaagaact gtttgatggc cttaactgca caattggctt gtgccagatt gagatgtgtt 9780
gaactgggca gtggaggtgg gggtgagggg caactccctc tcctattggg ggcagagtgc 9840
tggctgcatc aggcagtgat cagataggca ctgatcgggt cggttcaccc tctgatatag 9900
cccgtatgca gacttccttt gaagagagag gagtcaggct cattaattaa aggagcctgg 9960
cttgagaggc ctgtaattat aggtgctaga aatctgaatt ttaagaggtt gtgcagctgc 10020
attttgaaaa atgtagggag gcaccgagaa gtactggttg tgggcaagaa tgtggctgaa 10080
ccagattttg gaggttcagg ttttggcaga attcccctcc aaattcatat ttattctttc 10140
tttccatttt gttctttttt ttttttctaa atgggaagca gaacctgtga tttttttttt 10200
ttttaaatga aggttacacc ctgtgaccga gaggctagca ttgtcagagt atatgggggg 10260
cagaaaggag ttgctgtgaa ctagaagatg cttagttttt gaggaaacgg cacatctttg 10320
ggccttaggg attcagtatt tgaaaaaaat taaataattt ctttcttgaa atgagtgagg 10380
ggagcaggga gcagaagccc aatttgaatg cagtctaggg aagtaagtcc caggcaaatg 10440
gtggactcta gaacctttgt cccaacttga cttccccaga agtgtctctt cctctgtcat 10500
gcgttacttt tccatgtgaa ccctgagtta caaacagaaa atttcagacc tttatgggaa 10560
gtcctgacct gtgccttttc tctctcattt cctcagttat ttgtaggttg accttttgtt 10620
ttgggtgctt tttcgctctc ctctccttaa aaaaaaagta tccctctttg cttcttgtca 10680
aaaaccttta tttcggtccc tgactatttg catctctgtt aattaagcat ggctaatttg 10740
atatgtagat gggatgggga gggttaaggg ttagatgaat agaggtctac tccctgcctc 10800
ccccgggtgg tgggcagggg tgctccacag acctgcaaga ctgaacagct acacacggct 10860
gatggcaaac ccgatgccag ggggaacctc ctctctgcct cagtggtttt gtctccttgg 10920
ggatattgag agcgtcatcc cgtttcacag actggaccct ctgtgaaggg ctgaaaaaat 10980
aaaatataaa ggccagttct ctgtgctctt ttcttacatt cttgtagtgt atatattggg 11040
aaatccacgg gtgaaccctt acactgtaga atagaacaca agtgtccaag actctcactt 11100
ggtgcattta caatatgcaa attccaattc caattagatt gaaatctcta tttagcagag 11160
tggttttgtg aaataaataa cttttgaaga aggaggcctg ctatatgtaa taaatatata 11220
tttttccttc agttgcctgc atggtgcttc ctgcgttccc agcccttgcc cgaaatgctg 11280
attccgaagc atctttgggc tctttacaga agagatgctt ctccttccct tcctggcaca 11340
aggatttcat ccttgattta cagtgctcag cttgttagtg ccggtgaagg ggagagatgg 11400
tgtgaaggtc gttatgtccg ttttgtcctc ttggagagaa agtgagggag tgtgtttctt 11460
gccgtgtgct tgtcgtggta gtggtggagt tggagaggcc cccacttctg tcaaaaggag 11520
tcgagccaaa ctctcatcct ctcttcttca ccctgtatac tacacgttgt ccaacagggg 11580
gtgaatgatt tcaaaagcgg aattcagaat ttcaggaaga gtagcacacc accgagtaga 11640
gggcctcccc ctaacacccc ccttttcatc attctcccct cctcagccca gccgtgacaa 11700
actgacagat aattaacaga tcagaccctc ctattaggcg tgccctgtgc tggaagctgc 11760
tctggcttta tgcagtcctt tagcagctct gtgtgtgtgg gtggtgcctc actaagtggc 11820
catagcagag ggcagggagt gctttgggaa aagcaaacag aatgctcttt ggaagagaga 11880
gtgcttaaga gggagagcgg gggcagtaag gtgcctggct tgaaagcacc tggcgaaaag 11940
actcggactt tcagggttgt gatccggaat accaccgcct gtctcagtga atggtgcgct 12000
gggttataat aggatgcctt attacaaaaa acattttgaa ggccacttcc caggtcaggc 12060

tatgcattag gtgcactctt ccttcggtgt aacactgtgc tggacttgca aaattggaag 12120
tgtgcagcct gttgtgtttc agccttggca ttaaatattt ctacagagac actgccctat 12180
gtggctttct ctcagctacc taacttttct ttccctggtg gaaagaagaa ttcattgtga 12240
gaatattcat ttctacttac tagtgacttt taaaaactac ttttctttgt ttttggacac 12300
agggacttgc tgtgctgctc aggctggagt gcagtgtgca aacacggctt actgcagcct 12360
tcaccttccg gccgaagtga tcctcccgcc tcgacctcca tgtagctggg accatagatg 12420
tgtgccacca cacccagctc atttgaaatt tttgttgttg ttgttgaaca gaagtggtga 12480
gagtaaaata aaaataaatt aaataaataa tatgttttgg tagtgacagg gtctcacttt 12540
gttgcccagg ctgggtctca aattcctggg ctcaggcgat cctctcactt cagtctccca 12600
cagtgctggg attacaggca tgagctgttg tgcctggcct tcttcttctt tttttttttt 12660
tttttttttt ttttttttgt tttttaaact agagacaagg tctcactctg tcaccccagg 12720
ctggagtgca gtgctgcggt cataggtcac tgcagcctca accttctgag ctcaagcgat 12780
cctcctgccc cagcctccca agtagctagg accacaggca tatgccaaca tgcccagcta 12840
atttttaaat tttttgaaga gacaggattt cactgtgttg tctaggctgg tctcaaactc 12900
ttggcctcaa gtgatcctcc caccttggcc tcccagagtg ctggattata gtcatgaacc 12960
accatggccg gccctgaaaa ctacttttct acttccctta tggtggccac ccttctcatc 13020
ttcatgattt gaatattaac ataaagataa atttagcaac atgtaggggc tacacgtagg 13080
ctcaggtcct taggcctggc atgttggagc agaaagaaac acctaattgc atcaaaacca 13140
ctctaagttc atgtatgtgg agcccatcaa gctgaaagca tccagacagc atctgctgtt 13200
atcaaatcac tcgtgaatcc agctaattaa aatagagctt catttttctg aaccattgtt 13260
tggcaggaat tcgaaacggg tctaacccaa tttctgcatg tttatgagcc tttattatat 13320
cagaggtact tttctgtaaa tgtacagggt agcgttaaat gaattgatcc acacagtatc 13380
actgtagacg cactatggac ttgctaatga tttctcttcc agctttctgg tttccagaca 13440
tactcttgaa agctttatct tctctttttc tatcctctgc tctttgccgt tgtctttttt 13500
tttttttttt ttaacatttt gaactttcct tttacttttt aatcttcttt tttctttgga 13560
aacaggaaaa ctatcccctt aaagaagttt tcttttccat ttctcttttt cttcctctct 13620
tcctgttttt gtttttcttc tagtggataa gatttgtctt ttcttccttt atttcttgct 13680
gttttctatt ctctcctttt cttttctttt tctctcttat cttttctccc ttttatactt 13740
tctcccattc cttttctcta accttctctt tctccttttt ctcttctttc tatctcctcc 13800
cattttccct aataaaatat ccttatacct ttgtctccac atgcaaatac tgactaggga 13860
agatgggggt gcccgtggag gcagagctat ctggctgctc taagatgatg tcagctgagt 13920
ttacttctct gaatttgctt tcatgcttta ttttaggttt taaaaagatt tttcaagcca 13980
catctactct actttatagt ctgtaatcct actttgaaga tgaaaagtgt tacataaatg 14040
ctaactaatt attccttata ccactttgtg agtctctaag cagtattagt tccagtttac 14100
agatgaggaa gcaggaacag gaaaggtaag tagctctgaa agttactgaa gaagttggtg 14160
actgagttag aattaaacct ggctcatctt tggctttgct ttttgattac caccttatgg 14220
gtgttgactt ttttcccccc taagaatcat acatatttaa ggtgaggttg agtgaatacc 14280
atccccttgg ccccttctca ttatatactt ttaaccttct gaagtttcat ttgtagtctt 14340
tagatgttaa atgaatttct aggtaattcc tgggaaagaa ggatgctgaa ccacttgtgg 14400
gctggtaaaa accctgtcta ttagtcttaa agcagagtgc tcactttctc tttccaacag 14460
cttccagtaa gtgggcccac gtcacacact ggccccagtg atcgtagcgt tgtaggaagc 14520
gctgccatat ttgattgaat ttagccattc tggaatgacg tgttcatgaa gtcggctctt 14580

gcattaccgt ggcctttatt attgctaatg cattctgaca ttacaggtgt agccttggct 14640
ctcccagaaa gggcaaggct gctcttacca aacgaaagag cagaatttat gaaactgtta 14700
cacagtgagg ccagtgttca cctttagtta ctaagcagag taagggaaat aaatgctttt 14760
atagaaaaga tagccagtga cagcagtcta tcctgattcc tcacccagca ctgtttcaaa 14820
aagtggatca attaatattg ttgccagaca gagaacgagg accaatggtt aggaggttca 14880
ggaaagtaga tttcaactca gtttaaagag gcactcctga aaatgagctg acagtctctt 14940
aagggagtag tttccctgct cattatgagc attcaagagg aggcttcctg atcatttgtc 15000
agggttgttg gagaggggag tccctaggtc atgggtggga ccagatggct caagatctct 15060
tttggcttgt ttcctcatct tagattgcta cagcacaaac agcatctttc ctgcttctat 15120
tttgacttta caagtggctg gtgtaccgtg aggacctgtg aatgctcagt agctattcat 15180
tatttaatct ctggcttcct gcttcttaat ctaacatgaa tcaaagctgt tcttgttcaa 15240
ttcaggaaaa ccaccatctc tggcacctcc tagaatgagg cattctggat agttacgttt 15300
tctggatggt catcaggagc catttgaaag cagaacaaac aaaacacttt aaaacttttg 15360
gctgggaatt atgtaaaatg gattacatac tttcttgctt tattaagctg aatatctgga 15420
atataattta atcgtgatga ctcagtattg tcattttaga tgaacgtgtt tatatggtgt 15480
tataagcata gctgtgtttt caaaacctgt tgaaatatag atgtgttgta tatttgcccc 15540
tacttgaaat gactccaccg attctaaatg accccagact aataagcttt ttgaattttt 15600
ttgtttgctt ttcatggatg tttctctctt ggcatttgct gttgggcttt tttttgctgt 15660
cacatctttg tttggtgttt ctgtactagc tggctccttc ctctctgttc ttatttgcag 15720
cttttatctt ctatgatttg gataccagat aactaagctt gtatataaaa taaatagtaa 15780
gtagcgtgag taaagcttcg cagcgtataa aatatatgga atttgtaata tcttttttgg 15840
ctgccctgat ttcttctgct attccctgtc tcctccttct ctgttggttt tctccttttt 15900
ttgaattggg tgtcagcttg gagagatgaa atctggaaag actgggtctt gtccttgacc 15960
taagcccata tctctggccc aggtatcagc ggctgcctca tggaatcaac ttagaaatta 16020
taaaatggca caggtagggg gtcctgaagg tcatctgttt aaccccttat tttatagcag 16080
tgaaggaatc tgaggcactg agtggggaca taagaggcca ggttcatgac agtatttgtg 16140
taaggtaaaa aaaaaaaaaa atcttgtagg aaaattctct gctggcttat cagcaagttc 16200
agaattaaag tacataaacc aaaaaaaggc ttctgtttgt ggcagcgttt ggctgtgttt 16260
gcactcaaga ctattatcga tattctagga gaagctttaa tcctttcatt caaacttaca 16320
agaaatgctg tttcctttta gttaaaaaaa aaaaaaaaaa aaatccaaga gagcatttga 16380
tttgaggaac caaaagtcta gaaaatgtat ccgggagtcc tcgtcaattt ggggcataat 16440
gaaatataaa aaatactcaa taaaagctcc tttattactt taactgttta aatgtggagt 16500
ttttagccat caggttaatg ttttggtatc atttttttat ttgattgtga gcacttttcc 16560
aggtagctct gccactcgag cgctaatcag attcccacag ataaatggtg ctagctgcag 16620
cctgacatca ctgcgttttt gcttcaacat tattttattt tatttggttt tcctgtaaaa 16680
gttaataact ccatgtccat gtgtttaaac aggcaagaag agagagagag aaaggacagt 16740
gagcagaaaa ggatgaggga tgtttcagga agcttgtgca aaattgagga aatttaggga 16800
tttttgtaat ttccaggtat cttaccttat gaacagtatg tgaagaacca agaaggttac 16860
aaagcaagat ggtaattcat aatccttcag ataacaaaaa gtgaacttct ctgtttctca 16920
gacagagctg ggaagaattt gaaaggagtt accattgatt taaaggagga agatgctcaa 16980
taaactgatt tgcttaatgg tgagaattgt gtaaattttg tgttattcat aagtaaatta 17040
catgctcatc tggcttctcc tagatgacca agatggggaa gggcatcaga tatagggcat 17100

aaagaccctc cttttcatct tcccattatt agtaggacac agtgactctt ggccaagatg 17160
gctctggtgc tcagtaggcc tttgtggaat catttgtttg attgattgat tgattgattg 17220
atttttagag acggggtctc actctgtcac ccaggctaga gtgaagcagt acaatcatag 17280
ctcagtgtaa cctcaaactc ctgagctcaa gccatcctcc cacctagctc cccaagtagc 17340
tggagcgcca ctacgccaag ctatttaaaa aaaacaaatt tttttttgaa acaatggagt 17400
ctcgttatgt tgcccaggct ggtcccgaac tcttggcctc cagccatcct cctgcctcag 17460
cctcccaaag tgctgaatta taggcgtggg ccaccatgcc cggctggatc atttattttt 17520
taaagtgtgt gtttgtttat tggtagctgt gctggctaat aatagggatg tttatggtat 17580
ttagtgaaag gttgctgaat ggttcaaagc catgaggact acctttgggt tcttcttgtg 17640
tgtgatggat ttcctcactg agctgaaaat ttcttaattg gggtttatca gagaaggcaa 17700
ctggactgat gtctcttgat tcagtttctc ttccaaactt gtaccttgat actaaagaca 17760
gtccgtgggt acaatctgat gatggctgtg ataatcagtt tcacagccct tatgtcatta 17820
cttctgaaag atgtgtaaaa aaaaaaaaag acattttcct catctgcgtc agcggtatag 17880
cttgtattgc tttcctgtgt ttcacacttt gtgttgatat gttacttgtg aagtcgtcta 17940
caagggtgtg acctgtatta gataccgctt ttttagaaaa tggaacctga ccaccaactt 18000
gtccctgtgg agagcccatt tctggccact ctctggggct ggtgtgcttg gtgaggaagt 18060
tgactttcct ctgacagtag ctgattgtat atgactgttt gaatagtaag tttccaatcc 18120
ttgtcattct actgtcaatt tgaagatttt tttttcttca aaatggagtg tcactctgtc 18180
acccaggctg gagtgcaatg gcgtggtctc agctcactgc aacctccgcc tcctgggttc 18240
aagcgattct ctcatttcag cttcccgagt agctgggact gcaggcacat gccaccacac 18300
ccggctaatt tttgtatttt tagtatagat ggggtttcac taaattggcc aggctgatct 18360
cgaactcttg accttgtgat ccgcccgcct tggcccccta aagtgttgag gttgcaggcg 18420
tgagccactg cgcctggctg attttttttt tttttgagac agagtctcat tgtgtcttcc 18480
aggctggagt gcagtggtgt gatcttggct cactgcaacc tctgtctccc aggttcaagc 18540
gattctccta cctcgacctc cctagtagct gggactatag gtgcgtgctg ccacacccgg 18600
ctaatttttg tatttttagt agagacgggt ttcaccgtgt tagccaggat ggtctcgatc 18660
tcctgacctc gtgatccgcc cgcctcagcc tcccaaagtg ctgggattac aggcgtgagc 18720
cactgcgccc ggctgatttt tttttttttt tttttgagac agagtcgtgc tctgttgctc 18780
aggctggagt gcagtggcgt gatagctcgc tgcagcctcc acctcctggg ttcaagtgat 18840
tctcctgcct tagcttccca agtaactggg attacaagtg tgaaccacca cgcccagcta 18900
agttttgtat ttttattaca gacggtgttt caccatgttg gtcaggctgg tctcaaactc 18960
ctgatctcag gtcatctgcc caccttggcc tcccaaagtg caatttgaag atttttaagt 19020
ctgccagttg actgcattat gaattacact tttggaaccc atactttagt ttttttgttt 19080
aggccataaa agaatcaaag gtaatacagg ttgagtattc ttgatcccaa atgcttggga 19140
ccaaaagtgt tggattttgg attttttcag attttggaat atgtacataa tacttactgg 19200
ttgagcatct caaatctgaa aatcccaaat ccgaaatgct ccagtgagca tttcctttga 19260
gcatgacctt tgactgtcat gttggcactc aaaaagttct ggattttgga gcattttgga 19320
ttttggattt tcagattagg gatgctcatc ctgtagcagg atctaggact tggtggcatc 19380
ccgttagtag cagatcttga ttgttgactt tcttctgttt tctggtgggt ctaaagaaaa 19440
ttcttaggct ggacacggtg gcccacacct gtaaatccca gcactttcgg aggctgaggt 19500
gggcagatca cttgaggtta ggagttcgag acccgcctgg ccaacatggt gaaaccttgt 19560
ctctactaaa aatagaaaaa ttagccaagc gtggtggcat gtgcctgtag tcccagctac 19620

tcaggaggct gagacaggag aatcgcttga acccaggagg cagaggttgc agtgccaaga 19680
ttgtaccact ttactccagc ctgggtgata gagtgagact ctgtctcaaa aaaaaaaaaa 19740
aaaagaaaat ttgtgggcca ggcatggtgg ctcacgcctg taatcccaca ctttgggagg 19800
ccgaaactgg cagatcacga gatcaggaga ttgagaccat ccttgccaac atggtgaaac 19860
cctgtctcta ctaaaaatac aaaaattagc tgggtgtggt ggcatgtgcc tatagtccca 19920
gctactcggg aggctgaggc acaagaattg cttgaacccg ggaggtggag tttgcagtga 19980
gccgagatca cgccactgca ctccagcctg gtgacagagc gagactctgt ctcaaaaaaa 20040
aaaaaaaaag aaaaaatttg tttctaaatt tgaactcatt ctaggctatg agtagttctt 20100
tttttttgta ttcaaattta ttttttagca ttaggtcatt ctctaatttt cttatggggt 20160
gtttggtaga attgcctttt atttatttat ttttttgttt gtttgtttcc aagacagagt 20220
ctcactctgt cacccaggct ggagtgcagt ggtgcaatct tggttcattg caacctctgc 20280
ctcccaggtt caagtaattc tcgtgcctca gccaccctag tagctaagac tatgcaccac 20340
cacacctggc taatttttgt atttttttta gtggagatgg ggtttcgcca tgttggccag 20400
gctcttctca aactcctggc ctcaactggt ccactcgcct cggcctccca aagtgttggg 20460
attacaagcg tgagccactg tgcttggcct agaagtgcct tttaaagact ccttgaaggt 20520
tttagttttg tgcagagtgt gggttgatgg tacacataat tttttggtcc agagcttatc 20580
ttccttggaa ttcaggagcc catcttcttg cccttagggc aggtggccct aagcccttga 20640
gggggatcct gccttgcacc agctggtggt cacctattca gtagctgacc cccagtgatg 20700
ccttaatgtt tgaggttgcc attatccatg acacaacctt tcttcgtctc cttcctaatc 20760
atcccgcagt tcaatagtgc atgactagag agcttcacca atctccatct ggccgaactt 20820
caaaagaagt ggaagctgaa gcagggtttc atctgctgag catctgaagc tgctggaggt 20880
taggagaaat acctttcaga aagcatgaaa ttcaaccagt ggtggagaga aagagactag 20940
gaaatagagg cagtgttatc attagggagg tgctggggac tgtgtgccac atatttttag 21000
tgttggagtc acccgccacc gtcactgtgt gagctcacag ttctctcagg gagcatcttg 21060
gacactgagt cattacattc ctttagagtg atttaactca acagcttgac accttcatta 21120
attttacaac acgaagcctg ggtgctctct gcttcctctt taacagtgca gggaaattag 21180
aggaatattg aaaatataaa accaggggat actggaagaa ggagcagtca aggagctgtt 21240
gaaaaacatc atgttaagga attgaactac ttgtctttcc ttggtatctc ttgggatcga 21300
gcacccagct cttgccactc gtctctggca cataaacctt ttatttccgt tcctggcacg 21360
ctggccaaac ctccaacttg cagccagctc gtttgccttc tctgctgagg tcactgctac 21420
cgtgatccgt gtataatggc attttcctcc tcttttcagt atgaaggcta ttacagtttc 21480
ttctctaaac aggacttcaa acaatttgct gccagttgca tttcaaaggc agggaaaagg 21540
caaatgtcat ccttttttcc tcccctcttt gggtaactgc tttgttggtt gcatcaaaaa 21600
ttagaggggc tgctatctaa attcaggtgg ggatgcgtgg ggacaaggca tttaaatgtg 21660
cttttctttc tccctgaagg agaaaaacgc ttttccttct caacctttcc taaaaaggaa 21720
ctttgatctt cccaacccac aagcaagggt ttcatttctt ccaggccggc gtgtactcca 21780
cagctccttc tgtggcgtgg tgaccgtcac ccgctgcatc agcagctctg cttttcaagc 21840
tgtgctcctc cactgcacca gcgccctttg ggctggaagt tggcacccag agtcctcggc 21900
tggacacgga cacctctcca aagtcaccct tacgtcccta agatcactgt cccttgacat 21960
taaatagtgc gtggtggttt aagatgcttt ttatttcaat atacatttct ggaagaacta 22020
ctttaatcaa ataacaaaac aatgacattc tggcaagcag ttcatgcaat atttttaact 22080
aaggccaagg gaaaaaaaat aaatggcaga gatatttcac tttgaaaggt agtttttgtg 22140

catgtgaagt tgccactttt cataaaggct tttactacat atatcaatga gttttaagag 22200
agactagggt caataatgca tgctttagaa ttccctaaat acagatttac acaatagcct 22260
taaaatatat tctgttctat catgtcaact cctgtgcact tctaccagcg gaattttatt 22320
tctttaatgt gaaaaacaaa aggacagagg aaagtaaagt aaaacaaagg aaattagggc 22380
ccacaagctt aattagtata gtgttgtaac ccaaatcaac aatgggaagg acacagtgac 22440
aaaacccagg aaattccaag tcgaaagaga ggctctttcc tttaactcct tgagtgccag 22500
ggcacttgct agccagctcc ttacccaggt gcaggaggct acctgagtac aactcactgt 22560
ctttattgtt gaacttcctg gcttttgttg actttccctt ctcctttcct cccttaatgt 22620
acatgaaaca tacctttgtg tgtgcgttaa ttatagataa atgggctaaa attgcctctt 22680
ggttttgtaa ccctcagaga aggggaaaga gtacaaaaaa aattcctgtc actgtcatct 22740
gttcattttt aatgagcaat ttttaaaaat tgaaaataaa agaggaaaat gaataggtgt 22800
tgaaccagcc cgtgatatgt aaatgccaac tagtaattac ctaaaacctc aattttcatt 22860
ctgtttcatc ttgcaaaaca tttcattttc cctgagtgaa aacacttaat ttgggctgtg 22920
cgagttagcg gagcggccgc cccggcctgc gcctccgccg gctccggcta gggggcactt 22980
tggctctgcg cttctgccgc ctgcgggctc gtccacctca aggttattcc cctcttgcca 23040
ttccagtggt tttactccga gtgtgcaggc ccctcccccc tactcccact agtctggttt 23100
cggggagcct gttcctcctt gtccttgcag aggcgcgtat tgaattttgg ctgaccgagc 23160
cttcctctgc atattaactt tcccctctta cctggctatt gggcccttgg cgtccctgag 23220
ttgcagtcac actctgagag cagctttctg gaagctttct ttctcccttt gaggttgcac 23280
acagaactgc ctctagtttt tgtttattcc tgctgctttt ttagtctttc ttttcatttt 23340
ggcagctggg gaaatcctgg attgaggctc acacacattt gccctcctgc cacactgaga 23400
atgacaattc cgacgcttgc tttcttttgc atacttgtag atttttcttc tactctggtt 23460
taggatatgt caccttttgg atatgttcgt ggaatgtggc ttgtggttgc cgctgcttcc 23520
ttccctcccc agcctgttcc ctagctagaa agagagcgtg tagacttgag aaagaacagg 23580
gagcccggca ttgtgaatgg tagatcagag aggagcctgc atgtagaaaa ccagaattat 23640
gttgtcctag gaccctggat ccgagagtgc tctgcctgga atctcaagga acacggcctc 23700
tttagtgaga tatatagctt cataattagc ccgttgcatt ttttcacgag tgctagcatc 23760
agcgttctac gatgtgtttc tctgattaac agctgctttg caactatttc cctgtgccgc 23820
cgatgaccat atttgtcaag gaactcctga cagagtactg atttcttact aaaatgcagc 23880
atttctgttt gaagagggtt ctctcattct tggcagtgcc tggcttttta agtcagtcct 23940
tgttaaaact cgagttggct ttggtctaac gcttgttaac aggattctgg gtatctactt 24000
tagtgtatga acgtaggaac ctttcacagg caatttaatc ttctgagcag cgcacgtgga 24060
gcagtgcggc gctggaatgg ggtttcgggc ccgtagatta tattggtcat tttcttttgc 24120
cctctagttg tgatctctgt gggttaaaag gacttggctt tggttgttag tgtcattaat 24180
ttttgaatat ttttaatatt ttaagatggc taatgaatgc tcacatctta ggagatagat 24240
gtgtatgtag tacacaggag tgctggaatt ggtggcattc caaataagtg tcactggaga 24300
caatgcatca ggtttacatg tcaggtgcaa agtgctaagg ctccctcaaa ccccaggttc 24360
attattaggc aggctctgtc ctcctgtccc cctcgtgcag aaaataaagt gtgtattctg 24420
aagattacaa tgtgcaggtc ttttgtagga gagtggaaaa cacataggaa gcatctcttt 24480
ggtgaggact gtcaggaacc tcagttcttc tcactgggga ttacttaccc tacacaatgc 24540
atttaacagc acatagttca tagtagataa cccctgagta tactgtattc cattgattta 24600
tacagttaca aatcctgctc tctcaaatac aaatctctct caattggcca gtcatcttag 24660

atctaaaggt gtttttcaac ctgaagtccc ccccgctttt ttccaagttg ctgacagtga 24720
ctccagtgac tccggtgtaa ccattgttat gagactgcaa agacaagaat ttactcctct 24780
tccttgcacc tcagttttat ctctaagtga cacatagtga aaattatgaa atttccccaa 24840
aattgtggaa ttggtggtgg cagggttgtt ttattttgct ttgggtggag aaggggacag 24900
aaggtagggt cagagggatg gggggcgggg agaaactggt ctgttttctt tggagtttgt 24960
tgtctttatt ttgattaaac tagctattgg taattcgagg tgcactcatt aagaactcag 25020
tgcaagagga agggaaagga acgctgattc catatttatc acaggctcac cagcaagcag 25080
cttcgacggc ggcatcagca gcttagtgcc agaattctcc ctggcttgct gcactcccac 25140
aacatgtgca ataaagttcc tgtctccgtt ttacatcttt gacattgagg cggaagattt 25200
ggcctgaatt tatgtaattt agttgggcac aaatataacc catgaatagt cacattcatc 25260
ttctaattag aaaatcaaat gataatacag gacaggctgc aggcacccca tcatcgaatc 25320
caacaaaagc gtgttgtgtg tgttgggcct ctgcagagga aagtcagttg aattgcttca 25380
tgaaattaat gaaaagtgcc caacaatagg tgggtgaaag gcacaaaccg agtgccctgt 25440
cagttattta gtgtgtgaag atgttagcat tatgggtaaa gagttttgca aaatcttctc 25500
ccatcgatgt atgaatcatg gggctataaa attctagatt ggacaggtcc ttccccccat 25560
tttgtagtgc cagttccttg cggggaggga gggagaagag tcaagagtaa ctgaagaaat 25620
aattagtgaa aagaggagag aggttttcat atgttcacac ttacatgtag ccacttctca 25680
gggtgtcttt cttattcagc acattaggtt ggggctattt gagtgcttta tatttttgca 25740
tctagtgtag tgatgtctat gctgttaaat atgtgatttg cctgtcgcct gcattttcag 25800
acaaagatca agaggaacat aaacaagccc caaatatact ttatgtaggt gtttggcagt 25860
attattttat atgacttgaa attagtgaat ggaaacttta aaagaaattg ttcatatttt 25920
tgcaagtttg aagtgtgccg ggactttgtc aaattcccgt taaaataaat acgtcaattt 25980
catctccctt ccaaattcat ttatcttgct tttttaaaag gtgttttcta ctttacctga 26040
tgtttaaatt ggattcgctc gtttatgctg ttctttgcta tatcccccac gttcttctta 26100
aatgaattac taatgacaga gcactccatc attccctctc tgcagccact ttaaggaaag 26160
catggtgatc taaacctttt aacctattaa agatgatttt tcccaattga atcagaccca 26220
ccacaggtgt aataactaat catttaaact acacctgcta ttatgtattt ttaagccatt 26280
atgcatgttg gatagatgat aattcatgtt tattgcattg tccgatggat ccgttttgcc 26340
tacactaggc tgattaatgt atttaaagaa ataagagatg cattttttta aaaaaaattg 26400
atggaatgcg ccaagcactg cattgagtgc accattaaac ccatcagaac tttgctctct 26460
gaccagaaat gcatatacca aggactatat taaaaaagca aggtgaaacc tgtgtgttcc 26520
agtggtggga ttctgttttc tttctctgtc atggaaaatt ttggaaggat gaaggaaagc 26580
tttctgtttc tatgggtaaa tgataggagc tgtactacta gcatcttctg cttaagacct 26640
gttcatccac atttccgctg acacctggta cacgtgtcac acacagcatg cagactaccg 26700
catgatacga gtagtgtaat tttcttgtgt gttgaaggtg tatttttttc tccaggtgaa 26760
gaacaactat ccccacgcca tctgagctgt gcagactggc gctgaccctg ggggccagtg 26820
actgggtacc tgttcattct ctttccagag caggggcctc gcttccgttg gtttttctgc 26880
ttgtctctca tttctcctct ctccttttcc ccctctctcc tgttatagct ccttgtgtac 26940
tgttgccttc ttctccccac ctttcctacc tctacccatc cccttttctt ttttctcttt 27000
tttccttctt cccttctcct atctcttcat taaaatgcat cagtgttgta gatgtttgtt 27060
agatttctct tgattttggt gtgacctgag gcgatctggt tgagtatgcc gtttggggtc 27120
tctagaatct cgagcattca tttttcttta gtccagaact ggtctattac tgtggttcat 27180

gacggtggtt agtactgtcg gcagcattgg aggacagatg ataatctgtg cggagctggg 27240
cttggctgtc aggggacact agtatgtcct gttggtgaca ggcccagacc tgaaaagtca 27300
agatactgag ttcatccttt aggggaatgt ctaggtaagc ctcccacgga ggggttgcag 27360
ggaaaatgaa tacaggcttt tccctggctt tttttctttg gtttatcctc atatcttttg 27420
ttccccaaaa gtctgtctgg tctcaggatt acattcatat aaaatatggg gaaggagatg 27480
atttgtaact tgtatttcac ttacagtcac agtactttct gtttgactgc ttcccttagt 27540
ttgaaggatc ttagaaaact tccttacatc tagccttaga aattttttct ttcaacaact 27600
tatgagtgaa ctcagggcct gttgacttta tctgtaacat gagaccatat tattcctttg 27660
attttgatga ggcgaataat agcagtcatt taaaacagcc cctttcctac ctggggcagg 27720
tgggcgtttc ccatccatag ggctggtgtc tggggtggaa agtagacaag ccaggcgtaa 27780
acacacactt cgcgtgtgct gagtgatgtg aaggttaata cctgcagcgg tgccccttcc 27840
atcccctgcc cggcttatgt ggagatagga agatttcatt aaataatatt cattaccaga 27900
ggcaggtaga caatctgtca atctgtcagc ttttagccat gaaactccaa actttgccct 27960
ttgaagggtc ttcagaactc agctccattg cagaaggggg gccagagtga atggcagtgg 28020
gtgttgtctc cgacataatc ccttccagcc gggacgccct caacactttg ccaggctctg 28080
gagagagtgt ggtctctgcc cttcacttgc ttgcagcgta gttggggagg caggctgaac 28140
attagaagat ggtgtaatta tgtcttcaat tatatggtac acagtacagg cactgaagaa 28200
ttgaagagaa tagggagatc agagatggct tgcttattta ggcaaggctt cgaggaatca 28260
gaacagacta taaaagggct gtgctgatag agaacgtgtg tgtgtgtgtg tgcgtgtgta 28320
cgtgcgcgtg gggtaacgtt tagacaggtg gaggcaggaa agggaggcct tccagaaaga 28380
cagcatacag ggcacaaggg cggcgacatg ggtgcgcatg gccacacctg ccggggagta 28440
tgaagaatca gctcgaatgg agatcaggag gaacaggaag gattgtcgca ggcctgaagc 28500
tcggcagagg gattagattt gacagactca gatacaggaa gccagtgaag acagggaaac 28560
aacatcgcct ttcatttagg tagattttaa gtttaagaaa taactggtta taaatttgac 28620
cacaatagta tgcttgtctc tgtggagctg ggaaaaactc ctaggatttt taatcttgaa 28680
attcagacta ttgaagtgaa gtttactcct ctattttctc tctctcacca gattgccacg 28740
gcagtgaagt ttctacagaa ttcccgggtc cgccagagcc cacttgcaac caggagagca 28800
tttctaaaga agaaaggtac aggttccaca gggctgtgca gcacggcctg cagggggact 28860
ggggctgggg cagatggaag ccacggtgca aggcaggtgc tgagctgcca ggacttccta 28920
gagctgccca ctcatggggc agtggggctg aggcgaaccc cccagtgggg catgcaggtt 28980
tctttcctga cgatggagcg gcaagatgac acgattcggg taattaagca cggatgactt 29040
agtccagtcc atcatggcaa aggcagcatt cagtagcaga gtcggcccat tggatgagac 29100
tgtggaggac ttctggtttt tggtctgctc tgtgtcagtg accctggctg caggatgact 29160
agcttaggtt aggagttcag ctgcttcaat agagaatgag ggagaaagaa atgaagggcg 29220
agggaaacga gatgactaac aaggtcaggt taaactcaag cttggggtca gtccgattta 29280
cagattgaag cggtaaatta gtggttttat ggtatttctg taaacaggga taaagtggac 29340
cctgacaaat tcaatattgt ctgaagagac aatctattct ggttctgttg gacttcaggg 29400
tatttttctt tttttgtaaa atgaaaacta caaagaaacc tgacttttca attttttata 29460
catgtaattt tctagaaatc taggaagtca tttacacatc cttatatacc atgaggggca 29520
aaagtaagct ttcttcctcc caaagcaaaa ctctttttcc ttaaggagct ggaatgccag 29580
ccttgaaatt ctgagttttg agctttcagt catttttggc tggaataggt gggtgatatt 29640
tcctaggtct gctctgtgat gtcccgctga agggatgcag catgaaccat tggtcccttc 29700

atgcgatcat gtcccggggc tgcactaaca gggtttgggc agaagaagcc caaacatttc 29760
accacaggca gctgcttcgc ggcagcccca aggctgggtc cctgcgacag agttggtaag 29820
gagcactgca cggctgctca ctggctgcat tgtgccagca cccccccccc ccccccgcca 29880
tcattaggag attattaaaa tttggagtgt gttggctggc ctcgccactc cgcaagcatc 29940
atgccataaa agagtttttc cttgaaatat gtgtacctat ggggagtaat taatcataaa 30000
agcttaggga tttcagttct ctcatcaaag cattgaagct ggggaaagct gagcctaaaa 30060
gctgagctga ttacccgggg taacctgaga caaaatctat ggttgccgag agatccttag 30120
ctccagtata ttgtgcaata ttataactgc ctgatcttct cttccaccga caatgaccag 30180
ttagtcctca ttttctctca aacgcagctg ggttagaatt tcatttgcag aaggtattta 30240
gagcaattta gagacaaatg tctctggtgt ttgctcggca cttccccacc gcaggctttg 30300
ctgagcagtt ttagtatcag gagctccatc taatctgttt aattttaata aatccctgac 30360
tgggagaggg agtgaaaaaa aaaagagaga gagaaactct ggaccaaata atacttcttt 30420
tgagttactt ggctaataat tactgtagag agaaaactgt tttgtgcaat tatgattcgt 30480
tgctccttta gcaaaaaaaa tccaactgat caattcatcc aagagtcatt gtgcacccta 30540
tgccctgtcc ctggactccc atcaggaatt tagcagagaa ccgaacagac aaaatccctg 30600
ccctcgcaga gcttaaattc tagtggaggg aaggcagacg attaacaaat gggaaaatga 30660
aataggtcgg atgtcagatg ccaagcgcca tggatactga tgaatgaagc agggagccgg 30720
ggtgaggaat gccagagttg gtgtgggctg gactgtgatc ttaaacaaca tgaatgtcag 30780
agtgtctcta agagggtggc acatgagcaa agactggaag gtgactgttt attggtacag 30840
tggacataat tccttcttga cccctaattt gggttagagt tttagaattt ttaggcatct 30900
ggcaccttga gatgctatcc tgcccttcgt cctctaaaga gaaccatgtg agtttatccc 30960
gtacttaaac ctggaatctc tgaggttaat cacccatctc cattatcctc taaaatgctg 31020
cttcagtcag taattatcca tttccgagtt gaatcccaat ggagtaggtt ctggcaggac 31080
cttttctggg cagagaattc caaggtttga tcggtgtctg ggaagaaaaa gccccttcgc 31140
agatttaagc ttcacttttc aaatttcggg ctgttccagg gtcaggtttc cgtgctcagc 31200
acggcctggc accgtctccg atggggatga gggtgaacat ctactgggtc cctaacagta 31260
gttgtggcag gaaagcaaat tggctcgctg gccgtctgga ccaggagggg cctgggttct 31320
ggcacccgtg ctcttggact ccctgcagaa gccttgcagt gctgggcttg gagcatgggc 31380
tctgggaggg ctctcagctc ctggagtctt ggcttttcct agcaatgtca cttcagggaa 31440
ttgcagtgga gcactgactt gaattataat cttgactttc ttttttttaa taaacatctc 31500
tttcctgaaa aggcaaatcc aattttgttt ttcttttaat attatttaat atttaatata 31560
gaaattgagg tccctgtagt gcatgtcagt tcagtggaaa atgaggcgca tcagctgagt 31620
ttgtttcatt gtgaaaccgc agctgggagc aggggctgca tcctgtccgt gttaccgacg 31680
ggttaagtgt tgtgctctgt gacttctggt gggggtgctg ggtgagggtg agtagggaat 31740
gagtggtata gggatggaag agcattggtt tgcaaaggtc tttcctgtta cttttttttt 31800
gttttttgtt ttgagatctc tttcggatgc tttaaaatct gaacatggag ccattaatga 31860
gaaacgtgaa gttattagaa tcatttatca ttttctttga agtttgtact gccattttgt 31920
gtaatttaca acatttcttc cttttatcgg gaatattttg gatgaaagaa aaaagcagct 31980
gctcaccttc agagtattag aataaatgtt tttgttttcc ttccaaccac ttgattctgc 32040
agatgacaca tcaactgcgg agtcagccac acatcaagat aattggactg tgtgtggggt 32100
ttaagccctc agaaccagtc tatatataga agcagttctt gccaggcagg catcatatct 32160
ggggctttaa gcaggctgca atccaggagg ttagagtaac gtaaaaacag caatagaatc 32220

agtccagaaa gttagaggct tggaaattgt cgtcaatagg cttgtgttga tttgacaccc 32280
tatccagcca caaaatgtag gaggcaatct gcttccaaag atgtttggca ggctgaggat 32340
gaggtgggta atagtgatgc ttgcttgtga ggggggtgag gtgggggagg gggatagagt 32400
tgtctgttaa cttactctaa catatctgtt gcaaagcaag cttctaggaa agacaaacat 32460
catctatttt tctgtataac cctgcatagt ggggcctggg tgctgttggc tcacacttac 32520
tgtgtccttc tcaagtgtct aagtaaaggg ctttttcctg cctcatccaa gaaatggtcc 32580
tggttggggt aggcaaatga aacaaataaa acacaccacc atactttctc attccaaaag 32640
gtgaaactag atcttatcct tctactgttc ctggaagtag aaaccccggt agcaaaaccg 32700
cctccttgct ggaaatgaac ttgaagaaac ctggcggtcc caaccttggt agtgggctag 32760
aggtaaaggg cttctctgta gaatatttca agggggagta aattcttaca cagatttcaa 32820
tttaactctc ttttattact tttctttttt ttatcattta cgtctatttc atgaggtagt 32880
ttgcatgtcc ttgaatggaa tctcgttacc atgaaagctt ttttagcatt gatttcataa 32940
cagtcttaat ttaatagctc cgtcattact gagaagctct gggagtgggg ccctggcttt 33000
ccaggtcact gaccttttaa ttacaaacct tgttctgatg gccaggttat cgactggaca 33060
gctcccagct tgtgccccca ggatgaaaac gaaaataact gtgtttcctc ccccatctcc 33120
aggaaaaaaa aaaattgtaa cctcttgtgt ttctaaaatt aattttctcc ttcagcttta 33180
ctttataatt aattctttaa tgtgtcttag tgaagccaga agtcttttca caaagctttt 33240
cttcctagag cttgggtcca gcagatagct tggggggagg gcacggaaat cttgccagct 33300
cccacccctt gcttgcgtgt ggcccatttc ctcatcctca gaaactcctg cggctgagct 33360
ggaaggaata caattggctc tgtgaggggc tgtctggccc ttctgatttt ttgttaacga 33420
gacatggatt gtggcatcaa gatttagatt cattcctctg tttgttggag tcattgaagc 33480
cagtatatcc tggacatttt ttaaagaggt ccccattctg agaaaagaca ggagttgaat 33540
gtcttattga ttcttacctt tctgttcgtt atagacgacc agaggaaaca aatgcccgac 33600
acggattcga ctcagtcata agtgtgaacc aaataggccg atctgggttc tctcactgac 33660
tgaagaggaa gagaaataag agaggacagt gggcaaaatg tagggtgaca accaagggtt 33720
ctggtttgcc cagaattgcc ctgggttcaa ccctgaagtt cccatgttgt ggacagcccc 33780
gtggtcctag acaaacaggt caccttagcg gtaaaagcct ttctcaggag tgagagctac 33840
caggggagac aaaacgggtt tggttttgga acctggagga agaaggcaaa atgagaagag 33900
tccactggca gtgagtcccg gagaggcccg cctgcagcag cgtggcatct tccgcaccca 33960
cttcctgctc tttctcccgt agccctgccc tagtgtgggt ccagtgcaga gcctctgggg 34020
gcggggccgt gccctgcagg tgagcgagga gctgctcgtg cggagcaggc agggctgtgt 34080
ggcagccgcg cttgttcctg aggatgattg agcatctcca tccatctgtt gtgaacggac 34140
cgtcttcctt ccctgccagt cttgcacctg catattcgtc acctgctgtt ctggcccagc 34200
ctattcatca caccgactgg tcccccgcat caattttccc gctccccctg ttatccaaac 34260
ttgccttgaa ctccacagat ttttctcggc atccaggacg agacctacgg cagctaggcc 34320
agccgaatgt tgtagaaaca gagatgccct ggcttcccca ttcagagcgg gagctgatag 34380
cacttgctgc cccaagaagg ctgagctgag gggtttcctc cacttcaccc tgcgatgggg 34440
aaatagtgca gaggagtttg cggggaggga acactttcag atttacccag ttacttttat 34500
gttttccctg caactgaaga ctgattcttt tcttaaactt gcgtgcctgt aacaatactg 34560
caatgaaaag gaaagtgaca aaatgacatt ggcatgtatt agtaaaaaaa aaattttttt 34620
tactttttga aaaggtaagg catatacatg gtcttaaaaa ttcaaacagt agagaagggt 34680
atacagtaaa aaataagtct tctcttctca ttctctcatt cctgttcccc acccgttctc 34740

ttttgcagag gatgctacag tttcttatgt atcattccag agataatcta tccacatgaa 34800
agcatgttaa tatgtattac tcccccacag aaacacgaat gctttctaaa tacagtggta 34860
gcatatttta cactttactc gtatacttgg tgttatatct cggcagtcat tccacatcga 34920
tacatactga cttgcttcat tcttttcagt ggctgcataa tattccatta tatgcatgtg 34980
ttatcctttt aaaaactagt cccggctggg cgcggtggct cacgcctata atcccagcac 35040
tttgggaggc agaggcggga ggatcacgag gtcagaaaat tgagaccatc ctagctaaca 35100
cggtgaaacc ccgtctctac taaaaataca aaaaattagc tcggcgtggt ggtatgcgcc 35160
tgtagtccca gctactcgcg aggctgaggc aggagaatcg ctttaacccg ggagacaaag 35220
gttgtagtga gccgagatcg tgtcaccgca ctccagcctg ggtgacagag caagactcca 35280
tctcaaaaat agaaacaaaa acaaacaaaa aattagcccc aaatttgtga gcatttaggt 35340
tgattcctat cttctgctgt ctcaagcaat ggcccaatga atattcttgt cgtatacatt 35400
gttgaacgcg tgttggtttg gcaccatgct gaagtcagag catttttgca tatggtaagc 35460
aggatagggg atatttttct tgcttaaatt tcatgggatt atgtttcata gtaaagaaga 35520
taaaactttt ttaaggtaaa catggactat aggtagaagg aagttaataa gaggaaaaag 35580
agtaacaaat tgcggttaac acttagcaca gatttggatt ttgaagggat ggacattctg 35640
gttatgtgaa aaattcttgc tttctttttt tgttttgaga cggagtctcg ctctgtcgcc 35700
cagggtggag tgcaatggca caatctcgac tcactgcagc ttctgcttcc caagttcaag 35760
cgattcatct gcctcagcct cccgagtaac tgggattaca ggggcctgcc accgcaccca 35820
gctaattttt gtagtttttg gtagagatgg ggttttacca tcttggccag gctggtcttg 35880
aactcctgat ctcatgattc acctgtctcg gcctctcaaa gtgctggtat tacaggcatg 35940
agccaccact cccggcctgc tcagttttga cctagccatt gagggaggca gtgggcacag 36000
agagaaaaga gcaggaaggc cttcgttagt gaggggaggc tcaggcagag acgcatgtgc 36060
cagtcgctgg attggcgtgt gaggaaaaag aagggaagag aattgaggct ggtgagatag 36120
gaagtgaggg aggagctggg gttaggaccc aatattatat aacagtattg cctaggctgg 36180
aacgtaatgg catgatcata gctcactgaa gccttgaact tctgggctca agcgatcctt 36240
ctgccttagc ctcccgagta gctgggacta caggcacgtg ccaccacacc tggtgagttt 36300
ttttgtagag acagggtctc actgtgtttc ccaggctggt ctcaaactct cggcctcagg 36360
tcatccttct gccttgcctt cccaaagcat tggaattaca gacatgaacc acggcacctg 36420
gcccatggag cacagagaaa ctctgtttga ggaagtttac tctcggctct gtattgaaac 36480
tgacttggcc tggcaataat aagaccagcc acaaagatgg tcctgctgag tccgtcttca 36540
ggtgtggggg atctgggccg ggttggtagc agtagagatg gaaatagaaa tgacagcata 36600
tttatgtaga cagttctaca gtttacaaaa cactatcaca tagttgattt tttcactatg 36660
accctagtat gtagtgggca ggttaatgaa tattagttga ttttttcgct gtgaccctag 36720
tatgtagtgg gcaggttaat gaatatttgt taaatgaatg aaaacattat ttaatgaatc 36780
tcagagtggc tcttttctcc cattttacat agatgaagaa attgaggttt agaaaggcta 36840
agttgagcta atacatggca gaagtagaac tagaatctat gtcttctgac caagattctt 36900
tgaactatac cacgtacttt ctggagagga aggacagaac cacagacatg gcagaagaaa 36960
gagtaagcca tgctcaggag ggtgtgtgtg gcaaagggat gggccaaaga tgactctcag 37020
ggttgctttt tttgtgttaa gcagctttat tgaggtgtaa ttgacataca gtaaactaca 37080
catatttaat gtgtggtttt aaaaagttat gacatatgta taattgggaa actatcatca 37140
caattaagat aatggacatg cccagcatcc ccaaaagttt cctcctaccc cttggtaatc 37200
ccttcctcct gtggtccccc caacccccat atcctggttt gcaagtatgc attgatctgc 37260

tatcactata gattagcctt ttttttttct ttttcttttt ctgagacagg atcttactct 37320
gtcacctagg ctggagtgca gtggcgtgat tacggttcac tgcagccttg acctccccgg 37380
gatcaagtga tcctcccatc gcagcctccc aggtagctgg gccacaggca cacgccacca 37440
aacccggcta attttttttg tattttgggg agatagggtt tcaccatgtt gcccaggctg 37500
gtctcgatct cctgggctca agtgatctgc ccaccttggc ctcccaaagt gctgggatta 37560
caggtgcaaa ctactgtgcc tggccctagt ttgcattttg tagaaattta tataaatgga 37620
attatacagt gtgtaatttt ttttgttctg gcttttccac tcagcataat tactttgaag 37680
tttggccatg ttgttgtata tactagtggt tcattccttt ttattgctga atactattcc 37740
attatatggc tatactacct gttgatggac atttgggttg ttttcagatt ttggatatta 37800
aaaataaagg tgctgtcaat atttataagt ttttgtaggg acatataatt tcttttgggt 37860
acatacttta ggatggaatg gctggatcat ataataggtg tgtgtttaac attttaagaa 37920
acttctcaac tgtttttgaa agttgttata ccattttaca ttctcaccag tcatgtatga 37980
gtgtaccagc attttgagcc tggagaatag gaagatgaag gtgacaaatt ttctagtaat 38040
gaagccactt gactttggaa atattcttaa gagtgactaa ataataggtc atgtttggac 38100
tacttcagct ttagaggcaa cagtgcataa actatctggg tttgaatcct agcttcacca 38160
ctctttagct gtgtggtcct gcataaaata cttaacttcg tggtacattg gtttctttat 38220
ctgtaaaatg ggattaatga taatatttac tgcaaaaaat tcttgtaaga attaaatgaa 38280
aattggctgg gtgcggtggc tcaagcctgt aatcccaaca ctttgggaga ctgagacaga 38340
aggattgctt tagatggaag ggttgagacc agcctagaca acacagtgag atcctgtctc 38400
tacaaaaaaa attaaaaatt agctgggcgt ggtggcgcat gcctgtggtt ccagctactt 38460
gagaggctaa ggcaggagga ttgcttgagc cttggagttt gagcttacag ggagctatga 38520
ttgtgccact gcactgtagc ctgggtgaca gagcaagaca ctgtctcaaa aaaaaagatt 38580
gaggaggggg tagaattaaa tgaaaatgat atggaaagtg cttagcagag tgtctggcac 38640
caaaataagg gcttaattga tgacaacaat catcaatagt atatgttctc ttagaagata 38700
tattcttaat gggctatagt cagtttgctg taattgtatc ataagaattc gtgcatcagg 38760
aacctctagg aattttgagc agagagggat ttaatacagg cagttaggcc gggtgcagtg 38820
gctcaagcct gtaatcccag cactttggga ggcccaggca ggtggatgac ttgaggtcag 38880
gagtttgaga ccagcctgac caacatggcg aaaccctgtc tctactaaaa atacaaaatt 38940
agccgggtgt ggtgacgctt acctgtaaac caagctactt tggaggctga ggcaggagaa 39000
ttgcctgaac ctggaggcag aggttgcagt gagctgagat tgcgccattg cactccagcc 39060
tgggtgacag agcgagactc tgtttcaaaa aaaaaaaaaa aaaaaaaggc taggtgcggt 39120
gctcacgcct ataatcccag cactttggga ggccaaggtg ggcagatcac aaggtcagga 39180
gattgagacc atcctggcca acaccgtgaa aacctgtctc tactagaaaa aaaaaaaaaa 39240
atcagccgtg cgtggtggtg ggcacctata gtccccgctg ctcaggaggc tgaggcagga 39300
gaatggcgtg aacccgggag gcggaggttg cagtgagccg agattgcgcc actgcactcc 39360
agcctgggtg acagagtgag actccatctc aaaaaaaaac aaaaaacgaa caactacaac 39420
aacagcaaaa aaaccagatg gttaggcact tactcactta ttgaaaaggc taggagggta 39480
gacccaaggt taggccttca ggaatgactg ccagataatt tgccaggtta ggtgctacct 39540
ctgtcctgat caagcagtgg ggaatcaaga ggctgtcact ggagctgtgg agctcaagtg 39600
catgtccttg cagctgtgat ccagggacca agaagttgct gctgccccca ctgcaagtgt 39660
tgtgtgacac ctggaaaagc aacgacagga cattggaatg tgggcttggc ttctacttct 39720
gtgaagattg gctcttgacc cccatgaagc tggagactgg acattacaat aggaaacctc 39780

agtgtttcca atatcctgcc tgcccatctg ctgatatttg cagcaatcaa aagcagcaag 39840
aacagcagtc ctctgcctta tttccacttt ccaaattgtc catgagtgca cctaattggt 39900
ggtacctaat tcccatccag aactctagct gccatggagg gaagcatttt gtgcaatatg 39960
gcgcaaatta tgggtgcgtt agtccccgcc ttaaccacgg aggagacgtt ccaagacctc 40020
cagtggatgc ctgaaacctc ggctagtacc gaactctgta tgtactatgt ttttcttatg 40080
cttgcatacc tttggtaaag tttaatttat aagttaggta cactaagaga ttaacagtgt 40140
taactaataa taaaattgaa cagttaaaac aatatactgt cataaaagta atgtgaatgg 40200
actcaccacc tctcccaaat tatcttattt tcaggtaact gagactgcaa aaagcaaaac 40260
cacagataaa gggagacttc tgtgtatatg tgacagaagg atacttttat gcttttttag 40320
tctattatta tttttttaga agacttgggc ctttgttagc aactgtaatg atatacttcc 40380
agtttgtcca agagataaat attgatgtat tctgtatcat cactggcttt cgagatgatt 40440
ttaagaaaac ttgttggctg ggtgcagtgg ctcacgtctg taatcccagc actttgggag 40500
gctgaggcag gcagatcacc tgaggtcagg agttcgtgac tggtctggtc aacatggtga 40560
aaccctgtct ctactaaaaa taaaaaaaat tagccaggcg tggtggcgca tgcctgtcat 40620
cccagctact tgggaggctg aggcaggaga atcgcttgaa ccctggaggc ggaggttgca 40680
gtgagctgag atcgcgccac tgcactccag cctgggtgac agagtgagac tctgtctcaa 40740
aacaaacaaa caaaacaaaa caaaaagaaa aatttctttc ttatctggcc tcacttaggg 40800
atggaagttc ttttaatgag atttatctgc taagggtaga caagagagtt tccaccaaag 40860
ggactacatg atatgcgggc ttattggaac tgttggaaaa tgttagaagc agggattttt 40920
gaaacttaac ataaggtata acataaggca atctctactg aaaaacattt tatgttgatt 40980
ggactgtaat tagaaagatt taaaaataac tttggatagt attcattaat ttaccaatcc 41040
attgttatat tcaacacttt cgagctgatg tcttttaaaa atctaagttt atctgttggg 41100
gggtggtgta tgaattagtg tatctgtaat tgtatgaata cggagtattc ttatctttgt 41160
atgtgatggt gtgcttctat tttaaattta tttttaacta tagataggct tgctaggttg 41220
gtcattccac agcttggtct tctttccgtt tttgaagtaa aggtaactca ctaatggtcc 41280
tcagtaaaca gagtgatggg gcaagctgac ttatatttca attgttctga gaattgaaca 41340
actctactaa tgtttccttt ccaacttcaa acatacctgt tcagtttctt caacatcctg 41400
ataggagctg atctcctcag aactctcccc tctcatcaga cagaggactt gggcatgctc 41460
taatctctta ctgttaatta ttctttttag ttttaaagcc ctttaaagta atctttctgt 41520
aattagattg ttgagtagat tgtctttagt aatatggtgg tcttaaattt tgtgtggatt 41580
ttcagattgt taaatggttg cagaggcttt cattcatgaa gttcaagttt actatatcat 41640
ggtatgtcag gaaaaaaaaa atcatgaccc tctgtgataa attaaatatc acaaccacgt 41700
aaatgtcatc ttttcaatga aactgaaagt gcataggaat gggactcttt ttcagtctgt 41760
tttggcttta agctgtaaga aaaatatttt aacactgaca gtagtaaacc tatgaagcta 41820
aagaacaaag ccatactctc cccagcttta taagtcacac gcggaattgt gaattaaatt 41880
attctaattg caaaatttgg ggtcttggcc gaaagaacac aggttgaatt tgaagaagag 41940
gcagctagaa aaatcggtgt aacttttacg tgaagataat ttgcacagaa tgtaattgaa 42000
agagaataaa tatggagctc catgatgtcc tctttgagtc tatttctcat atttacttac 42060
attttttttt cccaatcaaa ggctctcctt taggcagtcc tgaaaagagg agacacagtg 42120
ctgaaataga aataaaggtt aagtatctcc gatccaaaat gcttgggacc agaaatgttt 42180
cagtttggat tttttcagat tttggaatat ttgcagaata ttgtgttggt tgaacatccc 42240
tattccaaaa tcctccaatg agcatttcct ttgaatgtca tgttggcact caaaacgttt 42300

cagattttgg attattttgg atttcagatt tttggatcag ggatgctcaa cctgtaacag 42360
gcagaaataa tagaaaataa cagaaataac ataaaatagc actgttcttt ggaagatctt 42420
agtaccttat ttggaaatat ttttactatt cattcatcca ccacccatcc atccatccat 42480
ccatctttcc atccaacaag tatttattga gtgcatgtat cttacaaggc atggtgctgg 42540
ccctgggagt gaggatgtaa tgttaccatt tacataaaac aaacagaggc ctggggtgga 42600
agcagactcc acagaaatat gaacaaacaa gcaaaataca gataagcaag gtgctgtgag 42660
agagaataat ggagagggtg gggtaggttt gcattgggaa ggttgctgag accagaagaa 42720
tgataagtgg ttctgtgaag aatgagaatc ccgggcagaa aggacagcaa gctctcaaag 42780
actccaaggc aggaaaggag cttgatttga tctaggaact cctgcagcag tgagtgaaaa 42840
tgagagtgac attttatagc ttccatctgg agacaccatg g 42881

<210> 83
<211> 614
<212> DNA
<213> Homo sapiens

<400> 83
gtaaagatgg gggtacagaa aacaattaaa tagaacaaaa acaactgttt ccttttcctg 60
tgattcaaga agggcttaga tcttctactc agcatccttt tactaatgcc ctccattggc 120
tctcacccca acatttcctt ttttatagct tattttgtaa tgcctcctta attatccttt 180
aatagaagcc accgctgata agctacctac actcatacag aagcattaat ataatgcccc 240
agatgtactg tttcaggggc aaaaaggaaa ataatttcca acaaagtggt gtgtgtttca 300
ctgtcagatg cttgcactta cacacngaat cgctgggcat ccgacagagg ctgattggca 360
catgggggca cgggggattg gncagctnaa accccggncn gcagcggttg cccctttggg 420
aaaatgggga ttttcccaaa aacaagtaaa accggggtnt ggtccttgga ttttaccaga 480
gtangctacc atttcctagc tttgggatnc ccgnccatna aggggccaat ttcacttggg 540
ccggtcgttt tnaccaacgn tcgtgactgg ggnaaaancc cttggcgttt acccccaact 600
tnaatngnct ttgg 614

<210> 84
<211> 23241
<212> DNA
<213> Homo sapiens

<400> 84
ataataaact gtcactgttc tcatcatgaa tgaatctcaa aaatatgtgt taagtaaaag 60
aagcctggga gactgaggtg ggaggctcac ttgaggtcag gagttcaaga ccagcctggg 120
cgacacagcg agacccgacc tctacaaaaa ataattagct gggcatcata gtgcatgact 180
atagtcccag ctactcggga ggctgaggct ggaggatcac ttgaacccag gaggtcaagg 240
ctacagtgag ctgtgataac acctctccac tccagactgg gtgacagagt aagaccctgt 300
ctcaaaaaac aaacaaacaa acaaacaaag tttaaggctg gacgcggtgg ctcccagcac 360
tgtgggaggc cgaggtgggt ggatcacttg aggtcagggg ttcgagacca tcccggccaa 420

catggcaaaa ccctgtctct attaaaaata caaaaaaatt agccaggcat ggtggcgggc 480
acctgtaatt ccagctactt gggaagctga gacaggagaa ttgcttgaac ccaggaagcg 540
gaggttgtag tgagccaaga ttgcaccact gcactccagc ctgggcaaca gagcaagact 600
ctgtctcaaa caaaaaaaaa aaggaaggaa aggaaaaaaa aaaaagacta aagagacaaa 660
tcaatgcaat ctctgtttct tcactagatt ctgatttttt ttaagtgtga aaatttgagt 720
gcagattctg ttttaaataa tactatttat caacacttaa atttcttgtg tgataatgat 780
attgtggtta tacaggggaa taaccttact cttatgagag ataaggtgaa atattctagg 840
atgaaatatc cttgatgtct gtaacttacc acaaatcctt ccaaaaaaaa aaatatatat 900
atagagagag agagagagag aaagagagag agagactgac ataaagcaaa ttcagcaaaa 960
tgtgaacagt gcatcttggt taagttttta tgggaatttt acagaagttc tcagtattat 1020
cctttcaact tttctatacg tgagaaattt ttcaaaataa aaagttgagg ccaggtgcca 1080
ggctcacacc tgtaatccca acactgtggg aggccgaggc aggtggatca cctgaggtca 1140
agcattcaag actagcctgg ccaacatggc aaaaccccat ctctactaaa aatacaaaaa 1200
aattagctgg gcatggtggc gggcacctgt agtcccagct actcaggagg ctgagacagg 1260
agaatcgctt gaacccagga ggcagaggtt gcagtgagcc gagattgtgc cactgtactc 1320
cagatcggga gacagagcaa gactccgtct caaaaaaaaa aaaaaaaaaa aaaaagttag 1380
ccaggcgtag tggcacacac ctgtagtctc agctactcag gaggctgagg caggtgaagc 1440
aggagaaccg cttgaacctg ggaggctgag gttgcagtga gccgagattg agccactgta 1500
ctccagcctg ggcaacagag ggagactcca tctcaaaaac aaaacaaaac aaaaaaaaaa 1560
aacattgaga gggcaaatac cttgtctgac ctgctgttat tcaagagttc aacagactct 1620
caaaactaca tgtaaagact gggagaagaa aactaactct gagcacttac tatgcagcag 1680
gtgctcttca catctgtgat ttcagtcaac actaaaaggg taattatttt ttattttatt 1740
tatttatttt ttaagtgata gggttgcact ctgttgccca ggctggagta caggggttta 1800
ttcacatatg tgatcatcgt gcactacaga ctccaactcc tgggctcaag caatcctcca 1860
gcctcagtct cctgagtagc tggaactaca gacatgcacc accacaccca gctttaaggg 1920
taactattat tgctctcgtt ttacagagga gaaaacatac tcagaaaggt ggagtgataa 1980
gtccaatagt tgtgagtgat gaagttccac caaaacctgc caggatgcga agccatgctc 2040
ttccactctt tcactctgct cttctccctg cctgcttctg tagcctctca atgcaaaatt 2100
tactgtaccc agtgaacctc cacagatagg aaatctacct tctgcaataa tactcgatac 2160
tcagtaactg tgaagtaatc agattgaacc atgcctctgc tgttcagcga aggaagaaaa 2220
gctagtagga aagttgggaa atagagataa cgtttctcat tcctacgggc tccaacgtgt 2280
acacactaga aaggacttca atgagagatc agccatgctg gctgggcgtg gtggctcatg 2340
cttataatct cagcactttc tctctctctg tctcttcccc cgtccccaaa cagggtattg 2400
ctctctcacc caggctggag ggcaatggca caatcaccgc tcactgcagc ctcaacctcc 2460
caggcccaag tgatgctctc acctcagcct ccaaagtagc tggaaccaca ggcaagcact 2520
accatgcctg gctaattttt taattttcag taggaacgag gtctccctat attgcaaagg 2580
ctggtctcaa actcctggcc tcaagcgatc ctcccaactc agcctcccaa agtgctaaga 2640
ttacaggtat gagccatcac gcccagccag gaactcacca ctacaaaaat tgttttaaaa 2700
atacattagc caggccaggc acagtggctc acacctgtaa tcccagcact ttgggaggcc 2760
gaggtgggcg gatcacgagg tcaggagatc gagaccatcc tggctaatac ggtgaaacat 2820
cgtctctact aaaaatgaaa taaaaaaaaa atagccgggc gtggtggcag gtgcctgtag 2880
tcccagctac tctggaggct gaggcaggag aatggttcga acccgagagg cggagcttgc 2940

agtgagttga gatcgcacca ctgcactcca gcctgggcga cagagcgaga ctctgtctca 3000
aaaaaaaaaa aaatacatta gccaggcatg gtggtgcaca cctgtagtcc cggttactca 3060
ggaggctgag acgaaaggat tgcctgagcc tgggcgggca aggatgcagt gagccatgat 3120
cacaccaccg cactctggtg acagagtgag accctgtctc aatcaataga tcaaccacat 3180
aaaacagagc aatgtgggga acacagaaca aaggaagcaa taaatgaaat tccagcaagt 3240
cgtcttgagg atagactaaa aagactatac cattatgaaa aagtaagtat gttcctgaaa 3300
tgcaaaagtc atgaaggaca gctagaataa acactagcat tcagggcatt ccttgaaatt 3360
ttaaggaatt acttctgaat aaaagaactt tactagacaa gtagtcaaca catgaaaaat 3420
cataccccaa aagatgtcgt aatagaactt gaagaaaggt ttagatccat ttgtgagtga 3480
aaactcagag cttattaaat tgaataataa taataataga caacacattg tttcaggcac 3540
tgttctggca tttttacata aataactcat ttaaggccag gtgcagtggc tcacgcctgt 3600
aataccaagc tctttgggag gccgaggtgg gcaggttacc tgaggtcagg agtttgagac 3660
cagcttgtcc aatatggtga aaccccattt ctacttaaaa aaaaaaatta gccaggcctg 3720
gtggtgcata cctgtagccc cagctacttg ggaggctgag gcaggagaat cacttgaacc 3780
tgggaggcgg aggttgcagt aagccgagat cgcgccactg cactccagcc tggtcaacag 3840
agtgagactc ggtctcaaaa ataaataaat aaataaataa ctcatttagt cctcagagca 3900
actctgtgag ataggtccta gtattttccc tgttttgcac atggagaaat taagtctaga 3960
aaaattaagt agatctgtcc gagatgacat tgctggtaag ttagtggagt taggattcca 4020
agccagtggg tctggcttta gagcctatac ttgtaaccgc tattctacac tgcctctcaa 4080
gggaagagaa ggttattcac ctttaatagc tggaaaccag agattgatat gacaaggcta 4140
accaacaaac tttctgtgaa agacagaata ctggctcagc cccacagttc taaccctaca 4200
caacaaggcg tgggggaatt agctaaacaa tcctttcaat cctactctgc tttaatcgga 4260
gaaatgggaa actggattga tcaagacttg aaaatgaaac aggcaattgc tgaagagcat 4320
tggttcattc aaaaagcgtg catttgcttc ttgttagata gccatagagt ctgttatgaa 4380
aagttcaagt tttggccagg cactgcagct cacgcctgta atcccagcac tttgggaggc 4440
tgaggtgggc ggatcatgag gtcaggagat caagaccatc ctggctaaca cggtgaaacc 4500
ccatctctac taaaaacaca aaaaattagc tgggcatggt ggcgggcgcc tgtagtccca 4560
gctactaggg aggctgaggc aggagaatgg cgtgaaccca ggaggcggag gttgcagtga 4620
gcagagatcg cgccactgca ctccagcctg ggtgacacag cgagactctg tctcaaaaaa 4680
aaaaaaagaa aagttcaagt ttttccccca ggctgccagc atgttctgtg gcaatcctac 4740
ctccaccagc tttggaaagg agggcaaagg agaagcagag ctgaaaatca aacaagtgat 4800
ggccatgccc ccttaaagga ccttagatcc ttttctatta aatacatata cagagagggc 4860
aaacttgcaa aaccaaaaga atccttgggc ataacagtgg ctccaagtgg caagattttt 4920
agtactgttt acagcactgg caaattaaat gtcctataag aatcaggaac agagccgggc 4980
acggtggctc acgcatataa tctcagcact ttgggaggct gaggcgggca gatcacttga 5040
agtcaggagt ttaagaccag cctggccaac atggtgaaac ctatcgctac taaaattaca 5100
aaaattagtc aggtgtggtg gcaggcaccc gtagtcccag ctacttggga ggctgaggca 5160
ggagaattgc tcgaacccgg gaggcagtga gccgagattg cgccaatgca ctccagcatg 5220
ggcgacagag agagactccg tcttaagaaa gaaaaaagaa tcaggaactg aggacaccct 5280
tcaatcccag cctgtgggca ttcctaacac aaaacaattc agagaaaccc agaccccaag 5340
atgccttagt atttcagcaa actaaatgtg gtaaggcaag aaaggagagg ccctcctctc 5400
gagacacttg ccaaactcca gaacacctga cagtgagaag gccagaatca gtccttgtta 5460

tctcccatgt tttacaatca agacatcttc ttcctgaatc attcacactg ctaatagaaa 5520
ctggtatggg ggaacacacc aactccttta agttcaattc ttgtaacaga cgtgcagaat 5580
aagacagtaa gaaacactaa gcggtggtct tctgcctctg aaggaagtga aatactagta 5640
agttgctgaa aactcttaga ggtgcttcaa ggagatggga gaggaggaat gaggccccaa 5700
gttgaaacta ttttcttctt cctcattcta atcctaacta aactcaaaaa tgtatggatg 5760
actagtggag tcacacaggt tcaatacagg ctcaacagag cacacgattt ggggcatgtc 5820
acaaaccctt agtattcatt tctgtgaaat ggaggttagt agcctgcact tcataaaggt 5880
tgtagtgaac actaaacgag ataatagatg taatttgtgc tcaacatgac accacacagg 5940
cagttaaatg taagatgtct cttagccctt ttctcctcac cccaccctac cccacctttg 6000
gactataaac cccttcggag accttatcat ccactgcctc ccggccgcag aggcacaggc 6060
gtcaacgctg gtccttatgg tccccttatt ggagtgggat ttgtctgaat ctgcaggtga 6120
aaagctagag cggagccctc ggccaacaaa accaaaccca gggaagcgag gatgggtcct 6180
cagccaaaag cttggtggcg tctgttagtc acagtgcgtc cctttcctat caacgcactg 6240
agagggctcg actagattat ttctgactat ccaacttgac gagagaagcg caaaggtgac 6300
cacagatgaa gaggctggca gccgctccag gaggctgagg ccgtgaggga gtgggaaccc 6360
taatggaaac aggcccctcc ccggagggac agccgggaac gatcgatcta tcctctcctt 6420
ctcaagccct cgcagctcaa ggctggggac ttcccaaggg tcccgtcacc ctccccaaca 6480
agctcaagtg ggaccagagg tgctggcagg cgggaagtgg ggacccgggg gcacagccaa 6540
acaccagagc ccgggctccg gaatgtggtc aaagaggggt caccgggggg aggggccggg 6600
tggggaggac ctggggaggg gcggggctca ggagggggca agaggtcggg gtccccagct 6660
tcggagactc ggggtcccta cccggctccc agccgtatgc tgagcgcccc ctccagccgg 6720
ggtctccgcc gcacagcggg gcagtcccac tccccttacc tggctcggct gctctgcctg 6780
ctccgaggac gccatctttc tgaggcctga ctaatcaacc gccgctgggc gaggtttctc 6840
ccaggcgccc tcgctctcgc cctcccgccc agactaaggt agccggaaca gagcctgtcg 6900
atgtagggga ctggggcaga ggggtggagc ccgggtgtcc gggtcacctt gcatagtttc 6960
cgttaagact ctgctttcca ggaagatccc aagtctatat cttctttgcg ttgttatatg 7020
tgtattattc tggttttgct tttttttttt gagacggagt ttcgcacttg ttgcctaggc 7080
tggagtgcaa tggcgcgata tcggctcacc gcaacctcag cctcccggtt tcaagtgatt 7140
ctcctgcctc agcctcccga gtagctggga ttgcaggcat gcgccaccat gcccggctaa 7200
cattgtattt ttagtagaga tggagtttct ccatgttggt caggctgctc tcgaactccc 7260
gacctcaggt gatctgcccg cctcggcctc ccaaagtgtt gggattacag aagtgagcca 7320
cagcgcccgg cccatattat tcttattgag ttaatctctg cccatcccca cagtgccatg 7380
ggcacattta gggacttgcc aatggatgaa tgcgaagaaa cttggaaatt aatttttaga 7440
catcagttca gttccattca ttgagtgcct actatatgct agggactttg gtaggccttg 7500
aaataaaaac attcaagact caatccctac tcccacgccc aggccaaaca cacttacaat 7560
tacagtatta tggggatagg actcggataa gtgtgcaggt ctattgggag aacataaaag 7620
ggggcatttt gaagagaaga ggcttctaca aaacgtcatt attttacact ggttgaattg 7680
ttagattttt ttaattgtta gattttaaat taaatattgg acgggagcag tggctcacac 7740
ctgtaatccc aacactttag gaggccaagg caggaggatc ccttgagccc aggaggttga 7800
ggctgcagtg aactatgatt atgccactgc actccagcct gggcgacaga gcgagacctt 7860
gtctaaaaat aagataagaa aataaaaaat gaaattatat gaaaaaggat acatacatta 7920
gagttaaccg ccagctccta ttatagttgt gttttgatca atatagactg cttaaaaggg 7980

atgacttaat gaataattcc tcctactaaa cgctgtttta aatgtctaaa cttttcttga 8040
gataatatcc aatttcctct cccacgtttt ttttgtttgt ttttgctttt gtttttgttt 8100
ttgcttgaga cgaagtcttg ctcctgacgc ccaggctgga gtgcaatggt gcgatctggg 8160
ccgactgcaa cctccgcctc ccgggttcaa gcgattcacc tgcctcagcc tcccaagagc 8220
tgggactaca ggcgtgcgcc atcacgcccg gctaattttt gtatttttag tagagatagg 8280
gtttcaccat gctggccagg ctggtctcaa actactgacc tcagatcatc tgtccgcctc 8340
ggcctcctaa agtgctgaga ttacaggggt gagccaccgc ccccggggcc cttgcccacg 8400
tttttataaa caaaatatga ggtgggagga ttgcttgagc ccaggagatc aaaactgcag 8460
tgagccatca tcgcgccact gcactccagc ctgggagaca gagcgaaacg ctgtctcaaa 8520
aaaagaaaaa agaaacaagg aaggaaagtg tcccccgaat gaataaatat gagcagataa 8580
ttacttctag tttacataaa atgccctctt ttaagagagg aactttttga tcatttttat 8640
tactattctt ttaatcttag atgtttattt ttcaggagga aaaaaacaaa cttttccata 8700
gtagcagtat taaaacaaag atgaatatgt tgtcaagagg catctgaaca atctccaaca 8760
gtgccgagtc gcatttcccc tttcctctcc gatgcatttg agactttaat gcttactata 8820
tttatttaag cgttttgttc tgagacccca aactaatttg cacagttgaa gcaggaaata 8880
gaatgggtgg aaacatgcgg gtgtctgtat tagcattatg catctcatat gcgaatagga 8940
atgctctaaa aattcaatac agtatacatg cgtgtgcatc gtcatgcaaa ataagtctct 9000
attggcctta tatttgaacg cgcgaatgca gctcctaaaa gttaaaaaca actcctaggc 9060
tgaatcgcgc gatagaacga aagcggcttt aaacccagag actacagttt ccggcatgct 9120
tcgacgccac acgtgattag cggcaactag aactacattt cccggcgtgc cctggtctca 9180
cgtgtgattt gctgcggaac gaactacatc tcccggcagg ctgcggaagg gggtcgagta 9240
gaaggaccgc cgctccggcc tcccgcgact tctcgaaggt gggcaggtcc caccttgtgg 9300
aggatggagg tgaccgggga cgccggggta ccagaatctg gcgagatccg gactctaaag 9360
ccgtgtctgc tgcgccgcaa ctacagccgc gaacagcacg gcgtggccgc ctcctgcctc 9420
gaagacctga ggagcaaggg ttggctcggg accccgggcg agggtgtggg ggagccaggg 9480
ccgggtgagg acgtccggcg ggggcgaggc ggggtcagga gaggcgactt ggggattctt 9540
gtatctcggg tggaggcttg ggacagaaac ggtccgagtg ggcatagcga tttacagatt 9600
taaggcgctt gagcgtaatt aatctcgtgt gagactccac ggtggtggga agacagccag 9660
gaagcaatgt gcaaattaag ccagactcgg cttaggtctc ttgcctgaaa tcacttcgat 9720
gttggaccaa acggacccca ctgggttcct gccactccgc cacactcaga tggtacaaga 9780
acccccagct gagcccacgg cggtgtgggg aggggtttcc accgtccgct ctggcaggaa 9840
cctatgcagg aaagagggag ctgctactga ccatttctgg aggtccagac aaggaacgtg 9900
agttgtaagt gacggagccg acggaagttc cgagcccggc cccatacctt taccagctgc 9960
ttggccttgc acttaaccag ctctcaaccc tcagttttct catcagcaaa ttgggtataa 10020
tatttatagc cctgcttcaa agggttgttg gttctctcaa ataagatgat atatttaagt 10080
gacttacaag tcttattagc cagtagcaac aaatcgctta cccaaagaag ttttacaggt 10140
tacatgtgtg agccagcccc aggcatgtag tcacagtgtg tgtcagaggg cacagttgct 10200
tcctctcagt caccaacaga aatcatatgg aaattttcag agacccatga acagccaaag 10260
cattataaat gctttggtaa catggatttt gcatataagc atttatgtat tttttttcct 10320
gacattagat tactttactt ctaattcccc ttcagtgccg tggctggatg tgcgtggcac 10380
agaggctggt gttctctgca gtccagcgca tacatgcctt gctgttctat tgggtggtag 10440
acaacaccgc tgaaggatat ttgtagtctc ttggctattt ctgatcactc cagtcccgtt 10500

agtatttgaa gcattgctat cactaaatgg aatagtacag acctgtcctg gagaaagcct 10560
ctccgccaat gataatgata tgaaaagcta ctgatagaag cttccacttc ttttatactt 10620
tcccataggt tagaaaagaa gctgctaagg aagatactgt aaagaacaaa aagcattttc 10680
tttttttttt tttttttttg agacggagtg tcactctgtc acccaggctg gagtgcagtg 10740
gcgctatctc ggctcactgc aagctccgcc tcccgggttc actccattct cctgcttcag 10800
cctcccgagt agctgggact acaggcgccc accaccacgc ccatctaatt ttttgtattt 10860
tttagcagag acggtttttc accatgttag ccaggatggt ctcgattgcc tgctttgtga 10920
tccacccgcc tcggcctccc aaagtgctgg gattacagac gtgagccacc gtgcctggcc 10980
tttttggtgt attttttgac atttttacac ataggagaat ttaagaggac aactgagggc 11040
cgggtgcggt ggctcacacc tgtaatccca gcactttggg aggccgaggt gggcggatca 11100
cgaggtcagg agatcgagac catcctggct aacacggtga aaccccgtct ctactaaaaa 11160
tacaaaaaaa ttagccgggc gtggtggtgg gcgcctgtaa tcccagctac tccggaggct 11220
gaagcaggag aatggcgtga acccgggggg cggagcttgc agtgagccca gatagcgcct 11280
gggcactcca gcctgggcga cagagtgagc tccgtctcaa aaaaaaaaaa aaaaaaaaga 11340
ggaaaactga ataaatgaat gagagactgc ttgtttgcaa ggaatcaaac tagactttga 11400
ttggttgttc actccagtaa aaactcaaat gcctaccata catgagtgtg gagtagctgg 11460
agtaaacata ctttcaggag ccagactttg tgggtttaaa tcccagcttt gccacttact 11520
gtgtgatctt agacaagtta cttaacctct ctgagccttg ggttcatcat ctataaaaca 11580
gaactaataa aagtacctac tttacaggat tattatgaga ttaaatactg tatatgaaaa 11640
gcacttaggc caggcgcggt tgctcatgcc tgtaatccta gcactttggg aggccgaggc 11700
aggtagatca ttaagtcggg agttccagac cagactggcc aagatggtga aaccccgtct 11760
ctactaaaaa tacaaaaatt agccaggcac ggtggccggt gcctgtagtc ccagctactc 11820
gggagactaa ggcagagagt tgcttgaacc cgggaggcgg aggttgctgt gaaccaagat 11880
tgtactactg cacgccagcc tgtctcaaaa aaaaaaaaaa agaaagaaag aaaagcactt 11940
agggctgagc acagtggctc atgcctctaa tcccagcact ttgggaggcc aaggcgggtg 12000
gatcatgagg tcaggagttc gagaccagcc tggtcaacat ggtgaaacca cgtctctact 12060
aaaaatacaa aaattagccg gacatggtgg tgcacgccta taatcccaac tactcgggag 12120
gctgaggcag gaaaatcact tgaacctggg aggcagaggt ttcagtgagc cgagatcgcg 12180
ccagtgcact gcagcctggg taacagagtg agaccctgct acaaaaaaaa agaaaagaac 12240
acttagaaca gaatcaggca cgtgatgagt gttctggtag tgctagaagt gatgattgtg 12300
attctagtta ttacctacca ggagttctgc tcagctctgg agccatacat acttgagatg 12360
cagggataga tgtaatttcc ttacggttat ttgtcatttg ccaaatattt tattcttttc 12420
agcctgtgac attctggcca ttgataagtc cctgacacca gtcaccctgg tcctggcaga 12480
ggatggcacc atagtggatg atgacgatta ctttctgtgt ctaccttcca atactaagtt 12540
tgtggcattg gctagtaatg agaaatgggc atacaacaat tcaggtaaga aactctagcc 12600
atgtataaaa cggcctatgc agtacagcat ccatcatgat tactttcact gcacagacaa 12660
ttgccatttg tctggctttt taagcatttg ttcaacagat atttactgag cactttctat 12720
gtgccagaca tggtgctgag tcctggggat aaagatgaat gaggccaggc acggtgggca 12780
gatcacctga gctcaggagt ttgagaccag cctggccaac atggcgaaag tccatctcta 12840
ctgaaaatac aaaaactagc tgagcatggt ggcaggcacc tgtaatccca gatacttggg 12900
aggctgaggc gggagaattg cttgaacctg ggaggtggag gttgcagtga gctgagattg 12960
tgccactgca ctccagcctg ggcgacagaa cgagactctg tctcaaaaaa aaaaaaaaat 13020

agatgaatga gacatgggcc cagttctcaa ctaactcatg gacagacagc aactgaaatg 13080
cagcctggca gggagctcag tgctgcaggc tgcagtccag ctgttgaggt ctggatcctg 13140
gctctaccat ttactaacca ggtgaccttg tgcaaattac ataacttctc agtgccttgt 13200
tgacataata attatagaac ctgtccccta agattgttgt aaagataata tagtacatat 13260
caaaccaggg tttctcaatg acactgctga catttggggc tggagttgtg aagaggatgc 13320
tttgtgtatg ataggacgtt tagtagcatc ctagatgtta cgaacctccc cccacaactg 13380
tgataaccaa gaataaatga ctccagatat tgtcagatgt ctcctggggg gcaaaattgc 13440
tcccacttga gaaccactga tatgaagtgt ttagatctta cctggcacat agttaaggac 13500
tcaataattg ttacctgtcc ctgccatcat catcatcaca ggagatagaa aggggactaa 13560
cattggtgac caaggatcaa gaagtgtctt gagaggggat tagttttagg gagggactta 13620
aaaaaaaaaa aagtgtcctg aagtgtgcca caaaattgag gaagtcatcc tacagcagtg 13680
gccctaccag ccacctttgt gctcaggcct aagggacagc aatcgatggc tacagctctg 13740
ccagttaaag aagggaccaa tccccaagca cagagaaatg ctggcacaaa tgaccgtttt 13800
cgtataaaaa acaggacaca cctggacaca cagaaaccag aaagggaagt gtgattggat 13860
tccttgtagg tgtgaccaca cattgggttt aaatggcttt ttctatggaa gaatacagag 13920
ccgttgcaca cagagataaa gatgaaagag ggtcaggcac ggtggcttat gcctgtaatc 13980
ccagcacttt gggagaccga ggtgggcaga tcacttgaag tcaggagttt gagaccagcc 14040
tggccaacat ggtgaaagcc cgtctctact aaaaatacca aaattagctg gtcatggtgg 14100
tgggcacctg taatcccagc tacttgcaag gctgaggcag gagaatcgct tgaactcggg 14160
cagtggaggt tgcagtgagc caagatcgtg ccactgcact ccagcctggg caacagagtg 14220
agactctgtc tcaaaaaaaa aaaaaatgaa agaagaggga aaatcaaggt cctggatgga 14280
tattgaccct cctgtgaatc cttgagataa gatgcttctt tcccattgaa attaatctag 14340
atcctcaaga ggtctgtgtg tgctggggga ggaggtgaaa acagcacttc aggttgttct 14400
gtgactggca ttttctccca tgtgtcagat ggaggtacag cttggatttc ccaagagtcc 14460
tttgatgtag atgaaacaga cagcggggca gggttgaagt ggaagaatgt ggccaggcag 14520
ctgaaagaag atctgtccag catcatcctc ctatcagagg aggacctcca ggtaaagcct 14580
cctccacaac ctgcaacaat gttcatccag cctctgcccc ccgccccagc accttcttag 14640
cttccatact tgccatagca atttctttgt tttaatctta atttttaaat aattcttaaa 14700
ttacaactaa gatttttcat cttctcattc tcacctagag taatttttca accctcgtca 14760
cgaagcagaa ttacctgtgg attttttttt tttttttttt tttttttgag acacagtctt 14820
actctgtcgc ccaggctgga gtgcagtggt gcaatctcag ctcactgcag tctccacctc 14880
ccgggttcaa gcaattctcc tgtctcagcg tcccaggtag ctggaattac aggtgcacac 14940
caacacgcct ggctaatttt ggtattttta gtagagacag cgtttcatca tgttggccag 15000
gctgctcctg agttcaagtg atccacctgc ctcagcctcc caaagtgctg agattatagg 15060
cgtgagccac cgtgcccggc cagatttttt ttttaataca aatgcctggg atccacccta 15120
aacctagtta ggtggacgct ggggtcaggg atgccctgct agagaatgta gcgttggagt 15180
agatggcttc acatcccagc gctgtcacca cgactttgag ccagtagctt agcttctctc 15240
cttgtgtttt ctctctagta agattgggat aggccaggtg cagtggctca cgcctgtaat 15300
ctcagcactt tgggaggccg aggcgggcag atcacgaggt caggagatcg agaccatcct 15360
ggctaacatg gtaaaacccc atctctacta aaaatacaaa aaattagccg ggcctggtgg 15420
tgtgcacctg tacctgtagt cccagctact cgggatgctg aggcagaaga atcgcctgaa 15480
cccaggaggt ggagcttgca gtgagccgag atcgcaccac tgcactccag cctgggtgac 15540

agagagagac tccatctcaa aaagaaaaaa aagattggga taaaaacagt atgttttcat 15600
tcaaagaaaa tttctctttg agacagggtc ttgctttgtc acccagtgca gtggcatgat 15660
catagctcac tgcatcctcg acttcctggg ctcaagccat cctcccacct caacctctca 15720
ggtagctgag actgtaggct tgcaccacca cactgcgcta tttttctttt tttttttttt 15780
ttaatttttg taggccaggt gcagtggctt atacctgtaa tcctagcatt ttgggaggcc 15840
aaggtgagca gattgcctga gttcaggagt ttgatgtggt aaaaccctat ctctacaaaa 15900
attagctagg tgtggtggcg tgtgcctgta gttccagcta ctcaggaaac tgaggcagaa 15960
ggatcacttg agcccaggtg gtcaaggctt cagtgagcca tgactatacc acaccaacct 16020
gggcaacaaa gcaagaccct gtctcaaaaa taaataaata aataaataaa gccaggcgtg 16080
gtggctcaca cctataatcc tagaactttg ggaggccggg gcgggcagat cacgatgtca 16140
agagatcaag accatcctgg caaacatggt gaaaccccgt ctttactaaa aatacagccg 16200
ggcacggtgg cttaacgcct gtaatcccag cactttggga ggctgaggag ggtggatcac 16260
aaggtcaaga gttcaagacc agcttggcca agatggtgaa atcccgtctc tactaaaaat 16320
ataaaaatta gccaggcgtg gcggcaagca cctgtaatcc cagctactca ggaggctgag 16380
gcaggagaat cacttgacct gggaggggga ggtagcagtg agcagagatc acgccactgc 16440
attttagcct gggagacaga gcaagactct gtctcaaaaa acaaaaaaat tacaaaaatt 16500
agctgggcgt gatggcgtgt gcctgtagtc ccagctactc aggatgctga ggcaggagaa 16560
tagcttgaac ccaggaggcg gaggttgcgg tgagccgaga tcacgtcatt gcactccaac 16620
ctggtgacag agcaagactc agtctcaaaa aaaataatta attaatgttt tatagagagg 16680
aggtcttcac aggttgccta ggctggtctt gaactcctgg gctcaggcag tcctcccacc 16740
ctggcctccc aaagtgctgg gattattgat atgagcctct gtgcttgacc caaaacatat 16800
ttattgagcc ccatcctgtg cctgtcatgg ttctatgcac tgggtgtgca gtggcgaaca 16860
agccagacac agtctctgct ctaacggtac ttgtattctg atgaagggag gcagaaagca 16920
acaggaaagc atatcagaca gtggtatgca gagagtgatg gaggctactc tagactggaa 16980
cacggaggaa ggcctctttg aggaaatgat ttcttggtgt tttttgtttt ggtttggttt 17040
ggtttgagac agggtctagc tctgtcaccc aggctggagt gcagtagtgc aatctcagct 17100
cactgcacca tctgcctgct gggctcaagc gatcctccca cctcagcctt ccaagtagct 17160
gggaccacag gcgtactcca ccatgcctgg tgaattttta tatttttggt aaagacaggg 17220
tttcaccatg ttgcccaggc tggtcttaaa ctcctgagct caagcagtcc tcctacctca 17280
gcctcccaga gtgctgggat tataggtgta agccactaca cccgaccctt cttcccaatt 17340
ttaacactaa aagaaaatta gaaaaattgg ccgggcacgg tggctcacac ctgtaatccc 17400
agcactttgg gaggccgagg cgggtggatc acaagatcag gagatcgaga ccatcctggc 17460
taacacggtg aaaccccgtc tctactaaaa atacaaaaaa ttagccaagc gtggtggcag 17520
gcacctgtag taccagctgc tcaggaggct gaggcaggag aatggcgtga acccaggagg 17580
cggagcttgc agtgagccga gatcgtgcca ctgcactcca gcctgggtga cagagcgaga 17640
ctctgtctca aaaaaaaaaa aaattagaaa aattacatgc ttttctcttg actgtctgcc 17700
aactatcata gtaggtaact ttgggaaatg gatgcattga gttctgaaat ggatgtattt 17760
gagaggtaga acaccgtgtt tagaaagtcc ttgtaggccg ggcgcagtag ctcacacctg 17820
taatcccagc actttgggag gccaaggcgg gtggatcacc tgaggtcagg agttcaagac 17880
cagcctggcc aacatgacaa aaccccgtct ctactaaaaa tacaaaaatt agctgggctt 17940
ggtagcacat gcctgtaatc ccagctacta gggaggctga ggcaggaaaa ttgcttgaac 18000
ccaggaggcg gaggttgcag tgagccgaga tcacaccatc gcactccagc ctgggcaaca 18060

gagcagactc cgtctcaaaa aaaaaaaaga aagtcagaaa gtccctgtag atttctaagc 18120
gtctgtgttt gtttgttaga tgcttgttga cgctcctgct cagacctggc tcaggaacta 18180
cgtcagagtt gtgccaccgt ccagcggctg cagcacacac tccaacaggt gcttgaccaa 18240
agagaggaag tgcgtcagtc caagcagctc ctgcagctgt acctccaggc tttggagaaa 18300
gagggcagcc tcttgtcaaa gcaggaaggt aggactctct gcaggcagag tcactctgag 18360
aattcagggc tgatggaggc gtcccttggg aggacactat ttgtgatgta gccagggcca 18420
ctgaagccgc ggctgctggg atgcgggacg ggcacgtttc tctcttgccc ggccagttat 18480
tctggcgagt tgttcatgtg gctgtgtggt cacaccacgt ctgatctctg tctcgtgtct 18540
cctttgcaga gtccaaagct gcctttggtg aggaggtgga tgcagtagac acgggtatca 18600
gcagagagac ctcctcggac gttgcgctgg cgagccacat ccttactgca ctgagggaga 18660
agcaggctcc agagctgagc ttatctagtc aggatttgga ggtgggcgga aaccagggtc 18720
actgagctac agaggaggac atgccctggg atgtagtagt atcatgcaga ggtgtgtggg 18780
cccttttgtt cacctctgca gactgtgaat cctagctgcc agtttgccta ttatatgcca 18840
aggcatttgc aaaaatctca ttaatctaaa tcaaaatagc tttaaagaaa aatgcataca 18900
cttcctcaga tcatcaaaca gactctggtc caaggttggt aatgaaatga ctgttcctga 18960
cagggaggaa tagcagggcc caatcttctg agatggcttc tgggtctttc catggtcaga 19020
gaagatctat agtccgtcct gaggtctgtc aatgtcacag gaaaaggcaa acttgagggg 19080
atcgtcgcct gctggctaag accagggagc taaaaacttg aggaagggaa cctgcctggg 19140
tgggtgctac ttctgattca ttgtccttgt ccctgtcata agtacctccc tattgtagat 19200
agaagggaag gaaactgttg acttgagctt ggctaaattt cctgaaggtg gaggacctta 19260
gagttgctcc aagtaaactt tccaaggact tcttcctgtt ggtgttgtaa gaaaaggccc 19320
atgcttgtcc gggcacagtg gcttacgcct gttaatgcca gtactttggg aggccgaggc 19380
aggtggatca cctgaggtca ggagttcgag accagcctga ccaacatggc gaaaccccat 19440
ctctactaaa aatacaaaaa ttagcctggc atggtggtgg gaggctgaag caagagaatc 19500
acttgaaccc aggaggtgga ggttgcgtga gctaagatcg cgccactgca ctccagcctg 19560
ggcgacaaga gtgaaactcc gtcttaaaaa agcccatggc aggctgggcg cggtggctca 19620
cgcctgtaat cccagcactt tgggaggcca aggtgggcgg atcacaaggt caggagatcg 19680
agaccatcct ggcgaacacg gtgaaacccc gtctctacta aaaaaaatac aaaaaaatta 19740
gccaggcgtg gtcgtgggca cctgtagtcc cagctactca gggggctgag gcaggagaat 19800
ggcgtgaatc cgggaggcgg agcttgcagg gagccgagat agtgtcactg cactccagcc 19860
tgggtgacag agcgagactc cgtctcaaaa aacaaaaagc ccgtggcaat taatggtaaa 19920
ggaaacccgg catttcagtg taaagaggta acataaacgg atactgcctc attgcactcc 19980
ccaaaagaga gtcaggagag cagcaggaga ggcgcacaga aggcccagtt ctcaggggtt 20040
tttattcttg ctgcagttgg ttaccaagga agaccccaaa gcactggctg ttgccttgaa 20100
ctgggacata aagaagacgg agactgttca ggaggcctgt gagcgggagc tcgccctgcg 20160
cctgcagcag acgcagagct tgcattctct ccggagcatc tcagcaagca aggcctcacc 20220
acctggtgac ctgcagaatc ctaagcgagc cagacaggat cccacatagc agcagcggga 20280
agtgtgccaa ggaagctctg tggcgttgtg ttattggtag acaccctcag cctcatcatt 20340
tgactaccta tgtactactc taccccctgc cttagagcac cttccagaga agctattcca 20400
ggtctcaaca tacgccgttc caccaatttt ttttttagcc ccaccagctt caggacttct 20460
gccaattttg aatgatatag ctgcaccaac aatatcccgc ctcctctaat tacatatgat 20520
gttctctgtt caaaagtaat tggcagtgat tggccaggcg cagtggctca cgcctgtaat 20580

cccagcactg ggaggccgag gggggcggat cgtgaagtca ggagatcgag accatcctgg 20640
ctaacatggt gaaaccctgt ctctactaaa aatacaaaaa aaattagcca gccatggtgg 20700
cgggcgcctg taatcccagc tacttgggag gctgaggcag gagaatggca tgaacctggg 20760
aggcagagct tgcagtgagc tgagattgcg ccactgcact ccagcctggg caacagagcg 20820
agactccgtc tcaaaaaaaa aaaaaaaaaa agtaattggc agtgactatg ggcgcactgc 20880
ctaacattta gccctgcccc atatggaaca tgttaaaaaa aaaaaaagcc aggccgagcg 20940
tgttggctca cgcttgtaat cccagcactt tgggaggccg aggcgggtgg atcacgaggt 21000
caggagatcg agaccatcct ggctaacaca gtgaaacgtg tttttactaa aagtacaaaa 21060
aactagctgg gcgtggtggc aggagcctgt agtcccagct actcgggagg ctgaggcagg 21120
agaattgctt gaacccggga ggcagaagtt gcagtgagcc gagattatgc cactgtactc 21180
cagcctgggt gacagagtga cactctgtct caaaaaaaaa aaaaaaaagg ataggcacga 21240
cagtggctca cacctttgat cccagcactt tggaggccga tgcaggcgga tcacatgagg 21300
tcaggagttc aagaccaacc tggccaatat ggtgaaaccc cgtctctact aaaaatacaa 21360
aaattagctg ggcatgatgg cgggtgcctg taatcccagc tactcggtag gcagaggttg 21420
cagtgagctg agagcgcacc actgcactcc agcctgggtg acagagctag actccgtatc 21480
tcaagaaaaa aaaaaagttt tgttttgttt ttttgagaca gagtctcact ctgtcaccta 21540
gtctggagtg cagtggcaca ttctcggctc actgcaaccc ccacctccca ggttcaagcg 21600
attctcctgt ttcagcctcc tgagcagctg ggattacagg cacgtgccac catgcccggc 21660
taatttttgt atttttagta gagacagggt tttgtcacat tggccaggct ggtctcaaac 21720
tcctgacctc aggtgatcca cccacctcag cctcccaaag tgctgggatt acaggtgtga 21780
gccaccaccc ctggccagaa aaaaaagctt ttaaataaag taattgtcag cttaggcaac 21840
atagcaagac cctgtctcta caaaaaaaaa atttaagtta gccaggcatg gtggcactga 21900
cctataatcc tagctactct ggaggctaag gcgagagtat cacttgagcc caggagctca 21960
aggctgatca tgccaccata ctccagcctg gatgatagag taagacccta cttttttttt 22020
ttttttttta aaaaggtaac catcaagggc aatgaaaaga aatggtcgta tttccaattc 22080
attatcctta ttctgaagat agtacggcag aattttaagc agagatagtg gtgaagatgg 22140
tgacattata gagtgttgac tatggccaca atcttgtttc tccagttaat gaattttttt 22200
atgtgtttgt ttaaagatac atattaagct tgtagaccat agggacatac ggagagtcca 22260
ttgctaatat ctcactcagt attgtgaaat tctatctcac caccgtgaaa ctcttcagtt 22320
ttctaattgc tttatcagca gggggtataa aaggtcatga aagcaatttc cacatgctgt 22380
ggctccaggt ctctgggtgt gaagcagagc aagcctggtt tgtcctcctc ctgtctccac 22440
acagacggct tctgcaggtt tggtaatcta cagtacactc cttgcaggga aaaggtgatg 22500
agtcatcatg gacttatttg accacttttt atgcatgctt agaggaaaac agaatactgt 22560
taagagattc atctgctagt tattaagtaa agaaatatca caataggccg ggcgcagtgg 22620
ctcacacctg taatcccagc attttgggag gccaaggtgg gcggatcacc tgaggtcagg 22680
agttcgagac cagcctagcc aacatggtga aaccccgtct ctactgaaat tataaaaaat 22740
tagccgggtg tagtggtaca cgcctgtagt cccagttact tgggaggctg aggcatgaga 22800
attgcttgaa cccaggaagt ggaggtggag gtgagccgaa attgtgccac tgtactccag 22860
cctgcaacag agtgagacac tgtcacacaa aaaagaaaga aatatcacaa tatgtcacaa 22920
taggccgggc gcagtggctc acacctgcag tcccagcact ttgggaggcc aaggcagatg 22980
gatcacctga ggtcaggagt ttgagaccag cctggccaac gtgacaaaac ccagtctact 23040
aaaaatacaa aaattagcca ggcgtgatgg tgggcacctg taatcccagc tactcaggag 23100

gctgagacat gagaatcgct tgaacccagg aggtggagat tgcactgagc tgagatcctg 23160
ccactgggct ccagcctggg tgacagaatg agactctgtc tttaaaaaaa aaaaaaaaag 23220
aaaatcacaa taagtcctag g 23241

<210> 85
<211> 23241
<212> DNA
<213> Homo sapiens

<400> 85
   ataataaact gtcactgttc tcatcatgaa tgaatctcaa aaatatgtgt taagtaaaag 60
   aagcctggga gactgaggtg ggaggctcac ttgaggtcag gagttcaaga ccagcctggg 120
   cgacacagcg agacccgacc tctacaaaaa ataattagct gggcatcata gtgcatgact 180
   atagtcccag ctactcggga ggctgaggct ggaggatcac ttgaacccag gaggtcaagg 240
   ctacagtgag ctgtgataac acctctccac tccagactgg gtgacagagt aagaccctgt 300
   ctcaaaaaac aaacaaacaa acaaacaaag tttaaggctg gacgcggtgg ctcccagcac 360
   tgtgggaggc cgaggtgggt ggatcacttg aggtcagggg ttcgagacca tcccggccaa 420
   catggcaaaa ccctgtctct attaaaaata caaaaaaatt agccaggcat ggtggcgggc 480
   acctgtaatt ccagctactt gggaagctga gacaggagaa ttgcttgaac ccaggaagcg 540
   gaggttgtag tgagccaaga ttgcaccact gcactccagc ctgggcaaca gagcaagact 600
   ctgtctcaaa caaaaaaaaa aaggaaggaa aggaaaaaaa aaaaagacta aagagacaaa 660
   tcaatgcaat ctctgtttct tcactagatt ctgatttttt ttaagtgtga aaatttgagt 720
   gcagattctg ttttaaataa tactatttat caacacttaa atttcttgtg tgataatgat 780
   attgtggtta tacaggggaa taaccttact cttatgagag ataaggtgaa atattctagg 840
   atgaaatatc cttgatgtct gtaacttacc acaaatcctt ccaaaaaaaa aaatatatat 900
   atagagagag agagagagag aaagagagag agagactgac ataaagcaaa ttcagcaaaa 960
   tgtgaacagt gcatcttggt taagttttta tgggaatttt acagaagttc tcagtattat 1020
   cctttcaact tttctatacg tgagaaattt ttcaaaataa aaagttgagg ccaggtgcca 1080
   ggctcacacc tgtaatccca acactgtggg aggccgaggc aggtggatca cctgaggtca 1140
   agcattcaag actagcctgg ccaacatggc aaaaccccat ctctactaaa aatacaaaaa 1200
   aattagctgg gcatggtggc gggcacctgt agtcccagct actcaggagg ctgagacagg 1260
   agaatcgctt gaacccagga ggcagaggtt gcagtgagcc gagattgtgc cactgtactc 1320
   cagatcggga gacagagcaa gactccgtct caaaaaaaaa aaaaaaaaaa aaaaagttag 1380
   ccaggcgtag tggcacacac ctgtagtctc agctactcag gaggctgagg caggtgaagc 1440
   aggagaaccg cttgaacctg ggaggctgag gttgcagtga gccgagattg agccactgta 1500
   ctccagcctg ggcaacagag ggagactcca tctcaaaaac aaaacaaaac aaaaaaaaaa 1560
   aacattgaga gggcaaatac cttgtctgac ctgctgttat tcaagagttc aacagactct 1620
   caaaactaca tgtaaagact gggagaagaa aactaactct gagcacttac tatgcagcag 1680
   gtgctcttca catctgtgat ttcagtcaac actaaaaggg taattatttt ttattttatt 1740
   tatttatttt ttaagtgata gggttgcact ctgttgccca ggctggagta caggggttta 1800
   ttcacatatg tgatcatcgt gcactacaga ctccaactcc tgggctcaag caatcctcca 1860
   gcctcagtct cctgagtagc tggaactaca gacatgcacc accacaccca gctttaaggg 1920
   taactattat tgctctcgtt ttacagagga gaaaacatac tcagaaaggt ggagtgataa 1980
   gtccaatagt tgtgagtgat gaagttccac caaaacctgc caggatgcga agccatgctc 2040
   ttccactctt tcactctgct cttctccctg cctgcttctg tagcctctca atgcaaaatt 2100
   tactgtaccc agtgaacctc cacagatagg aaatctacct tctgcaataa tactcgatac 2160
   tcagtaactg tgaagtaatc agattgaacc atgcctctgc tgttcagcga aggaagaaaa 2220
   gctagtagga aagttgggaa atagagataa cgtttctcat tcctacgggc tccaacgtgt 2280
   acacactaga aaggacttca atgagagatc agccatgctg gctgggcgtg gtggctcatg 2340
   cttataatct cagcactttc tctctctctg tctcttcccc cgtccccaaa cagggtattg 2400
   ctctctcacc caggctggag ggcaatggca caatcaccgc tcactgcagc ctcaacctcc 2460
   caggcccaag tgatgctctc acctcagcct ccaaagtagc tggaaccaca ggcaagcact 2520
   accatgcctg gctaattttt taattttcag taggaacgag gtctccctat attgcaaagg 2580
   ctggtctcaa actcctggcc tcaagcgatc ctcccaactc agcctcccaa agtgctaaga 2640
   ttacaggtat gagccatcac gcccagccag gaactcacca ctacaaaaat tgttttaaaa 2700
   atacattagc caggccaggc acagtggctc acacctgtaa tcccagcact ttgggaggcc 2760
   gaggtgggcg gatcacgagg tcaggagatc gagaccatcc tggctaatac ggtgaaacat 2820
   cgtctctact aaaaatgaaa taaaaaaaaa atagccgggc gtggtggcag gtgcctgtag 2880
   tcccagctac tctggaggct gaggcaggag aatggttcga acccgagagg cggagcttgc 2940
   agtgagttga gatcgcacca ctgcactcca gcctgggcga cagagcgaga ctctgtctca 3000
   aaaaaaaaaa aaatacatta gccaggcatg gtggtgcaca cctgtagtcc cggttactca 3060
   ggaggctgag acgaaaggat tgcctgagcc tgggcgggca aggatgcagt gagccatgat 3120
   cacaccaccg cactctggtg acagagtgag accctgtctc aatcaataga tcaaccacat 3180
   aaaacagagc aatgtgggga acacagaaca aaggaagcaa taaatgaaat tccagcaagt 3240
   cgtcttgagg atagactaaa aagactatac cattatgaaa aagtaagtat gttcctgaaa 3300
   tgcaaaagtc atgaaggaca gctagaataa acactagcat tcagggcatt ccttgaaatt 3360
   ttaaggaatt acttctgaat aaaagaactt tactagacaa gtagtcaaca catgaaaaat 3420
   cataccccaa aagatgtcgt aatagaactt gaagaaaggt ttagatccat ttgtgagtga 3480
   aaactcagag cttattaaat tgaataataa taataataga caacacattg tttcaggcac 3540
   tgttctggca tttttacata aataactcat ttaaggccag gtgcagtggc tcacgcctgt 3600
   aataccaagc tctttgggag gccgaggtgg gcaggttacc tgaggtcagg agtttgagac 3660
   cagcttgtcc aatatggtga aaccccattt ctacttaaaa aaaaaaatta gccaggcctg 3720
   gtggtgcata cctgtagccc cagctacttg ggaggctgag gcaggagaat cacttgaacc 3780
   tgggaggcgg aggttgcagt aagccgagat cgcgccactg cactccagcc tggtcaacag 3840
   agtgagactc ggtctcaaaa ataaataaat aaataaataa ctcatttagt cctcagagca 3900
   actctgtgag ataggtccta gtattttccc tgttttgcac atggagaaat taagtctaga 3960
   aaaattaagt agatctgtcc gagatgacat tgctggtaag ttagtggagt taggattcca 4020
   agccagtggg tctggcttta gagcctatac ttgtaaccgc tattctacac tgcctctcaa 4080
   gggaagagaa ggttattcac ctttaatagc tggaaaccag agattgatat gacaaggcta 4140
   accaacaaac tttctgtgaa agacagaata ctggctcagc cccacagttc taaccctaca 4200
   caacaaggcg tgggggaatt agctaaacaa tcctttcaat cctactctgc tttaatcgga 4260
   gaaatgggaa actggattga tcaagacttg aaaatgaaac aggcaattgc tgaagagcat 4320
   tggttcattc aaaaagcgtg catttgcttc ttgttagata gccatagagt ctgttatgaa 4380
   aagttcaagt tttggccagg cactgcagct cacgcctgta atcccagcac tttgggaggc 4440
   tgaggtgggc ggatcatgag gtcaggagat caagaccatc ctggctaaca cggtgaaacc 4500
   ccatctctac taaaaacaca aaaaattagc tgggcatggt ggcgggcgcc tgtagtccca 4560
   gctactaggg aggctgaggc aggagaatgg cgtgaaccca ggaggcggag gttgcagtga 4620
   gcagagatcg cgccactgca ctccagcctg ggtgacacag cgagactctg tctcaaaaaa 4680
   aaaaaaagaa aagttcaagt ttttccccca ggctgccagc atgttctgtg gcaatcctac 4740
   ctccaccagc tttggaaagg agggcaaagg agaagcagag ctgaaaatca aacaagtgat 4800
   ggccatgccc ccttaaagga ccttagatcc ttttctatta aatacatata cagagagggc 4860
   aaacttgcaa aaccaaaaga atccttgggc ataacagtgg ctccaagtgg caagattttt 4920
   agtactgttt acagcactgg caaattaaat gtcctataag aatcaggaac agagccgggc 4980
   acggtggctc acgcatataa tctcagcact ttgggaggct gaggcgggca gatcacttga 5040
   agtcaggagt ttaagaccag cctggccaac atggtgaaac ctatcgctac taaaattaca 5100
   aaaattagtc aggtgtggtg gcaggcaccc gtagtcccag ctacttggga ggctgaggca 5160
   ggagaattgc tcgaacccgg gaggcagtga gccgagattg cgccaatgca ctccagcatg 5220
   ggcgacagag agagactccg tcttaagaaa gaaaaaagaa tcaggaactg aggacaccct 5280
   tcaatcccag cctgtgggca ttcctaacac aaaacaattc agagaaaccc agaccccaag 5340
   atgccttagt atttcagcaa actaaatgtg gtaaggcaag aaaggagagg ccctcctctc 5400
   gagacacttg ccaaactcca gaacacctga cagtgagaag gccagaatca gtccttgtta 5460
   tctcccatgt tttacaatca agacatcttc ttcctgaatc attcacactg ctaatagaaa 5520
   ctggtatggg ggaacacacc aactccttta agttcaattc ttgtaacaga cgtgcagaat 5580
   aagacagtaa gaaacactaa gcggtggtct tctgcctctg aaggaagtga aatactagta 5640
   agttgctgaa aactcttaga ggtgcttcaa ggagatggga gaggaggaat gaggccccaa 5700
   gttgaaacta ttttcttctt cctcattcta atcctaacta aactcaaaaa tgtatggatg 5760
   actagtggag tcacacaggt tcaatacagg ctcaacagag cacacgattt ggggcatgtc 5820
   acaaaccctt agtattcatt tctgtgaaat ggaggttagt agcctgcact tcataaaggt 5880
   tgtagtgaac actaaacgag ataatagatg taatttgtgc tcaacatgac accacacagg 5940
   cagttaaatg taagatgtct cttagccctt ttctcctcac cccaccctac cccacctttg 6000
   gactataaac cccttcggag accttatcat ccactgcctc ccggccgcag aggcacaggc 6060
   gtcaacgctg gtccttatgg tccccttatt ggagtgggat ttgtctgaat ctgcaggtga 6120
   aaagctagag cggagccctc ggccaacaaa accaaaccca gggaagcgag gatgggtcct 6180
   cagccaaaag cttggtggcg tctgttagtc acagtgcgtc cctttcctat caacgcactg 6240
   agagggctcg actagattat ttctgactat ccaacttgac gagagaagcg caaaggtgac 6300
   cacagatgaa gaggctggca gccgctccag gaggctgagg ccgtgaggga gtgggaaccc 6360
   taatggaaac aggcccctcc ccggagggac agccgggaac gatcgatcta tcctctcctt 6420
   ctcaagccct cgcagctcaa ggctggggac ttcccaaggg tcccgtcacc ctccccaaca 6480
   agctcaagtg ggaccagagg tgctggcagg cgggaagtgg ggacccgggg gcacagccaa 6540
   acaccagagc ccgggctccg gaatgtggtc aaagaggggt caccgggggg aggggccggg 6600
   tggggaggac ctggggaggg gcggggctca ggagggggca agaggtcggg gtccccagct 6660
   tcggagactc ggggtcccta cccggctccc agccgtatgc tgagcgcccc ctccagccgg 6720
   ggtctccgcc gcacagcggg gcagtcccac tccccttacc tggctcggct gctctgcctg 6780
   ctccgaggac gccatctttc tgaggcctga ctaatcaacc gccgctgggc gaggtttctc 6840
   ccaggcgccc tcgctctcgc cctcccgccc agactaaggt agccggaaca gagcctgtcg 6900
   atgtagggga ctggggcaga ggggtggagc ccgggtgtcc gggtcacctt gcatagtttc 6960
   cgttaagact ctgctttcca ggaagatccc aagtctatat cttctttgcg ttgttatatg 7020
   tgtattattc tggttttgct tttttttttt gagacggagt ttcgcacttg ttgcctaggc 7080
   tggagtgcaa tggcgcgata tcggctcacc gcaacctcag cctcccggtt tcaagtgatt 7140
   ctcctgcctc agcctcccga gtagctggga ttgcaggcat gcgccaccat gcccggctaa 7200
   cattgtattt ttagtagaga tggagtttct ccatgttggt caggctgctc tcgaactccc 7260
   gacctcaggt gatctgcccg cctcggcctc ccaaagtgtt gggattacag aagtgagcca 7320
   cagcgcccgg cccatattat tcttattgag ttaatctctg cccatcccca cagtgccatg 7380
   ggcacattta gggacttgcc aatggatgaa tgcgaagaaa cttggaaatt aatttttaga 7440
   catcagttca gttccattca ttgagtgcct actatatgct agggactttg gtaggccttg 7500
   aaataaaaac attcaagact caatccctac tcccacgccc aggccaaaca cacttacaat 7560
   tacagtatta tggggatagg actcggataa gtgtgcaggt ctattgggag aacataaaag 7620
   ggggcatttt gaagagaaga ggcttctaca aaacgtcatt attttacact ggttgaattg 7680
   ttagattttt ttaattgtta gattttaaat taaatattgg acgggagcag tggctcacac 7740
   ctgtaatccc aacactttag gaggccaagg caggaggatc ccttgagccc aggaggttga 7800
   ggctgcagtg aactatgatt atgccactgc actccagcct gggcgacaga gcgagacctt 7860
   gtctaaaaat aagataagaa aataaaaaat gaaattatat gaaaaaggat acatacatta 7920
   gagttaaccg ccagctccta ttatagttgt gttttgatca atatagactg cttaaaaggg 7980
   atgacttaat gaataattcc tcctactaaa cgctgtttta aatgtctaaa cttttcttga 8040
   gataatatcc aatttcctct cccacgtttt ttttgtttgt ttttgctttt gtttttgttt 8100
   ttgcttgaga cgaagtcttg ctcctgacgc ccaggctgga gtgcaatggt gcgatctggg 8160
   ccgactgcaa cctccgcctc ccgggttcaa gcgattcacc tgcctcagcc tcccaagagc 8220
   tgggactaca ggcgtgcgcc atcacgcccg gctaattttt gtatttttag tagagatagg 8280
   gtttcaccat gctggccagg ctggtctcaa actactgacc tcagatcatc tgtccgcctc 8340
   ggcctcctaa agtgctgaga ttacaggggt gagccaccgc ccccggggcc cttgcccacg 8400
   tttttataaa caaaatatga ggtgggagga ttgcttgagc ccaggagatc aaaactgcag 8460
   tgagccatca tcgcgccact gcactccagc ctgggagaca gagcgaaacg ctgtctcaaa 8520
   aaaagaaaaa agaaacaagg aaggaaagtg tcccccgaat gaataaatat gagcagataa 8580
   ttacttctag tttacataaa atgccctctt ttaagagagg aacttttgga tcatttttat 8640
   tactattctt ttaatcttag atgtttattt ttcaggagga aaaaaacaaa cttttccata 8700
   gtagcagtat taaaacaaag atgaatatgt tgtcaagagg catctgaaca atctccaaca 8760
   gtgccgagtc gcatttcccc tttcctctcc gatgcatttg agactttaat gcttactata 8820
   tttatttaag cgttttgttc tgagacccca aactaatttg cacagttgaa gcaggaaata 8880
   gaatgggtgg aaacatgcgg gtgtctgtat tagcattatg catctcatat gcgaatagga 8940
   atgctctaaa aattcaatac agtatacatg cgtgtgcatc gtcatgcaaa ataagtctct 9000
   attggcctta tatttgaacg cgcgaatgca gctcctaaaa gttaaaaaca actcctaggc 9060
   tgaatcgcgc gatagaacga aagcggcttt aaacccagag actacagttt ccggcatgct 9120
   tcgacgccac acgtgattag cggcaactag aactacattt cccggcgtgc cctggtctca 9180
   cgtgtgattt gctgcggaac gaactacatc tcccggcagg ctgcggaagg gggtcgagta 9240
   gaaggaccgc cgctccggcc tcccgcgact tctcgaaggt gggcaggtcc caccttgtgg 9300
   aggatggagg tgaccgggga cgccggggta ccagaatctg gcgagatccg gactctaaag 9360
   ccgtgtctgc tgcgccgcaa ctacagccgc gaacagcacg gcgtggccgc ctcctgcctc 9420
   gaagacctga ggagcaaggg ttggctcggg accccgggcg agggtgtggg ggagccaggg 9480
   ccgggtgagg acgtccggcg ggggcgaggc ggggtcagga gaggcgactt ggggattctt 9540
   gtatctcggg tggaggcttg ggacagaaac ggtccgagtg ggcatagcga tttacagatt 9600
   taaggcgctt gagcgtaatt aatctcgtgt gagactccac ggtggtggga agacagccag 9660
   gaagcaatgt gcaaattaag ccagactcgg cttaggtctc ttgcctgaaa tcacttcgat 9720
   gttggaccaa acggacccca ctgggttcct gccactccgc cacactcaga tggtacaaga 9780
   acccccagct gagcccacgg cggtgtgggg aggggtttcc accgtccgct ctggcaggaa 9840
   cctatgcagg aaagagggag ctgctactga ccatttctgg aggtccagac aaggaacgtg 9900
   agttgtaagt gacggagccg acggaagttc cgagcccggc cccatacctt taccagctgc 9960
   ttggccttgc acttaaccag ctctcaaccc tcagttttct catcagcaaa ttgggtataa 10020
   tatttatagc cctgcttcaa agggttgttg gttctctcaa ataagatgat atatttaagt 10080
   gacttacaag tcttattagc cagtagcaac aaatcgctta cccaaagaag ttttacaggt 10140
   tacatgtgtg agccagcccc aggcatgtag tcacagtgtg tgtcagaggg cacagttgct 10200
   tcctctcagt caccaacaga aatcatatgg aaattttcag agacccatga acagccaaag 10260
   cattataaat gctttggtaa catggatttt gcatataagc atttatgtat tttttttcct 10320
   gacattagat tactttactt ctaattcccc ttcagtgccg tggctggatg tgcgtggcac 10380
   agaggctggt gttctctgca gtccagcgca tacatgcctt gctgttctat tgggtggtag 10440
   acaacaccgc tgaaggatat ttgtagtctc ttggctattt ctgatcactc cagtcccgtt 10500
   agtatttgaa gcattgctat cactaaatgg aatagtacag acctgtcctg gagaaagcct 10560
   ctccgccaat gataatgata tgaaaagcta ctgatagaag cttccacttc ttttatactt 10620
   tcccataggt tagaaaagaa gctgctaagg aagatactgt aaagaacaaa aagcattttc 10680
   tttttttttt tttttttttg agacggagtg tcactctgtc acccaggctg gagtgcagtg 10740
   gcgctatctc ggctcactgc aagctccgcc tcccgggttc actccattct cctgcttcag 10800
   cctcccgagt agctgggact acaggcgccc accaccacgc ccatctaatt ttttgtattt 10860
   tttagcagag acggtttttc accatgttag ccaggatggt ctcgattgcc tgctttgtga 10920
   tccacccgcc tcggcctccc aaagtgctgg gattacagac gtgagccacc gtgcctggcc 10980
   tttttggtgt attttttgac atttttacac ataggagaat ttaagaggac aactgagggc 11040
   cgggtgcggt ggctcacacc tgtaatccca gcactttggg aggccgaggt gggcggatca 11100
   cgaggtcagg agatcgagac catcctggct aacacggtga aaccccgtct ctactaaaaa 11160
   tacaaaaaaa ttagccgggc gtggtggtgg gcgcctgtaa tcccagctac tccggaggct 11220
   gaagcaggag aatggcgtga acccgggggg cggagcttgc agtgagccca gatagcgcct 11280
   gggcactcca gcctgggcga cagagtgagc tccgtctcaa aaaaaaaaaa aaaaaaaaga 11340
   ggaaaactga ataaatgaat gagagactgc ttgtttgcaa ggaatcaaac tagactttga 11400
   ttggttgttc actccagtaa aaactcaaat gcctaccata catgagtgtg gagtagctgg 11460
   agtaaacata ctttcaggag ccagactttg tgggtttaaa tcccagcttt gccacttact 11520
   gtgtgatctt agacaagtta cttaacctct ctgagccttg ggttcatcat ctataaaaca 11580
   gaactaataa aagtacctac tttacaggat tattatgaga ttaaatactg tatatgaaaa 11640
   gcacttaggc caggcgcggt tgctcatgcc tgtaatccta gcactttggg aggccgaggc 11700
   aggtagatca ttaagtcggg agttccagac cagactggcc aagatggtga aaccccgtct 11760
   ctactaaaaa tacaaaaatt agccaggcac ggtggccggt gcctgtagtc ccagctactc 11820
   gggagactaa ggcagagagt tgcttgaacc cgggaggcgg aggttgctgt gaaccaagat 11880
   tgtactactg cacgccagcc tgtctcaaaa aaaaaaaaaa agaaagaaag aaaagcactt 11940
   agggctgagc acagtggctc atgcctctaa tcccagcact ttgggaggcc aaggcgggtg 12000
   gatcatgagg tcaggagttc gagaccagcc tggtcaacat ggtgaaacca cgtctctact 12060
   aaaaatacaa aaattagccg gacatggtgg tgcacgccta taatcccaac tactcgggag 12120
   gctgaggcag gaaaatcact tgaacctggg aggcagaggt ttcagtgagc cgagatcgcg 12180
   ccagtgcact gcagcctggg taacagagtg agaccctgct acaaaaaaaa agaaaagaac 12240
   acttagaaca gaatcaggca cgtgatgagt gttctggtag tgctagaagt gatgattgtg 12300
   attctagtta ttacctacca ggagttctgc tcagctctgg agccatacat acttgagatg 12360
   cagggataga tgtaatttcc ttacggttat ttgtcatttg ccaaatattt tattcttttc 12420
   agcctgtgac attctggcca ttgataagtc cctgacacca gtcaccctgg tcctggcaga 12480
   ggatggcacc atagtggatg atgacgatta ctttctgtgt ctaccttcca atactaagtt 12540
   tgtggcattg gctagtaatg agaaatgggc atacaacaat tcaggtaaga aactctagcc 12600
   atgtataaaa cggcctatgc agtacagcat ccatcatgat tactttcact gcacagacaa 12660
   ttgccatttg tctggctttt taagcatttg ttcaacagat atttactgag cactttctat 12720
   gtgccagaca tggtgctgag tcctggggat aaagatgaat gaggccaggc acggtgggca 12780
   gatcacctga gctcaggagt ttgagaccag cctggccaac atggcgaaag tccatctcta 12840
   ctgaaaatac aaaaactagc tgagcatggt ggcaggcacc tgtaatccca gatacttggg 12900
   aggctgaggc gggagaattg cttgaacctg ggaggtggag gttgcagtga gctgagattg 12960
   tgccactgca ctccagcctg ggcgacagaa cgagactctg tctcaaaaaa aaaaaaaaat 13020
   agatgaatga gacatgggcc cagttctcaa ctaactcatg gacagacagc aactgaaatg 13080
   cagcctggca gggagctcag tgctgcaggc tgcagtccag ctgttgaggt ctggatcctg 13140
   gctctaccat ttactaacca ggtgaccttg tgcaaattac ataacttctc agtgccttgt 13200
   tgacataata attatagaac ctgtccccta agattgttgt aaagataata tagtacatat 13260
   caaaccaggg tttctcaatg acactgctga catttggggc tggagttgtg aagaggatgc 13320
   tttgtgtatg ataggacgtt tagtagcatc ctagatgtta cgaacctccc cccacaactg 13380
   tgataaccaa gaataaatga ctccagatat tgtcagatgt ctcctggggg gcaaaattgc 13440
   tcccacttga gaaccactga tatgaagtgt ttagatctta cctggcacat agttaaggac 13500
   tcaataattg ttacctgtcc ctgccatcat catcatcaca ggagatagaa aggggactaa 13560
   cattggtgac caaggatcaa gaagtgtctt gagaggggat tagttttagg gagggactta 13620
   aaaaaaaaaa aagtgtcctg aagtgtgcca caaaattgag gaagtcatcc tacagcagtg 13680
   gccctaccag ccacctttgt gctcaggcct aagggacagc aatcgatggc tacagctctg 13740
   ccagttaaag aagggaccaa tccccaagca cagagaaatg ctggcacaaa tgaccgtttt 13800
   cgtataaaaa acaggacaca cctggacaca cagaaaccag aaagggaagt gtgattggat 13860
   tccttgtagg tgtgaccaca cattgggttt aaatggcttt ttctatggaa gaatacagag 13920
   ccgttgcaca cagagataaa gatgaaagag ggtcaggcac ggtggcttat gcctgtaatc 13980
   ccagcacttt gggagaccga ggtgggcaga tcacttgaag tcaggagttt gagaccagcc 14040
   tggccaacat ggtgaaagcc cgtctctact aaaaatacca aaattagctg gtcatggtgg 14100
   tgggcacctg taatcccagc tacttgcaag gctgaggcag gagaatcgct tgaactcggg 14160
   cagtggaggt tgcagtgagc caagatcgtg ccactgcact ccagcctggg caacagagtg 14220
   agactctgtc tcaaaaaaaa aaaaaatgaa agaagaggga aaatcaaggt cctggatgga 14280
   tattgaccct cctgtgaatc cttgagataa gatgcttctt tcccattgaa attaatctag 14340
   atcctcaaga ggtctgtgtg tgctggggga ggaggtgaaa acagcacttc aggttgttct 14400
   gtgactggca ttttctccca tgtgtcagat ggaggtacag cttggatttc ccaagagtcc 14460
   tttgatgtag atgaaacaga cagcggggca gggttgaagt ggaagaatgt ggccaggcag 14520
   ctgaaagaag atctgtccag catcatcctc ctatcagagg aggacctcca ggtaaagcct 14580
   cctccacaac ctgcaacaat gttcatccag cctctgcccc ccgccccagc accttcttag 14640
   cttccatact tgccatagca atttctttgt tttaatctta atttttaaat aattcttaaa 14700
   ttacaactaa gatttttcat cttctcattc tcacctagag taatttttca accctcgtca 14760
   cgaagcagaa ttacctgtgg attttttttt tttttttttt tttttttgag acacagtctt 14820
   actctgtcgc ccaggctgga gtgcagtggt gcaatctcag ctcactgcag tctccacctc 14880
   ccgggttcaa gcaattctcc tgtctcagcg tcccaggtag ctggaattac aggtgcacac 14940
   caacacgcct ggctaatttt ggtattttta gtagagacag cgtttcatca tgttggccag 15000
   gctgctcctg agttcaagtg atccacctgc ctcagcctcc caaagtgctg agattatagg 15060
   cgtgagccac cgtgcccggc cagatttttt ttttaataca aatgcctggg atccacccta 15120
   aacctagtta ggtggacgct ggggtcaggg atgccctgct agagaatgta gcgttggagt 15180
   agatggcttc acatcccagc gctgtcacca cgactttgag ccagtagctt agcttctctc 15240
   cttgtgtttt ctctctagta agattgggat aggccaggtg cagtggctca cgcctgtaat 15300
   ctcagcactt tgggaggccg aggcgggcag atcacgaggt caggagatcg agaccatcct 15360
   ggctaacatg gtaaaacccc atctctacta aaaatacaaa aaattagccg ggcctggtgg 15420
   tgtgcacctg tacctgtagt cccagctact cgggatgctg aggcagaaga atcgcctgaa 15480
   cccaggaggt ggagcttgca gtgagccgag atcgcaccac tgcactccag cctgggtgac 15540
   agagagagac tccatctcaa aaagaaaaaa aagattggga taaaaacagt atgttttcat 15600
   tcaaagaaaa tttctctttg agacagggtc ttgctttgtc acccagtgca gtggcatgat 15660
   catagctcac tgcatcctcg acttcctggg ctcaagccat cctcccacct caacctctca 15720
   ggtagctgag actgtaggct tgcaccacca cactgcgcta tttttctttt tttttttttt 15780
   ttaatttttg taggccaggt gcagtggctt atacctgtaa tcctagcatt ttgggaggcc 15840
   aaggtgagca gattgcctga gttcaggagt ttgatgtggt aaaaccctat ctctacaaaa 15900
   attagctagg tgtggtggcg tgtgcctgta gttccagcta ctcaggaaac tgaggcagaa 15960
   ggatcacttg agcccaggtg gtcaaggctt cagtgagcca tgactatacc acaccaacct 16020
   gggcaacaaa gcaagaccct gtctcaaaaa taaataaata aataaataaa gccaggcgtg 16080
   gtggctcaca cctataatcc tagaactttg ggaggccggg gcgggcagat cacgatgtca 16140
   agagatcaag accatcctgg caaacatggt gaaaccccgt ctttactaaa aatacagccg 16200
   ggcacggtgg cttaacgcct gtaatcccag cactttggga ggctgaggag ggtggatcac 16260
   aaggtcaaga gttcaagacc agcttggcca agatggtgaa atcccgtctc tactaaaaat 16320
   ataaaaatta gccaggcgtg gcggcaagca cctgtaatcc cagctactca ggaggctgag 16380
   gcaggagaat cacttgacct gggaggggga ggtagcagtg agcagagatc acgccactgc 16440
   attttagcct gggagacaga gcaagactct gtctcaaaaa acaaaaaaat tacaaaaatt 16500
   agctgggcgt gatggcgtgt gcctgtagtc ccagctactc aggatgctga ggcaggagaa 16560
   tagcttgaac ccaggaggcg gaggttgcgg tgagccgaga tcacgtcatt gcactccaac 16620
   ctggtgacag agcaagactc agtctcaaaa aaaataatta attaatgttt tatagagagg 16680
   aggtcttcac aggttgccta ggctggtctt gaactcctgg gctcaggcag tcctcccacc 16740
   ctggcctccc aaagtgctgg gattattgat atgagcctct gtgcttgacc caaaacatat 16800
   ttattgagcc ccatcctgtg cctgtcatgg ttctatgcac tgggtgtgca gtggcgaaca 16860
   agccagacac agtctctgct ctaacggtac ttgtattctg atgaagggag gcagaaagca 16920
   acaggaaagc atatcagaca gtggtatgca gagagtgatg gaggctactc tagactggaa 16980
   cacggaggaa ggcctctttg aggaaatgat ttcttggtgt tttttgtttt ggtttggttt 17040
   ggtttgagac agggtctagc tctgtcaccc aggctggagt gcagtagtgc aatctcagct 17100
   cactgcacca tctgcctgct gggctcaagc gatcctccca cctcagcctt ccaagtagct 17160
   gggaccacag gcgtactcca ccatgcctgg tgaattttta tatttttggt aaagacaggg 17220
   tttcaccatg ttgcccaggc tggtcttaaa ctcctgagct caagcagtcc tcctacctca 17280
   gcctcccaga gtgctgggat tataggtgta agccactaca cccgaccctt cttcccaatt 17340
   ttaacactaa aagaaaatta gaaaaattgg ccgggcacgg tggctcacac ctgtaatccc 17400
   agcactttgg gaggccgagg cgggtggatc acaagatcag gagatcgaga ccatcctggc 17460
   taacacggtg aaaccccgtc tctactaaaa atacaaaaaa ttagccaagc gtggtggcag 17520
   gcacctgtag taccagctgc tcaggaggct gaggcaggag aatggcgtga acccaggagg 17580
   cggagcttgc agtgagccga gatcgtgcca ctgcactcca gcctgggtga cagagcgaga 17640
   ctctgtctca aaaaaaaaaa aaattagaaa aattacatgc ttttctcttg actgtctgcc 17700
   aactatcata gtaggtaact ttgggaaatg gatgcattga gttctgaaat ggatgtattt 17760
   gagaggtaga acaccgtgtt tagaaagtcc ttgtaggccg ggcgcagtag ctcacacctg 17820
   taatcccagc actttgggag gccaaggcgg gtggatcacc tgaggtcagg agttcaagac 17880
   cagcctggcc aacatgacaa aaccccgtct ctactaaaaa tacaaaaatt agctgggctt 17940
   ggtagcacat gcctgtaatc ccagctacta gggaggctga ggcaggaaaa ttgcttgaac 18000
   ccaggaggcg gaggttgcag tgagccgaga tcacaccatc gcactccagc ctgggcaaca 18060
   gagcagactc cgtctcaaaa aaaaaaaaga aagtcagaaa gtccctgtag atttctaagc 18120
   gtctgtgttt gtttgttaga tgcttgttga cgctcctgct cagacctggc tcaggaacta 18180
   cgtcagagtt gtgccaccgt ccagcggctg cagcacacac tccaacaggt gcttgaccaa 18240
   agagaggaag tgcgtcagtc caagcagctc ctgcagctgt acctccaggc tttggagaaa 18300
   gagggcagcc tcttgtcaaa gcaggaaggt aggactctct gcaggcagag tcactctgag 18360
   aattcagggc tgatggaggc gtcccttggg aggacactat ttgtgatgta gccagggcca 18420
   ctgaagccgc ggctgctggg atgcggacgg ggcacgtttc tctcttgccc ggccagttat 18480
   tctggcgagt tgttcatgtg gctgtgtggt cacaccacgt ctgatctctg tctcgtgtct 18540
   cctttgcaga gtccaaagct gcctttggtg aggaggtgga tgcagtagac acgggtatca 18600
   gcagagagac ctcctcggac gttgcgctgg cgagccacat ccttactgca ctgagggaga 18660
   agcaggctcc agagctgagc ttatctagtc aggatttgga ggtgggcgga aaccagggtc 18720
   actgagctac agaggaggac atgccctggg atgtagtagt atcatgcaga ggtgtgtggg 18780
   cccttttgtt cacctctgca gactgtgaat cctagctgcc agtttgccta ttatatgcca 18840
   aggcatttgc aaaaatctca ttaatctaaa tcaaaatagc tttaaagaaa aatgcataca 18900
   cttcctcaga tcatcaaaca gactctggtc caaggttggt aatgaaatga ctgttcctga 18960
   cagggaggaa tagcagggcc caatcttctg agatggcttc tgggtctttc catggtcaga 19020
   gaagatctat agtccgtcct gaggtctgtc aatgtcacag gaaaaggcaa acttgagggg 19080
   atcgtcgcct gctggctaag accagggagc taaaaacttg aggaagggaa cctgcctggg 19140
   tgggtgctac ttctgattca ttgtccttgt ccctgtcata agtacctccc tattgtagat 19200
   agaagggaag gaaactgttg acttgagctt ggctaaattt cctgaaggtg gaggacctta 19260
   gagttgctcc aagtaaactt tccaaggact tcttcctgtt ggtgttgtaa gaaaaggccc 19320
   atgcttgtcc gggcacagtg gcttacgcct gttaatgcca gtactttggg aggccgaggc 19380
   aggtggatca cctgaggtca ggagttcgag accagcctga ccaacatggc gaaaccccat 19440
   ctctactaaa aatacaaaaa ttagcctggc atggtggtgg gaggctgaag caagagaatc 19500
   acttgaaccc aggaggtgga ggttgcgtga gctaagatcg cgccactgca ctccagcctg 19560
   ggcgacaaga gtgaaactcc gtcttaaaaa agcccatggc aggctgggcg cggtggctca 19620
   cgcctgtaat cccagcactt tgggaggcca aggtgggcgg atcacaaggt caggagatcg 19680
   agaccatcct ggcgaacacg gtgaaacccc gtctctacta aaaaaaatac aaaaaaatta 19740
   gccaggcgtg gtcgtgggca cctgtagtcc cagctactca gggggctgag gcaggagaat 19800
   ggcgtgaatc cgggaggcgg agcttgcagg gagccgagat agtgtcactg cactccagcc 19860
   tgggtgacag agcgagactc cgtctcaaaa aacaaaaagc ccgtggcaat taatggtaaa 19920
   ggaaacccgg catttcagtg taaagaggta acataaacgg atactgcctc attgcactcc 19980
   ccaaaagaga gtcaggagag cagcaggaga ggcgcacaga aggcccagtt ctcaggggtt 20040
   tttattcttg ctgcagttgg ttaccaagga agaccccaaa gcactggctg ttgccttgaa 20100
   ctgggacata aagaagacgg agactgttca ggaggcctgt gagcgggagc tcgccctgcg 20160
   cctgcagcag acgcagagct tgcattctct ccggagcatc tcagcaagca aggcctcacc 20220
   acctggtgac ctgcagaatc ctaagcgagc cagacaggat cccacatagc agcagcggga 20280
   agtgtgccaa ggaagctctg tggcgttgtg ttattggtag acaccctcag cctcatcatt 20340
   tgactaccta tgtactactc taccccctgc cttagagcac cttccagaga agctattcca 20400
   ggtctcaaca tacgccgttc caccaatttt ttttttagcc ccaccagctt caggacttct 20460
   gccaattttg aatgatatag ctgcaccaac aatatcccgc ctcctctaat tacatatgat 20520
   gttctctgtt caaaagtaat tggcagtgat tggccaggcg cagtggctca cgcctgtaat 20580
   cccagcactg ggaggccgag gggggcggat cgtgaagtca ggagatcgag accatcctgg 20640
   ctaacatggt gaaaccctgt ctctactaaa aatacaaaaa aaattagcca gccatggtgg 20700
   cgggcgcctg taatcccagc tacttgggag gctgaggcag gagaatggca tgaacctggg 20760
   aggcagagct tgcagtgagc tgagattgcg ccactgcact ccagcctggg caacagagcg 20820
   agactccgtc tcaaaaaaaa aaaaaaaaaa agtaattggc agtgactatg ggcgcactgc 20880
   ctaacattta gccctgcccc atatggaaca tgttaaaaaa aaaaaaagcc aggccgagcg 20940
   tgttggctca cgcttgtaat cccagcactt tgggaggccg aggcgggtgg atcacgaggt 21000
   caggagatcg agaccatcct ggctaacaca gtgaaacgtg tttttactaa aagtacaaaa 21060
   aactagctgg gcgtggtggc aggagcctgt agtcccagct actcgggagg ctgaggcagg 21120
   agaattgctt gaaccgggga ggcagaagtt gcagtgagcc gagattatgc cactgtactc 21180
   cagcctgggt gacagagtga cactctgtct caaaaaaaaa aaaaaaaagg ataggcacga 21240
   cagtggctca cacctttgat cccagcactt tggaggccga tgcaggcgga tcacatgagg 21300
   tcaggagttc aagaccaacc tggccaatat ggtgaaaccc cgtctctact aaaaatacaa 21360
   aaattagctg ggcatgatgg cgggtgcctg taatcccagc tactcggtag gcagaggttg 21420
   cagtgagctg agagcgcacc actgcactcc agcctgggtg acagagctag actccgtatc 21480
   tcaagaaaaa aaaaaagttt tgttttgttt ttttgagaca gagtctcact ctgtcaccta 21540
   gtctggagtg cagtggcaca ttctcggctc actgcaaccc ccacctccca ggttcaagcg 21600
   attctcctgt ttcagcctcc tgagcagctg ggattacagg cacgtgccac catgcccggc 21660
   taatttttgt atttttagta gagacagggt tttgtcacat tggccaggct ggtctcaaac 21720
   tcctgacctc aggtgatcca cccacctcag cctcccaaag tgctgggatt acaggtgtga 21780
   gccaccaccc ctggccagaa aaaaaagctt ttaaataaag taattgtcag cttaggcaac 21840
   atagcaagac cctgtctcta caaaaaaaaa atttaagtta gccaggcatg gtggcactga 21900
   cctataatcc tagctactct ggaggctaag gcgagagtat cacttgagcc caggagctca 21960
   aggctgatca tgccaccata ctccagcctg gatgatagag taagacccta cttttttttt 22020
   ttttttttta aaaaggtaac catcaagggc aatgaaaaga aatggtcgta tttccaattc 22080
   attatcctta ttctgaagat agtacggcag aattttaagc agagatagtg gtgaagatgg 22140
   tgacattata gagtgttgac tatggccaca atcttgtttc tccagttaat gaattttttt 22200
   atgtgtttgt ttaaagatac atattaagct tgtagaccat agggacatac ggagagtcca 22260
   ttgctaatat ctcactcagt attgtgaaat tctatctcac caccgtgaaa ctcttcagtt 22320
   ttctaattgc tttatcagca gggggtataa aaggtcatga aagcaatttc cacatgctgt 22380
   ggctccaggt ctctgggtgt gaagcagagc aagcctggtt tgtcctcctc ctgtctccac 22440
   acagacggct tctgcaggtt tggtaatcta cagtacactc cttgcaggga aaaggtgatg 22500
   agtcatcatg gacttatttg accacttttt atgcatgctt agaggaaaac agaatactgt 22560
   taagagattc atctgctagt tattaagtaa agaaatatca caataggccg ggcgcagtgg 22620
   ctcacacctg taatcccagc attttgggag gccaaggtgg gcggatcacc tgaggtcagg 22680
   agttcgagac cagcctagcc aacatggtga aaccccgtct ctactgaaat tataaaaaat 22740
   tagccgggtg tagtggtaca cgcctgtagt cccagttact tgggaggctg aggcatgaga 22800
   attgcttgaa cccaggaagt ggaggtggag gtgagccgaa attgtgccac tgtactccag 22860
   cctgcaacag agtgagacac tgtcacacaa aaaagaaaga aatatcacaa tatgtcacaa 22920
   taggccgggc gcagtggctc acacctgcag tcccagcact ttgggaggcc aaggcagatg 22980
   gatcacctga ggtcaggagt ttgagaccag cctggccaac gtgacaaaac ccagtctact 23040
   aaaaatacaa aaattagcca ggcgtgatgg tgggcacctg taatcccagc tactcaggag 23100
   gctgagacat gagaatcgct tgaacccagg aggtggagat tgcactgagc tgagatcctg 23160
   ccactgggct ccagcctggg tgacagaatg agactctgtc tttaaaaaaa aaaaaaaaag 23220
   aaaatcacaa taagtcctag g 23241

<210> 86
<211> 12312
<212> DNA
<213> Homo sapiens

<400> 86
   cctaggagtt cgagaccaac ctggccaaca tggcaaaacc ccgtctttgc taaaaataca 60
   aaaattagct ggccgtggtg gtgcacgcct atagtcccag atactcagga gactaaggca 120
   agagagtcac ttgaacccgg gagacagagg ttgcagtgag cggagattgc accactgcac 180
   tccagcctgg gtgacacagc gagacaccgc ttcaaaaaaa gaaatgtttt ggtgggaaaa 240
   aaaatgagaa ccacttatcc acagtgaacc tgccgacaag tacaccaaag tattctaacc 300
   acaaaagtca cagaagacac aaatgctgag gagactgtgc caggaataca tgttagaggt 360
   ctttggcaga caggcaaccg gctgtagcta ttcttttctt ttcttttttt tttttttttt 420
   tttttttttg agacagaatc tggctctgtc gcccaggctg cagggcaata gcgcgatctc 480
   ggttcactgc aacctctgcc acctgagtag ccaggattat aggtacctgc cactacaccc 540
   agctaatttt tttttttttt taagtagaga cggggttttg ccatgttagc caggctagtc 600
   tcgaactcct gacctcaggt gatccacccg cctcggcctc ccacagtgct gggattacag 660
   gcgtgagcac cgtgccttgc ctattcttac agagatcaca gtactggctc cctgaatcca 720
   tcaaaatacg ccttctgatt accagcctct ggttaattgg cttgtaaacc aaagagaaaa 780
   tcttgggtgt cccaggggta agatggatga gcttccccag gactgagatc aaatacatga 840
   tgtagaaccc cctggacaag gttccatctg agcctcagga agaaacctga gggcactttg 900
   gcacttggag agcaaaacta ctgagacgca acctagatct gcaactgcag agcatcttta 960
   ttttgaagaa ttcagcatac taaaaaatag taatcccaga aaaaggtgat aaagtagaaa 1020
   ttgctgtcat cagttacaga aaaaattgta gtttggggcc aggcacgttg gctcacacct 1080
   gtaatcccag cactttggga ggccaaggtg ggcagatcac aaggtcagga gatccagacc 1140
   atcctggcca acatggtgaa accctgtctc cactaaaaat acaaaaatta gctgggcatg 1200
   gcggcgcgta cctgtaatcc cagctactct ggaggctgag gcaggagaat cgcttgaacc 1260
   cgggaggtgg aggatgcagt gagccgagat cacaccactg cactccagcc tgggtgacag 1320
   agcaagactc catctcaaaa aaaaaaaaaa aaaaaattgt tctttgggat tattttggtg 1380
   gagagatgca ggaaagagga cagaaagaaa aaacaaaaag aacctacacc taaaagttaa 1440
   aggaagagac gatggccaag gactcatcca ttagagagaa cttggtgtca aagagcaaaa 1500
   agctaaaatt ctagtttagc caaaatccat aagcactgtt taaagtagat gagctatagt 1560
   attgctgaac attgagaaac tgaattgaaa caagtgagca gatgtgtaaa tcaaacctta 1620
   tttactgctt acttcaaata accaaaaatc atatgactaa atggaaagct caaaagttta 1680
   tttaacccaa gtttcagtat ccaaacaaga tgggaactga aattacccca tgcacatgtg 1740
   gacaattgtc cgcccattct aaaatactta tagaccaggc agggacttgg tcctactagg 1800
   acagcacaag aagctggatc cagagactgt gtgggcctaa ggacaggcac acaactgtgg 1860
   ctagaaaacc agagtctgtg ggaaattcac aggttttcac agatctggga gaagctggag 1920
   gtctgtactc tggaggcctg tccgaactcc ttgcctcagc aggcaaagct cagtgaagag 1980
   agtataaaga gaaaaaagta accagccatt ttaactttgg cggagttttg tcaacaacag 2040
   agaatttcaa cttggagttt gcagaatttc tcaagactgg tctgttcacc aatctctagc 2100
   cctaatgaca tgatattaga gagcaatact cctctgagac ggtcaatgtt gctgggttgg 2160
   tttgatttgg aggccaggat cgaggcagga ttgaacgggc aagaaaagga aacacgcagc 2220
   ctcactactt tgctagaatc aaggccagga tttggctaca aacacgaatg tgtcgtttag 2280
   agactacaag attacacttc tcaccacaaa atctcgagac agtttttatg attcagagag 2340
   gacagaactg aaaatcaagt cttctttgcc tagcaaataa aacaaaacaa tcatgcacat 2400
   attgactaca tcacaatgtc acctgcaccg cactgactac taaatagaac ttcttctgtc 2460
   gtaatttact tttattttca ttcttttgtt ttgttttgtt ttgttttgtt ttgttttgtt 2520
   ttagagacag ggccttgctc tgttgctcag gctggagtgc agtggcctga tcatagctca 2580
   ctgcagcctc aaactcctga gctcaagcaa tcctctggct tctgcctcct gagcagctga 2640
   gagtataggt atgaggcacc aagcccggct aatgttaaaa tttataatag agatggggtc 2700
   tctctatttt gtgcaggctg gtctcaaact cctggcctca agtgatcccc cgaccttggc 2760
   ctcccaaagt gctgggatga taggcatgag ccactgcacc tggccagtaa cttacttttt 2820
   taataagggg aaaaattatt tcaaagtgat catctagtct gggacaaaga ataagattat 2880
   ttgcctttag tgtagagacc aggcaacttc cattaaggaa ttgtatgaaa aatatgcatt 2940
   cagttaaaaa caaaaagaat atgtatttcc cgaagcttaa atgaaaaaat taacaataat 3000
   aaacacaaaa acaaaacaac ttagcagtgt gttcccccaa cactgaaata tcccattcct 3060
   caaggcctgg cttcaagttt cccaaaagcc atccctaagt ggccctcgcc ctccgcagag 3120
   ctcttcctgc tctgaataac ttggggctct cttctacgcc caaccaggca gctctgcacc 3180
   aaatgataca caatgttgtg ctatttgcta aaattacata cagtcgtgtg cagtttgctc 3240
   ttatttcata tgtataaatg ccttttcttc ccaaagtaaa acacgtgaat tactcaagaa 3300
   caaggtttag ttttattcct caattgtctg ttccccatcc tcaggcctga gctgagctaa 3360
   gctgagcatg caggagacag caaatacaca tggcctgaga aagctccctc cctgtggaag 3420
   ggctgactgc tgtgtgtttt cacaaaccgc aacaccacca cactgagctg tggggctttc 3480
   tgtgcaaggg ggctgcccat cttgtgcccc cgccctgcca cacgagtcct cagcgggctc 3540
   ctctgcctga caccctccca acaccactcc caggaggaag aagcagttca cacaagacca 3600
   cgcgactcag ctggtgcccc cgtttaatca ggctgctgca aatgcttggc caaaagcaat 3660
   aagggcatga ctgagagact gtgggagttt aatataaaaa atttaataaa caaaaaagtg 3720
   ggggaaggag aaagggtcta ccttttaata gggccctaat tacccaactc ataggatcag 3780
   cccaaatgag atgggagttt atttcggggg tttttttaat tattatttta tatatatata 3840
   taaattgccg ctatgtgttt tctttcctct gagcaattat gcagcaagta cgtaaagagg 3900
   agaaaaaata ttaattaaat ataaggtaat tgtggagttg gcctggagag aatgagtctg 3960
   aactctggac gatcacaggc tactagggtt tgaagactgc tcagggcctc ccagaaaaga 4020
   aagaaataaa aatacaacca accaacaagc agatgcacag tacagcagga agagcagggc 4080
   acagaaacac tagcagagca aggggtctgc agggctccaa ataccagaaa tcagtgagct 4140
   ggccaactgc tcagggctcc caaagagctt gtcgaatcaa gcccccaaag atggactggc 4200
   cactcctccg atgaccagtt aaatgggtac atcaaaattt gaaaagagag aggaaggaaa 4260
   ggactgcagc ggcctatggt gttcaatctg cacttcagtc taaaacaacc acaacttcgg 4320
   cctgaggggc actgaggcag gccccaggga agcaggcaga cgtccggaga gactagcaag 4380
   gtggggcctc ctgggctttc ccaccaccat gacctcaagg aaggaactgg gcaataccaa 4440
   aagagtgaaa cgaggccacc gtgggagtgc tgggatataa gcccgtaact ggtgacgtaa 4500
   cagcaggtga catgaatgac aacactgctc ccctgaaaca gtcccagcag tgcttgtatc 4560
   acctcggcct ttctacagac agctcaactg gatccaactc gccctcagcc tgggaaagcc 4620
   ttcgtacaca ctggaattca gacgtgttca tacccgctaa gtccctcacg tcatctcctc 4680
   ccgcgactgc cagaagggga gtgaagcaga acaggtgaaa ccacaggccc tgctgaaaca 4740
   aagccttggc agtacagccc tacgcctgct atcgctacgc aggaagaaaa agctctgaaa 4800
   agttaaacct agagctggct ctcccatcaa acttatctgg tttatacaga gccagtctta 4860
   ctacccagcc aagaggactt acagcaggcc ctgaaaagca aaaaagaatg aaaatggggt 4920
   gggcagcctg ggactctggc caccaaatga aaagcacttg ggagagaaaa ttacctgcag 4980
   tgcttaatag gcctcttccc tatccatagt ctaagaagac agcgttggga atgacaatca 5040
   cacaattact gaccaggcaa aaccagaatt ccatcgctta cctgttcttt aaagaccact 5100
   gaggcgctcg caccctgagg ggcagagcag agggcggcta cttcaaacaa tctagaagcc 5160
   cagccacctc actccacctc agcctcccac gggcgcagga atctcactaa ttactattga 5220
   tcagccacaa acaaagtggt gcaggaccat ctggggtgga tgcttttagt cctagaaagg 5280
   accagccttt actaaggaga gatctcactc gtccccacac gcgagaagag aagtcagagg 5340
   tcactgacag aggcagtctc taggtaacag ctacttttaa agggacacta ctgacgcaga 5400
   accataggaa cagaaggttt cttttcactc gcgtggcatt ttaattacta gaaagtgata 5460
   ccacatatct tgggtactta ccagcggctc tcgaggcagc acattttcac ttcctggagt 5520
   agagcttggc tgcagagaaa aatggaaaca aaaataagat cacagtgcca cccaatggac 5580
   atcaaaaaac caaagatgtt ccgttctcaa acgatgcacg ctggcacctc tttctcacat 5640
   ctctcactgg tttttcacga cacaagagta ttttgcagtg gactacctgt aaaaagtagc 5700
   ccaaagagtc tgaattcacc agggaagatg aagactgttt ctgggttcac ttgtcacacc 5760
   actccaagaa caaggcttgg ttctgctcag gaaggcccac gatcacctac tgagcagcaa 5820
   gggctgggga ccatcccaaa atccacggga agagtagagt ccagagaaaa ggcctgaaga 5880
   gagagcacaa cacctcctct ttctgccacc tccccaacgt caggggactg ggaagaaaag 5940
   tcgcgcagca gtggagaacc agattccacc accagggggc cccagctgcc cacgctcagg 6000
   attttctctg ggcatctagt ttctggagcc cagaagccag taaactacac aggttcctat 6060
   tcatcacctg ccactaagga caccacccca caatagtaaa gaaagcccca ggcttaaatc 6120
   cttcttggaa ataagctagt gacaggctgg catataagca gaccaacctc caggcgagca 6180
   ggcagcactg tggccgaggc cctggctcta gcccgtttcc tcttccttgg ggccactacc 6240
   tcaccctcac aggcgccttc gacccttgac tgatctggcc aaaatttaga gaaagggaga 6300
   ggcagaggta gagacagatt ctgaagaaat ttacaaatct ccaaaagaat aaagtaagca 6360
   ggtatgctgc taagtgagct atgattagtt tactgtgact gatggtcagg ccaattgcca 6420
   tctatctaca gttaatatcc agacaagtgc agcaaaatct gtgtgtccca ggagacagcc 6480
   tgcagcctcc attctcccgg ctattacaga tcagatggaa gtgccaggcc gccttggaaa 6540
   aggacagagg cccggtctgc agagggttcc caaaaattcc tcatgcccag ctactaccac 6600
   attagctggc acacagcaca gtggcaagca ctctgctggg tgaaggcctc tatccctcca 6660
   tccgaagtcc tgtcccaaga gcctcaccac cccaggacaa gaggtcagcc agcaggaaac 6720
   gatctagaaa gtgagggtcc gtctcaggga atgtgagcaa gcttcccctg cctccctgcc 6780
   tcctgtcctc ccaaagctcc aggcattggt aacttcaaac ctttagagct ccagcccaca 6840
   cccctctgag atacacatcc gactgccttc ctgcctcttc ttggagttaa gctgccctgg 6900
   agcagactca cagtcctctc tgacacatat gtcttcttcc agcattccct cttggcatga 6960
   atgggacaaa cttcaccaaa tgatgtaaac tagaatccaa aaggccatct tgagacagct 7020
   ctttctccct cgctcccaga gctaactcat tacatcaagt ctgccatact ccttcctcaa 7080
   tatccatgga agagtggtgt aaatgccagc actttgggag gcccaggagg atggattgct 7140
   tgagcccagg agtttgaaac cagcctgggc aacatggcaa atcctcgtct ctacaaaaaa 7200
   tataaaaatg ggccaggtgt ggtggtgtgc gcttgtactc ccagctattt gggaagctgg 7260
   ggcagaagga tcccttgagc ctgtaaattc aaggctgcag tacaccatga tcacaccact 7320
   gcactccagc ccaggtgaca gagccagacc ctgtctcaaa aaaaattaaa aagtccatgg 7380
   aaacaatcca tccgtttttc tccatctcta ctgccatcac ttcagtccaa gctaccaaaa 7440
   tcccctgcct ggaccactgc gtcagctttc tcatcagtgc tggcgctctt ccaattcata 7500
   ttctgggatg caaacagact atcatttcca aaggagaatc tgctcgtccc atcccttgtc 7560
   cacccactca ccccagtaaa cacaacaaat ttaagggctt ctccttgctc ttagaagaca 7620
   gaaaaacctc agcgtggcct acaaggcctc acgtctactc tgactacacc tcaggtctca 7680
   agcggcacca cattgcccac ttctgccccc tgacagacgc actggccttt gctttctctg 7740
   tcactgcgcc tttgcacatg cccttccttc cctttagttc atccttcaga tccagctaag 7800
   acacttcctc agggaagcat tccttctctg acatccatga ctaggtcaga tctccctgtg 7860
   acacattcag gtaacaccac ctgcctctcc ttcaaggacc tgagcagatg tgcaacttta 7920
   catctgtgtg actatcggac tgatgcctgt ctctctcacc agattataaa ctccacgtgt 7980
   gcagaaactg cctgtgtttg cttatcactg tatccctggg ggctaggaca gtgccaggca 8040
   cagaggaggc cttcaataga aatttgctga atgaatgaat gggcaacaaa aaaacagaac 8100
   aatggcaaca actcagctga atggattatt tagattcttc cgttcattat cttcctgagg 8160
   gtttcctcta ctccatttgc tagatactaa gtgctaacat tcacttagct ctttcctcct 8220
   ttaattctca caataagtat atcaaattac tgctgatgag gaaactgagg caaaagaggt 8280
   taaataactt gcccatgatt actcagatag taactggtac tacagggatt tgaacccttc 8340
   tacttaatca aaataagcaa gttttgttta aaccacattt tattaagcaa caaccttaat 8400
   gctaccccat aaataataat gtaggttatt tgaccagaca aaacacacaa aagcaaacag 8460
   ctttagtatt taggaagccc cattctgact taacccaaaa ctttcttcct aaaataaaac 8520
   taatcactat gaattgtttc caccaaggaa ctgctcttaa aattagttca tcccttgaat 8580
   agctagtatt taccagaggg aaaaacttca aacagtacac agtgccaaat aagtctcttt 8640
   catcataaac cctttagtac cttttaaatt ataagccatg taaatgaatt gttttagaaa 8700
   attcaatatt aaaagtaaaa aagaaagctt ccctctcatt ccaaccctag gggacccaca 8760
   ctgacgctgg ttccttatat attctgccaa ggatattcaa agcatataag aagcatggcc 8820
   ggccgggtgc agtggctcat gcctgtaatc ccagcacttc aggaggccaa ggtgagcgaa 8880
   tcacttgagg ccgggagtta gagatgagcc tggccaacat ggcgaaaccc cgtctctaca 8940
   aaaaaaatgc aaaaataagc tgggtgtggt ggtgcatgcc tgtagtccca gctactcggg 9000
   aggcagaggc acaataatcg cttgaacccg ggaggcggag gttacagtga gctgagatca 9060
   tgccactgca ctccaatcta ggtgacagag tgagactctg tctccaaaaa aaaaaaaaaa 9120
   aaaaaaaagg agcatggcca agtgtggctt acaccttaaa cccagcacag cactctggga 9180
   gggtacaatg ggaggatccc ttaaggccag gagtttgaga ccagcctggg caacacagcg 9240
   agaccccatc tctacaaaaa atttaaaaat tagccgggtg tggtggtgca tcctatagtc 9300
   ccagctactc aggaggctcg cttgatccca ggaggcagag attgcagtga gctatgatgg 9360
   tgccattgta ttccagcttg gtcaacagag caagactctg tctcaaaaag aaaaaaaggg 9420
   tgcatgtgta tgcatattcc taccatggaa tttgaaacag caacactcca aattcctcaa 9480
   gtcatggctt cccagagcca cagtcccagt atgaagctgg gaatacaaat gtgagccacc 9540
   acacctggcc ttttttttat ttttttaatc atggcctacc ctgtgaacat ttttttaaaa 9600
   tcttccacca tgctagctgt ttaaacaatc gaagcaggcc gggcacagta gctcacgcct 9660
   gtaatcccag cattttggga ggtcgaggca ggcagatcac ctggtcagga gttcaagacc 9720
   agcctggcca acatagcaaa acctcatctc caactgatcc tggaaatctt cacagagtag 9780
   tccatgatta aaaaaaaaaa aaaaacaagg ccgggcatgg tggttcacac cagcactttg 9840
   ggaggccgat gtgggaggat cgcttgggcc caggagttcg agaccagcct gagcaacatg 9900
   gtaaaaccct gtctctacta aaaaatacaa aaatttgcca cgcatggtgg cacatgcctg 9960
   taagtcccaa ctacccagga ggttgaagtg ggaggatcaa ctgagcccag gaggttgagg 10020
   ctgtggtgag ctgtgatcac atcactgcac tccagcctgg gtaacagagt aagactgtct 10080
   cagaaaaaaa aaaaaaaaaa aaggcttaga aaccacccac aggcatctat tgtaacgatg 10140
   taagaaaagg acctaaggca atccatgccc aggatcctta gaggcctgaa tggggagagc 10200
   aggaagagaa gaaagcaaaa gcagaaaact gctttaaaaa aacaagtcag gcaaagacta 10260
   cggcggcaga gagagaaatg cttttctttt ggtagagaca aaggtgctct cactcctgct 10320
   gccaaggcgc tgaccttccc caactggaag gcgttcccag atcctgacct gctgtccaac 10380
   aaagaacaac ctcacaagca gctcacagat ttgagcaact ggcgaaagct ccaggtctag 10440
   gagaacccat cctgcatctg cccagtaact gcgacttcag ataggagaac ccatcctgca 10500
   tctgcccggt gactgtgact ccagataggt cctaagccag tctgtgtagt tctctgctgg 10560
   gaagaataag attcgatccc accaatatcc tttgagatgc agtggcatga cagaaagtac 10620
   acaagctttg gcattaagaa ctcagtgatt tgggggcacg ctgctcaacc tctctgagcc 10680
   tcagtttcct catctatggg aataacggga ataataccac cacctcacat ttgggattag 10740
   aggagatgat aagttatttc atgtcactcc tctgctcaaa gtcaaccaat gcacttccac 10800
   ttcgggtcat gatggagtga tagggaacag atttgccctc ccactgtaaa caactagaaa 10860
   actaagccaa ataaataaaa ccactgtgtg tacacattgg acagcaggca gtgcaggact 10920
   acgatccctg aaagcagaga aacaaattac ttgggcccta aaatcactct ggcttcctgc 10980
   caggagacac atttcaggcc ctggagagag acggggatcc ccagcagagc acgccaccca 11040
   cttaagttga agagacagag atcagagttc agggaggcca aggcagctga aagctgcagg 11100
   acagacttca gaaagaaaga gctatgcaga aaaagtggtc cagaaatctg tctggaggtt 11160
   ccctcaggtc tctgctgaat actaaaattt gtgcatgcat agggtgaaac tccacagtga 11220
   agaagaagct aggagctgac atttcttaga actctcaggg gaatcatgag tcccaagcag 11280
   ccagactgga gtgtcctcac agggcattca ggagagacca gaggtcaaat gatccctaga 11340
   ttaaagatta tccagatcca ctctaacaaa gctaaaaaac aggactcaaa atgataacag 11400
   taaaccataa aaaactgcac agaagaaaaa agctttaagc actctttaaa gaccacaaaa 11460
   tccataacct caacaatact gaactcacaa tatccagcat ccaataaaaa atttcaacac 11520
   atggccaggc acggtagctc acgcctgtaa tcccagcact ttgggaggct gaggcgggcg 11580
   gatcacctga ggtcacgagt tcgagactag cctgaccaac atggagaaac cccgtctcta 11640
   ctaagaatac aaaaattagc cgggtgtggt ggtgcatgcc tgtaatccca gctacttggg 11700
   aggctgaggt aggagaatca cttgaaccca ggaggcgggg gttgcggtga gccgagatcg 11760
   cgccattgca ctccagccta ggcaacaaga gcaaaactca gtctcaaaaa cataaaattt 11820
   aatttaaaaa tttcaacata tgtcaagaag caggaaaatg taaccaataa caataagaaa 11880
   aaccagacga tataactagt agacaagaac agtaagacag gtgttacaac tatactcaag 11940
   tatttaaatg aaaacatgtc acagtaagaa gagaaatgta aggtataaaa agaaaataga 12000
   ggccgagcac agtggctcac gcctgtaatc ccagcacttt gggaggccga ggcggatgga 12060
   tcacgaggtc aggagatcga gaccatcctg gctaacacgg tgaaacccct tctctactaa 12120
   aaatacaaaa aaaaaattag ccaggcatgg tggcgggcac ctgtagtccc agctactcag 12180
   gaggctgagg caggagaatg gggtgaaccc aggaggcaga gcttgcagtg aaccgaggtc 12240
   acgccactgc actccagcca gggcgacaga gtgacattcc gtctcaaaaa aaaagaaaat 12300
   agaatttcta ga 12312

<210> 87
<211> 12730
<212> DNA
<213> Homo sapiens

<400> 87
tctagagagc gcagtccaga ctgcagtggg agggcgggca gagggctcca gaaatggcac 60
caagaaaaca gtaagttgcc tgatgtcttt gatggtgccg aggggaattt tacagcttga 120
gctgagtgag gattaatcag tgataagtac acagctaagc aatctgcccc atccccaaat 180
agagtccatt aactctatga aaaaaaaatt ttacaaaaga gaaagcacaa ctatcacata 240
cttcatggct cacttgtaaa aaatagttac atacttttca catttattat aattagtatt 300
catgatttta ctaaagcatg aatattgctt tagtactata atgcttagaa tttgatattt 360
ataatattag cactgtttat atttaatgca tccaatacta ctaaaataaa ctagatctcc 420
atcaatctct acttcaaagc caggcacagt ggctcatgcc tgtaaatccc agcactttgg 480
gaggccaagg tgggcagatc acttgaggtc aggagttcga gaccagcctg gccaatgtgg 540
tgaaacccca tctctacaaa aaatacaaaa attagcaagg tgtggtgacg tgcatctgta 600
atcccagcta cttgggaggc tgaggtatga gaatcacttg aacccaggag gcagaggttg 660
cagtgagccg agatcacgcc actgcactcc agccagggca acagagtgag actctgtctc 720

aaaaacaaaa caaaacaaaa caaaaaacac tctaccttaa tttttccaaa tatcctcaaa 780
ttcatgatgt ctgagggcat gaacacaaag gcatgaacaa attcatcatc tctgagggct 840
tgaacacata cttacggcta ttctgctaac tgctggataa aataatggca caaagaatct 900
agcacattta aaaaatacta cactacaact ggctggaatt catattaata atataataat 960
gattcactgt tatctattaa tatactttat catattagat caaaagacaa taattatatg 1020
attatttctg taagtgataa aggacattag atataattcg atatccattc ttagtaaaaa 1080
ctctaataca ataagaatac aagaatactt cctgaatatg attatctgtt agtgggagtg 1140
ggggggggga aagagtatct acttgtgtat atgtgtgtca ttctccaaag ctgacatcat 1200
cttttattaa aaagatataa aagcatatta tttaaaatca gaaacacatc atacgttcac 1260
aatcatcagt tttatttatc actgttctgg aggtatttgc tgatgcaact ggacaagaaa 1320
agtagtaata gatataagca acggaaaaga agaggggaga gtgttattta catatagcac 1380
ctggaaaaac tgacatgaat taacgtacca taaacaatat aagtatctag ctaggtgtca 1440
acattcaaaa tcagtatgta aaaactgcct tcaaattaaa gaactggctg aaagacataa 1500
tgaaagaata catcccattc aaagtagtaa cgaaaaagat acatggctgg gtgcagtggc 1560
tcatgcctgt aatcccatac ttcgtgatgc tgaggcagga ggatcacttg agcccaggag 1620
tttgcgacca gcctgggcaa catagtgaga ccccgtctct acaaaaaata agaaaattag 1680
gccaggtaca gtggctcaca cctgtaatgt cagcactttg agaggatgag gcaggcggat 1740
cacttgaggt caggagttca agaccagcct ggtcaacatg gcgaaacccc gtctctacta 1800
aaaatacaaa aattagcccg gcgtggtggt gcatgcgtgt aatcccagcc acttgggagg 1860
cagaggcagg agaaccactt cagtctcctg aggtggaggg tgcagtgagc caagatcatg 1920
ccactgccct ccaatctggg tgacacagca agactgtcta aaaaaaaatc tttatttttc 1980
attttttact gggcaagcta attttgaaaa ttatatagaa ataagaatag ccaagaaaat 2040
tccaaataat tgaaaaaatg ctgaggcaaa attaatttta acagataata aaacatgtaa 2100
gatagtgtga cattggacac aaagaaacat ttcaagtgaa cagagtacgt ccagatatat 2160
acaggaagtt aacataggat aaagcttcat tgcaaatcaa tggagaaaaa ctgcagagat 2220
gggaaactac agccagggag acaaatttag cccattgtct atttttggat ggcctgcaag 2280
ctaagaatgg tttttacatt tttttaaagt taggaggcaa aaaaagaatt taatgataca 2340
tgaaaattag atgaaattca aatttcagtg tccataaata aagttttatt tctcattagt 2400
ttatacactg tcaacggctg cttttgtgct accacagtgg cgatgagtag tgttcagaga 2460
ccatacaaat tgcaaaacct taaagtattt actatctggc tctttacaga aaaagtgtgt 2520
caacccttgc aataatggat gctggattat tttattagct ggaaaaaact gaacttgaat 2580
ccttatactc tatacactga aataaattgc tgattactta aaaaaaaaca acttgaggtt 2640
taaaaaataa tactggtggc caggagcggt ggctcacgtc tgtaatccca gcactttggg 2700
aggccgaggc gggcggatca cctgaggtcg ggagtttgag atcagcctga ccaacatgaa 2760
gaaaccccgt ctctactaaa aaacaaaaca aaacaaaaca aacaaacaaa caaaaaaatt 2820
agccaggcat gatggcacat gcctgtaatc ctagctactc gggaggctga ggtaggagaa 2880
tcccttgaac tcaggaggcg gaggttgcag agagtcaaga tcatgccatt gcactccaac 2940
ctgggcaaca agagtgaaat tccacgtcaa aaaaaaaaag gaagtcataa aagaaatgat 3000
agataaataa aaaaattctg cctggacaaa agcatcataa acaaagtcaa gacaaatttc 3060
aaactaatag catatatttg taacacaaat cccagacatg ggctattttt ccttagtata 3120
taaaattata aatcaatttt aaaacattaa tctcctaatc ccaagaaagg gtaaaatacc 3180
tgaatagtca atttgtagat acggaaataa aaatggctct taagttcaaa agagaaattc 3240

aaatcaaaac agcactgaga ggctttctgc atctaaccaa gatgaaatag cttgtaaaag 3300
ttcaacaata ctgctgagaa caactgaaaa acaaactgat tgaaagtact ggggagctgc 3360
caaggcaacc aagacttgag agtccaagat ctcagtgaga agggaactgc agaaaggtga 3420
gcaaataccc tataagatgc tgaggcagag cttttgtcca tcttcagatg gactagagag 3480
atgtaagtaa atagacagac atgtaaataa ataaacaaat ggagttcaca aagcagaact 3540
cctgggctta agagatcttc ccacctcagc ctcccaagta gctgggacta caggcatgtg 3600
ctaccacacc catctaattt ttgtgttttt ttgtagcaat gaggtcgccc aggctggtct 3660
caaactccta gcctcaagca atcctcctgc ctcagcctcc caagtagctg ggattacagg 3720
ctcaagccat tgcactcagt tacaattttt aaattgagta aggctagagc ttaagatcca 3780
aagttgagaa tataaaatta taaatccatt ttaaaacatt aatctcctaa tcctaagaaa 3840
gggtaaaata catgaatagt caatttgtag atatggaaat aaaaatggct cttaagttca 3900
aaagagaaat tcaaatcaaa acagcattga gaggctttct gcgtctaact gagatgaaat 3960
agcttgtaaa agttcaacag cagtaaacaa aaattagggt tcaccccacc aaggaaaagg 4020
agccatgaag aactttaggt tctttgttgg gacccataga gggctatacc ctaggaacat 4080
ggattgacca ggggaagaca gacttatttt ctaaaaccct gcaacctgac tcaggccaaa 4140
tcctgattga tcaaggtcat aagtcccact ttacacaagc ataaagtaaa cttcagagga 4200
aaatagtatc tttcagagct tctcaattat tcactgtcat gcaataaaat attttaagat 4260
atacaaaaaa aggctgggcg cagtggctca tgcctgtaat cccagcactt tgggaggccg 4320
aggcgggcag atcacgaggt caggagttcg agaccagcct ggccaatgtg gtgaaacctc 4380
gtctctacta aaaatataaa aattagtcgg gtgtggtggt gcacacctgt agtcccaact 4440
acttgggagg ctgaggcaga agaatcactt gaacctggga ggcagaggtt gcagtgagct 4500
gagatcgtgc cactgcactc cagcctgggt gacagagtaa gactccgtct caaaaaaaaa 4560
aaaaaaaaag atatacaaaa aataggacca agtgggggaa aatgaattta aaaaacccaa 4620
actcatagat agcccagtgt taaaatttat caaagacttt aaaagaacta attaatatat 4680
tcaatgaaac aggtgacaaa acagagaagt ttactagaaa actaaactta aaaggcatca 4740
ggttaggcat agtggcccat gcctataatc tcaggacttt gagaggccaa ggcaggtggc 4800
atcacttgag ctcaggaatt tgagaccagc cttggcaaca tggcaaggcc ctgtctctac 4860
aaaaaaaaaa aaaaaagaaa gaaaaagaaa aatcaggcca ggtgtggttg ctcacacctg 4920
tagtcccagt tacttgggag actaaggtag gaggatggct tgagccttga ttgtgccacc 4980
acactccagt ctgggcaaga gagtgagaca gtgaaatctt tgtctcaaaa aaaaaaaaaa 5040
atcaaatgca aacaaggtaa ctgtaaaatg caataattga aattaagagc tcaacagaca 5100
ggtttaacag caaactggat atagcagaag aaagggttag taaactgaaa gatcagtaaa 5160
gaaaaagaaa agcggatgga caatgtagaa cagagcaaaa gacataaaga acccggaaag 5220
aaggtctaac acaattacaa ctggagtcca ggaaagaaga gaatgagaac aagacagaac 5280
aatattaaaa gagagaataa ttaataattt tgtaaaacta acaaggcaat caggccgaat 5340
attcaaagat tctatgttac ataggataac tacaaagaaa accacctcag gaacatcaca 5400
taggaaaact acttttgaaa aaaacaccaa agattgagaa aaaaatctaa aagctgccag 5460
aagataaaaa catgtttcac cttcttcaaa ggagcaataa caagactgaa agatgacaat 5520
agaagccaga aaataatgaa ataacttttt tttttttctt ttttgagacc gagtcttgct 5580
ctgtcgccag gctgtagtgc agtggcgtga tgtcggctca ctgcaagccc cgcctcctgg 5640
gttcacgcca ttctcctgcc tcagcctccc gagtagctgg aactacaggc gcccgccacc 5700
atgcccggct aattttttgc atttttagta gagacggggt ttcaccgtgg tctcaatctc 5760

ctgacctcgt gatacacccg cctcggcctc ccaaggtgct gggattacag gtgtgagcca 5820
ctgcgcccgg cccaactttt ttattttttg agacggagtt tcgctcttgt tgcccaggct 5880
ggagtgcaat ggcacgatct cggctcacag caacctccgc ctcccgggtt caagggattc 5940
tccggcctcc gcctccggag tagctgacag gcatgtgcca ctatgcccag cacatgcctg 6000
gcatacaaat acaaaaatgc aaaaattttg tatttttagt agagatgggg tttcttcatg 6060
ttggtcaggc tggtctcaaa ctcccatctt cagatgatcc gcctgcctcg gcctcccaaa 6120
gtgctgggat tacaggcgtg agcaaccgcg cccggcctga aataacattt ttaaagtgtg 6180
gaaataactg ccaatctaga gttctatacc agcaagaata catttcaata gtaagaggat 6240
tagaaaatca ccattcagcg gccaggcgcc gtggctcacg cctgtaatcc cagcactttg 6300
ggaggccaag gtcagtggat cgcaaggtca ggagttcgag accagcctga ccaacatggt 6360
gaaaccccgc ctctaccaaa aaaaaaaaaa aaattagcca ggcatggtgg tgtgcacctg 6420
taatcccagc tactcaggag gctgaggcag gagaattgct tgaacccggg agatggaggt 6480
tgcagtgagc cgagatcatg ccattgcact ccaccctggg cgacagagag agactctgtc 6540
tctaaaaaaa aaaaaaaaga aagaaagaaa aaaagaaaat caccattcag caatcctcat 6600
aataactgat atgaggattc agcaatcttc ataataattg attcagtgag gaatcattag 6660
caatgttcat gaagctgata aaaacaggct actcatatag tgtcgaagga tcgaagtatc 6720
tcctcaaaag gtacttatta attataaagg gaaaaaatat aactctgcag tggagaaact 6780
tggcagatac cacttcaacc aacagatcaa agtgaaatca cagaacaaat caaaatcaat 6840
gtccttttat gatgctggaa agacacaata ccactcctgt gtattcttac caaaaatgca 6900
caaatgaaat ctagtcatga ggaaacatca gataaatcca aattaagtac agtgtacaaa 6960
actggcctct atcctacaaa aatgtcaagg taataaaaga caaaggaaga ctgcaggaca 7020
ttgttccaaa ttaagggggg taaagagata ggacaatcat gcaactcacg atcatggatt 7080
agatggtggg ctagaaaaaa atctttttct ttttctataa agggcattat taggacaact 7140
gactaaattt gaatatggtc tgtacatctg atcatagaat tgtatagtta tcttcttgat 7200
tttgatacat acacatgtgt tgggggtggg tggagaaaag aagacaagaa aagagagaaa 7260
aagaaaaaga gctgggcctc atgactcatg cctgtaatcc cagctactgg ggcagcaggg 7320
ggcagtgcgc aggggagatg aagggattgc taaagaccag gagttcaaga ccagcctggg 7380
caacacagta agaccttgtc tctaaaaaat aaaataaaat aaaaaattat tcttaaatta 7440
gccaggcgca gtggcataca cctataatcc tagctattcg ggaggctgag gcaggaagat 7500
agccagagtg agaccttgtc tcgggtaaaa aggaagaaaa atgacaataa tagattatag 7560
cactctgaac taaagagtac ataataacac tcaaatgaaa gtgagaagag aaagaaacag 7620
aagaggaaag aggagagagc tctttacaga aaaataatag gtaataaatg agtaataata 7680
cagttacata atcacaactg ttaaagttcc agagaaacag tcagttgaaa caagaatctc 7740
caatggatac taaaaccact gaatgaaagg ctgttgagta accagataac atgatctcaa 7800
agaagcactt ctcagattac ttgttaatta caaaagggaa aatgtacctt taatatggag 7860
agttctggta gtcacctcat taaccaatca aattagcatc accaatactg gaagaatctg 7920
atctttctca ccttttattg taatgcaata ataagcacac aacattactt actaactgtt 7980
cttgccaaaa atgtttaatc ttaatcttat caagaggaaa caatgagaca aatgcagaat 8040
gtgtgatatt atataaaaca attgattgga actcctcaaa aaatgttaat gacaagtaaa 8100
atgagaagag acaagagggc agttctagat aaagagacat ggcagtgaaa tgcacagcac 8160
aagccctgac tgtcccctga atctgagata caaaagaaat gtgggggcaa ctgggaaaat 8220
gtaaatatgg gatatatagc agatgatctt atggaattag tgttaatttt cttctatgtg 8280

gtaacatggt gtggttatgt gtggaagaat ggccgtatct gaggaggtat atgctgaagt 8340
aattacaggt gaagtgtcat gatatctaca atttattttt acatggtcag attagggttt 8400
gtgtgtgtgt atatgtgtgt gtgtagagag tgagacaaag taaaagtggc aaaatgggcc 8460
aggcacagcg gcttacccct gtaatctcgg cactttggga ggccgagggc aggcgaatca 8520
caaggtcagg agttcgagac cagcctggcc aatatggtga aaccccgcct ctactaaaaa 8580
tacaaaaatt agctgggcgt ggtggtgtgc gcctataatc ccagctactt gggaggctaa 8640
ggcaggagaa ttgcttgaac ctgggaggca gaggttgcag tgagccaaga ttgcaccact 8700
gcactccaac ctgggcgaca gagcaagact ccgtctcaaa aaaaaaaaaa aaaagggggc 8760
aaaatgttaa caactaagaa actgaataaa aggtatctgg gaggctgggc gtggtggctc 8820
atgtctgtaa tcccaacact ttgggaggtt gaggcaggca gatcacctga ggtcagaagt 8880
ttgagaccag cctgggcaac atggcgaaac cccatctcta ccaaaaaaca aagaaacaaa 8940
aaattagctg ggtctggtgg tgcacacctg tggtcccaga tggcaccact gcactccagc 9000
ctgggtgaca gagtgagacc ccatctccaa aaaagaaaga aagaaaaggt aattgggtgt 9060
tcatacgcta ttccactttt ctggaaattt gaagctgtta aatataaaag atggaaggcc 9120
gggcatggtg gctcacacct gtaatcccag cactttggga agcccaggct ggcagatcac 9180
ctgatgttgg gagttcaaga ccagtctgac caacatggag aaacaccgtc tctactaaaa 9240
atacaaaatt agctgggcac ggtggcgggc gcctgtaatc ccagctactc gggaggctga 9300
ggcaggagaa ttgcttgaac ccgggaggca gagattgcag tgagccaaga tcgtgccatt 9360
gcattccagc ctgagcaaca aaagtgaaac tccgtctcaa aaaaaagaaa aaatagatgg 9420
aggaaaaaaa tcagattaga aagaatacaa gcagacaaca ggacttgatc ctaggagcaa 9480
tgggaactac ccaaaggagt gcagggaaga cacacgccga aagcagaact ggaatcttat 9540
catctggcag caacgcagag tggagcaggg gaggcgagac tgaaaacgca agaactcagc 9600
ctcgcagctc cccaggccta agatcatcaa atgaccccct gttccatcct ttaccacaat 9660
ctgtctcagg gtgagggcag cagcctgatg acacagaagc tgctgtcagt tgtcctctga 9720
gagaagagaa gtctctagtt ctgtagaata aataatcact tctgatgatg tcctacagcg 9780
tccatgtttc atttactctt tcggccaaaa acttgccctt tccactcagg ctgtacaccg 9840
ttgggtggtg gtggggggag gctgccgtgg tgttgagagc tgcagcagcc gtggcaatgg 9900
accgccagcc cccgcaccat cctctccagg cagtggcttt gtctcacaca ggggtctact 9960
cactgatctg tttgccaagg gactgggagt tatttccaat gcctttcagc ttttccgctt 10020
gacaaaatag aaataatgtg tattttcttc tctaaaataa ggtggttttc agcatgtcaa 10080
gccatttttg gccaaagaat aaatactgga ccaaataggt gaaaaactaa aaataaaata 10140
gatgaaaaag tcctgcaaag caaggacttt gaaaagtcta ggaatcatca tggctatgtc 10200
tcttatattc ctatgctctt catctctaaa ttaatgcttt aaagaaaagg gggtgattgt 10260
acctcccaat gtacctcctt gtgcactcat atttaattaa agctgttcta gaagggccag 10320
tcaaaataat aataataaag tcatacgaca ttggaaaagg aaaatgttat tttcatgtga 10380
tatgctactg agaaaaccta agagaaccaa caaaaacaat taggactatt aaaaataata 10440
aaataattct aaaaacacag aaatcaacag ctttcctaac tattggcaaa gatcaattag 10500
aaaatataat ggaaataaat atcctaaagc aagaataaaa aattaaaaat acaggtactc 10560
agaattaaag gaaatgtgca ggaactatgc aagaactgta caaaaccatc agtgaggggc 10620
attacatatt ttacttgaaa gataccccac atccgtggat ggaaagagtg catactgcaa 10680
agacaccaat tctttccaaa gtaatttctt tctttctttt tttttttttt tttttgagac 10740
agggtctcat tctgtcgccc agactggagt gcagtggtgc aatctcagct caccacaatc 10800

tctgcctccc aggctcaagt gattctcctg cctcaggctc ctgagtagct gggattacag 10860
gtgtgtacca ccatgcccag ctaatttttg tatttttagt agagagatgg ggtttcacta 10920
tgttagtcag gctggtcttg aactcctaac ctcaaatgat ctacctgtct cggcctccca 10980
aagtgctggg attacaggca tgagccacag cgtccagctt ccaaagtaat ttctatgttg 11040
aatgtaatta tcatcaaaat ttcaaaggga tcttctttct tgattcgcag tgggtagggg 11100
tggggtggga tcaagaagaa gatgataaaa gtgattctaa aatgtcttaa gaaatataca 11160
agaaggccgg gcacggtggc tcacacgtgt aatcgcagca ctttgggaag ccgagggtgg 11220
gcggatcacc tgaggtcagg agttcaagac cagcctgacc aacatgaaga aaccccatct 11280
ctattaaaaa tacaaaattt ttatattttt gtacaaaaga gtacaacgaa tactaacaaa 11340
tacagtgtgt aacaaatcta taataataaa aacattgcag cattagcaca acaacagaca 11400
agtacagcaa tgaatgagaa cagaatgtcc ctgatcacac atcagtatat ttaagaattt 11460
agcagatgaa aaaggtgaca tgtcaaatca gtggggaaag aaataaatcg ttctataaat 11520
ggtgctgaga ctatccagtt ggtcaacagg aaaaaataaa gttatattcc ctgttacgca 11580
actagcaaca acacaaattc cataaaagaa aactaaaatc atttaaaaaa aaaaaaaggt 11640
aggcaaatag ttctatgccc ttgcagtggg ggaagggttt tccatgctta gacaaaggaa 11700
gaaactgtga acttgacaat gtggaagttt gagacttgtg ctcggggcac tggccaactg 11760
gtgttgaggc tgtaagctga aatcaccttt ttaaagcaca ggttagcaat gtacattaaa 11820
atgaaaacaa gtccattttc tttaacctag gattagcagc tctgaggttt tttgcttttt 11880
gtttttgaga cagagtctca ctctgttgcc cagcctgaag tgcagtggca cgatctcggg 11940
tcactgcaaa ctccacctcc agggttcaag caattcttct gcctcagcct cccgagcagc 12000
tgggattaca ggcgcccgcc accacagctg gctatttttt gtatttttag tagagacgag 12060
gtttcaccat gttggccagg ctggtctcaa actcctgacc tcaagtgatc cgcccacctc 12120
ggcctcccaa agtgctggga ttacaggcgt gagccattgc acccagccta actctgaggt 12180
ttttacccca caaaggtaat cacaaaagta atacctattt cccaaggata cttaaagaag 12240
agttgctata attgtgaaaa atgaagataa tcaaggggac tgggttacaa taaattatgg 12300
catatatttt cagcagaaca caaggcagtt actaaaattg atgctagaga tctgtattga 12360
tggataccag ttacatccaa gacctagtat caagcaggga aaaaaaaaag aaagatgaga 12420
gaagaggcaa aatctaaggt gactggcttt acttaatgac ttactcaaat gactttaagg 12480
taagactcaa cagttccaag agaggccaaa aggcaaagca ctctaacctc agcttccaga 12540
tttgtgccct ccctctggcc caagtcacag attcttccag gtgcctcagt ctctcagtct 12600
ataaaatggg gataaaaatc accagtgcca ttaccatact ctcccctgtc cccagggtgc 12660
tctgaggatt aattagctgg tatttgtgaa gtgtgttcct gtactgagtt attactgaaa 12720
agtttccatt 12730

<210> 88
<211> 9602
<212> DNA
<213> Homo sapiens

<400> 88
cctaggaata gactcagatg tgtgcagttt taattttaat aagacctgtc aaaccttctt 60
ccaggttgtt tccactcaca ttccctcaac cttgcctaca tgatgctatt aagcttttta 120

tctttgccat tctgaaaggt taaaaaattg tctcgttatt ttatttattt atttatttga 180
gacagaatct cactctgtca ctcaggctgg tgccatctcg gctcactgta acctccgcct 240
cccaggttca agcaattctc gtgcctcagc ctaccgagaa gctgggatta caggagtgtg 300
ccaccagccc tgctaatttt tgtatttttg tagatacagg gtttcactat gttggccagg 360
ctggtctcaa actcctggct tcacgtgatc catctgcctt ggcctcccaa agtgctggga 420
ttacaggcgt gagccaccac acacagcctc attataattt taaggaaact tttgttggca 480
ttgaatatat acagaaaagc atgcacattt ttaagtgtac agcttaatga attttcccac 540
ggtgaacaca cttatgtaac taccatccag atcaaaaaat agagtaacag cctctagcaa 600
catcccttgt ctcctcccag tcaccaccta acttctaata ccacagattc agtttgcttg 660
cttttgaatt ttatatatgt gggataatac ggaaagcatt cacacctgta atcccagcac 720
tttgggaggc cgaggcggtg gatcatgagg tcagaagatt gagaccatcc tggctaacat 780
ggtgaaaccc cgtctctact aaaaatacaa aaaaaattag ctgggcatgg tggtgggcac 840
ctgtagtccc agctactcgg gaggctgagg caggagaatg gcgtgaaccc aggaggtgga 900
gcttgcagtg agcagagatc acgccactgc actccagcct gggcaacaga gcgagactct 960
gtctcaaaaa aaaaaaaaaa aaaaggtaaa cggctccttt tgttcaacat tataacatta 1020
tgcttgcgag tcatccttgt tgttacacat agccacagtt cattcaaatt gttgtattgt 1080
agtcttatac agtaaaagaa tgtatttcta gttttggacc atctatgaat aatgctgcta 1140
tcaacattct taatatgtat ttttttaggg gaaatgctcg attatagagt gcacatcttc 1200
tcagctttaa cagatactgc caacaatgtc caaaatggtc acagcaattc acactcccac 1260
cagcagagta gaagagctct agctgtccca cacccagcag caaagttctc agtcttttcc 1320
attttagtca ttctggtggg tgtgaaggat agcacattct gattgcaatt tgcatttctc 1380
tgacaacgac cgagactgaa tactttttgt ttgtttatca tcttcattga gtacagcgtt 1440
ctacgtaata aggcaagttt gttcattgtg ttgttagaat ctggattgct ttgtctgttt 1500
gtactaccaa acatggagaa agaagttttg aaagaagttt tgaaacgtcc cattatgata 1560
gatttgtcta gtcctccttt caggtctgtc cagttttgct ttacgtatat tgaagctatg 1620
tttgttaggt gtgtaactat tttgaaatgt tcatcttgtt ggtgattgac cattttatta 1680
ttctgaaatt tttctatctc tgactcactt ttttttattt gttttgagac ggagtctcgc 1740
tctgtcgccc aggctagagt gcagtggcgt gatctccact caccgcaatc tttgtctccc 1800
agattcaagc aattctcctg cctcagactc cggagtagct gggattacag gcatgcgcca 1860
ctgcatagag acagggtttc gccatgttgg ccaggctggt cttgaactcc tgacctcagg 1920
tgatctgctc acctcggcct cccaaagtgc tgggattata ggtgtgaacc accacgcctg 1980
gccttctgat tctactttta agaccacctg tctggtatta gtaccgagtt tcttgtggtt 2040
agtgtttgca tggtatatct ttttccatca cttgacattt aatccttaaa tatctttata 2100
ctaaaggtgt gtctgatgca agcagcattg agtcttttaa atctaatctt gaccaactgc 2160
ttttaatgga gtatttactc catttatatt taatgtaaat gctgaacttt gggggtttat 2220
atctttcata ttacaatttt gttttccttt tgacctgttt agtgctctct ttctctccct 2280
gtcttctttt taattcagtt tttctctcta ttaccttgta gtcaaggtgg tttctgatgg 2340
atttctctac tgcaaagtta ctatcttttc tttcagagtt aataaatagc tcggaaaaga 2400
cactttgctc tttctcttga aaccttcatc tcattttagc acccgtcaat ggaatttgtc 2460
tgcaacagta attactggat gtttgcctaa tgggagatcc tcaattaagg gtgaaagggg 2520
tcctgagact aaaacacttg cgaaccaacg ccctagacat cacaatatat cctttactta 2580
ctatagtgtg atataaatga gaaattttac tgttccccca aagatcctcg cacatataac 2640

tttccctcca cccctccttt tgtgtactgg catgcatttc aattataatg tatgttaaaa 2700
tccacaagaa agacattctt attttacaca gtcaatattc gttgatatgt gtccttttgt 2760
ttgtccttca ttctttcctg ccatttctat gtttccatgt aagatcattt tctctctgcc 2820
tgaaaactcc tttagtgttt ctttcagtac agtgtgctgg tgatgaattt tctctttttg 2880
tttgcctgga aatgtctgta ttttccttca cttttaaaag acattctgtt atgaaataat 2940
ttctgattta cagaaaagct gctgaaagca tagtacaaag aattctcata tatctttcac 3000
ccaggttctt taaatgttaa attcatcaca ctcgctttat tattctctat tgatctgtct 3060
atatgattac tttccctgaa ctgtttgaga ctaagttaca gacatcacac cctacccctt 3120
aaatatttca gtaagcatgt cttaatttgg ggtgggagtc ggggaagctc atgcttgtat 3180
ataaccacag aataattatc aaaatcaaga agttaacaat gttacattta tatctgtagc 3240
tcagatcttt cctctgaact tgattcataa tatccctctg cccacctgat acctccactt 3300
ggatgtctga tatctcaaac ttaatatgtc taacactgaa ctcctaatct tgccccacca 3360
aactgctcca ttcctagtta atgacaactc caaacttgta gtcacacaag gccaaaaacc 3420
ttggagtcat ccttaatttc tacttctctt tcatatccag atcccacaaa tcaggctatt 3480
ctattggatt tacttccaaa aaaatatcca gggaatctga tcacatctta acactagact 3540
agtctcccag cttccaccct ggtgatccta ttaaacctta catcagatgg tataactcct 3600
tgcttaaaac actctgatga ttttccattt tactcaaaat aaaaccaaag tccttaccaa 3660
gatactgttg ccctacacaa ccttactccc tgtgaatgct gtgacctcgt gtctcactgc 3720
tatccacctg gcctaccatt cttgttcctg gaatttgcca gacacaactt cctcttcaag 3780
cttctgcatc ggtcattccc ttttcctgaa actctttcta cccagaaatc tgcatggttt 3840
gcttcctcgt tttcttcaag tttttgctca aaatgttatc ttagaggggc ctttccaaac 3900
catcccattt aaaatagcaa attcctaaga agaatcctct attgctctta ctcatgttta 3960
tattttattc tatttactgt cttctccctc ccactctacc attacatttg gggttatttt 4020
attcactgct atgttcctag tacctaaaac acctaggttc tactggcacc tagtaggtat 4080
tcaataaaaa tgtgtaaaaa gccaggcgtg gtagctaata cctgtaatct cagcactttg 4140
ggaggctgag gcaggaggat cacttgagta caggagttca agaccagcct ggacaacata 4200
gtgcaacctc acctttttaa aaaaaaaaaa aaaaaaagct gggtgcaatg gtgtatattt 4260
gtagtctcag ctactaggga ggctgaagca ggaggactgc ttgagcccag gaggtcaagg 4320
ctgcagtgag ccatgactgc accactgcac tccagcctgg gtgacaaaac aagactctat 4380
ctcaaaaaaa aaaaaagtgt agaaagaata aataaacgga aaccccaata tgtaaaacag 4440
acaaaagtaa tgttcaggtt ttgagaggtc cggaggggta tggagaaaaa tcagagtccc 4500
accttcttga ttccctcttt gcccctgaaa tatctcctca gaatctccaa gaatccccag 4560
aggtaagtat aaaaaccgtt accaggccag gtatggtggc tcatggctgt aatcccagca 4620
ctttgggagg ccgaggaggg acagatcact tgaggtcaag gagttcaaga ccagcctggc 4680
caacatggtg aaacccccgt ccctaataaa aatatagaaa ttagccaggt gtggtggcag 4740
gcgcctgtaa tcccagctac tcaggaggct ggggtggcag aattgattga atccgggagg 4800
cggaggtcac agtgagctga gatcgtgcca ttgcactcca gcctgggcta cagagtgaga 4860
ctccatctca aaaccaaacc aaacaaaaca aaacaaaatg cctgttacca tatattcacc 4920
ctgtactaaa taaagcaaac tagtttattc atcagcccct gatatgcctt atactctgga 4980
gagagtcagg catcagtctt ccacccctga tgttccagcc atcagagtag cttcttccca 5040
accctactct gagcttgtca gttgttccgg gttatctctt ccatcaccaa agctcctctg 5100
tgatcaccct aggtagaagt acttttctac agcaattact atctagacaa tactcctctg 5160

gttgtcaaac atagatggct ttgtaacctt tcttttatct tatatggttt tctgaggatt 5220
ttaatggtgt tgaactttta ttctctgcct tattcattac tgtatcccca aaacctaaaa 5280
tactgatcaa cccatagtgg tcattcacca aataattgtt caatgtgtga ataaatgtga 5340
ataaagcaca ctggtgcttc atgaattgca gattttcaat agctgtaaga tatatttgta 5400
gtacttcatg ataatccctt ttttcccctc ttaaaaaaaa aaagctgttt atcataaaac 5460
tcattatcac tgtcatcatt aacatttaac attattcttg gagtttcatc ctatgtaata 5520
aagcaggaaa taaaattaaa atgtctatga actagagaca agtttacaaa ataatcaatt 5580
gaccatatac ctataaaatt taatcatgag aaagtgggat ggcaagatat agtacataaa 5640
attaaaaatc agtagcactc ctatgtacca gcaataatta aaaaataaaa tgaagagaaa 5700
ttcctttcat aatagccatg agaaatataa aattactata gtcttacaaa atgttagatc 5760
agtataaaga aatgttgcaa agagaaataa agggagacaa acgaaaaagc ctttcaaatt 5820
attttatttt tattttataa attattataa atttataaat tataaattaa ataaaatata 5880
aattattata aacttataaa ttattttata agtttaacac aatttcaatt aaaaaatccc 5940
aattatttta aattatctca gatgttcctc aagcttatct gggagaatgc acaaataaga 6000
ataacaagga ttagccaggc acggtggctc acgcctgtaa tcccagcact ttgggaggcc 6060
aaggtgggtg gatcataagg tcaggagttc aagatcagcc tggccaagat ggtgaaatcc 6120
tgtctctact aaaaatacaa aaatgagcag ggtatggtgg cgggcgcctg taatcccaac 6180
tactcgggag gctgagacag agaattgctt gaaaccagga gacacaggtt gcagtgagcc 6240
gagattgcac cactgcactc cagcctgggc gacagagcca gactctgtct caaaaaaaaa 6300
aaaaaaaaag gataacaaag atcatcgttt aacaccaagg tcagaggcaa caagaatgga 6360
aacaggtttc attctgccct catgggattc cagactgctc ttaattttcc ttttctgact 6420
acctgccatg tagacttcaa gctccagcct cagacgtggc aacagcatac tatttcccat 6480
tggttctgct tctctggttg aacccttcct gagacagaga agactgttct tttcctttgt 6540
cgctcatatc aataggacac ttatttgtta atttatgtgt cagctgcttc ctccactaga 6600
agcataacat agtggttaaa agcaaaaatt agaaaaaaag gtggcttatg ttttaatcct 6660
ggctctgaca cttaatagct atgagtcagg tccttcatct gtaaaatggg aataaagttg 6720
ttataaagat taaataagct actactttta tagatcaatg cctggcatat ataagcagca 6780
tatgaacttg atgaataaaa atataataaa aataatgcac tgaaaaaaaa aagaacagct 6840
aggaatgttc ggtagtccat tcagtatcct cttctggata tttaaatatt tcttagtatt 6900
tttttttaag tggtccctct agggattaca atatacattc ttatcagtct actctgaatt 6960
aatattacac catgttacat attaagaact ttaatatgat acatataatt gtatgtacta 7020
tactgtatta ttgtttatat tgataataca ttttattaag aatcttggcc gggtgcagtg 7080
gctcacgcct gtttgggtgc agtggctcac gcctgtaacc ccaacacttt gaaaggtcaa 7140
ggcaggtgga tcacctgagc tcaggagttc gagaccagcc tgggcaacat ggtgaaactc 7200
tgtctctact aaaaatacaa aaattaacca ggcaaggtag catgcacctg taatcccagc 7260
tacccgggag gccaaggcag gagaatcgct tgaacctggg aggcagaagt tgcagtgagt 7320
tgagatggtg ccactgcacg ccagcctggg tgacagagtg agatcctatc tcaaaaaaaa 7380
gaaaaagagt cttaattcca tttacacccc ttctgtcctt tgtgctcttg ttgtaaaatg 7440
ttcattacat ataaatatat gagtatacta taaatctaac actacattat tgttattgct 7500
ttagtcaact gacttttaca gaactgaaga attaaaataa aatagtattg tatattttcc 7560
tatgtattta acattcccac ggctctttat cccttcctga agacccaaat ttccatctag 7620
tattgtcaga ggcatgtgaa ccagaacaac tccatcttga gtaggagctg ggtaaaatga 7680

ggctgaaacc tactgggatg catttccaaa cagctgaggc attctaagtc acagggtgag 7740
acagaaagtc ggcacaaaat acaggtcata aagaccttgc tgataagaca ggttgcagta 7800
aaggagctgg ccaaaaccca ccaaaaccaa aatggccatg agagtaacat ctggttgtcc 7860
tcactgctac actcccacca gtgccatggc agtttacaga cgccatggca acatcgggaa 7920
gttaacctct atagtctaaa agggggaagc atgaataatc caccccgtct ggcatctcat 7980
caagaaataa ccataaaaat gggcaaccag cagctcccag ggctgctctg tctgtggagt 8040
agccattctt ttattccttt actttgttaa taaccttgct ttcactttgc accgcagact 8100
cgcccagaat tctttcttgt gtgagatcca agaaccctct cttagggtct ggattgggac 8160
ctcttcccta acagatttct ctttcttgta acagtattat ctcctcttca atgggaagat 8220
ctcacacaga tatttattca atgcatgtct gctggtgaca atttctctca gcttttatct 8280
tataatgtct ttattttttt ccttcatgtt tagtggtatt ttcactgtac agagaattct 8340
agataaagtg tcttttttct ttcgccattt tgaaaacatc attccattgt cccgtgtgct 8400
acactgtctc tgattatgag tcagccataa ttctcatcat tgttcccttt ttatttaata 8460
ttagcagttt cactgtgata cacctcgatg caattttctt tgttatttat cctacttgta 8520
attggccaca cttcctgggc tgctgtttgt gtggggttac tttaatcaaa attggaaatt 8580
tctcagccaa tattgcttca aatacttttt ctgcctcatc cttctctgct ttttgggagt 8640
actgctgtca ccctatgtta gacagcttga tttttgtcct gcaggtcatt aaggctctgg 8700
tcctattttt attccagcta tttttcctct ccatacttca gtttggatga tttctgttta 8760
cctgtcttca ggttcactga tgcttccttc tgttatgccc aatctgctgt gtctctcacc 8820
cagggaaatt tcagtgtcag gttttgtgtt tttcagttct aagatttaca tttggttctt 8880
tttcacaact tccttatctc cactgcaact cctcacctct ttaccctttc acccattttt 8940
ttcatgtaaa ttcttttaac atatttgttc atagttaaag ttactgttca cctgtgaatt 9000
tgtttctatt aactgagttt tcatttcagt aagggtcaca gggtcctgtt tcttctcaca 9060
tctagtaatt ttttaatttt atactggatg ttgaaggtga tagtttgtag aggctctgga 9120
ttctgttttc ttctgcagaa ttcagttttt tctagtagac agttaaatca cctgtagatc 9180
tggtgtaggc acaattttat gctttatttt tgttttgccc ttagtcctag ggaaaatgcc 9240
atagccctgg ggagtagtat ttgcccctaa ggggtagccc ttctgggatt tcaaagcaaa 9300
gtccaaggtg tttatcgaat cgcctctaac ttgacaagac tccacctcca aattctctct 9360
ctcccacagc aggcagctac tgaaatctct gctggaatct gtggcacttc ccctgtgcat 9420
gggcagttca gcagttagtc tagcatgtga ggggtgttta tatgtacttc ggggtgatcc 9480
ccagcatggt tctctccttt ctatgattgc catcttcaat ttcccgacac acagtctcaa 9540
aatatcccct gattcctcaa accaatatga cacagctttc tgcttgagtt ctatttgcta 9600
gc 9602